# EUROPEAN PATENT APPLICATION

(11) **EP 4 438 603 A1**
(43) Date of publication of application: **02.10.2024**
(21) Application number: 22897927.4
(22) Date of filing: 25.11.2022
(51) Int. Cl.: C07D 471/10, A61K 31/497, A61P 13/08, A61P 35/00

(54) **CHIMERIC COMPOUND FOR TARGETED DEGRADATION OF ANDROGEN RECEPTOR PROTEIN, PREPARATION METHOD THEREFOR, AND MEDICAL USE THEREOF**

(30) Priority: 25.11.2021 CN 202111413454; 28.12.2021 CN 202111624368; 27.01.2022 CN 202210097592; 03.03.2022 CN 202210203856; 06.09.2022 CN 202211083887
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: YANG, Fanglong, Shanghai 200245 (CN); JIA, Minqiang, Shanghai 200245 (CN); CHEN, Gang, Shanghai 200245 (CN); CHI, Jiangtao, Shanghai 200245 (CN); HE, Feng, Shanghai 200245 (CN)
(74) Representative: Regimbeau
(86) International application number: PCT/CN2022/134333
(87) International publication number: WO 2023/093845

(57) **Abstract**

A chimeric compound for targeted degradation of androgen receptor protein, a preparation method therefor, and a medical use thereof. Specifically, the present invention relates to a spiro compound represented by general formula (I), a preparation method therefor, a pharmaceutical composition containing the spiro compound, and uses thereof as a therapeutic agent, in particular a use as an androgen receptor degradation agent and a use in the preparation of a drug for treating and/or preventing androgen receptor mediated or dependent diseases or conditions.

R-X¹-X²-X³-X⁴-A (I)

## Description

### TECHNICAL FIELD

The present disclosure belongs to the field of pharmaceutics and relates to a novel proteolysis targeting chimera (PROTAC) compound, a preparation method therefor, and pharmaceutical use thereof. Specifically, the present disclosure relates to a spirocyclic compound represented by general formula (I), a preparation method therefor, a pharmaceutical composition comprising the spirocyclic compound, and use thereof as a therapeutic agent, particularly as an androgen receptor degrader, and use thereof in the preparation of a medicament for treating and/or preventing androgen receptor-mediated or -dependent diseases or disorders.

### BACKGROUND

PROTACs (PROteolysis TArgeting Chimera) are hybrid bifunctional small-molecule compounds. Their structures contain two different ligands: a ubiquitin ligase E3 ligand and a ligand that binds to a target protein, which are linked by a linker unit. PROTACs draw close the target protein and the ubiquitin ligase E3 in the cell to form a target protein-PROTAC-E3 ternary complex. Subsequently, the E3 ubiquitin ligase labels the target protein with a ubiquitinated protein tag and then initiates the powerful ubiquitination-proteasome system in the cell to specifically degrade the target protein, thereby playing a role in inhibiting the corresponding protein signaling pathway (Cell Biochem Funct. 2019, 37, 21-30). PROTACs have unique advantages over traditional small-molecule inhibitors: 1) PROTACs do not require long and high-strength binding to the target protein, and their degradation of the target protein is similar to catalysis: they can bind and degrade the target protein cyclically, so that the systemic drug exposure and the occurrence of toxic and side effects are reduced; 2) after being degraded, the target protein needs to be re-synthesized to recover its function; therefore, degrading the target protein exhibits a more efficient and lasting anti-tumor effect than inhibiting its activity and will not lead to drug resistance caused by mutation of the target protein; 3) PROTACs also have therapeutic potentials for the targets which are now considered undruggable, such as transcription factors, scaffold proteins, and regulatory proteins.

The discovery of CRBN-type E3 ligase ligands is related to the study of the mechanism of action of thalidomide. In 2010, scientists discovered cereblon, a binding protein of thalidomide, while studying the toxicity of thalidomide (Science 2010, 327, 1345). Cereblon is part of the E3 ubiquitin ligase protein complex. As a substrate receptor, it selectively acts on ubiquitinated proteins. This study indicated that the *in vivo* binding of thalidomide to cereblon might be the cause of thalidomide's teratogenicity. Subsequent studies found that this compound and related structures could be used as antiinflammatory agents, anti-angiogenesis agents, and anti-cancer agents. Lenalidomide and pomalidomide obtained by further modifying the thalidomide structure are much safer, and their teratogenic effects are significantly smaller. Lenalidomide was approved for sale by the FDA in 2006. In 2014, two groundbreaking papers published in Science pointed out that lenalidomide acts by degrading two special B-cell transcription factors, Ikaros family zinc finger structure proteins 1 and 3 (IKZF1 and IKZF3), which further reveals that the thalidomide structure may bind to the E3 ubiquitin ligase protein complex of cereblon, thereby playing a role in degrading the target protein (Science, 2014, 343, 301; Science, 2014, 343, 305).

The androgen receptor (AR) is a ligand-dependent trans-transcription regulating protein that belongs to the nuclear receptor superfamily, mainly found in the nucleus. AR molecules that are not bound by the ligand bind to the heat shock protein (HSP); however, upon binding to the ligand, the AR undergoes a conformational change and dissociates from the HSP, and its affinity for DNA increases (activation of the AR). Activated AR molecules bind in dimer form to a specific DNA sequence in the nucleus-the androgen response element (ARE)-and interact with other transcription factors, thereby regulating the expression of relevant genes and producing biological effects. Studies have shown that abnormalities in the AR signaling pathway are closely related to the development and progression of diseases such as prostate cancer, benign prostatic hyperplasia, Kennedy's disease, male infertility, androgen insensitivity syndrome, and male breast cancer. Prostate cancer is one of the most common malignancies. According to statistics, in 2018, there were nearly 1.3 million new cases and 359 thousand deaths worldwide, accounting for 13.5% of the incidence rate of malignancies in men, ranking second, and 6.7% of the mortality rate of malignancies in men, ranking fifth. A number of AR antagonists have been approved for sale and successfully used in the treatment of castration-resistant prostate cancer, and they have become a major treatment for prostate cancer. However, most patients develop resistance after 0.5-2 years of treatment, which leads to disease progression. In some patients with resistance, cancer cell growth still depends on the AR signaling pathway.

There is a need to develop more effective treatments for prostate cancer. Unlike AR antagonists, PROTACs can degrade the AR, so that they can inhibit the AR signaling pathway more effectively. PROTACs are likely to become a potential treatment for prostate cancer. Disclosed patent applications of AR protein targeted degradation PROTAC compounds include WO2015160845A2, WO2016197032A1, US2015291562A1, WO2018071606A1, WO2019023553A1, WO2016118666A1, WO2018144649A1, WO2020142228A1, WO2020198711A1, WO2021061644A1, and WO2021055756A1.

### SUMMARY

The present disclosure aims to provide a compound represented by general formula (I) or a pharmaceutically acceptable salt thereof,

R-X¹-X²-X³-X⁴-A (I)

wherein:
R is aryl or heteroaryl, wherein the aryl and heteroaryl are each independently optionally substituted with one or more substituents selected from the group consisting of halogen, alkyl, alkoxy, haloalkyl, haloalkoxy, cyano, -(CH₂)ₙNR^{a}R^{b}, nitro, hydroxy, hydroxyalkyl, aminoalkyl, alkoxyalkyl, cycloalkylalkyl, heterocyclylalkyl, cycloalkyl, heterocyclyl, aryl, and heteroaryl;
A is selected from the group consisting of 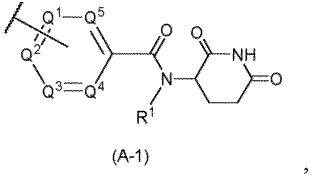 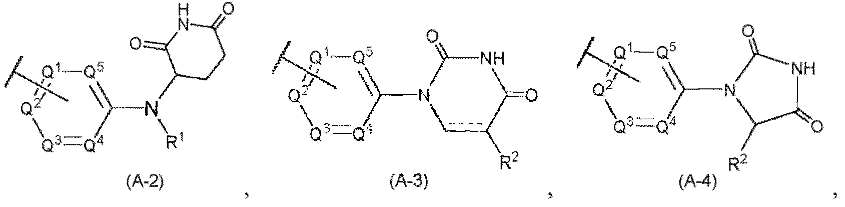 and 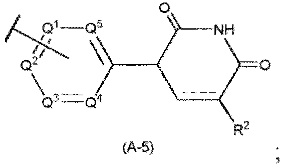
- - - - is a single bond or a double bond;
one of Q¹, Q², Q³, Q⁴, and Q⁵ is a carbon atom, and the other four are identical or different and are each independently a nitrogen atom or CR';
each R' is identical or different and is independently selected from the group consisting of a hydrogen atom, halogen, alkyl, alkoxy, haloalkyl, haloalkoxy, hydroxyalkyl, aminoalkyl, alkoxyalkyl, cyano, hydroxy, nitro, and -(CH₂)ₙNR^{c}R^{d};
R¹ is selected from the group consisting of a hydrogen atom, alkyl, haloalkyl, hydroxyalkyl, aminoalkyl, and alkoxyalkyl;
R² is selected from the group consisting of a hydrogen atom, halogen, alkyl, haloalkyl, hydroxyalkyl, alkoxy, haloalkoxy, hydroxy, cyano, aminoalkyl, and alkoxyalkyl;
X¹ is spirocyclyl, wherein the spirocyclyl is optionally substituted with one or more substituents selected from the group consisting of halogen, alkyl, alkoxy, haloalkyl, haloalkoxy, cyano, nitro, hydroxy, hydroxyalkyl, aminoalkyl, alkoxyalkyl, cycloalkylalkyl, heterocyclylalkyl, cycloalkyl, heterocyclyl, aryl, heteroaryl, -OR³, - C(O)R³, -C(O)OR³, -S(O)ₘR³, -NR⁴R⁵, -C(O)NR⁴R⁵, -S(O)ₘNR⁴R⁵, =O, and =S;
X² is selected from the group consisting of -C(O)-, -S(O)₂-, and -(CR^{2a}R^{2b})ₘ₁-;
X³ is selected from the group consisting of cycloalkyl, heterocyclyl, aryl, and heteroaryl, wherein the cycloalkyl, heterocyclyl, aryl, and heteroaryl are each independently optionally substituted with one or more substituents selected from the group consisting of halogen, alkyl, alkoxy, haloalkyl, haloalkoxy, cyano, -(CH₂)ₙNR^{e}R^{f}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
X⁴ is -J¹-J²-J³-J⁴-J⁵-, wherein J¹ is attached to X³;
J¹ is selected from the group consisting of -O-, -S-, -NR⁶-, -C(O)-, -S(O)₂-, -(CR⁷R⁸)ₘ₂-, alkenyl, and alkynyl;
J² is cycloalkyl or heterocyclyl, wherein the cycloalkyl and heterocyclyl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J³ is selected from the group consisting of -O-, -S-, -NR^{6a}-, -C(O)-, -S(O)₂-, -(CR^{7a}R^{8a})ₘ₃-, alkenyl, alkynyl, cycloalkyl, and heterocyclyl, wherein the cycloalkyl and heterocyclyl are each independently optionally substituted with one or more substituents selected from the group consisting of halogen, alkyl, alkoxy, haloalkyl, haloalkoxy, cyano, -
(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J⁴ is selected from the group consisting of a bond, cycloalkyl, heterocyclyl, aryl, and heteroaryl, wherein the cycloalkyl, heterocyclyl, aryl, and heteroaryl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J⁵ is selected from the group consisting of -C(O)NR^{6b}-, -NR^{6b}C(O)-, -O-, -S-, -NR^{6b}-, - C(O)-, -S(O)₂-, -(CR^{7b}R^{8b})ₘ₄-, alkenyl, alkynyl, cycloalkyl, and heterocyclyl, wherein the cycloalkyl and heterocyclyl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
R^{a}, R^{b}, R^{c}, R^{d}, R^{e}, R^{f}, R^{g}, R^{h}, R⁶, R^{6a}, and R^{6b} are identical or different and are each independently selected from the group consisting of a hydrogen atom, alkyl, haloalkyl, hydroxyalkyl, aminoalkyl, alkoxyalkyl, cycloalkylalkyl, and heterocyclylalkyl;
R^{2a}, R^{2b}, R⁷, R⁸, R^{7a}, R^{8a}, R^{7b}, and R^{8b} are identical or different and are each independently selected from the group consisting of a hydrogen atom, halogen, alkyl, alkoxy, hydroxy, amino, cyano, haloalkyl, haloalkoxy, hydroxyalkyl, aminoalkyl, alkoxyalkyl, cycloalkylalkyl, and heterocyclylalkyl;
R³ is selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
R⁴ and R⁵ are identical or different and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
or R⁴ and R⁵, together with the nitrogen atom to which they are attached, form heterocyclyl, and the heterocyclyl is optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
n is 0, 1, 2, or 3;
m is 0, 1, or 2;
m1 is 0, 1, 2, or 3;
m2 is 0, 1, 2, or 3;
m3 is 0, 1, 2, or 3; and
m4 is 0, 1, 2, or 3.

In some embodiments of the present disclosure, the compound represented by general formula (I) or the pharmaceutically acceptable salt thereof is provided, wherein:
R is aryl or heteroaryl, wherein the aryl and heteroaryl are each independently optionally substituted with one or more substituents selected from the group consisting of halogen, alkyl, alkoxy, haloalkyl, haloalkoxy, cyano, -(CH₂)ₙNR^{a}R^{b}, nitro, hydroxy, hydroxyalkyl, aminoalkyl, alkoxyalkyl, cycloalkylalkyl, heterocyclylalkyl, cycloalkyl, heterocyclyl, aryl, and heteroaryl;
A is selected from the group consisting of 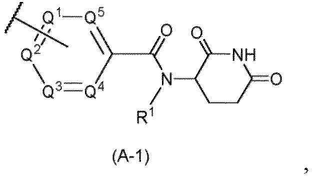 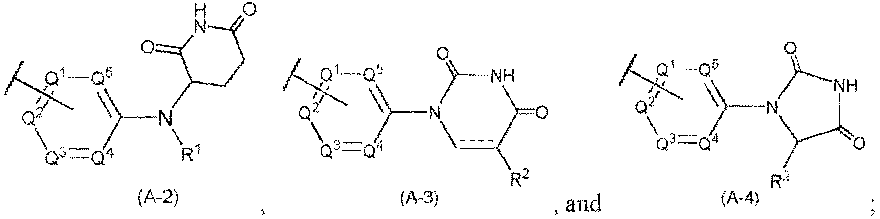
- - - - is a single bond or a double bond;
one of Q¹, Q², Q³, Q⁴, and Q⁵ is a carbon atom, and the other four are identical or different and are each independently a nitrogen atom or CR';
each R' is identical or different and is independently selected from the group consisting of a hydrogen atom, halogen, alkyl, alkoxy, haloalkyl, haloalkoxy, hydroxyalkyl, aminoalkyl, alkoxyalkyl, cyano, hydroxy, nitro, and -(CH₂)ₙNR^{c}R^{d};
R¹ is selected from the group consisting of a hydrogen atom, alkyl, haloalkyl, hydroxyalkyl, aminoalkyl, and alkoxyalkyl;
R² is selected from the group consisting of a hydrogen atom, halogen, alkyl, haloalkyl, hydroxyalkyl, alkoxy, haloalkoxy, hydroxy, cyano, aminoalkyl, and alkoxyalkyl;
X¹ is spirocyclyl, wherein the spirocyclyl is optionally substituted with one or more substituents selected from the group consisting of halogen, alkyl, alkoxy, haloalkyl, haloalkoxy, cyano, nitro, hydroxy, hydroxyalkyl, aminoalkyl, alkoxyalkyl, cycloalkylalkyl, heterocyclylalkyl, cycloalkyl, heterocyclyl, aryl, heteroaryl, -OR³, - C(O)R³, -C(O)OR³, -S(O)ₘR³, -NR⁴R⁵, -C(O)NR⁴R⁵, -S(O)ₘNR⁴R⁵, =O, and =S;
X² is selected from the group consisting of -C(O)-, -S(O)₂-, and -(CR^{2a}R^{2b})ₘ₁-;
X³ is selected from the group consisting of cycloalkyl, heterocyclyl, aryl, and heteroaryl, wherein the cycloalkyl, heterocyclyl, aryl, and heteroaryl are each independently optionally substituted with one or more substituents selected from the group consisting of halogen, alkyl, alkoxy, haloalkyl, haloalkoxy, cyano, -(CH₂)ₙNR^{e}R^{f}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
X⁴ is -J¹-J²-J³-J⁴-J⁵-, wherein J¹ is attached to X³;
J¹ is selected from the group consisting of -O-, -S-, -NR⁶-, -C(O)-, -S(O)₂-, -(CR⁷R⁸)ₘ₂-, alkenyl, and alkynyl;
J² is cycloalkyl or heterocyclyl, wherein the cycloalkyl and heterocyclyl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J³ is selected from the group consisting of -O-, -S-, -NR^{6a}-, -C(O)-, -S(O)₂-, -(CR^{7a}R^{8a})ₘ₃-, alkenyl, alkynyl, cycloalkyl, and heterocyclyl, wherein the cycloalkyl and heterocyclyl are each independently optionally substituted with one or more substituents selected from the group consisting of halogen, alkyl, alkoxy, haloalkyl, haloalkoxy, cyano, - (CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J⁴ is selected from the group consisting of a bond, cycloalkyl, heterocyclyl, aryl, and heteroaryl, wherein the cycloalkyl, heterocyclyl, aryl, and heteroaryl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J⁵ is selected from the group consisting of -C(O)NR^{6b}-, -NR^{6b}C(O)-, -O-, -S-, -NR^{6b}-, - C(O)-, -S(O)₂-, -(CR^{7b}R^{8b})ₘ₄-, alkenyl, alkynyl, cycloalkyl, and heterocyclyl, wherein the cycloalkyl and heterocyclyl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
R^{a}, R^{b}, R^{c}, R^{d}, R^{e}, R^{f}, R^{g}, R^{h}, R⁶, R^{6a}, and R^{6b} are identical or different and are each independently selected from the group consisting of a hydrogen atom, alkyl, haloalkyl, hydroxyalkyl, aminoalkyl, alkoxyalkyl, cycloalkylalkyl, and heterocyclylalkyl;
R^{2a}, R^{2b}, R⁷, R⁸, R^{7a}, R^{8a}, R^{7b}, and R^{8b} are identical or different and are each independently selected from the group consisting of a hydrogen atom, halogen, alkyl, alkoxy, hydroxy, amino, cyano, haloalkyl, haloalkoxy, hydroxyalkyl, aminoalkyl, alkoxyalkyl, cycloalkylalkyl, and heterocyclylalkyl;
R³ is selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
R⁴ and R⁵ are identical or different and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
or R⁴ and R⁵, together with the nitrogen atom to which they are attached, form heterocyclyl, and the heterocyclyl is optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
n is 0, 1, 2, or 3;
m is 0, 1, or 2;
m1 is 0, 1, 2, or 3;
m2 is 0, 1, 2, or 3;
m3 is 0, 1, 2, or 3; and
m4 is 0, 1, 2, or 3.

In some embodiments of the present disclosure, the compound represented by general formula (I) or the pharmaceutically acceptable salt thereof is provided, wherein R is 6- to 10-membered aryl or 5- to 10-membered heteroaryl, wherein the 6- to 10-membered aryl and 5- to 10-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, -(CH₂)ₙNR^{a}R^{b}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, and 3- to 8-membered heterocyclyl; R^{a} and R^{b} are identical or different and are each independently a hydrogen atom or C₁₋₆ alkyl; n is 0, 1, 2, or 3;
preferably, R is phenyl or 5- or 6-membered heteroaryl, wherein the phenyl and 5- or 6-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl;
preferably, R is phenyl, wherein the phenyl is optionally substituted with one or more substituents selected from the group consisting of halogen, C₁₋₆ alkyl, C₁₋₆ alkoxy, C₁₋₆ haloalkyl, and cyano;
more preferably, R is phenyl, wherein the phenyl is optionally substituted with one or more substituents selected from the group consisting of halogen and cyano;
most preferably, R is phenyl, wherein the phenyl is optionally substituted with one or more substituents selected from the group consisting of a chlorine atom and cyano.

In some embodiments of the present disclosure, the compound represented by general formula (I) or the pharmaceutically acceptable salt thereof is provided, wherein R is 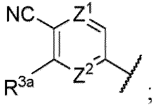 Z¹ is N or CR^{3b}; Z² is N or CR^{3c}; R^{3a}, R^{3b}, and R^{3c} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl; preferably, R is selected from the group consisting of 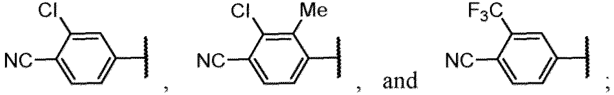 more preferably, R is

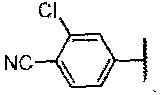

In some embodiments of the present disclosure, the compound represented by general formula (I) or the pharmaceutically acceptable salt thereof is provided, wherein X¹ is 6-to 14-membered spiroheterocyclyl, wherein the 6- to 14-membered spiroheterocyclyl is optionally substituted with one or more substituents selected from the group consisting of halogen, C₁₋₆ alkyl, C₁₋₆ alkoxy, C₁₋₆ haloalkyl, C₁₋₆ haloalkoxy, cyano, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, 6- to 10-membered aryl, 5- to 10-membered heteroaryl, - NR⁴R⁵, =O, and =S; R⁴ and R⁵ are identical or different and are each independently a hydrogen atom or C₁₋₆ alkyl.

In some embodiments of the present disclosure, the compound represented by general formula (I) or the pharmaceutically acceptable salt thereof is provided, wherein X¹ is 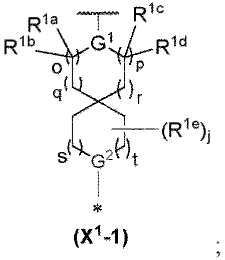
the bond with * is attached to X²;
G¹ is N or CH;
G² is N or CH;
R^{1a}, R^{1b}, R^{1c}, R^{1d}, and R^{1e} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, and -NR⁴R⁵;
or, R^{1a} and R^{1b}, together with the carbon atom to which they are attached, form 3- to 8-membered cycloalkyl or 3- to 8-membered heterocyclyl, wherein the 3- to 8-membered cycloalkyl and 3- to 8-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, -NR⁴R⁵, and =O;
or, R^{1c} and R^{1d}, together with the carbon atom to which they are attached, form 3- to 8-membered cycloalkyl or 3- to 8-membered heterocyclyl, wherein the 3- to 8-membered cycloalkyl and 3- to 8-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, -NR⁴R⁵, and =O;
or, R^{1a} and R^{1b}, together with the carbon atom to which they are attached, form C=O;
or, R^{1c} and R^{1d}, together with the carbon atom to which they are attached, form C=O;
R⁴ and R⁵ are identical or different and are each independently a hydrogen atom or C₁₋₆ alkyl;
o is 0 or 1;
p is 0 or 1;
q is 0, 1, 2, or 3;
r is 0, 1, 2, or 3;
s is 0, 1, 2, 3, or 4;
t is 0, 1, 2, 3, or 4; and
j is 0, 1, 2, 3, or 4.

In some embodiments of the present disclosure, the compound represented by general formula (I) or the pharmaceutically acceptable salt thereof is provided, wherein X¹ is 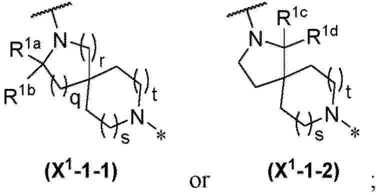 the bond with * is attached to X²; R^{1a}, R^{1b}, R^{1c}, and R^{1d} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
or, R^{1a} and R^{1b}, together with the carbon atom to which they are attached, form C=O;
or, R^{1c} and R^{1d}, together with the carbon atom to which they are attached, form C=O;
q is 0, 1, 2, or 3; r is 0, 1, 2, or 3; s is 0, 1, 2, 3, or 4; and t is 0, 1, 2, 3, or 4.

In some embodiments of the present disclosure, the compound represented by general formula (I) or the pharmaceutically acceptable salt thereof is provided, wherein X¹ is 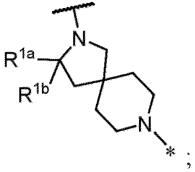 the bond with * is attached to X²; R^{1a} and R^{1b} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; or, R^{1a} and R^{1b}, together with the carbon atom to which they are attached, form C=O.

In some embodiments of the present disclosure, the compound represented by general formula (I) or the pharmaceutically acceptable salt thereof is provided, wherein X¹ is 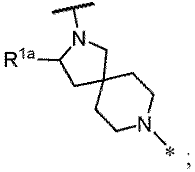 the bond with * is attached to X²; R^{1a} is selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl, preferably a hydrogen atom or C₁₋₆ alkyl, further preferably C₁₋₆ alkyl, and more preferably methyl.

In some embodiments of the present disclosure, the compound represented by general formula (I) or the pharmaceutically acceptable salt thereof is provided, wherein X¹ is 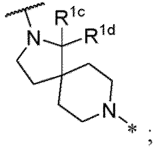 the bond with * is attached to X²; R^{1c} and R^{1d} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; or, R^{1c} and R^{1d}, together with the carbon atom to which they are attached, form C=O.

In some embodiments of the present disclosure, the compound represented by general formula (I) or the pharmaceutically acceptable salt thereof is provided, wherein X¹ is 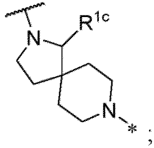 the bond with * is attached to X²; R^{1c} is selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl, preferably C₁₋₆ alkyl, and more preferably methyl.

In some embodiments of the present disclosure, the compound represented by general formula (I) or the pharmaceutically acceptable salt thereof is provided, wherein X¹ is selected from the group consisting of 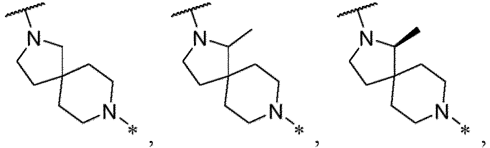 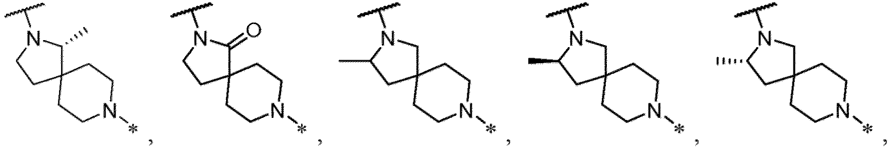 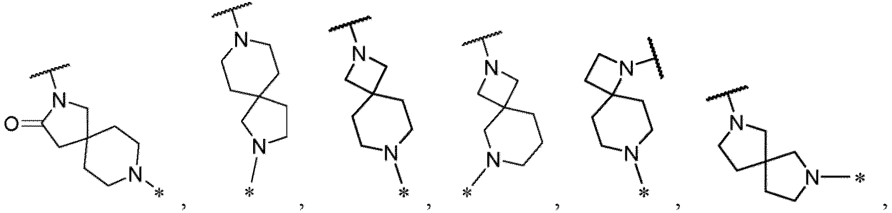 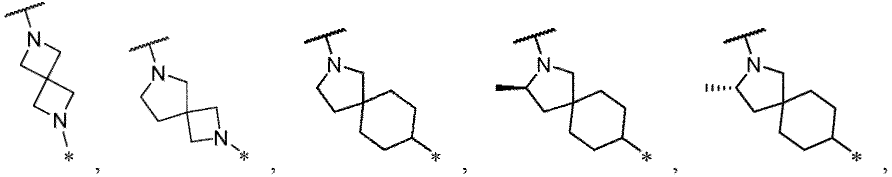 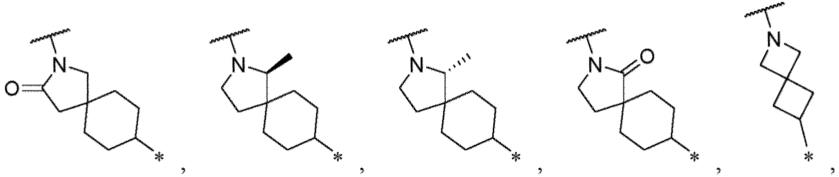 and 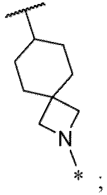 preferably, X¹ is selected from the group consisting of 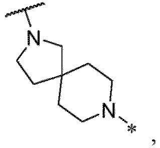 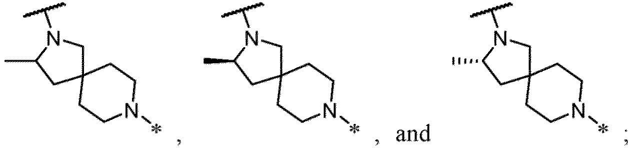 more preferably, X¹ is 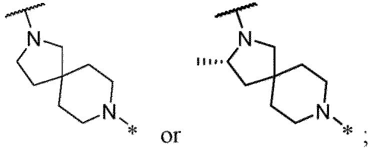 most preferably, X¹ is 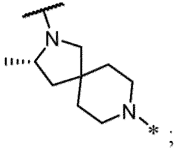 the bond with * is attached to X².

In some embodiments of the present disclosure, the compound represented by general formula (I) or the pharmaceutically acceptable salt thereof is a compound represented by general formula (II) or a pharmaceutically acceptable salt thereof: 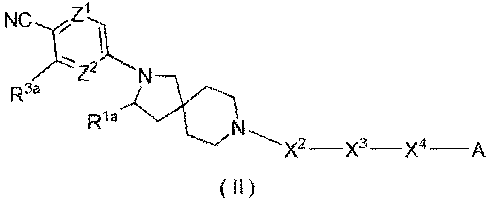 wherein:
R^{1a} is selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
preferably, R^{1a} is C₁₋₆ alkyl; more preferably, R^{1a} is methyl;
Z¹ is N or CR^{3b}; Z² is N or CR^{3c}; R^{3a}, R^{3b}, and R^{3c} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl; and
X², X³, X⁴, and A are as defined in general formula (I).

In some embodiments of the present disclosure, the compounds represented by general formula (I) and general formula (II) or the pharmaceutically acceptable salts thereof are compounds represented by general formula (II-1) or pharmaceutically acceptable salts thereof: 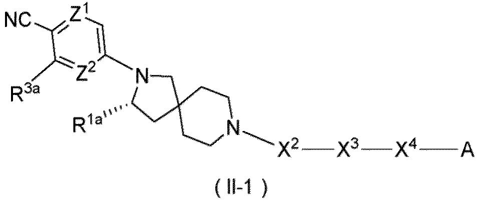 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably, R^{1a} is C₁₋₆ alkyl; more preferably, R^{1a} is methyl; and
Z¹, Z², R^{3a}, X², X³, X⁴, and A are as defined in general formula (II).

In some embodiments of the present disclosure, the compounds represented by general formula (I), general formula (II), and general formula (II-1) or the pharmaceutically acceptable salts thereof are provided, wherein X³ is 6- to 10-membered aryl or 5- to 10-membered heteroaryl, wherein the 6- to 10-membered aryl and 5- to 10-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, -(CH₂)ₙNR^{e}R^{f}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, 6-to 10-membered aryl, and 5- to 10-membered heteroaryl; R^{e} and R^{f} are identical or different and are each independently a hydrogen atom or C₁₋₆ alkyl; and n is 0, 1, 2, or 3; preferably, X³ is 6- to 10-membered aryl or 5- to 10-membered heteroaryl, wherein the 6-to 10-membered aryl and 5- to 10-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
more preferably, X³ is selected from the group consisting of phenyl, pyridinyl,
pyrimidinyl, pyridazinyl, and pyrazinyl, wherein the phenyl, pyridinyl, pyrimidinyl, pyridazinyl, and pyrazinyl are each independently optionally substituted with one or more substituents selected from the group consisting of halogen, C₁₋₆ alkyl, and C₁₋₆ haloalkyl; most preferably, X³ is phenyl or pyridinyl, wherein the phenyl and pyridinyl are each independently optionally substituted with one or more substituents selected from the group consisting of halogen, C₁₋₆ alkyl, and C₁₋₆ haloalkyl.

In some embodiments of the present disclosure, the compounds represented by general formula (I), general formula (II), and general formula (II-1) or the pharmaceutically acceptable salts thereof are provided, wherein X³ is phenyl or 6-membered heteroaryl, wherein the phenyl and 6-membered heteroaryl are each independently optionally substituted with one or more halogens; preferably, X³ is selected from the group consisting of phenyl, pyridinyl, pyrimidinyl, pyridazinyl, and pyrazinyl, and the phenyl, pyridinyl, pyrimidinyl, pyridazinyl, and pyrazinyl are each independently optionally substituted with one or more F; more preferably, X³ is phenyl, and the phenyl is optionally substituted with one or more F; most preferably, X³ is phenyl.

In some embodiments of the present disclosure, the compounds represented by general formula (I), general formula (II), and general formula (II-1) or the pharmaceutically acceptable salts thereof are provided, wherein X³ is 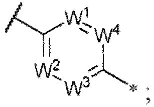 the bond with * is attached to X⁴; wherein:
W¹ is N or CR^{3d}; W² is N or CR^{3e}; W³ is N or CR^{3f}; W⁴ is N or CR^{3g}; R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, -(CH₂)ₙNR^{e}R^{f}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, 6-to 10-membered aryl, and 5- to 10-membered heteroaryl; R^{e} and R^{f} are identical or different and are each independently a hydrogen atom or C₁₋₆ alkyl; and n is 0, 1, 2, or 3; preferably, W¹ is N or CR^{3a}; W² is N or CR^{3e}; W³ is N or CR^{3f}; W⁴ is N or CR^{3g}; R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
more preferably, W¹ is N or CR^{3a}; W² is N or CR^{3e}; W³ is N or CR^{3f}; W⁴ is N or CR^{3g}; R^{3d}, R^{3e}, R^{3f}, and R^{3g} are identical or different and are each independently selected from the group consisting of a hydrogen atom, halogen, C₁₋₆ alkyl, C₁₋₆ alkoxy, and C₁₋₆ haloalkyl;
further preferably, W¹ is N or CR^{3a}; W² is N or CR^{3e}; W³ is N or CR^{3f}; W⁴ is N or CR^{3g}; R^{3d}, R^{3e}, R^{3f}, and R^{3g} are identical or different and are each independently a hydrogen atom or halogen (the halogen is preferably F);
most preferably, W¹ is CR^{3d}; W² is CR^{3e}; W³ is CR^{3f}; W⁴ is CR^{3g}; R^{3d}, R^{3e}, R^{3f}, and R^{3g} are identical or different and are each independently a hydrogen atom or halogen (the halogen is preferably F);
still most preferably, W¹ is CH; W² is CH; W³ is CH; W⁴ is CH.

In some embodiments of the present disclosure, the compounds represented by general formula (I), general formula (II), and general formula (II-1) or the pharmaceutically acceptable salts thereof are provided, wherein X³ is selected from the group consisting of 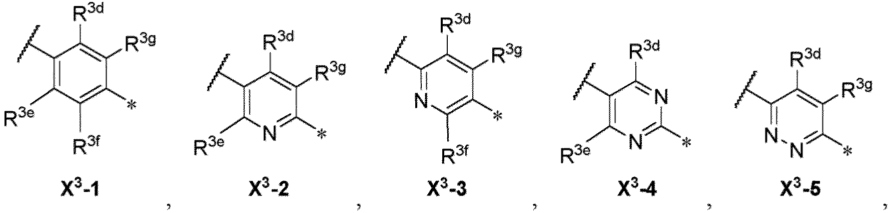 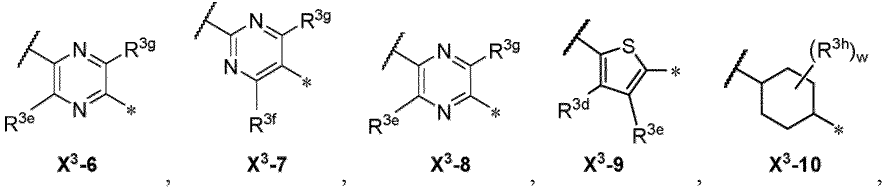 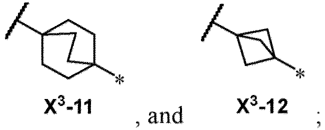 the bond with * is attached to X⁴; R^{3d}, R^{3e}, R^{3f}, R^{3g}, and R^{3h} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; and w is 0, 1, 2, or 3. In some embodiments of the present disclosure, the compounds represented by general formula (I), general formula (II), and general formula (II-1) or the pharmaceutically acceptable salts thereof are provided, wherein X³ is selected from the group consisting of 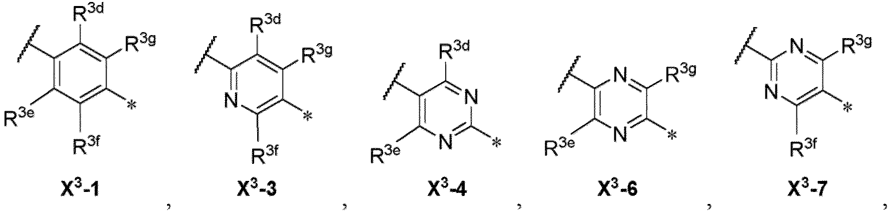 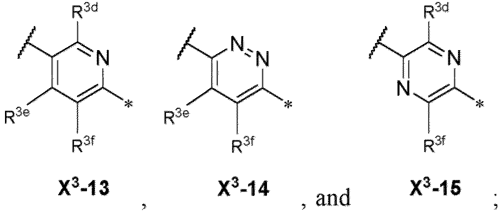 R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably from the group consisting of 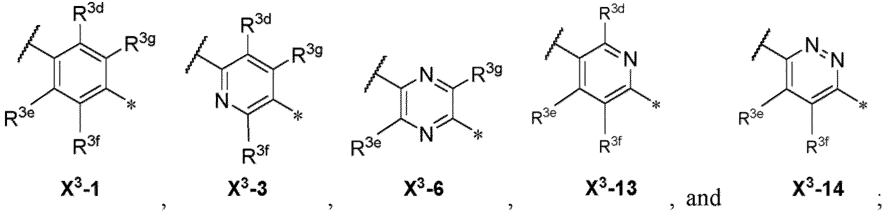 R^{3d}, R^{3e}, R^{3f}, and R^{3g} are identical or different and are each independently a hydrogen atom or halogen (preferably F); more preferably from the group consisting of 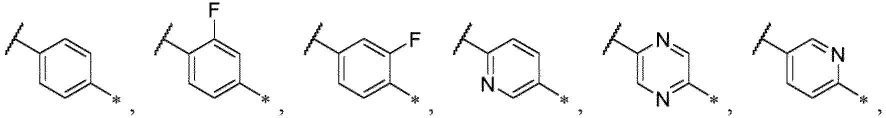 and 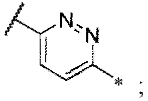 further preferably from the group consisting of 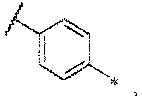 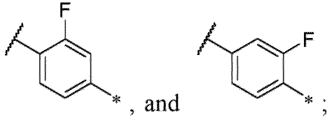 and most preferably 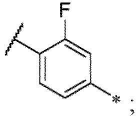 the bond with * is attached to X⁴.

In some embodiments of the present disclosure, the compounds represented by general formula (I), general formula (II), and general formula (II-1) or the pharmaceutically acceptable salts thereof are provided, wherein J² is 3- to 12-membered cycloalkyl or 3- to 12-membered heterocyclyl, wherein the 3- to 12-membered cycloalkyl and 3- to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, -(CH₂)ₙNR^{g}R^{h}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, and =O; R^{g} and R^{h} are identical or different and are each independently a hydrogen atom or C₁₋₆ alkyl; and n is 0, 1, 2, or 3.

In some embodiments of the present disclosure, the compounds represented by general formula (I), general formula (II), and general formula (II-1) or the pharmaceutically acceptable salts thereof are provided, wherein J² is 3- to 12-membered cycloalkyl or 3- to 12-membered heterocyclyl, and the 3- to 12-membered cycloalkyl and 3- to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O; preferably, J² is 3- to 12-membered heterocyclyl, wherein the 3- to 12-membered heterocyclyl is optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O;

further preferably, J² is 3- to 8-membered monocyclic heterocyclyl or 7- to 11-membered spiroheterocyclyl, and the 3- to 8-membered monocyclic heterocyclyl and 7- to 11-membered spiroheterocyclyl are each independently optionally substituted with one or more substituents selected from the group consisting of halogen, C₁₋₆ alkyl, and C₁₋₆ haloalkyl;
still further preferably, J² is 4- to 6-membered monocyclic heterocyclyl or 9- to 11-membered spiroheterocyclyl;
more preferably, J² is piperidinyl or piperazinyl.

In some embodiments of the present disclosure, the compounds represented by general formula (I), general formula (II), and general formula (II-1) or the pharmaceutically acceptable salts thereof are provided, wherein J² is selected from the group consisting of the following structures: 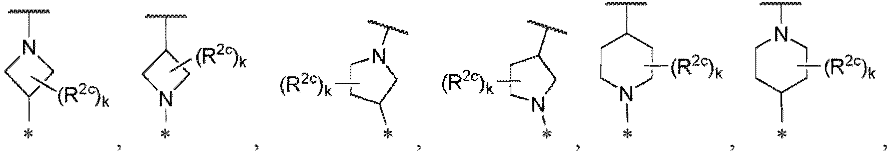 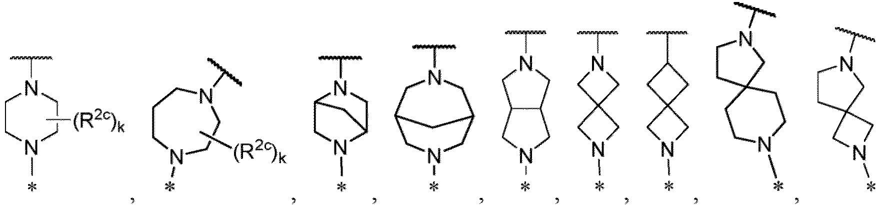 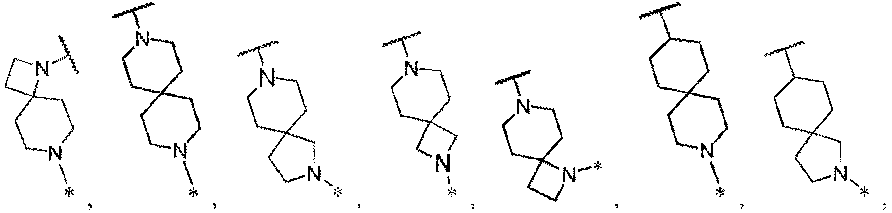 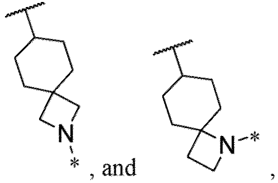 wherein the bond with * is attached to J³; each R^{2c} is identical or different and is independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and =O, preferably a hydrogen atom; and k is 0, 1, 2, 3, 4, 5, or 6.

In some embodiments of the present disclosure, the compounds represented by general formula (I), general formula (II), and general formula (II-1) or the pharmaceutically acceptable salts thereof are provided, wherein J² is selected from the group consisting of 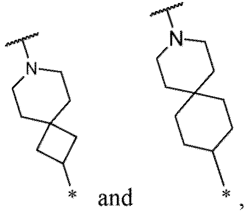 wherein the bond with * is attached to J³. In some embodiments of the present disclosure, the compounds represented by general formula (I), general formula (II), and general formula (II-1) or the pharmaceutically acceptable salts thereof are provided, wherein J² is selected from the group consisting of 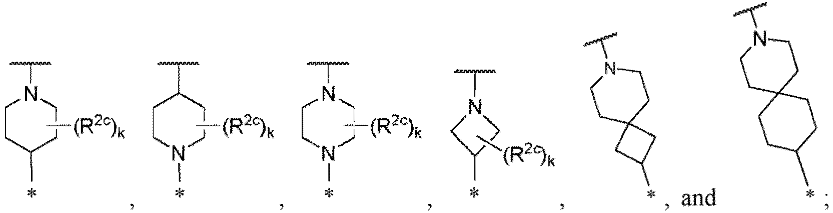 each R^{2c} is identical or different and is independently selected from the group consisting of halogen, C₁₋₆ alkyl, C₁₋₆ haloalkyl, and =O; and k is 0, 1, or 2; preferably, J² is selected from the group consisting of 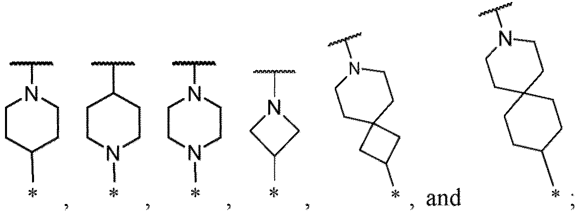 more preferably, J² is 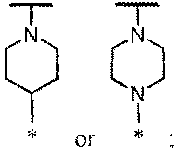 most preferably, J² is 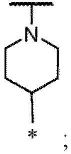 wherein the bond with * is attached to J³.

In some embodiments of the present disclosure, the compounds represented by general formula (I), general formula (II), and general formula (II-1) or the pharmaceutically acceptable salts thereof are provided, wherein R^{6a} is a hydrogen atom or C₁₋₆ alkyl; preferably, R^{6a} is a hydrogen atom or methyl.

In some embodiments of the present disclosure, the compounds represented by general formula (I), general formula (II), and general formula (II-1) or the pharmaceutically acceptable salts thereof are provided, wherein R^{7a} and R^{8a} are identical or different and are each independently selected from the group consisting of a hydrogen atom, halogen, C₁₋₆ alkyl, C₁₋₆ alkoxy, hydroxy, amino, cyano, C₁₋₆ haloalkyl, C₁₋₆ haloalkoxy, and C₁₋₆ hydroxyalkyl; preferably, R^{7a} and R^{8a} are identical or different and are each independently selected from the group consisting of a hydrogen atom, halogen, hydroxy, and C₁₋₆ alkyl; more preferably, R^{7a} and R^{8a} are both hydrogen atoms.

In some embodiments of the present disclosure, the compounds represented by general formula (I), general formula (II), and general formula (II-1) or the pharmaceutically acceptable salts thereof are provided, wherein J³ is selected from the group consisting of -O-, -S-, -NR^{6a}-, -C(O)-, -S(O)₂-, -(CR^{7a}R^{8a})ₘ₃-, C₂₋₆ alkenyl, C₂₋₆ alkynyl, 3- to 12-membered cycloalkyl, and 3- to 12-membered heterocyclyl, wherein the 3- to 12-membered cycloalkyl and 3- to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, -(CH₂)ₙNR^{g}R^{h}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, and =O; R^{g} and R^{h} are identical or different and are each independently a hydrogen atom or C₁₋₆ alkyl; R^{6a} is a hydrogen atom or C₁₋₆ alkyl; R^{7a} and R^{8a} are identical or different and are each independently selected from the group consisting of a hydrogen atom, halogen, C₁₋₆ alkyl, C₁₋₆ alkoxy, hydroxy, amino, cyano, C₁₋₆ haloalkyl, C₁₋₆ haloalkoxy, and C₁₋₆ hydroxyalkyl; m3 is 0, 1, 2, or 3; n is 0, 1, 2, or 3.

In some embodiments of the present disclosure, the compounds represented by general formula (I), general formula (II), and general formula (II-1) or the pharmaceutically acceptable salts thereof are provided, wherein J³ is selected from the group consisting of -O-, -S-, -NR^{6a}-, -C(O)-, -S(O)₂-, -(CR^{7a}R^{8a})ₘ₃-, C₂₋₆ alkenyl, C₂₋₆ alkynyl, 3- to 8-membered cycloalkyl, and 3- to 8-membered heterocyclyl; R^{6a} is a hydrogen atom or C₁₋₆ alkyl, preferably a hydrogen atom or methyl; R^{7a} and R^{8a} are identical or different and are each independently selected from the group consisting of a hydrogen atom, halogen, hydroxy, and C₁₋₆ alkyl, preferably a hydrogen atom; m3 is 0, 1, 2, or 3.

In some embodiments of the present disclosure, the compounds represented by general formula (I), general formula (II), and general formula (II-1) or the pharmaceutically acceptable salts thereof are provided, wherein J³ is -(CR^{7a}R^{8a})ₘ₃-; R^{7a} and R^{8a} are identical or different and are each independently selected from the group consisting of a hydrogen atom, halogen, hydroxy, and C₁₋₆ alkyl, preferably a hydrogen atom; and m3 is 0, 1, 2, or 3, preferably 0 or 1.

In some embodiments of the present disclosure, the compounds represented by general formula (I), general formula (II), and general formula (II-1) or the pharmaceutically acceptable salts thereof are provided, wherein J³ is -(CH₂)ₘ₃-; m3 is 0, 1, 2, or 3, preferably 0 or 1.

In some embodiments of the present disclosure, the compounds represented by general formula (I), general formula (II), and general formula (II-1) or the pharmaceutically acceptable salts thereof are provided, wherein J³ is selected from the group consisting of a bond, -O-, -S-, -NH-, -N(CH₃)-, -C(O)-, -S(O)₂-, -CH₂-, -CH(CH₃)-, CH₂CH₂-, - CH₂CH₂CH₂-, and ethynylene; preferably, J³ is a bond or -CH₂-; more preferably, J³ is - CH₂-.

In some embodiments of the present disclosure, the compounds represented by general formula (I), general formula (II), and general formula (II-1) or the pharmaceutically acceptable salts thereof are provided, wherein J⁴ is selected from the group consisting of a bond, 3- to 12-membered cycloalkyl, 3- to 12-membered heterocyclyl, 6- to 10-membered aryl, and 5- to 10-membered heteroaryl, wherein the 3- to 12-membered cycloalkyl, 3- to 12-membered heterocyclyl, 6- to 10-membered aryl, and 5- to 10-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, -(CH₂)ₙNR^{g}R^{h}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, and =O; R^{g} and R^{h} are identical or different and are each independently a hydrogen atom or C₁₋₆ alkyl; and n is 0, 1, 2, or 3.

In some embodiments of the present disclosure, the compounds represented by general formula (I), general formula (II), and general formula (II-1) or the pharmaceutically acceptable salts thereof are provided, wherein J⁴ is 3- to 12-membered cycloalkyl or 3- to 12-membered heterocyclyl, and the 3- to 12-membered cycloalkyl and 3- to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O;
preferably, J⁴ is 3- to 8-membered cycloalkyl or 3- to 8-membered heterocyclyl, and the 3- to 8-membered cycloalkyl and 3- to 8-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O;
more preferably, J⁴ is 3- to 8-membered heterocyclyl, and the 3- to 8-membered heterocyclyl is optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O;

further preferably, J⁴ is 4- to 6-membered heterocyclyl, and the 4- to 6-membered heterocyclyl is optionally substituted with one or more =O;
further preferably, J⁴ is selected from the group consisting of piperazinyl, piperidinyl, and azetidinyl, and the piperazinyl and piperidinyl are each independently optionally substituted with one or more =O;
most preferably, J⁴ is selected from the group consisting of piperazinyl, piperidinyl, and azetidinyl.

In some embodiments of the present disclosure, the compounds represented by general formula (I), general formula (II), and general formula (II-1) or the pharmaceutically acceptable salts thereof are provided, wherein J⁴ is a bond or 4- to 6-membered heterocyclyl, and the 4- to 6-membered heterocyclyl is optionally substituted with one or more =O; preferably, J⁴ is selected from the group consisting of a bond, piperazinyl, piperidinyl, and azetidinyl, and the piperazinyl and piperidinyl are each independently optionally substituted with one or more =O; more preferably, J⁴ is selected from the group consisting of a bond, 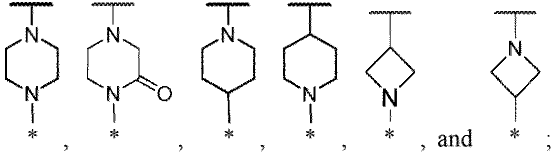 the bond with * is attached to J⁵.

In some embodiments of the present disclosure, the compounds represented by general formula (I), general formula (II), and general formula (II-1) or the pharmaceutically acceptable salts thereof are provided, wherein J⁴ is selected from the group consisting of the following structures: 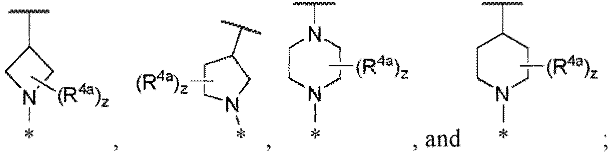 the bond with * is attached to J⁵; each R^{4a} is identical or different and is independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and =O, preferably a hydrogen atom; and z is 0, 1, 2, or 3.

In some embodiments of the present disclosure, the compounds represented by general formula (I), general formula (II), and general formula (II-1) or the pharmaceutically acceptable salts thereof are provided, wherein J⁴ is selected from the group consisting of the following structures: 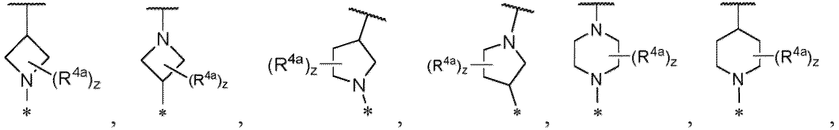 and 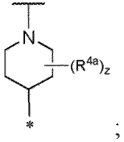 each R^{4a} is identical or different and is independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and =O, preferably a hydrogen atom; and z is 0, 1, 2, or 3; preferably, J⁴ is selected from the group consisting of 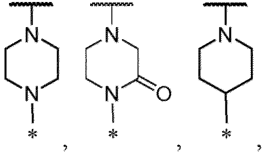 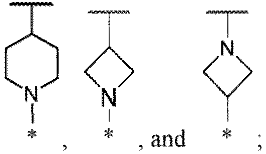 more preferably, J⁴ is 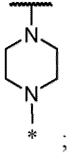 the bond with * is attached to J⁵.

In some embodiments of the present disclosure, the compounds represented by general formula (I) and general formula (II) or the pharmaceutically acceptable salts thereof are compounds represented by general formula (G) or pharmaceutically acceptable salts thereof: 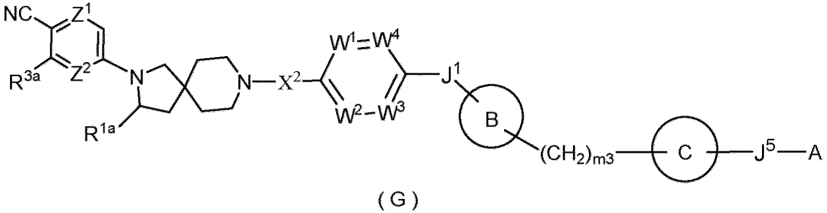 wherein:
R^{1a} is selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z¹ is N or CR^{3b}; Z² is N or CR^{3c}; R^{3a}, R^{3b}, and R^{3c} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl;
ring B and ring C are identical or different and are each independently 3- to 12-membered cycloalkyl or 3- to 12-membered heterocyclyl, and the 3- to 12-membered cycloalkyl and 3- to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O;
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
and m3, X², J¹, J⁵, and A are as defined in general formula (I).

In some embodiments of the present disclosure, the compounds represented by general formula (I), general formula (II), and general formula (G) or the pharmaceutically acceptable salts thereof are compounds represented by general formula (G-1) or pharmaceutically acceptable salts thereof: 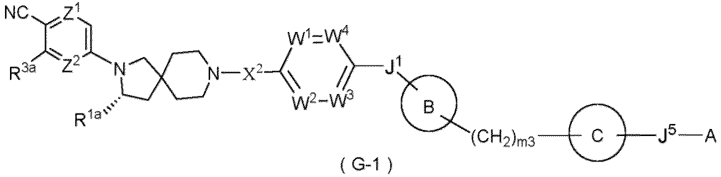 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; and
ring B, ring C, R^{3a}, W¹, W², W³, W⁴, Z¹, Z², m3, X², J¹, J⁵, and A are as defined in general formula (G).

In some embodiments of the present disclosure, the compounds represented by general formula (G) and general formula (G-1) or the pharmaceutically acceptable salts thereof are provided, wherein ring B is 3- to 12-membered heterocyclyl, and the 3- to 12-membered heterocyclyl is optionally substituted with one or more substituents selected from the group consisting of halogen, C₁₋₆ alkyl, and C₁₋₆ haloalkyl; preferably, ring B is 3- to 8-membered monocyclic heterocyclyl or 7- to 11-membered spiroheterocyclyl, and the 3- to 8-membered monocyclic heterocyclyl and 7- to 11-membered spiroheterocyclyl are each independently optionally substituted with one or more substituents selected from the group consisting of halogen, C₁₋₆ alkyl, and C₁₋₆ haloalkyl; more preferably, ring B is 4- to 6-membered monocyclic heterocyclyl or 9- to 11-membered spiroheterocyclyl; further preferably, ring B is selected from the group consisting of 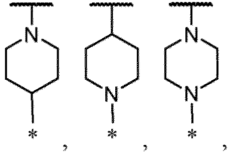 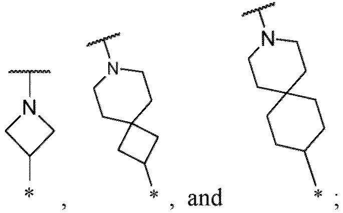 more preferably, ring B is 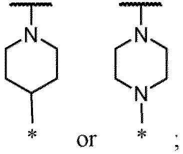 most preferably, ring B is 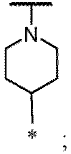 wherein the bond with * is attached to (CH₂)ₘ₃.

In some embodiments of the present disclosure, the compounds represented by general formula (G) and general formula (G-1) or the pharmaceutically acceptable salts thereof are provided, wherein ring C is 4- to 6-membered heterocyclyl, and the 4- to 6-membered heterocyclyl is optionally substituted with one or more =O; preferably, ring C is selected from the group consisting of piperazinyl, piperidinyl, and azetidinyl, and the piperazinyl and piperidinyl are each independently optionally substituted with one or more =O; more preferably, ring C is selected from the group consisting of 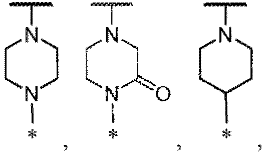 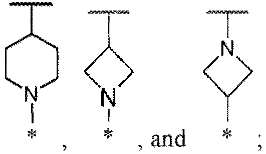 most preferably, ring C is 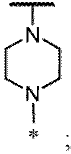 the bond with * is attached to J⁵.

In some embodiments of the present disclosure, the compounds represented by general formula (I), general formula (II), general formula (II-1), general formula (G), and general formula (G-1) or the pharmaceutically acceptable salts thereof are provided, wherein R^{7b} and R^{8b} are identical or different and are each independently selected from the group consisting of a hydrogen atom, halogen, C₁₋₆ alkyl, C₁₋₆ alkoxy, hydroxy, amino, cyano, C₁₋₆ haloalkyl, C₁₋₆ haloalkoxy, and C₁₋₆ hydroxyalkyl; preferably, R^{7b} and R^{8b} are identical or different and are each independently selected from the group consisting of a hydrogen atom, halogen, hydroxy, and C₁₋₆ alkyl; more preferably, R^{7b} and R^{8b} are both hydrogen atoms.

In some embodiments of the present disclosure, the compounds represented by general formula (I), general formula (II), general formula (II-1), general formula (G), and general formula (G-1) or the pharmaceutically acceptable salts thereof are provided, wherein R^{6b} is a hydrogen atom or C₁₋₆ alkyl; preferably, R^{6b} is a hydrogen atom or methyl.

In some embodiments of the present disclosure, the compounds represented by general formula (I), general formula (II), general formula (II-1), general formula (G), and general formula (G-1) or the pharmaceutically acceptable salts thereof are provided, wherein J⁵ is selected from the group consisting of -C(O)NR^{6b}-, -NR^{6b}C(O)-, -O-, -S-, -NR^{6b}-, - C(O)-, -S(O)₂-, -(CR^{7b}R^{8b})ₘ₄-, C₂₋₆ alkenyl, C₂₋₆ alkynyl, 3- to 12-membered cycloalkyl, and 3- to 12-membered heterocyclyl, wherein the 3- to 12-membered cycloalkyl and 3-to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, -(CH₂)ₙNR^{g}R^{h}, nitro, hydroxy, C₁₋₆ hydroxyalkyl, C₁₋₆ aminoalkyl, C₁₋₆ alkoxy C₁₋₆ alkyl, 3- to 8-membered cycloalkyl C₁₋₆ alkyl, 3- to 8-membered heterocyclyl C₁₋₆ alkyl, 3- to 8-membered cycloalkyl, 3- to 8-membered heterocyclyl, and =O; R^{g} and R^{h} are identical or different and are each independently a hydrogen atom or C₁₋₆ alkyl; n is 0, 1, 2, or 3; R^{6b} is a hydrogen atom or C₁₋₆ alkyl; R^{7b} and R^{8b} are identical or different and are each independently selected from the group consisting of a hydrogen atom, halogen, C₁₋₆ alkyl, C₁₋₆ alkoxy, hydroxy, amino, cyano, C₁₋₆ haloalkyl, C₁₋₆ haloalkoxy, and C₁₋₆ hydroxyalkyl; m4 is 0, 1, 2, or 3.

In some embodiments of the present disclosure, the compounds of general formula (I), general formula (II), general formula (II-1), general formula (G), and general formula (G-1) or the pharmaceutically acceptable salts thereof are provided, wherein J⁵ is selected from the group consisting of -C(O)NR^{6b}-, -NR^{6b}C(O)-, -O-, -S-, -NR^{6b}-, -C(O)-, -S(O)₂-, -(CR^{7b}R^{8b})ₘ₄-, C₂₋₆ alkenyl, C₂₋₆ alkynyl, 3- to 8-membered cycloalkyl, and 3- to 8-membered heterocyclyl; R^{6b} is a hydrogen atom or C₁₋₆ alkyl, preferably a hydrogen atom or methyl; R^{7b} and R^{8b} are identical or different and are each independently selected from the group consisting of a hydrogen atom, halogen, hydroxy, and C₁₋₆ alkyl, preferably a hydrogen atom; m4 is 0, 1, 2, or 3, preferably 0 or 1.

In some embodiments of the present disclosure, the compounds represented by general formula (I), general formula (II), general formula (II-1), general formula (G), and general formula (G-1) or the pharmaceutically acceptable salts thereof are provided, wherein J⁵ is a bond.

In some embodiments of the present disclosure, the compounds represented by general formula (I), general formula (II), general formula (II-1), general formula (G), and general formula (G-1) or the pharmaceutically acceptable salts thereof are provided, wherein J⁵ is -C(O)NR^{6b}- or -NR^{6b}C(O)-; R^{6b} is a hydrogen atom or C₁₋₆ alkyl;
preferably, J⁵ is -C(O)NR^{6b}- or -NR^{6b}C(O)-; R^{6b} is a hydrogen atom or methyl;
more preferably, J⁵ is -C(O)NH-*; the * end is attached to A.

In some embodiments of the present disclosure, the compounds represented by general formula (I), general formula (II), and general formula (G) or the pharmaceutically acceptable salts thereof are compounds represented by general formula (III') or pharmaceutically acceptable salts thereof: 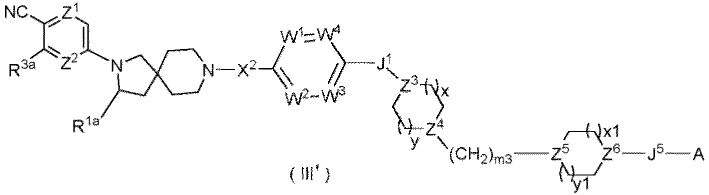 wherein:
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z³, Z⁴, Z⁵, and Z⁶ are identical or different and are each independently a N atom or CH; m3 is 0, 1, 2, or 3;
x, x1, y, and y1 are each independently 0, 1, or 2;
J⁵ is selected from the group consisting of -C(O)NR^{6b}-, -NR^{6b}C(O)-, -O-, -S-, -NR^{6b}-, - C(O)-, -S(O)₂-, -(CR^{7b}R^{8b})ₘ₄-, C₂₋₆ alkenyl, C₂₋₆ alkynyl, 3- to 8-membered cycloalkyl, and 3- to 8-membered heterocyclyl; R^{6b} is a hydrogen atom or C₁₋₆ alkyl; R^{7b} and R^{8b} are identical or different and are each independently selected from the group consisting of a hydrogen atom, halogen, hydroxy, and C₁₋₆ alkyl; m4 is 0, 1, 2, or 3; preferably, J⁵ is selected from the group consisting of a bond, 3- to 8-membered heterocyclyl, and 3- to 8-membered cycloalkyl;
Z¹, Z², R^{1a}, R^{3a}, X², J¹, and A are as defined in general formula (G).

In some embodiments of the present disclosure, the compounds represented by general formula (I), general formula (II), general formula (G), and general formula (III') or the pharmaceutically acceptable salts thereof are compounds represented by general formula (III'-1) or pharmaceutically acceptable salts thereof: 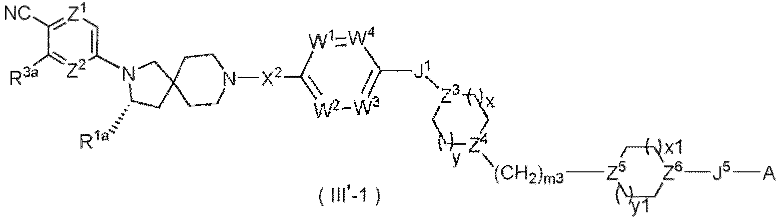 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; and
R^{3a}, W¹, W², W³, W⁴, Z¹, Z², Z³, Z⁴, Z⁵, Z⁶, x, x1, y, y1, m3, X², J¹, J⁵, and A are as defined in general formula (III').

In some embodiments of the present disclosure, the compounds represented by general formula (I), general formula (II), general formula (II-1), general formula (G), general formula (G-1), general formula (III'), and general formula (III'-1) or the pharmaceutically acceptable salts thereof are provided, wherein J⁵ is selected from the group consisting of a bond, 3- to 8-membered heterocyclyl, and 3- to 8-membered cycloalkyl; preferably, J⁵ is a bond or piperazinyl; more preferably, J⁵ is a bond.

In some embodiments of the present disclosure, the compounds represented by general formula (I), general formula (II), general formula (II-1), general formula (G), general formula (G-1), general formula (III'), and general formula (III'-1) or the pharmaceutically acceptable salts thereof are provided, wherein J⁵ is selected from the group consisting of a bond, 5- or 6-membered heterocyclyl, and -C(O)NH-*; preferably, J⁵ is selected from the group consisting of a bond, piperazinyl, and -C(O)NH-*; the * end is attached to A; more preferably, J⁵ is a bond.

In some embodiments of the present disclosure, the compounds represented by general formula (I), general formula (II), general formula (G), and general formula (III') or the pharmaceutically acceptable salts thereof are compounds represented by general formula (III) or pharmaceutically acceptable salts thereof: 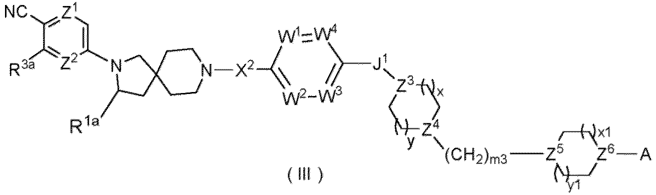 wherein:
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z³, Z⁴, Z⁵, and Z⁶ are identical or different and are each independently a N atom or CH, provided that at least one of Z³ and Z⁴ is a N atom;
m3 is 0, 1, 2, or 3;
x, x1, y, and y1 are each independently 0, 1, or 2;
Z¹, Z², R^{1a}, R^{3a}, X², J¹, and A are as defined in general formula (G).

In some embodiments of the present disclosure, the compounds represented by general formula (I), general formula (II), general formula (G), general formula (III'), and general formula (III) or the pharmaceutically acceptable salts thereof are compounds represented by general formula (III-1) or pharmaceutically acceptable salts thereof: 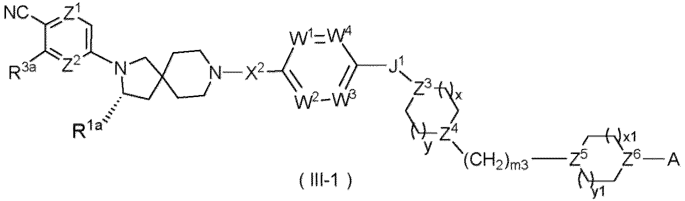 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; and
R^{3a}, W¹, W², W³, W⁴, Z¹, Z², Z³, Z⁴, Z⁵, Z⁶, x, x1, y, y1, m3, X², J¹, and A are as defined in general formula (III).

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein R^{2a} and R^{2b} are identical or different and are each independently selected from the group consisting of a hydrogen atom, halogen, C₁₋₆ alkyl, C₁₋₆ alkoxy, hydroxy, amino, cyano, C₁₋₆ haloalkyl, C₁₋₆ haloalkoxy, and C₁₋₆ hydroxyalkyl; preferably, R^{2a} and R^{2b} are identical or different and are each independently selected from the group consisting of a hydrogen atom, halogen, hydroxy, and C₁₋₆ alkyl; more preferably, R^{2a} and R^{2b} are both hydrogen atoms.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein X² is selected from the group consisting of -C(O)-, -S(O)₂-, and -(CR^{2a}R^{2b})ₘ₁-; R^{2a} and R^{2b} are identical or different and are each independently selected from the group consisting of a hydrogen atom, halogen, hydroxy, and C₁₋₆ alkyl, preferably a hydrogen atom; and m1 is 0, 1, 2, or 3.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein X² is a bond or -C(O)-; preferably, X² is a bond.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein R⁷ and R⁸ are identical or different and are each independently selected from the group consisting of a hydrogen atom, halogen, C₁₋₆ alkyl, C₁₋₆ alkoxy, amino, hydroxy, cyano, C₁₋₆ haloalkyl, C₁₋₆ haloalkoxy, and C₁₋₆ hydroxyalkyl; preferably, R⁷ and R⁸ are identical or different and are each independently selected from the group consisting of a hydrogen atom, halogen, hydroxy, and C₁₋₆ alkyl; more preferably, R⁷ and R⁸ are both hydrogen atoms.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein R⁶ is a hydrogen atom or C₁₋₆ alkyl; preferably, R⁶ is a hydrogen atom or methyl. In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein J¹ is selected from the group consisting of -O-, -S-, -NR⁶-, -C(O)-, -S(O)₂-, - (CR⁷R⁸)ₘ₂-, C₂₋₆ alkenyl, and C₂₋₆ alkynyl; R⁶ is a hydrogen atom or C₁₋₆ alkyl, preferably a hydrogen atom or methyl; R⁷ and R⁸ are identical or different and are each independently selected from the group consisting of a hydrogen atom, halogen, hydroxy, and C₁₋₆ alkyl, preferably a hydrogen atom; and m2 is 0, 1, 2, or 3.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein J¹ is selected from the group consisting of a bond, -O-, -S-, -NH-, -N(CH₃)-, - C(O)-, -S(O)₂-, -CH₂-, -CH(CH₃)-, -CH₂CH₂-, -CH₂CH₂CH₂-, and ethynylene; preferably, J¹ is selected from the group consisting of a bond, -O-, -S-, -CH₂-, and -C(O)-; more preferably, J¹ is selected from the group consisting of a bond, -S-, and -C(O)-; further preferably, J¹ is -S- or -C(O)-; most preferably, J¹ is -C(O)-.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein X² is -C(O)-; and J¹ is -S-.

In some embodiments of the present disclosure, the compounds represented by general formula (I), general formula (II), general formula (II-1), general formula (G), general formula (G-1), general formula (III'), general formula (III'-1), general formula (III), and general formula (III-1) or the pharmaceutically acceptable salts thereof are provided, wherein X² is a bond; and J¹ is -C(O)-.

In some embodiments of the present disclosure, the compounds represented by general formula (I), general formula (II), and general formula (G) or the pharmaceutically acceptable salts thereof are compounds represented by general formula (X) or pharmaceutically acceptable salts thereof: 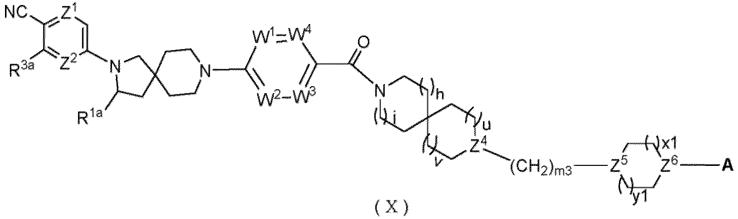 wherein:
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z⁴, Z⁵, and Z⁶ are identical or different and are each independently a N atom or CH;
m3 is 0, 1, 2, or 3;
h, i, u, and v are each independently 0 or 1;
x1 and y1 are each independently 0, 1, or 2;
Z¹, Z², R^{1a}, R^{3a}, and A are as defined in general formula (II).

In some embodiments of the present disclosure, the compounds represented by general formula (I), general formula (II), general formula (G), and general formula (X) or the pharmaceutically acceptable salts thereof are compounds represented by general formula (X-1) or pharmaceutically acceptable salts thereof: 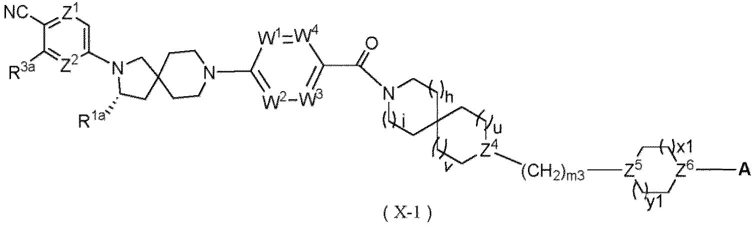 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; and
R^{3a}, W¹, W², W³, W⁴, Z¹, Z², Z⁴, Z⁵, Z⁶, h, i, u, v, x1, y1, m3, and A are as defined in general formula (X).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein Q² is a carbon atom, and Q¹, Q³, Q⁴, and Q⁵ are identical or different and are each independently a nitrogen atom or CR';
each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, C₁₋₆ alkoxy C₁₋₆ alkyl, cyano, hydroxy, and - (CH₂)ₙNR^{c}R^{d}; R^{c} and R^{d} are identical or different and are each independently a hydrogen atom or C₁₋₆ alkyl; and n is 0, 1, 2, or 3;
preferably, each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino;
further preferably, each R' is identical or different and is independently selected from the group consisting of a hydrogen atom, halogen, hydroxy, and C₁₋₆ alkyl;
more preferably, each R' is identical or different and is independently a hydrogen atom or halogen;
most preferably, each R' is identical or different and is independently a hydrogen atom or fluorine.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein:
Q² is a carbon atom, and Q¹, Q³, Q⁴, and Q⁵ are identical or different and are each independently a nitrogen atom or CR'; or, Q³ is a carbon atom, and Q¹, Q², Q⁴, and Q⁵ are identical or different and are each independently a nitrogen atom or CR'; wherein:
each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, C₁₋₆ aminoalkyl, C₁₋₆ alkoxy C₁₋₆ alkyl, cyano, hydroxy, and - (CH₂)ₙNR^{c}R^{d}; R^{c} and R^{d} are identical or different and are each independently a hydrogen atom or C₁₋₆ alkyl; and n is 0, 1, 2, or 3;
preferably, each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino;
further preferably, each R' is identical or different and is independently selected from the group consisting of a hydrogen atom, halogen, hydroxy, and C₁₋₆ alkyl;
more preferably, each R' is identical or different and is independently a hydrogen atom or halogen;
most preferably, each R' is identical or different and is independently a hydrogen atom or fluorine.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein R¹ is a hydrogen atom or C₁₋₆ alkyl; preferably, R¹ is a hydrogen atom.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein R² is a hydrogen atom or C₁₋₆ alkyl; preferably, R² is a hydrogen atom.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein - - - - is a single bond. In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is 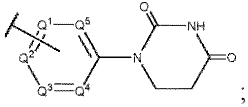 Q¹, Q², Q³, Q⁴, and Q⁵ are as defined in general formula (I); preferably, Q² is a carbon atom, Q¹, Q³, Q⁴, and Q⁵ are each independently CR', and R' is a hydrogen atom or halogen; more preferably, Q² is a carbon atom, Q¹, Q³, Q⁴, and Q⁵ are each independently CR', and R' is a hydrogen atom or fluorine; most preferably, Q² is a carbon atom, and Q¹, Q³, Q⁴, and Q⁵ are all CH.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of the following structures: 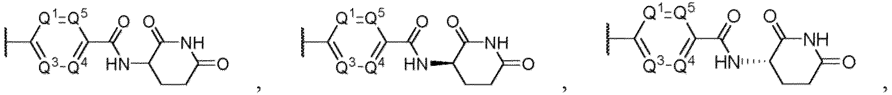 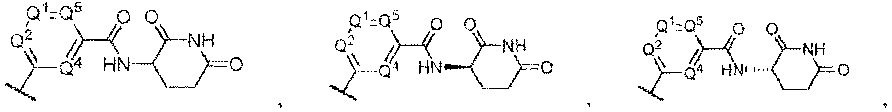 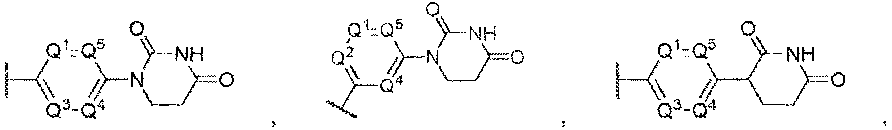 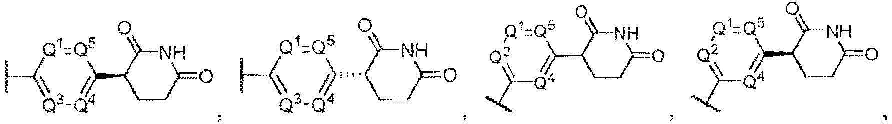 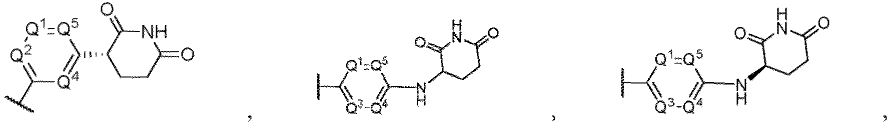 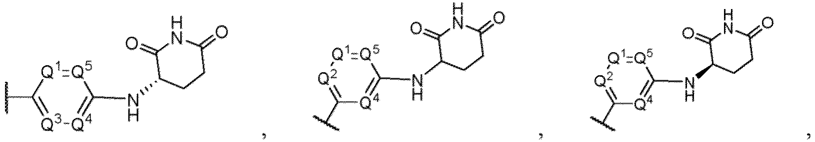 and 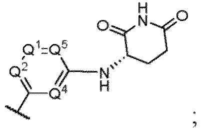 Q¹, Q², Q³, Q⁴, and Q⁵ are identical or different and are each independently a nitrogen atom or CR'; each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino, preferably a hydrogen atom, halogen, hydroxy, and C₁₋₆ alkyl, more preferably a hydrogen atom or halogen, further preferably a hydrogen atom or fluorine, and most preferably a hydrogen atom.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of

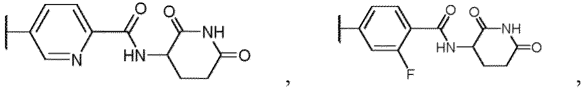

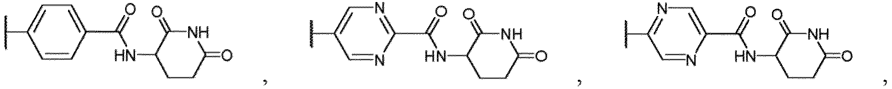

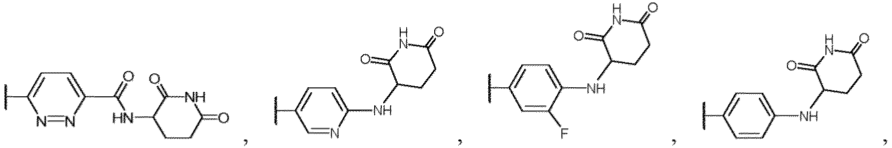

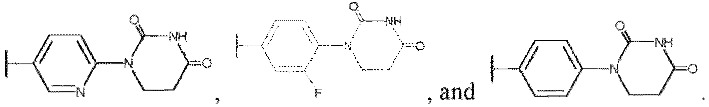

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of

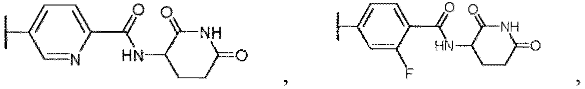

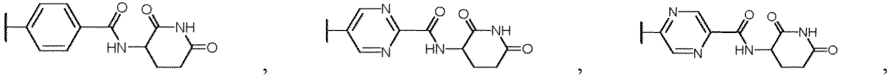

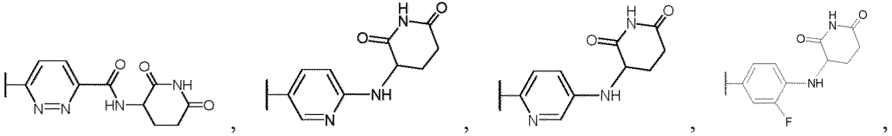

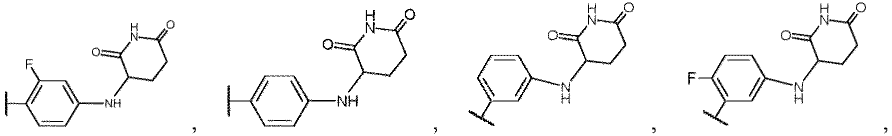

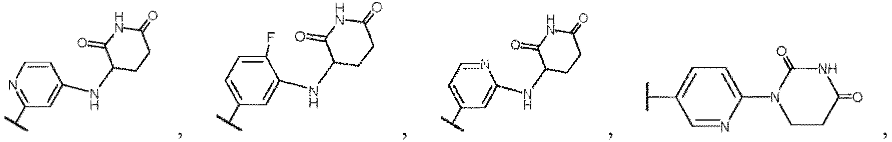

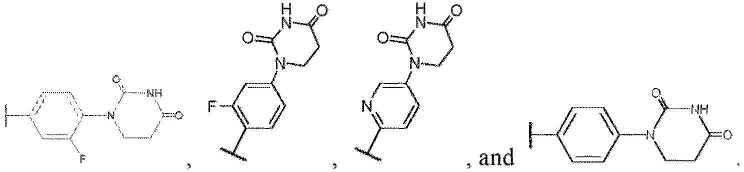

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of the following structures: 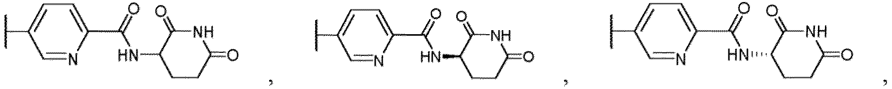 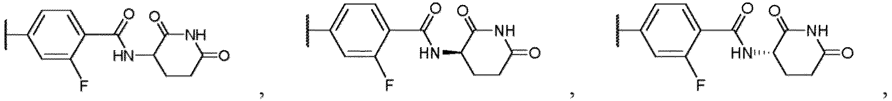 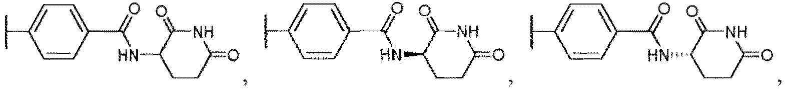 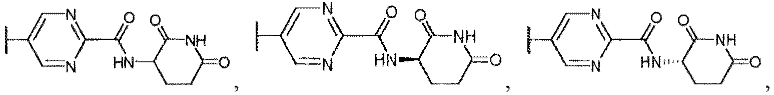 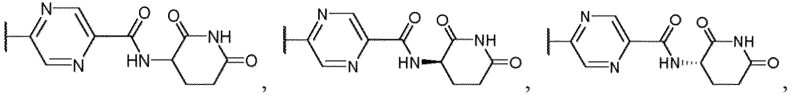 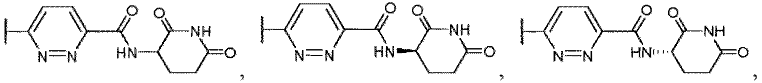 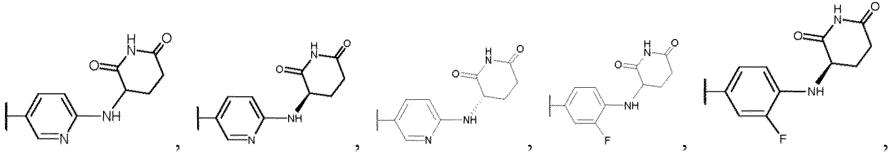 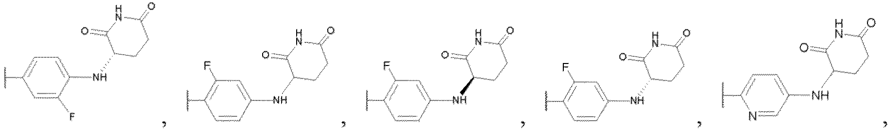 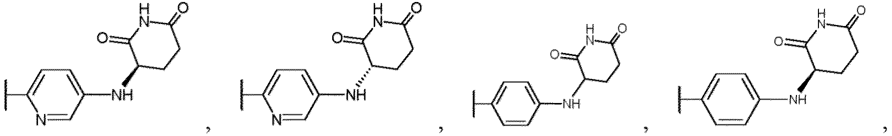 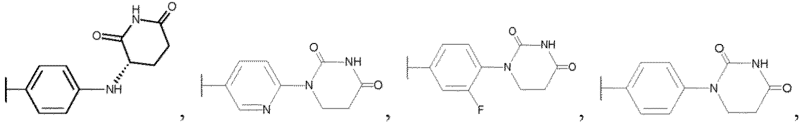 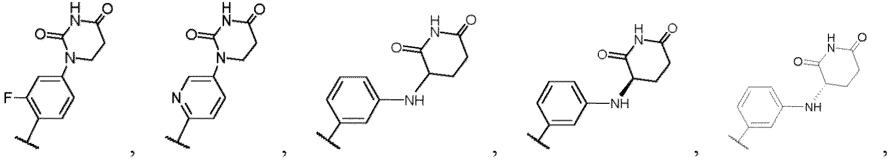 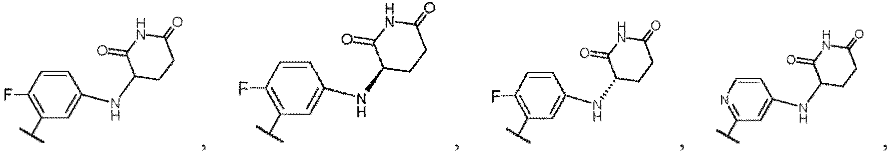 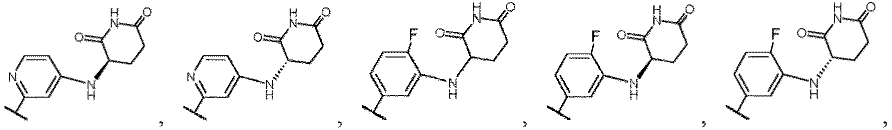 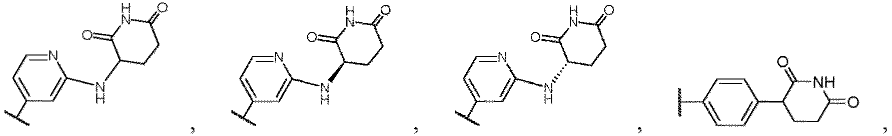 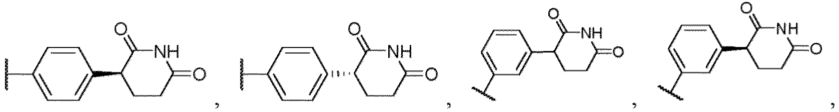 and

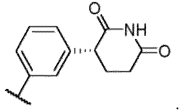

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of the following structures: 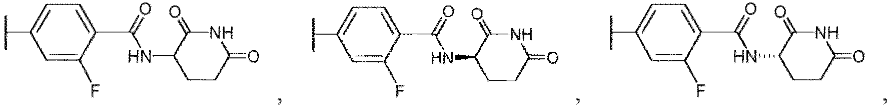 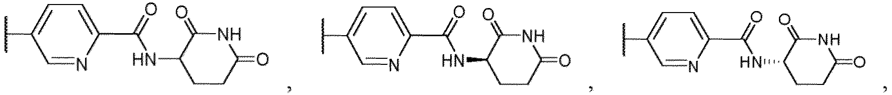 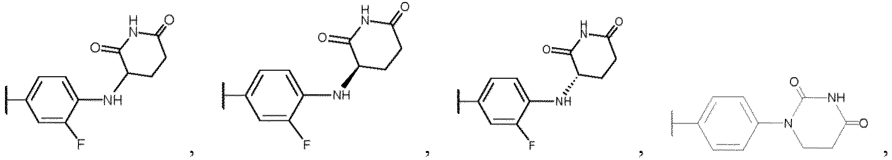 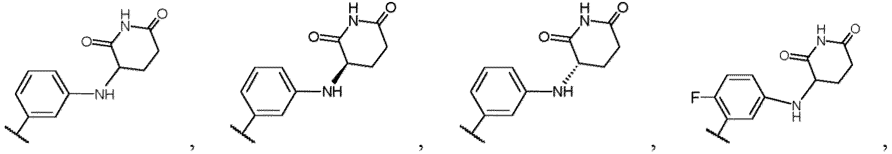 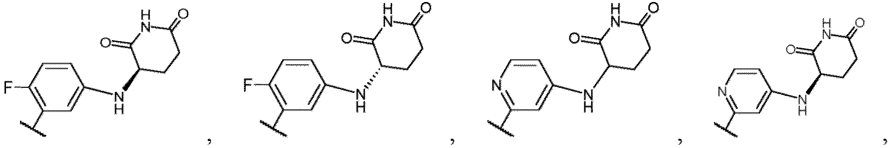 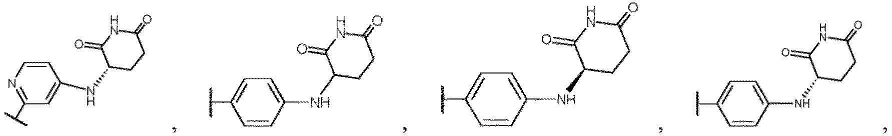 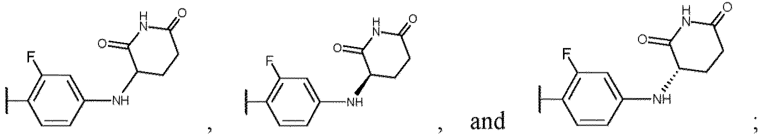 preferably

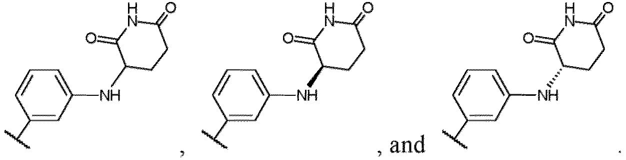

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of the following structures: 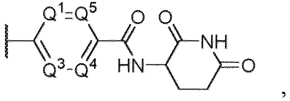 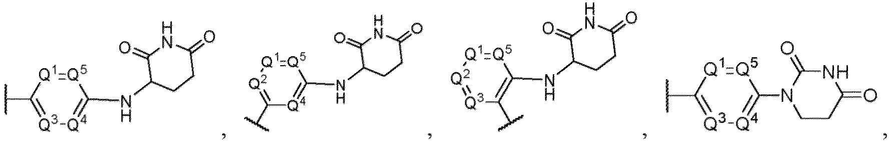 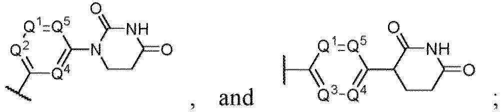 preferably 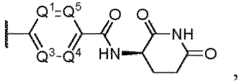 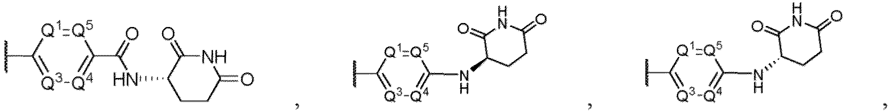 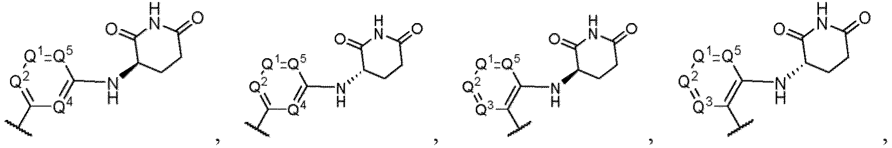 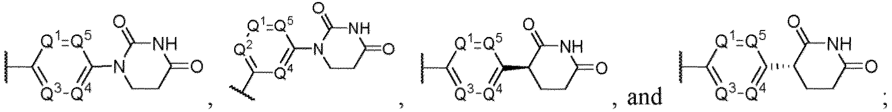 more preferably 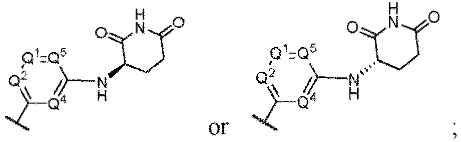 and most preferably 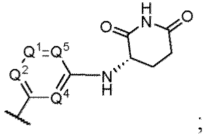 wherein: Q¹, Q³, Q², Q⁴, and Q⁵ are as defined in general formula (I); preferably, Q¹, Q², Q³, Q⁴, and Q⁵ are identical or different and are each independently a nitrogen atom or CR'; each R' is identical or different and is independently a hydrogen atom or halogen; more preferably, Q¹, Q², Q³, Q⁴, and Q⁵ are CR'; each R' is identical or different and is independently a hydrogen atom or F; more preferably, Q¹, Q², Q³, Q⁴, and Q⁵ are CH.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of the following structures: 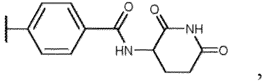 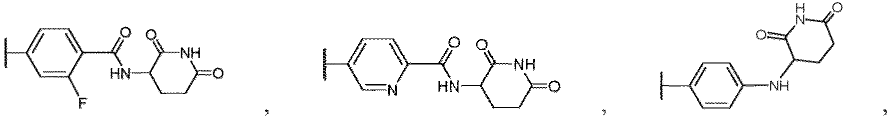 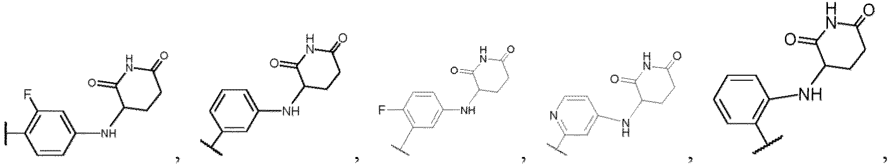 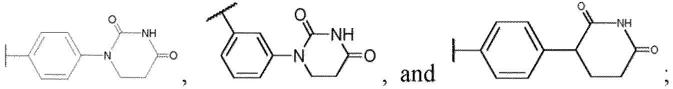 preferably from the group consisting of 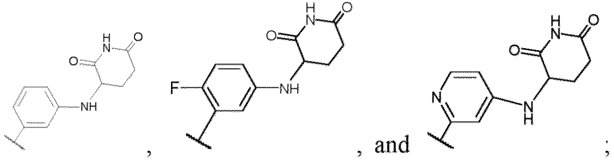 more preferably 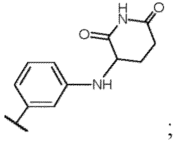 and most preferably

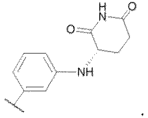

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), and general formula (X-1) or the pharmaceutically acceptable salts thereof are provided, wherein A is selected from the group consisting of the following structures: 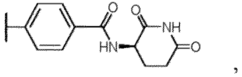 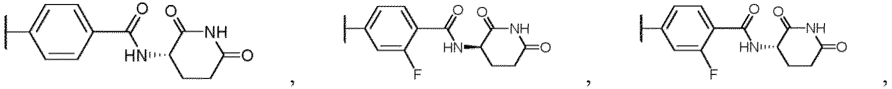 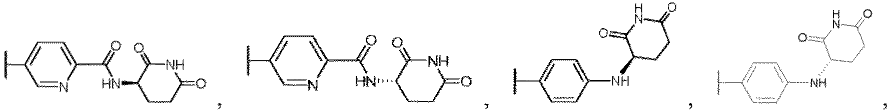 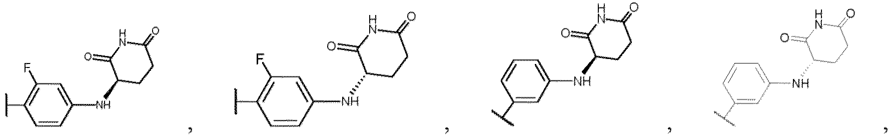 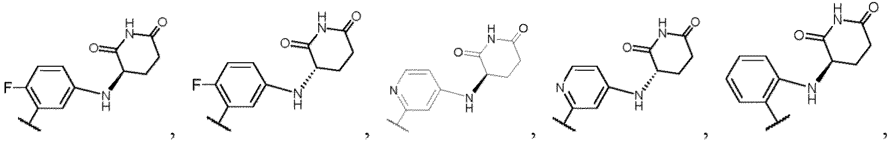 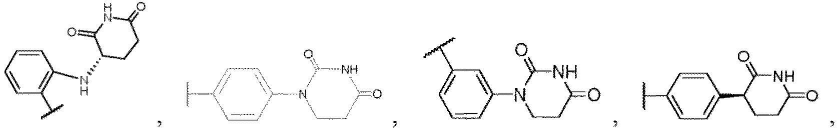 and 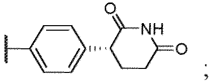 preferably from the group consisting of 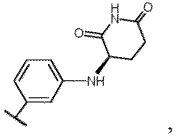 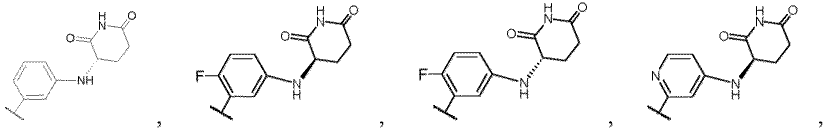 and 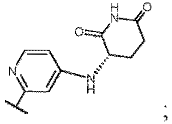 more preferably from the group consisting of 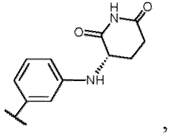 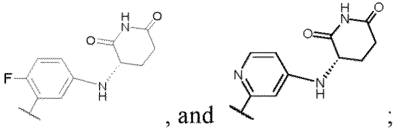 and most preferably

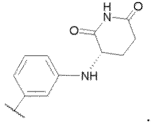

In some embodiments of the present disclosure, the compounds represented by general formula (I), general formula (II), general formula (G), general formula (III'), and general formula (III) or the pharmaceutically acceptable salts thereof are compounds represented by general formula (IV') or pharmaceutically acceptable salts thereof: 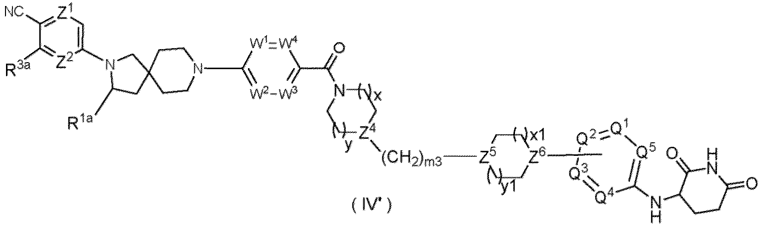 wherein:
Q² is a carbon atom, and Q¹, Q³, Q⁴, and Q⁵ are identical or different and are each independently a nitrogen atom or CR'; or, Q³ is a carbon atom, and Q¹, Q², Q⁴, and Q⁵ are identical or different and are each independently a nitrogen atom or CR';
each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino; and
Z¹, Z², R^{1a}, R^{3a}, W¹, W², W³, W⁴, Z⁴, Z⁵, Z⁶, m3, x, x1, y, and y1 are as defined in general formula (III').

In some embodiments of the present disclosure, the compounds represented by general formula (I), general formula (II), general formula (G), general formula (III'), general formula (III), and general formula (IV') or the pharmaceutically acceptable salts thereof are compounds represented by general formula (IV'-1) or pharmaceutically acceptable salts thereof: 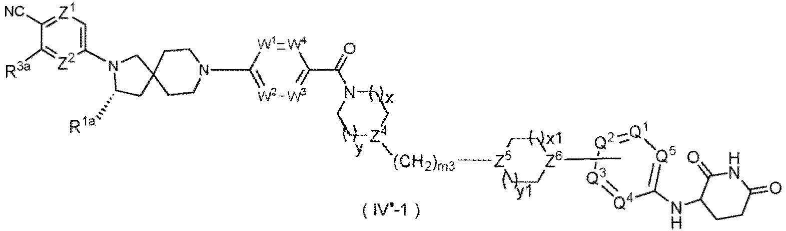 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably, R^{1a} is C₁₋₆ alkyl; more preferably, R^{1a} is methyl;
Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in general formula (IV').

In some embodiments of the present disclosure, the compounds represented by general formula (I), general formula (II), general formula (II-1), general formula (III'), general formula (III'-1), general formula (G), general formula (G-1), general formula (III), general formula (III-1), general formula (IV'), and general formula (IV'-1) or the pharmaceutically acceptable salts thereof are compounds represented by general formula (IV'-1-1) or general formula (IV'-1-2) or pharmaceutically acceptable salts thereof: 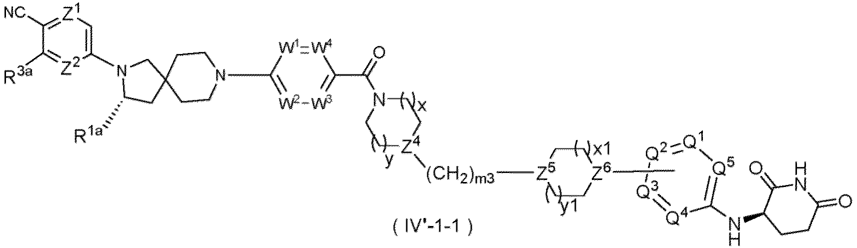 or 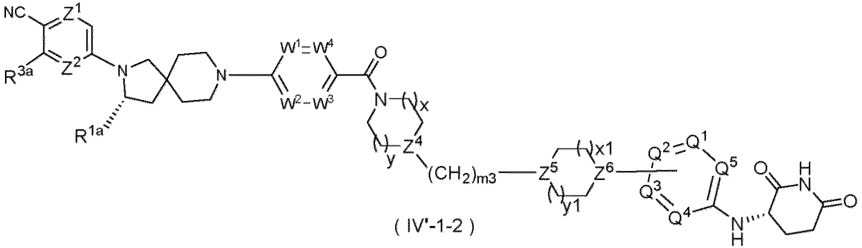 wherein:
Q¹, Q², Q³, Q⁴, Q⁵, R^{1a}, R^{3a}, W¹, W², W³, W⁴, Z¹, Z², Z⁴, Z⁵, Z⁶, x, x1, y, y1, and m3 are as defined in general formula (IV'-1).

In some embodiments of the present disclosure, the compounds represented by general formula (I), general formula (II), general formula (G), general formula (III'), general formula (III), and general formula (IV') or the pharmaceutically acceptable salts thereof are compounds represented by general formula (M) or pharmaceutically acceptable salts thereof: 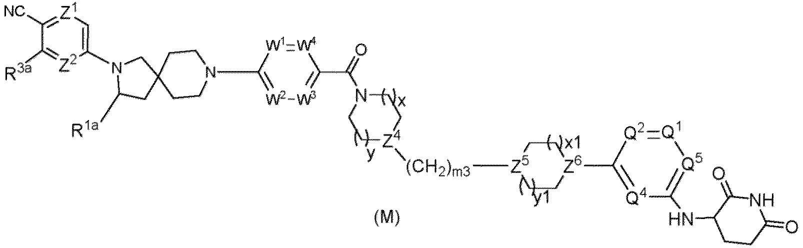 wherein:
Q¹, Q², Q⁴, Q⁵, R^{1a}, R^{3a}, W¹, W², W³, W⁴, Z¹, Z², Z⁴, Z⁵, Z⁶, x, x1, y, y1, and m3 are as defined in general formula (IV').

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (IV'), general formula (IV'-1), and general formula (M) or the pharmaceutically acceptable salts thereof are compounds represented by general formula (M-1) or pharmaceutically acceptable salts thereof: 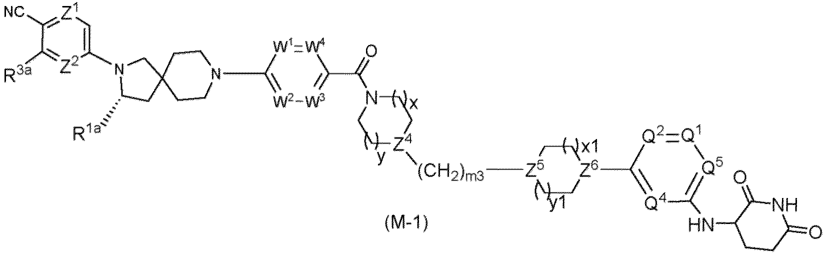 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably, R^{1a} is C₁₋₆ alkyl; more preferably, R^{1a} is methyl;
Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (IV'), general formula (IV'-1), general formula (M), and general formula (M-1) or the pharmaceutically acceptable salts thereof are compounds represented by general formula (M-1-1) or general formula (M-1-2) or pharmaceutically acceptable salts thereof: 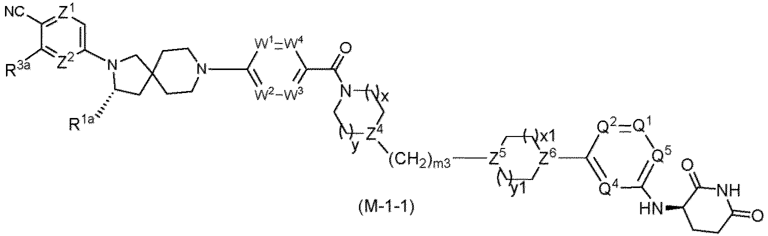 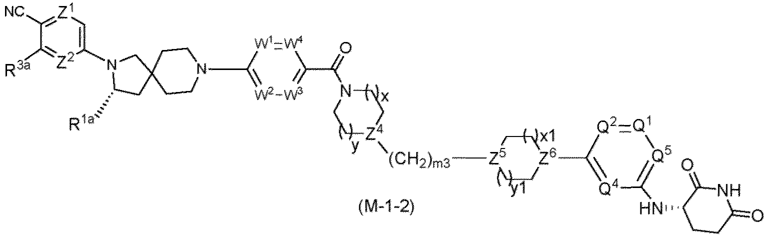 wherein:
Q¹, Q², Q⁴, Q⁵, R^{1a}, R^{3a}, w1, W², W³, W⁴, Z¹, Z², Z⁴, Z⁵, Z⁶, x, x1, y, y1, and m3 are as defined in general formula (M-1).

In some embodiments of the present disclosure, the compounds represented by general formula (I), general formula (II), general formula (G), general formula (III'), and general formula (III) or the pharmaceutically acceptable salts thereof are compounds represented by general formula (IV) or pharmaceutically acceptable salts thereof: 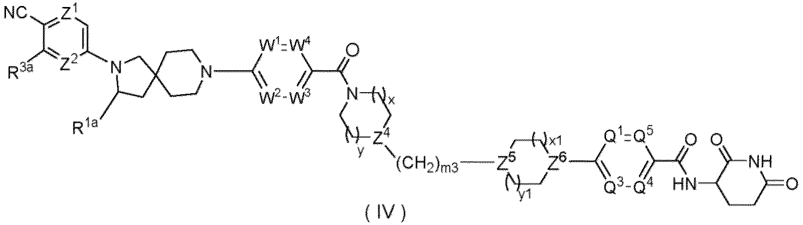 wherein:
Q¹, Q³, Q⁴, and Q⁵ are identical or different and are each independently a nitrogen atom or CR';
each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino; and
Z¹, Z², R^{1a}, R^{3a}, W¹, W², W³, W⁴, Z⁴, Z⁵, Z⁶, m3, x, x1, y, and y1 are as defined in general formula (III).

In some embodiments of the present disclosure, the compounds represented by general formula (I), general formula (II), general formula (G), general formula (III'), general formula (III), and general formula (IV) or the pharmaceutically acceptable salts thereof are compounds represented by general formula (IV-1) or pharmaceutically acceptable salts thereof: 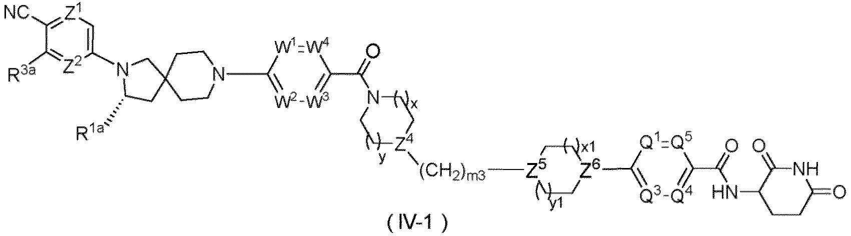 wherein:
R^{1a} is selected from the group consisting of halogen, C₁₋₆ alkyl, C₁₋₆ alkoxy, C₁₋₆ haloalkyl,
C₁₋₆ haloalkoxy, cyano, amino, hydroxy, and C₁₋₆ hydroxyalkyl; preferably, R^{1a} is C₁₋₆ alkyl; more preferably, R^{1a} is methyl;
Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (IV), and general formula (IV-1) or the pharmaceutically acceptable salts thereof are compounds represented by general formula (IV-1-1) or general formula (IV-1-2) or pharmaceutically acceptable salts thereof: 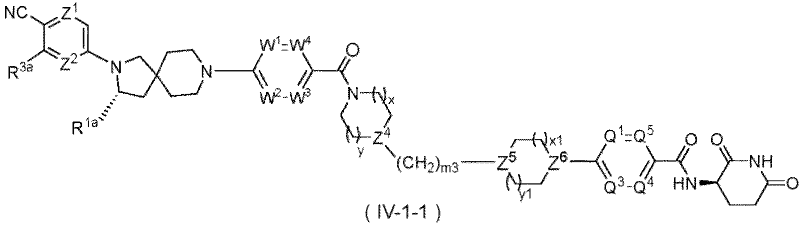 or 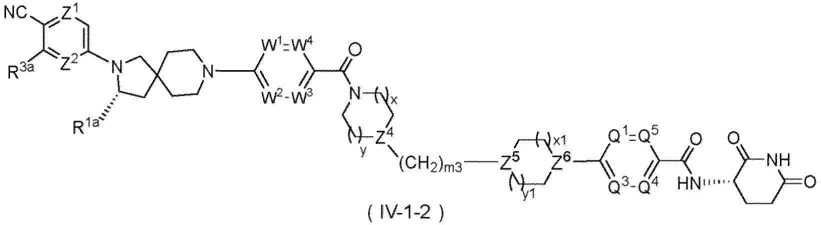 wherein:
Q¹, Q³, Q⁴, Q⁵, R^{1a}, R^{3a}, w1, W², W³, W⁴, Z¹, Z², Z⁴, Z⁵, Z⁶, x, x1, y, y1, and m3 are as defined in general formula (IV-1).

In some embodiments of the present disclosure, the compounds represented by general formula (I), general formula (II), general formula (G), general formula (III'), and general formula (III) or the pharmaceutically acceptable salts thereof are compounds represented by general formula (V) or pharmaceutically acceptable salts thereof: 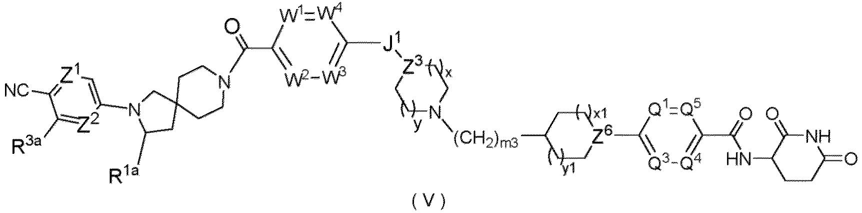 wherein:
J¹ is selected from the group consisting of a bond, O, S, -CH₂-, and -C(O)-;

Q¹, Q³, Q⁴, and Q⁵ are identical or different and are each independently a nitrogen atom or CR';
each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino; and
Z¹, Z², R^{1a}, R^{3a}, W¹, W², W³, W⁴, Z³, Z⁶, m3, x, x1, y, and y1 are as defined in general formula (III).

In some embodiments of the present disclosure, the compounds represented by general formula (I), general formula (II), general formula (G), general formula (III'), general formula (III), and general formula (V) or the pharmaceutically acceptable salts thereof are compounds represented by general formula (V-1) or pharmaceutically acceptable salts thereof: 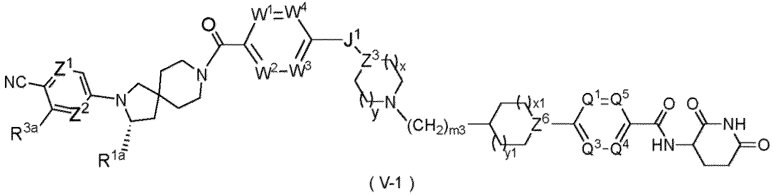 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably, R^{1a} is C₁₋₆ alkyl; more preferably, R^{1a} is methyl;
J¹, Q¹, Q³, Q⁴, Q⁵, R^{3a}, W¹, W², W³, W⁴, Z¹, Z², Z³, Z⁶, x, x1, y, y1, and m3 are as defined in general formula (V).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (V), and general formula (V-1) or the pharmaceutically acceptable salts thereof are compounds represented by general formula (V-1-1) or general formula (V-1-2) or pharmaceutically acceptable salts thereof: 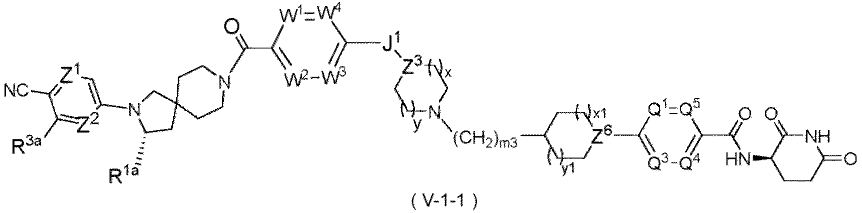 or 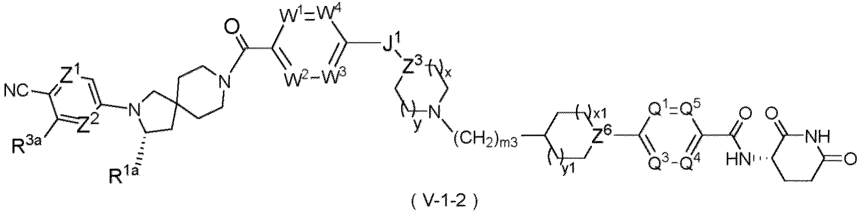 wherein:
J¹, Q¹, Q³, Q⁴, Q⁵, R^{1a}, R^{3a}, W¹, W², W³, W⁴, Z¹, Z², Z³, Z⁶, x, x1, y, y1, and m3 are as defined in general formula (V-1).

In some embodiments of the present disclosure, the compounds represented by general formula (II), general formula (II-1), general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein Z¹ is CR^{3b}; Z² is CR^{3c}; R^{3a}, R^{3b}, and R^{3c} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl;
preferably, Z¹ is CR^{3b}; Z² is CR^{3c}; R^{3a}, R^{3b}, and R^{3c} are identical or different and are each independently selected from the group consisting of a hydrogen atom, halogen, C₁₋₆ alkyl, C₁₋₆ alkoxy, cyano, and C₁₋₆ haloalkyl; further preferably, Z¹ is CR^{3b}; Z² is CR^{3c}; R^{3b} and R^{3c} are both hydrogen atoms; R^{3a} is selected from the group consisting of a hydrogen atom, halogen, C₁₋₆ alkyl, C₁₋₆ alkoxy, cyano, and C₁₋₆ haloalkyl;
more preferably, Z¹ is CR^{3b}; Z² is CR^{3c}; R^{3b} and R^{3c} are both hydrogen atoms; and R^{3a} is halogen;
most preferably, Z¹ is CR^{3b}; Z² is CR^{3c}; R^{3b} and R^{3c} are both hydrogen atoms; and R^{3a} is a chlorine atom.

In some embodiments of the present disclosure, the compounds represented by general formula (II), general formula (II-1), general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein Z¹ is CR^{3b}; Z² is CR^{3c}; R^{3a} is halogen or C₁₋₆ haloalkyl; R^{3b} is a hydrogen atom; R^{3c} is a hydrogen atom or C₁₋₆ alkyl; preferably, Z¹ is CR^{3b}; Z² is CR^{3c}; R^{3a} is Cl or trifluoromethyl; R^{3b} is a hydrogen atom; R^{3c} is a hydrogen atom or methyl.

In some embodiments of the present disclosure, the compounds represented by general formula (II), general formula (II-1), general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein is selected from the group consisting of the following structures: 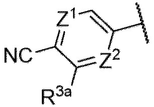 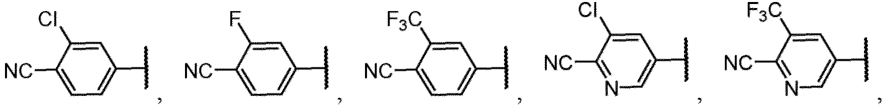 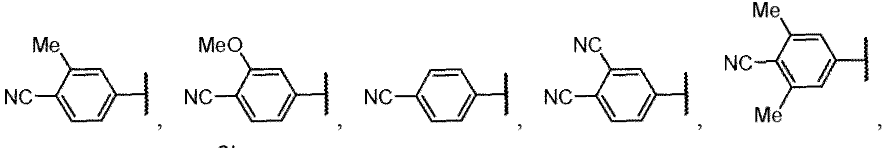 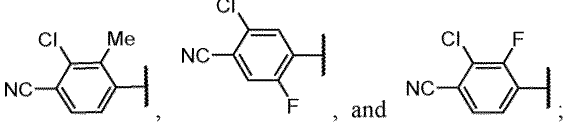 preferably, 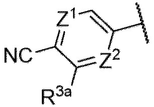 is selected from the group consisting of 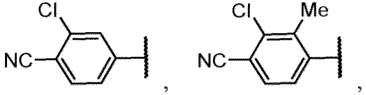 and 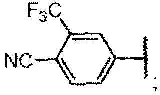 more preferably,

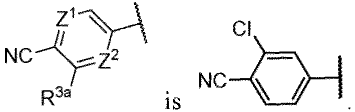

In some embodiments of the present disclosure, the compounds represented by general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein W¹ is CR^{3d}; W² is CR^{3e}; W³ is CR^{3f}; W⁴ is N or CR^{3g}; R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
preferably, W¹ is CR^{3d}; W² is CR^{3e}; W³ is CR^{3f}; W⁴ is N or CR^{3g}; R^{3d}, R^{3e}, R^{3f}, and R^{3g} are identical or different and are each independently selected from the group consisting of a hydrogen atom, halogen, C₁₋₆ alkyl, and C₁₋₆ haloalkyl.

In some embodiments of the present disclosure, the compounds represented by general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein W¹ is CR^{3d}; W² is CR^{3e}; W³ is CR^{3f}; W⁴ is CR^{3g}; R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
preferably, W¹ is CR^{3d}; W² is CR^{3e}; W³ is CR^{3f}; W⁴ is CR^{3g}; R^{3d}, R^{3e}, R^{3f}, and R^{3g} are identical or different and are each independently selected from the group consisting of a hydrogen atom, halogen, C₁₋₆ alkyl, and C₁₋₆ haloalkyl;
more preferably, W¹ is CR^{3d}; W² is CR^{3e}; W³ is CR^{3f}; W⁴ is CR^{3g}; R^{3d}, R^{3e}, R^{3f}, and R^{3g} are all hydrogen atoms.

In some embodiments of the present disclosure, the compounds represented by general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein W¹ is CR^{3d}; W² is CR^{3e}; W³ is CR^{3f}; W⁴ is N; R^{3d}, R^{3e}, and R^{3f} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
preferably, W¹ is CR^{3a}; W² is CR^{3e}; W³ is CR^{3f}; W⁴ is N; R^{3d}, R^{3e}, and R^{3f} are identical or different and are each independently selected from the group consisting of a hydrogen atom, halogen, C₁₋₆ alkyl, and C₁₋₆ haloalkyl;
more preferably, W¹ is CR^{3d}; W² is CR^{3e}; W³ is CR^{3f}; W⁴ is N; R^{3d}, R^{3e}, and R^{3f} are all hydrogen atoms.

In some embodiments of the present disclosure, the compounds represented by general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein:
W¹ is N or CR3^{d}; W² is N or CR^{3e}; W³ is N or CR^{3f}; W⁴ is N or CR^{3g}; R^{3d}, R^{3e}, R^{3f}, and R^{3g} are identical or different and are each independently selected from the group consisting of a hydrogen atom, halogen, C₁₋₆ alkyl, and C₁₋₆ haloalkyl; preferably, W¹ is CR^{3d}, W² is CR^{3e}; W³ is CR^{3f}; and W⁴ is CR^{3g}; or,
W¹ is CR^{3d}, W² is CR^{3e}; W³ is CR^{3f}; and W⁴ is N; or,
W¹ is N, W² is CR^{3e}; W³ is CR^{3f}; and W⁴ is N; or,
W¹ is CR^{3d}, W² is N, W³ is CR^{3f}; and W⁴ is N; or,
W¹ is CR^{3d}, W² is N, W³ is CR^{3f}; and W⁴ is CR^{3g}; and R^{3d}, R^{3e}, R^{3f}, and R^{3g} are identical or different and are each independently selected from the group consisting of a hydrogen atom, halogen, C₁₋₆ alkyl, and C₁₋₆ haloalkyl, preferably a hydrogen atom or halogen, more preferably a hydrogen atom or fluorine, and most preferably a hydrogen atom.

In some embodiments of the present disclosure, the compounds represented by general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein: W¹ is N or CR^{3d}; W² is N or CR^{3e}; W³ is N or CR^{3f}; W⁴ is N or CR^{3g}; R^{3d}, R^{3e}, R^{3f}, and R^{3g} are identical or different and are each independently a hydrogen atom or halogen (the halogen is preferably F);
preferably, W¹ is CR^{3d}; W² is CR^{3e}; W³ is CR^{3f}; W⁴ is CR^{3g}; R^{3d}, R^{3e}, R^{3f}, and R^{3g} are identical or different and are each independently a hydrogen atom or halogen;
preferably, W¹ is CR^{3d}; W² is CR^{3e}; W³ is CR^{3f}; W⁴ is CR^{3g}; R^{3d}, R^{3e}, R^{3f}, and R^{3g} are identical or different and are each independently a hydrogen atom or fluorine;
further preferably, W¹ is CF or CH; W² is CH; W³ is CH; and W⁴ is CH;
most preferably, W¹ is CF; W² is CH; W³ is CH; and W⁴ is CH.

In some embodiments of the present disclosure, the compounds represented by general formula (II), general formula (II-1), general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein R^{1a} is C₁₋₆ alkyl, preferably methyl.

In some embodiments of the present disclosure, the compounds represented by general formula (II), general formula (G), general formula (III'), general formula (III), general formula (X), general formula (IV'), general formula (M), general formula (IV), and general formula (V) or the pharmaceutically acceptable salts thereof are provided, wherein R^{1a} is a hydrogen atom or C₁₋₆ alkyl; preferably a hydrogen atom or methyl.

In some embodiments of the present disclosure, the compounds represented by general formula (V), general formula (V-1), general formula (V-1-1), and general formula (V-1-2) or the pharmaceutically acceptable salts thereof are provided, wherein J¹ is -S-.

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), general formula (X-1), general formula (IV'), general formula (IV'-1), general formula (IV'-1-1), and general formula (IV'-1-2) or the pharmaceutically acceptable salts thereof are provided, wherein Q² is a carbon atom, and Q¹, Q³, Q⁴, and Q⁵ are identical or different and are each independently a nitrogen atom or CR'; or, Q³ is a carbon atom, and Q¹, Q², Q⁴, and Q⁵ are identical or different and are each independently a nitrogen atom or CR'; or, Q⁴ is a carbon atom, and Q¹, Q², Q³, and Q⁵ are identical or different and are each independently a nitrogen atom or CR'; and R' is selected from the group consisting of a hydrogen atom, halogen, and C₁₋₆ alkyl;
preferably, Q² is a carbon atom, and Q¹, Q³, Q⁴, and Q⁵ are identical or different and are each independently a nitrogen atom or CR'; or, Q³ is a carbon atom, and Q¹, Q², Q⁴, and Q⁵ are identical or different and are each independently a nitrogen atom or CR'; and R' is selected from the group consisting of a hydrogen atom, halogen, and C₁₋₆ alkyl;
further preferably, Q³ is a carbon atom, and Q¹, Q², Q⁴, and Q⁵ are identical or different and are each independently a nitrogen atom or CR'; or Q² is a carbon atom, and Q¹, Q³, Q⁴, and Q⁵ are identical or different and are each independently a nitrogen atom or CR'; and R' is a hydrogen atom or halogen;
further preferably, Q³ is a carbon atom, and Q¹, Q², Q⁴, and Q⁵ are identical or different and are each independently a nitrogen atom or CR'; and R' is a hydrogen atom or halogen; more preferably, Q³ is a carbon atom, and Q¹, Q², Q⁴, and Q⁵ are each independently CR'; and R' is a hydrogen atom or halogen;
more preferably, Q³ is a carbon atom, and Q¹, Q², Q⁴, and Q⁵ are each independently CR'; and R' is a hydrogen atom or fluorine.
more preferably, Q³ is a carbon atom, Q¹, Q⁴, and Q⁵ are CH, and Q² is a hydrogen atom or F;
most preferably, Q³ is a carbon atom, and Q¹, Q², Q⁴, and Q⁵ are all CH.

In some embodiments of the present disclosure, the compounds represented by general formula (M), general formula (M-1), general formula (M-1-1), and general formula (M-1-2) or the pharmaceutically acceptable salts thereof are provided, wherein Q¹, Q², Q⁴, and Q⁵ are each independently a nitrogen atom or CR'; and R' is a hydrogen atom or halogen;
preferably, Q¹, Q², Q⁴, and Q⁵ are each independently CR'; and R' is a hydrogen atom or halogen;
more preferably, Q¹, Q², Q⁴, and Q⁵ are each independently CR'; and R' is a hydrogen atom or fluorine;
most preferably, Q¹, Q², Q⁴, and Q⁵ are all CH.

In some embodiments of the present disclosure, the compounds represented by general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein: Q¹, Q³, Q⁴, and Q⁵ are each independently a nitrogen atom or CR'; and R' is selected from the group consisting of a hydrogen atom, halogen, and C₁₋₆ alkyl;
preferably, Q¹, Q³, Q⁴, and Q⁵ are each independently CR'; and R' is a hydrogen atom or halogen;
more preferably, Q¹, Q³, Q⁴, and Q⁵ are each independently CR'; and R' is a hydrogen atom or fluorine;
most preferably, Q¹, Q³, Q⁴, and Q⁵ are all CH.

In some embodiments of the present disclosure, the compounds represented by general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein Q¹, Q³, and Q⁵ are CR⁰; Q⁴ is N or CR⁰¹; R⁰ and R⁰¹ are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino;
preferably, Q¹, Q³, and Q⁵ are CR⁰; Q⁴ is N or CR⁰¹; R⁰ is a hydrogen atom; and R⁰¹ is a hydrogen atom or halogen (the halogen is preferably F);
more preferably, Q¹, Q³, and Q⁵ are CR⁰; Q⁴ is CR⁰¹; R⁰ is a hydrogen atom; and R⁰¹ is a hydrogen atom or halogen (the halogen is preferably F).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), and general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein m3 is 0 or 1; preferably, m3 is 1.

In some embodiments of the present disclosure, the compounds represented by general formula (X), general formula (X-1), general formula (IV'), general formula (IV'-1), general formula (IV'-1-1), general formula (IV'-1-2), general formula (M), general formula (M-1), general formula (M-1-1), and general formula (M-1-2), general formula (IV), general formula (IV-1), general formula (IV-1-1), and general formula (IV-1-2) or the pharmaceutically acceptable salts thereof are provided, wherein Z⁴ is CH, Z⁵ is N, and Z⁶ is N or CH.

In some embodiments of the present disclosure, the compounds represented by general formula (V), general formula (V-1), general formula (V-1-1), and general formula (V-1-2) or the pharmaceutically acceptable salts thereof are provided, wherein Z⁶ is a N atom. In some embodiments of the present disclosure, the compounds represented by general formula (V), general formula (V-1), general formula (V-1-1), and general formula (V-1-2) or the pharmaceutically acceptable salts thereof are provided, wherein Z³ is CH.

In some embodiments of the present disclosure, the compounds represented by general formula (V), general formula (V-1), general formula (V-1-1), and general formula (V-1-2) or the pharmaceutically acceptable salts thereof are provided, wherein Z³ is CH, and Z⁶ is N.

In some embodiments of the present disclosure, the compounds represented by general formula (III'), general formula (III'-1), general formula (III), general formula (III-1), general formula (IV'), general formula (IV'-1), general formula (IV'-1-1), general formula (IV-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), and general formula (V-1-2) or the pharmaceutically acceptable salts thereof are provided, wherein x, x1, y, and y1 are each independently 0 or 1; preferably, x, x1, y, and y1 are 1.

In some embodiments of the present disclosure, the compounds represented by general formula (X) and general formula (X-1) or the pharmaceutically acceptable salts thereof are provided, wherein x1 and y1 are each independently 0 or 1; preferably, x1 and y1 are 1.

In some embodiments of the present disclosure, the compounds represented by general formula (I), general formula (II), general formula (G), general formula (III'), general formula (III), and general formula (IV) or the pharmaceutically acceptable salts thereof are compounds represented by general formula (VI) or pharmaceutically acceptable salts thereof: 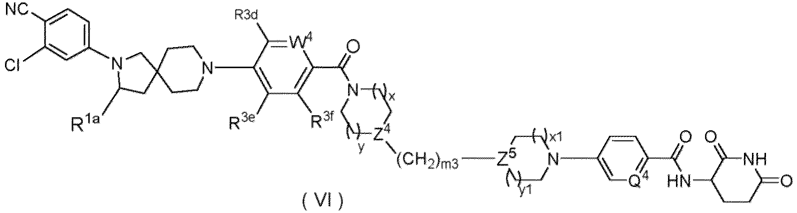 wherein:
R^{1a} is C₁₋₆ alkyl;
Q⁴ is a nitrogen atom or CR⁰¹; R⁰¹ is a hydrogen atom or halogen;
W⁴ is N or CR^{3g};
R^{3d}, R^{3e}, R^{3f}, and R^{3g} are identical or different and are each independently selected from the group consisting of a hydrogen atom, halogen, C₁₋₆ alkyl, and C₁₋₆ haloalkyl;
Z⁴ is N or CH;
Z⁵ is N or CH;
m3 is 0 or 1;
x, x1, y, and y1 are each independently 0 or 1.

In some embodiments of the present disclosure, the compounds represented by general formula (I), general formula (II), general formula (G), general formula (III'), general formula (III), general formula (IV), and general formula (VI) or the pharmaceutically acceptable salts thereof are compounds represented by general formula (VI-1) or pharmaceutically acceptable salts thereof: 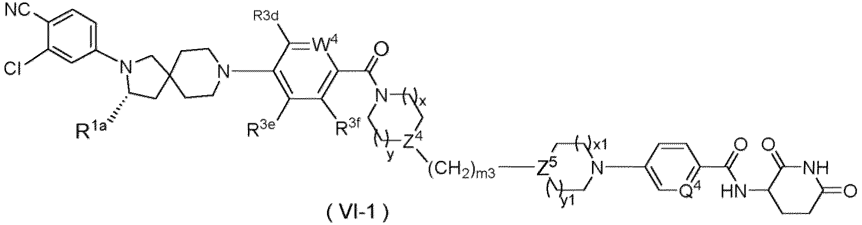 wherein:
R^{1a}, Q⁴, R^{3d}, R^{3e}, R^{3f}, W⁴, Z⁴, Z⁵, m3, x, x1, y, and y1 are as defined in general formula (VI).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (IV), general formula (IV-1), general formula (VI), and general formula (VI-1) or the pharmaceutically acceptable salts thereof are compounds represented by general formula (VI-1-1) and general formula (VI-1-2) or pharmaceutically acceptable salts thereof: 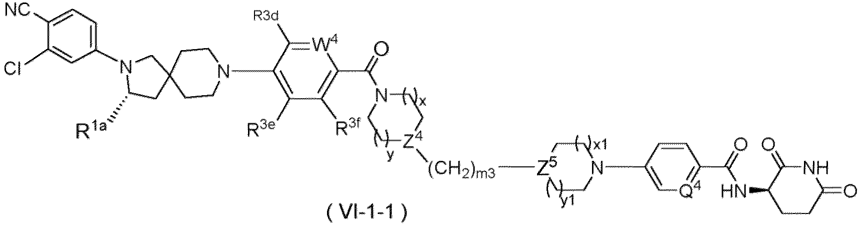 or 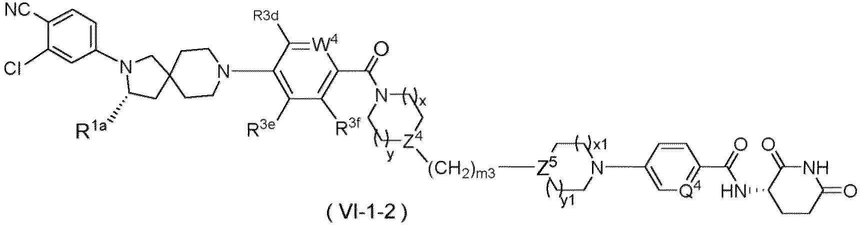 wherein:
R^{1a}, Q⁴, R^{3d}, R^{3e}, R^{3f}, W⁴, Z⁴, Z⁵, m3, x, x1, y, and y1 are as defined in general formula (VI-1).

In some embodiments of the present disclosure, the compounds represented by general formula (I), general formula (II), general formula (G), general formula (III'), general formula (III), and general formula (V) or the pharmaceutically acceptable salts thereof are compounds represented by general formula (VII) or pharmaceutically acceptable salts thereof: 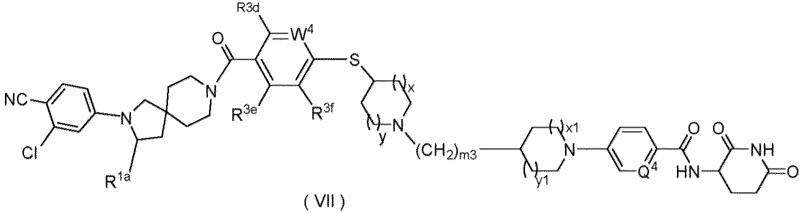 wherein:
R^{1a} is C₁₋₆ alkyl;
Q⁴ is a nitrogen atom or CR⁰¹; R⁰¹ is a hydrogen atom or halogen;
W⁴ is N or CR^{3g};
R^{3d}, R^{3e}, R^{3f}, and R^{3g} are identical or different and are each independently selected from the group consisting of a hydrogen atom, halogen, C₁₋₆ alkyl, and C₁₋₆ haloalkyl;
m3 is 0 or 1;
x, x1, y, and y1 are each independently 0 or 1.

In some embodiments of the present disclosure, the compounds represented by general formula (I), general formula (II), general formula (G), general formula (III'), general formula (III), general formula (V), and general formula (VII) or the pharmaceutically acceptable salts thereof are compounds represented by general formula (VII-1) or pharmaceutically acceptable salts thereof: 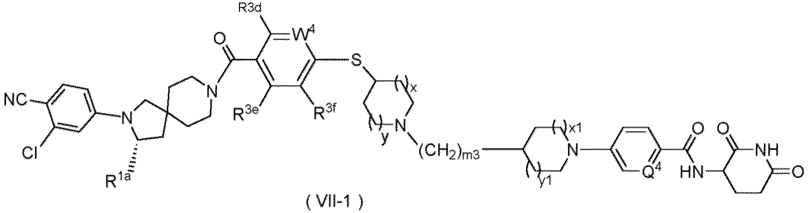 wherein:
R^{1a}, Q⁴, R^{3d}, R^{3e}, R^{3f}, W⁴, m3, x, x1, y, and y1 are as defined in general formula (VII).

In some embodiments of the present disclosure, the compounds represented by general formula (I), general formula (II), general formula (II-1), general formula (G), general formula (G-1), general formula (III'), general formula (III'-1), general formula (III), general formula (III-1), general formula (V), general formula (V-1), general formula (VII), and general formula (VII-1) or the pharmaceutically acceptable salts thereof are compounds represented by general formula (VII-1-1) and general formula (VII-1-2) or pharmaceutically acceptable salts thereof: 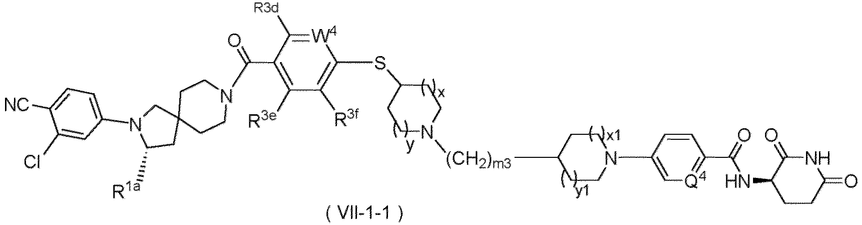 or 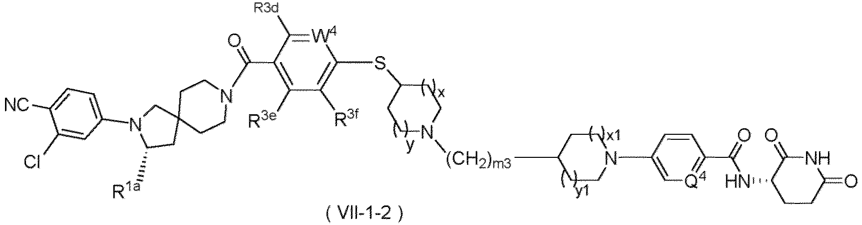 wherein:
R^{1a,} Q⁴, R^{3d}, R^{3e}, R^{3f}, W⁴, m3, x, x1, y, and y1 are as defined in general formula (VII-1).

In some embodiments of the present disclosure, the compounds represented by general formula (I), general formula (II), and general formula (II-1) or the pharmaceutically acceptable salts thereof are provided, wherein X⁴ is -J¹-J²-J³-J⁴-J⁵-, wherein J¹ is attached to X³; J¹ is selected from the group consisting of a bond, O, S, -CH₂-, and -C(O)-; J² is 3-to 12-membered heterocyclyl, and the 3- to 12-membered heterocyclyl is optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O; J³ is -(CH₂)ₘ₃-, and m3 is 0 or 1; J⁴ is 3- to 8-membered heterocyclyl, and the 3- to 8-membered heterocyclyl is optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O; J⁵ is selected from the group consisting of a bond, 3- to 8-membered heterocyclyl, and 3- to 8-membered cycloalkyl.

In some embodiments of the present disclosure, the compounds represented by general formula (I), general formula (II), and general formula (II-1) or the pharmaceutically acceptable salts thereof are provided, wherein X⁴ is -J¹-J²-J³-J⁴-J⁵-, wherein J¹ is attached to X³; J¹ is -S- or -C(O)-; J² is 4- to 6-membered monocyclic heterocyclyl or 9- to 11-membered spiroheterocyclyl; J³ is a bond or -CH₂-; J⁴ is a bond or 4- to 6-membered heterocyclyl, and the 4- to 6-membered heterocyclyl is optionally substituted with one or more =O; J⁵ is selected from the group consisting of a bond, 5- or 6-membered heterocyclyl, and -C(O)NH-*; the * end is attached to A;
preferably, X⁴ is -J¹-J²-P-J⁴-J⁵-, wherein J¹ is attached to X³; J¹ is -S- or -C(O)-; J² is selected from the group consisting of 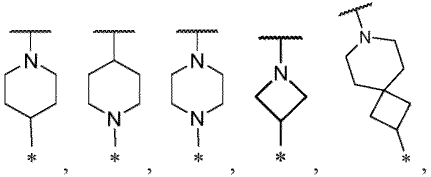 and 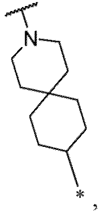 wherein the bond with * is attached to J³; J³ is a bond or -CH₂-; J⁴ is selected from the group consisting of a bond, 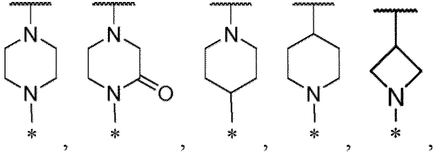 and 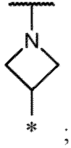 the bond with * is attached to J⁵; J⁵ is selected from the group consisting of a bond, piperazinyl, and -C(O)NH-*; the * end is attached to A; more preferably, X⁴ is -J¹-J²-J³-J⁴-J⁵-, wherein J¹ is attached to X³; J¹ is -C(O)-; J² is 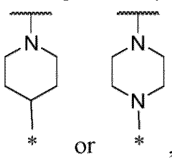 wherein the bond with * is attached to J³; J³ is -CH₂-; J⁴ is 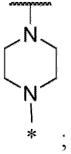 the bond with * is attached to J⁵; J⁵ is a bond.

In some embodiments of the present disclosure, the compounds represented by general formula (I), general formula (II), and general formula (II-1) or the pharmaceutically acceptable salts thereof are provided, wherein X⁴, i.e., -J¹-J²-J³-J⁴-J⁵-, is selected from the group consisting of the following structures: 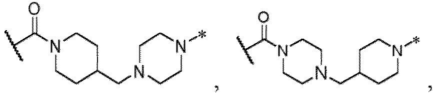 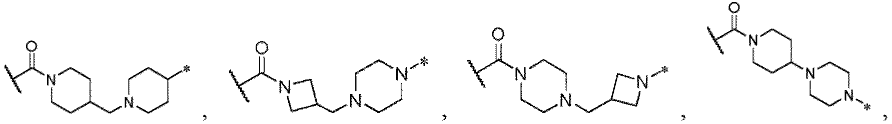 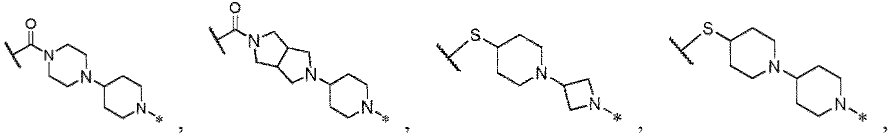 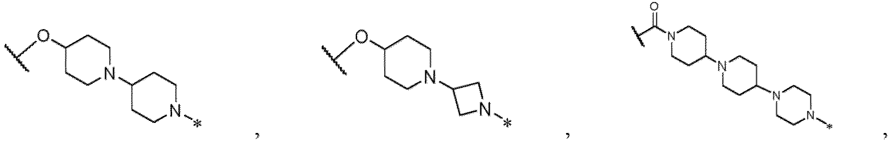 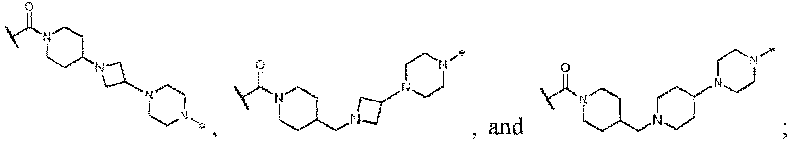 the bond with * is attached to A.

In some embodiments of the present disclosure, the compounds represented by general formula (I), general formula (II), and general formula (II-1) or the pharmaceutically acceptable salts thereof are provided, wherein X⁴, i.e., -J¹-J²-J³-J⁴-J⁵-, is selected from the group consisting of the following structures: 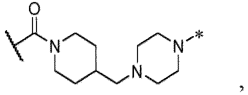 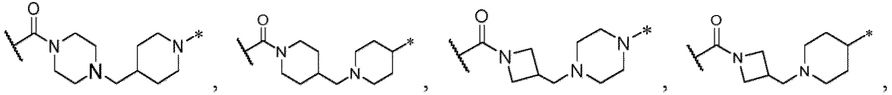 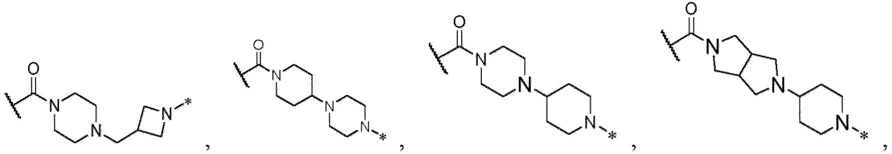 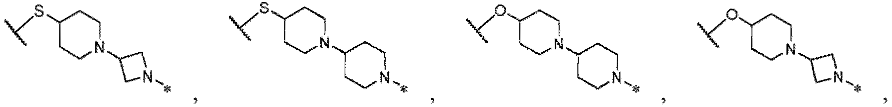 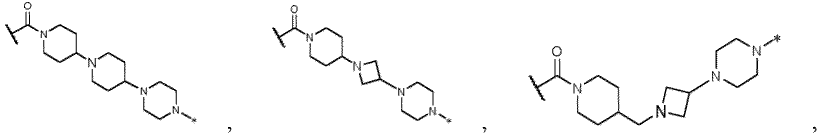 and 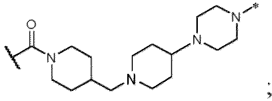 the bond with * is attached to A. In some embodiments of the present disclosure, the compounds represented by general formula (I), general formula (II), and general formula (II-1) or the pharmaceutically acceptable salts thereof are provided, wherein X⁴, i.e., -J¹-J²-J³-J⁴-J⁵-, is selected from the group consisting of the following structures: 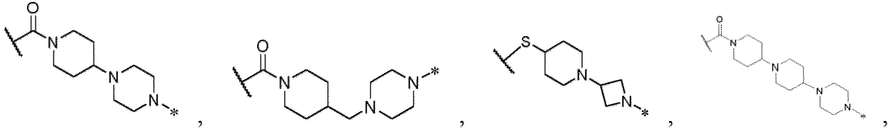 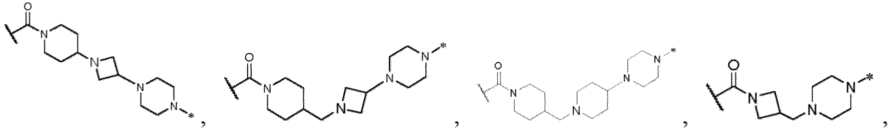 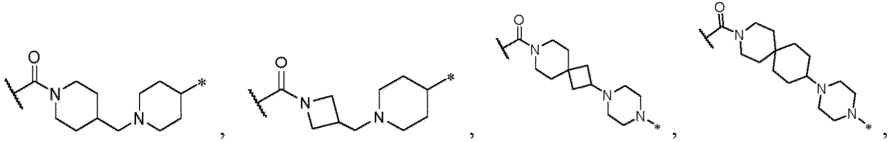 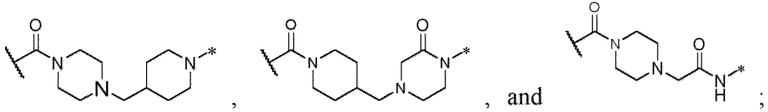 preferably 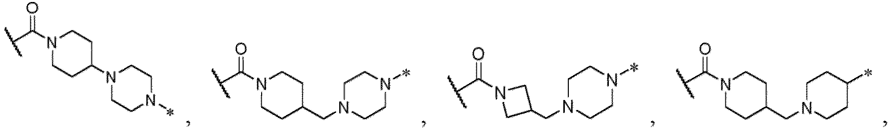 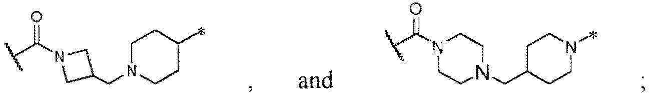 more preferably 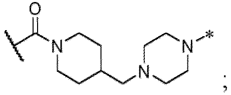 the bond with * is attached to A.

In some embodiments of the present disclosure, the compounds represented by general formula (I), general formula (II), and general formula (II-1) or the pharmaceutically acceptable salts thereof are provided, wherein X⁴, i.e., -J¹-J²-J³-J⁴-J⁵-, is selected from the group consisting of the following structures: 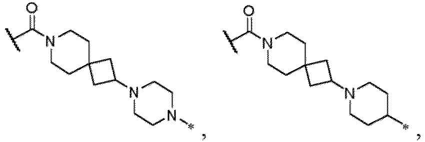 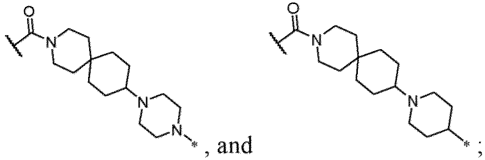 the bond with * is attached to A.

In some embodiments of the present disclosure, the compound represented by general formula (G) or the pharmaceutically acceptable salt thereof is provided, wherein Z¹ is CR^{3b}; Z² is CR^{3c}; R^{3a} is halogen or C₁₋₆ haloalkyl; R^{3b} is a hydrogen atom; R^{3c} is a hydrogen atom or C₁₋₆ alkyl; R^{1a} is a hydrogen atom or C₁₋₆ alkyl; X² is a bond or -C(O)-; W¹ is N or CR^{3d}; W² is N or CR^{3e}; W³ is N or CR^{3f}. _{>} W⁴ is N or CR^{3g}; R^{3d}, R^{3e}, R^{3f}, and R^{3g} are identical or different and are each independently a hydrogen atom or halogen; J¹ is -S- or -C(O)-; ring B is 4- to 6-membered monocyclic heterocyclyl or 9- to 11-membered spiroheterocyclyl; m3 is 0 or 1; ring C is 4- to 6-membered heterocyclyl, and the 4- to 6-membered heterocyclyl is optionally substituted with one or more =O; J⁵ is selected from the group consisting of a bond, 5- or 6-membered heterocyclyl, and - C(O)NH-*; the * end is attached to A; A is selected from the group consisting of the following structures: 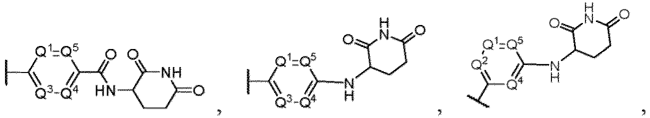 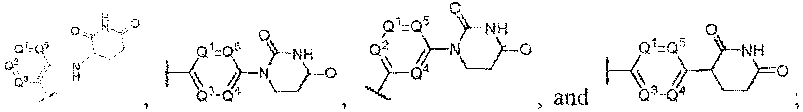 Q¹, Q², Q³, Q⁴, and Q⁵ are identical or different and are each independently a nitrogen atom or CR'; each R' is identical or different and is independently a hydrogen atom or halogen.

In some embodiments of the present disclosure, the compound represented by general formula (G-1) or the pharmaceutically acceptable salt thereof is provided, wherein Z¹ is CR^{3b}; Z² is CR^{3c}; R^{3a} is halogen or C₁₋₆ haloalkyl; R^{3b} is a hydrogen atom; R^{3c} is a hydrogen atom or C₁₋₆ alkyl; R^{1a} is C₁₋₆ alkyl; X² is a bond or -C(O)-; W¹ is N or CR^{3d}; W² is N or CR^{3e}; W³ is N or CR^{3f}. _{>} W⁴ is N or CR^{3g.} , R^{3d}, R^{3e}, R^{3f}, and R^{3g} are identical or different and are each independently a hydrogen atom or halogen; J¹ is -S- or -C(O)-; ring B is 4- to 6-membered monocyclic heterocyclyl or 9- to 11-membered spiroheterocyclyl; m3 is 0 or 1; ring C is 4- to 6-membered heterocyclyl, and the 4- to 6-membered heterocyclyl is optionally substituted with one or more =O; J⁵ is selected from the group consisting of a bond, 5- or 6-membered heterocyclyl, and -C(O)NH-*; the * end is attached to A; A is selected from the group consisting of the following structures: 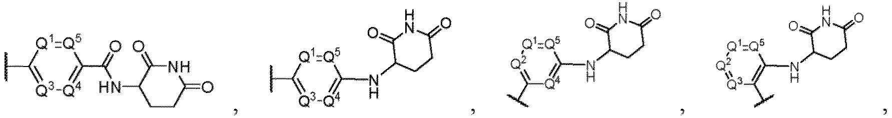 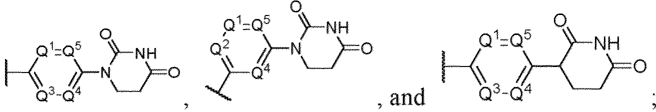 Q¹, Q², Q³, Q⁴, and Q⁵ are identical or different and are each independently a nitrogen atom or CR'; each R' is identical or different and is independently a hydrogen atom or halogen.

In some embodiments of the present disclosure, the compounds represented by general formula (M), general formula (M-1), general formula (M-1-1), and general formula (M-1-2) or the pharmaceutically acceptable salts thereof are provided, wherein 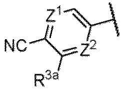 is selected from the group consisting of 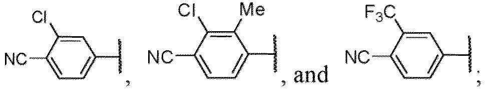 R^{1a} is C₁₋₆ alkyl; W¹ is N or CR^{3d}; W² is N or CR^{3e}, W³ is N or CR^{3f}; W⁴ is N or CR^{3g}; R^{3d}, R^{3e}, R^{3f}, and R^{3g} are identical or different and are each independently a hydrogen atom or halogen; m3 is 0 or 1; x, x1, y, and y1 are each independently 0 or 1; Z⁴, Z⁵, and Z⁶ are each independently CH or N; Q¹, Q², Q⁴, and Q⁵ are each independently a nitrogen atom or CR'; and R' is a hydrogen atom or halogen.

In some embodiments of the present disclosure, the compounds represented by general formula (IV'), general formula (IV'-1), general formula (IV'-1-1), and general formula (IV'-1-2) or the pharmaceutically acceptable salts thereof are provided, wherein: Z¹ is CR^{3b}; Z² is CR^{3C}; R^{3b} and R^{3c} are both hydrogen atoms; R^{3a} is halogen; R^{1a} is C₁₋₆ alkyl; W¹ is N or CR^{3d}; W² is N or CR^{3e}; W³ is N or CR^{3f}. _{>} W⁴ is N or CR^{3g}; R^{3d}, R^{3e}, R^{3f}, and R^{3g} are identical or different and are each independently selected from the group consisting of a hydrogen atom, halogen, C₁₋₆ alkyl, and C₁₋₆ haloalkyl; Z⁴ is CH; Z⁵ is N; Z⁶ is N or CH; x, x1, y, and y1 are each independently 0 or 1; m3 is 0 or 1; Q² is a carbon atom, and Q¹, Q³, Q⁴, and Q⁵ are identical or different and are each independently a nitrogen atom or CR'; or, Q³ is a carbon atom, and Q¹, Q², Q⁴, and Q⁵ are identical or different and are each independently a nitrogen atom or CR'; and R' is selected from the group consisting of a hydrogen atom, halogen, and C₁₋₆ alkyl.

In some embodiments of the present disclosure, the compounds represented by general formula (IV'), general formula (IV'-1), general formula (IV'-1-1), and general formula (IV'-1-2) or the pharmaceutically acceptable salts thereof are provided, wherein: 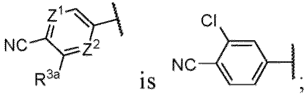 R^{1a} is methyl; W¹ is CR^{3d}, W² is CR^{3e}, W³ is CR³¹ I and W⁴ is CR^{3g}; or, W¹ is CR^{3a}, W² is CR^{3e}, W³ is CR^{3f}, and W⁴ is N; or, W¹ is N, W² is CR^{3e}, W³ is CR^{3f}, and W⁴ is N; or, W¹ is CR^{3d}, W² is N, W³ is CR^{3f}, and W⁴ is N; or, W¹ is CR^{3d}, W² is N, W³ is CR^{3f}, and W⁴ is CR^{3g}; R^{3d}, R^{3e}, R^{3f}, and R^{3g} are identical or different and are each independently a hydrogen atom or halogen; Z⁴ is CH; Z⁵ is N; Z⁶ is N or CH; x, x1, y, and y1 are each independently is 0 or 1; m3 is 0 or 1; Q³ is a carbon atom, and Q¹, Q², Q⁴, and Q⁵ are identical or different and are each independently a nitrogen atom or CR'; and R' is a hydrogen atom or halogen.

In some embodiments of the present disclosure, the compounds represented by general formula (IV'), general formula (IV'-1), general formula (IV'-1-1), and general formula (IV'-1-2) or the pharmaceutically acceptable salts thereof are provided, wherein: 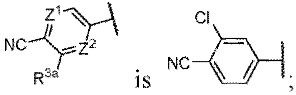 R^{1a} is methyl; W¹ is CF or CH, W² is CH, W³ is CH, and W⁴ is CH; Z⁴ is CH; Z⁵ is N; Z⁶ is N or CH; x, x1, y, and y1 are each independently 0 or 1; m3 is 0 or 1; Q³ is a carbon atom, Q¹, Q², Q⁴, and Q⁵ are each independently CR', and R' is a hydrogen atom or halogen.

In some embodiments of the present disclosure, the compounds represented by general formula (X) and general formula (X-1) or the pharmaceutically acceptable salts thereof are provided, wherein: 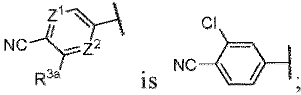 R^{1a} is methyl; W¹ is N or CR^{3d}; W² is N or CR^{3e}; W³ is N or CR^{3f}. _{>} W⁴ is N or CR^{3g}; R^{3d}, R^{3e}, R^{3f}, and R^{3g} are identical or different and are each independently selected from the group consisting of a hydrogen atom or halogen; Z⁴ is CH; Z⁵ is N; Z⁶ is N or CH; h, i, u, v, x1, and y1 are each independently 0 or 1; m3 is 0 or 1; A is 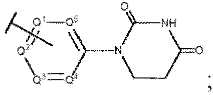 Q² is a carbon atom, Q¹, Q³, Q⁴, and Q⁵ are identical or different and are each independently a nitrogen atom or CR', and R' is a hydrogen atom or halogen.

In some embodiments of the present disclosure, the compounds represented by general formula (IV), general formula (IV-1), general formula (IV-1-1), and general formula (IV-1-2) or the pharmaceutically acceptable salts thereof are provided, wherein: 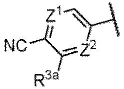 is 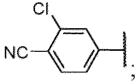 R^{1a} is methyl; W¹ is CR^{3d}, W² is CR^{3e}, W³ is CR^{3f}, and W⁴ is CR^{3g}; or, W¹ is CR^{3d}, W² is CR^{3e}, W³ is CR^{3f}, and W⁴ is N; or, W¹ is N, W² is CR^{3e}, W³ is CR^{3f}, and W⁴ is N; or, W¹ is CR^{3a}, W² is N, W³ is CR^{3f}, and W⁴ is N; or, W¹ is CR^{3d}, W² is N, W³ is CR^{3f}, and W⁴ is CR^{3g}; R^{3d}, R^{3e}, R^{3f}, and R^{3g} are identical or different and are each independently a hydrogen atom or halogen; Z⁴ is CH; Z⁵ is N; Z⁶ is N or CH; x, x1, y, and y1 are each independently is 0 or 1; m3 is 0 or 1; Q¹, Q³, Q⁴, and Q⁵ are each independently CR'; and R' is a hydrogen atom or halogen.

In some embodiments of the present disclosure, the compounds represented by general formula (V), general formula (V-1), general formula (V-1-1), and general formula (V-1-2) or the pharmaceutically acceptable salts thereof are provided, wherein: 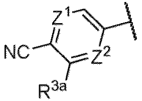 is 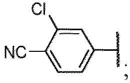 R^{1a} is methyl; W¹ is CR^{3d}, W² is CR^{3e}, W³ is CR^{3f}, and W⁴ is CR^{3g}; or, W¹ is CR^{3d}, W² is CR^{3e}, W³ is CR^{3f}, and W⁴ is N; or, W¹ is N, W² is CR^{3e}, W³ is CR^{3f}, and W⁴ is N; or, W¹ is CR^{3a}, W² is N, W³ is CR^{3f}, and W⁴ is N; or, W¹ is CR^{3d}, W² is N, W³ is CR^{3f} _{>} and W⁴ is CR^{3g}; R^{3d}, R^{3e}, R^{3f}, and R^{3g} are identical or different and are each independently a hydrogen atom or halogen; J¹ is -S-; Z³ is CH; Z⁶ is N; x, x1, y, and y1 are each independently is 0 or 1; m3 is 0 or 1; Q¹, Q³, Q⁴, and Q⁵ are each independently CR'; and R' is a hydrogen atom or halogen.

**Table A. Typical compounds of the present disclosure include, but are not limited to:**

| No. | Compound structure | Name |
|---|---|---|
| | 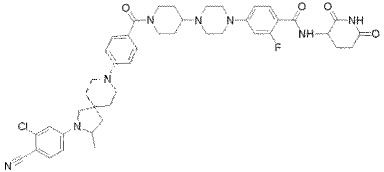 | 4-(4-(1-(4-(2-(3-Chloro-4-cyanophenyl)-3-methyl-2,8-diazaspiro[4.5]decan-8-yl)benzoyl)piperidin-4-yl)piperazin-1-yl)-N-(2,6-dioxopiperidin-3-yl)-2-fluorobenzamide |
| **1** | 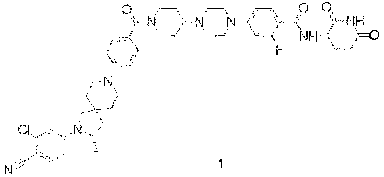 | 4-(4-(1-(4-((S)-2-(3-Chloro-4-cyanophenyl)-3-methyl-2,8-diazaspiro [4.5] decan-8-yl)benzoyl)piperidin-4-yl)piperazin-1-yl)-N-(2,6-dioxopiperidin-3 -yl)-2-fluorobenzamide 1 (a mixture of diastereomers) |
| | 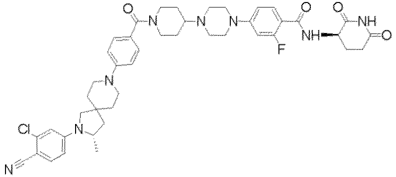 | 4-(4-(1-(4-((S)-2-(3-Chloro-4-cyanophenyl)-3-methyl-2,8-diazaspiro [4.5] decan-8-yl)benzoyl)piperidin-4-yl)piperazin-1-yl)-N-((*R*)-2,6-dioxopiperidin-3-yl)-2-fluorobenzamide |
| | 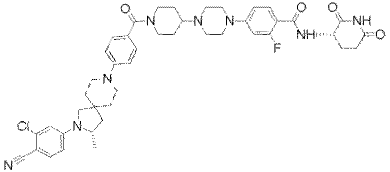 | 4-(4-(1-(4-((*S*)-2-(3-Chloro-4-cyanophenyl)-3-methyl-2,8-diazaspiro [4.5] decan-8-yl)benzoyl)piperidin-4-yl)piperazin-1-yl)-N-((*S*)-2,6-dioxopiperidin-3-yl)-2-fluorobenzamide |
| | 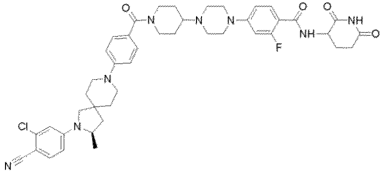 | 4-(4-(1-(4-((*R*)-2-(3-Chloro-4-cyanophenyl)-3-methyl-2,8-diazaspiro[4.5]decan-8-yl)benzoyl)piperidin-4-yl)piperazin-1-yl)-*N-*(2,6-dioxopiperidin-3-yl)-2-fluorobenzamide |
| | 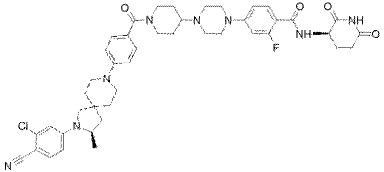 | 4-(4-(1-(4-((*R*)-2-(3-Chloro-4-cyanophenyl)-3-methyl-2,8-diazaspiro [4.5] decan-8-yl)benzoyl)piperidin-4-yl)piperazin-1-yl)-N-((*R*)-2,6-dioxopiperidin-3-yl)-2-fluorobenzamide |
| | 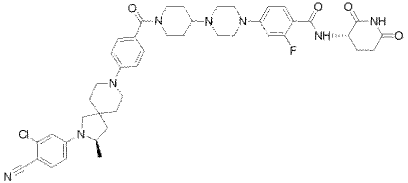 | 4-(4-(1-(4-((*R*)-2-(3-Chloro-4-cyanophenyl)-3-methyl-2,8-diazaspiro [4.5] decan-8-yl)benzoyl)piperidin-4-yl)piperazin-1-yl)-N-((*S*)-2,6-dioxopiperidin-3-yl)-2-fluorobenzamide |
| | 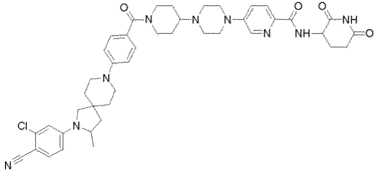 | 5-(4-(1-(4-(2-(3-Chloro-4-cyanophenyl)-3-methyl-2,8-diazaspiro[4.5]decan-8-yl)benzoyl)piperidin-4-yl)piperazin-1-yl)-N-(2,6-dioxopiperidin-3-yl)picolinamide |
| **2** | 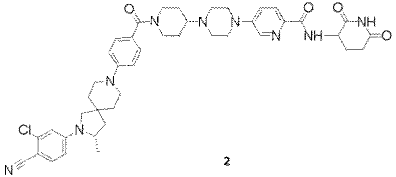 | 5-(4-(1-(4-((*S*)-2-(3-Chloro-4-cyanophenyl)-3 - methyl-2,8-diazaspiro[4.5]decan-8-yl)benzoyl)piperidin-4-yl)piperazin-1-yl)-*N-*(2,6-dioxopiperidin-3-yl)picolinamide 2 (a mixture of diastereomers) |
| | 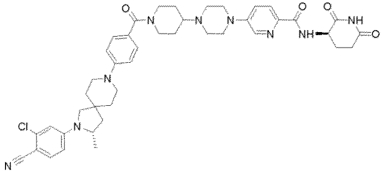 | 5-(4-(1-(4-((*S*)-2-(3-Chloro-4-cyanophenyl)-3-methyl-2,8-diazaspiro[4.5]decan-8-yl)benzoyl)piperidin-4-yl)piperazin-1-yl)-*N-*((*R*)-2,6-dioxopiperidin-3-yl)picolinamide |
| | 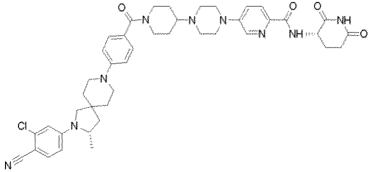 | 5-(4-(1-(4-((*S*)-2-(3-Chloro-4-cyanophenyl)-3-methyl-2,8-diazaspiro[4.5]decan-8-yl)benzoyl)piperidin-4-yl)piperazin-1-yl)-N-((*S*)-2,6-dioxopiperidin-3-yl)picolinamide |
| | 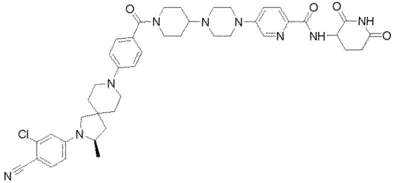 | 5-(4-(1-(4-((*R*)-2-(3-Chloro-4-cyanophenyl)-3-methyl-2,8-diazaspiro[4.5]decan-8-yl)benzoyl)piperidin-4-yl)piperazin-1-yl)-*N-*(2,6-dioxopiperidin-3-yl)picolinamide |
| | 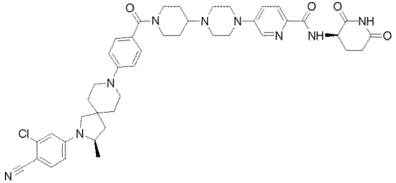 | 5-(4-(1-(4-((*R*)-2-(3-Chloro-4-cyanophenyl)-3-methyl-2,8-diazaspiro[4.5]decan-8-yl)benzoyl)piperidin-4-yl)piperazin-1-yl)-*N-*((*R*)-2,6-dioxopiperidin-3-yl)picolinamide |
| | 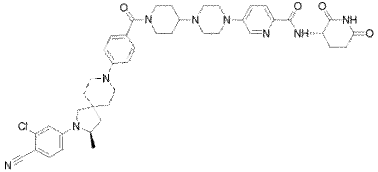 | 5-(4-(1-(4-((*R*)-2-(3-Chloro-4-cyanophenyl)-3-methyl-2,8-diazaspiro[4.5]decan-8-yl)benzoyl)piperidin-4-yl)piperazin-1-yl)-*N-*((*S*)-2,6-dioxopiperidin-3-yl)picolinamide |
| | 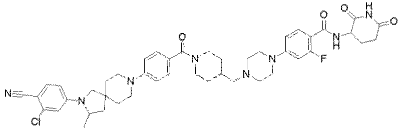 | 4-(4-((1-(4-(2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopiperidin-3-yl)-2-fluorobenzamide |
| **3** | 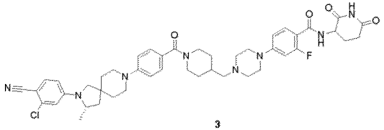 | 4-(4-((1-(4-((*S*)-2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopipridin-3-yl)-2-fluorobenzamide **3** (a mixture of diastereomers) |
| | 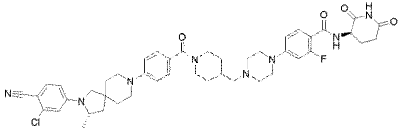 | 4-(4-((1-(4-((*S*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*R*)-2,6-dioxopiperidin-3-yl)-2-fluorobenzamide |
| | 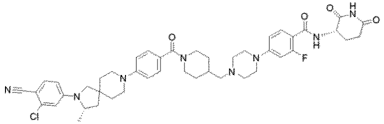 | 4-(4-((1-(4-((*S*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*S*)-2,6-dioxopiperidin-3-yl)-2-fluorobenzamide |
| | 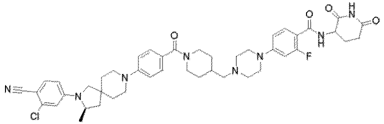 | 4-(4-((1-(4-((*R*)-2-(3-Chloro-4-cyanopheiryl)-3-methyl-2,8-diazaspiro[4.5]decan-8-yl)benzoyl)piperidin-4-yl)methyl)piperazin-1-yl)-*N*-(2,6-dioxopiperidin-3-yl)-2-fluorobenzamide |
| | 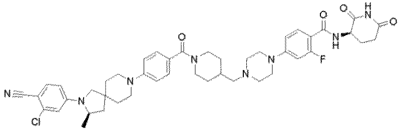 | 4-(4-((1-(4-((*R*)-2-(3-Chloro-4-cyanopheiryl)-3-methyl-2,8-diazaspiro[4.5]decan-8-yl)benzoyl)piperidin-4-yl)methyl)piperazin-1-yl)-*N*-((*R*)-2,6-dioxopiperidin-3-yl)-2-fluorobenzamide |
| | 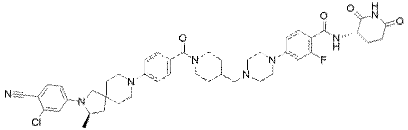 | 4-(4-((1-(4-((*R*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*S*)-2,6-dioxopiperidin-3-yl)-2-fluorobenzamide |
| | 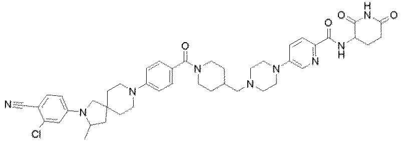 | 5-(4-((1-(4-(2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopiperidin-3-yl)picolinamide |
| **4** | 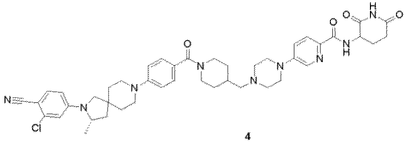 | 5-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopiperidin-3 yl)picolinamide 4 (a mixture of diastereomers) |
| | 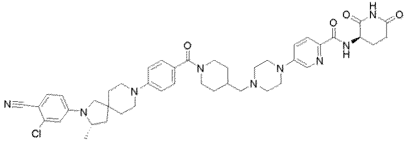 | 5-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl)-*N*-((*R*)-2,6-dioxopipcridin-3-yl)picolinamide |
| | 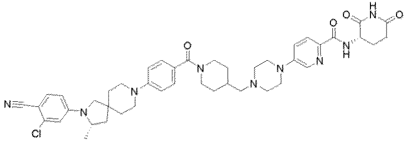 | 5-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl)-*N*-((*S*)-2,6-dioxopiperidin-3-yl)picolinamide |
| | 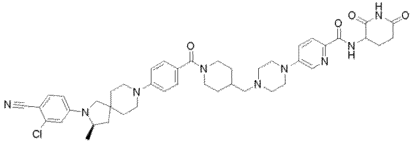 | 5-(4-((1-(4-((*R*)-2-(3-Chloro-4-cyanopheiryl)-3-methyl-2,8-diazaspiro[4.5]decan-8-yl)benzoyl)piperidin-4-yl)methyl)piperazin-1-yl)-*N*-(2,6-dioxopiperidin-3-yl)picolinamide |
| | 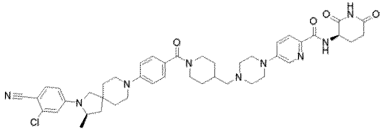 | 5-(4-((1-(4-((*R*)-2-(3-Chloro-4-cyanopheiryl)-3-methyl-2,8-diazaspiro[4.5]decan-8-yl)benzoyl)piperidin-4-yl)methyl)piperazin-1-yl)-*N*-((R)-2,6-dioxopipcridin-3-yl)picolinamide |
| | 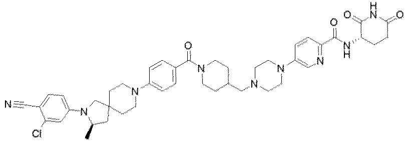 | 5-(4-((1-(4-((*R*)-2-(3-Chloro-4-cyanopheiryl)-3-methyl-2,8-diazaspiro[4.5]decan-8-yl)benzoyl)piperidin-4-yl)methyl)piperazin-1-yl)-*N*-((*S*)-2,6-dioxopiperidin-3-yl)picolinamide |
| | 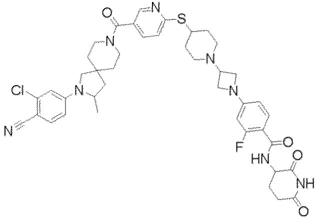 | 4-(3-(4-((5-(2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-(2,6-dioxopiperidin-3 -yl)-2-fluorobenzamide |
| **5** | 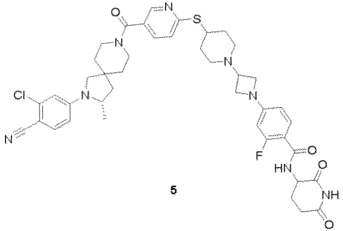 | 4-(3-(4-((5-((*S*)-2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-(2,6-dioxopiperidin-3 -yl)-2-fluorobenzamide 5 (a mixture of diastereomers) |
| | 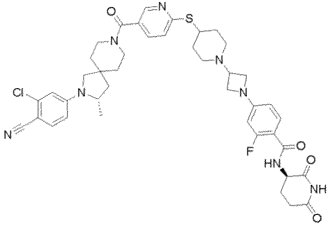 | 4-(3-(4-((5-((*S*)-2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-((*R*)-2,6-dioxopiperidin- 3-yl)-2-fluorobenzamide |
| | 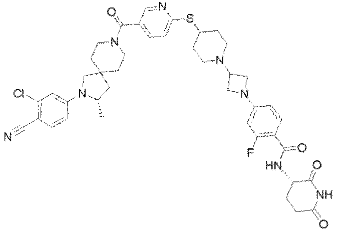 | yl)azetidin-1 4-(3-(4-((5-((*S*)-2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-((S)-2,6-dioxopiperidin- 3-yl)-2-fluorobenzamide |
| | 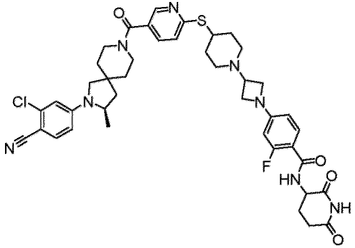 | 4-(3-(4-((5-((*R*)-2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-(2,6-dioxopiperidin-3 -yl)-2-fluorobenzamide |
| | 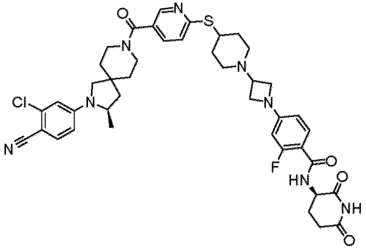 | 4-(3-(4-((5-((*R*)-2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-((*R*)-2,6-dioxopiperidin- 3-yl)-2-fluorobenzamide |
| | 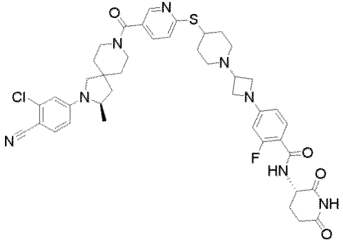 | 4-(3-(4-((5-((*R*)-2-(3-Chloro-4-cyanophenyl)-3-methyl-2,8-diazaspiro[4.5]decane-8-carbonyl)pyridin-2-yl)thio)piperidin-1-yl)azetidin-1-yl)-*N*-((*S*)-2,6-dioxopiperidin-3-yl)-2-fluorobenzamide |
| | 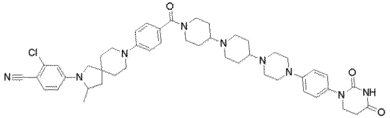 | 2-Chloro-4-(8-(4-(4-(4-(4-(2,4-dioxotetrahydropyrimidin-1*(2H)-*yl)phenyl)piperazin-1-yl)-[1,4'-bipiperidine]- 1-carbonyl)phenyl)-3-methyl-2,8-diazaspiro[4.5]decan-2-yl)benzonitrile |
| 6 | 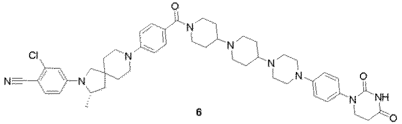 | (*S*)-2-Chloro-4-(8-(4-(4-(4-(4-(2,4-dioxotetrahydropyrimidin-1*(2H)-*yl)phenyl)piperazin-1-yl)-[1,4'-bipiperidine]-1'-carboiryl)phenyl)-3-methyl-2,8-diazaspiro[4.5]decan-2-yl)benzonitrile 6 |
| | 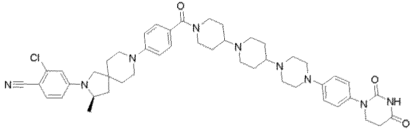 | (*R*)-2-Chloro-4-(8-(4-(4-(4-(4-(2,4-dioxotetrahydropyrimidin-1*(2H)-*yl)phenyl)piperazin-1-yl)-[1,4'-bipiperidine]-1-carbonyl)phenyl)-3-methyl-2,8-diazaspiro [4.5]decan-2-yl)benzonitrile |
| | 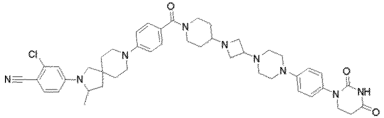 | 2-Chloro-4-(8-(4-(4-(3 -(4-(4-(2,4-dioxotetrahydropyrimidin-1*(2H)-*yl)phenyl)piperazin-1-yl)azetidin-1-yl)piperidine-1-carbonyl)phenyl)-3-methyl-2,8-diazaspiro[4.5]decan-2-yl)benzonitrile |
| 7 | 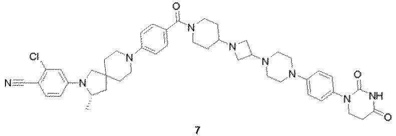 | (*S*)-2-Chloro-4-(8-(4-(4-(3 -(4-(4-(2,4-dioxotetrahydropyrimidin-1(2*H)-*yl)phenyl)piperazin-1-yl)azetidin-1-yl)piperidine-1-carbonyl)phenyl)-3-methyl-2,8-diazaspiro[4.5]decan-2-yl)benzonitrile 7 |
| | 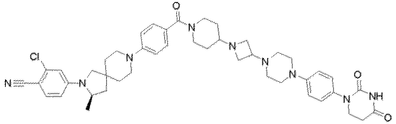 | (*R*)-2-Chloro-4-(8-(4-(4-(3-(4-(4-(2,4-dioxotetrahydropyrimidin-1*(2H)-*yl)phenyl)piperazin-1-yl)azetidin-1-yl)piperidine-1-carbonyl)phenyl)-3-methyl-2,8-diazaspiro[4.5]decan-2-yl)benzonitrile |
| | 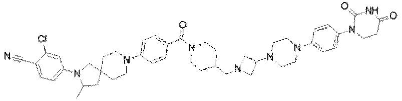 | 2-Chloro-4-(8-(4-(4-((3 -(4-(4-(2,4-dioxotetrahydropyrimidin-1(2H*)-*yl)phenyl)piperazin-1-yl)azetidin-1-yl)methyl)piperidine-1-carbonyl)phenyl)-3-methyl-2,8-diazaspiro [4.5] decan-2-yl)benzonitrile |
| 8 | 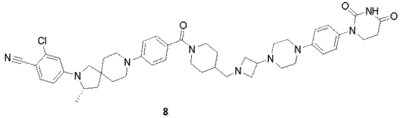 | (*S*)-2-Chloro-4-(8-(4-(4-((3 -(4-(4-(2,4-dioxotetrahydropyrimidin-1(2*H)-*yl)phenyl)piperazin-1-yl)azetidin-1-yl)methyl)piperidine-1-carbonyl)phenyl)-3-methyl-2,8-diazaspiro [4.5] decan-2-yl)benzonitrile **8** |
| | 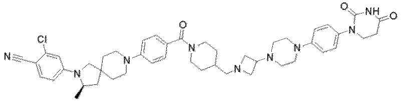 | (*R*)-2-Chloro-4-(8-(4-(4-((3-(4-(4-(2,4-dioxotetrahydropyrimidin-1*(2H)-*yl)phenyl)piperazin-1-yl)azetidin-1-yl)methyl)piperidine-1-carbonyl)phenyl)-3-methyl-2,8-diazaspiro[4.5]decan-2-yl)benzonitrile |
| | 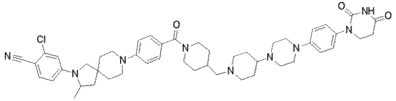 | 2-Chloro-4-(8-(4-(4-((4-(4-(4-(2,4-dioxotetrahydropyrimidin-1*(2H)-*yl)phenyl)piperazin-1-yl)piperidin-1-yl)methyl)piperidine-1-carbonyl)phenyl)-3-methyl-2,8-diazaspiro [4.5] decan-2-yl)benzonitrile |
| 9 | 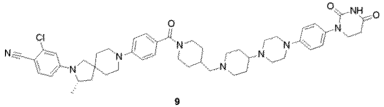 | (*S*)-2-Chloro-4-(8-(4-(4-((4-(4-(4-(2,4-dioxotetrahydropyrimidin-1*(2H)-*yl)phenyl)piperazin-1-yl)piperidin-1-yl)methyl)piperidine-1-carbonyl)phenyl)-3-methyl-2,8-diazaspiro [4.5] decan-2-yl)benzonitrile 9 |
| | 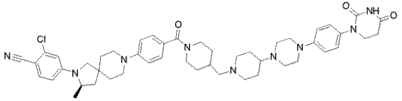 | (*R*)-2-Chloro-4-(8-(4-(4-((4-(4-(4-(2,4-dioxotetrahydropyrimidin-1*(2H)-*yl)phenyl)piperazin-1-yl)piperidin-1-yl)methyl)piperidine-1-carbonyl)phenyl)-3-methyl-2,8-diazaspiro[4.5]decan-2-yl)benzonitrile |
| | 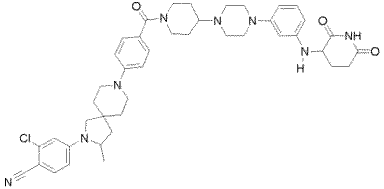 | 2-Chloro-4-(8-(4-(4-(4-(3-((2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile |
| 10 | 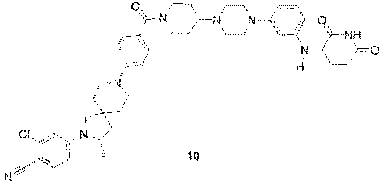 | 2-Chloro-4-((3*S*)-8-(4-(4-(4-(3-((2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile 10 (a mixture of diastereomers) |
| | 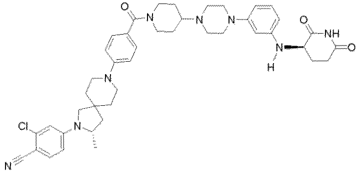 | 2-Chloro-4-((*S*)-8-(4-(4-(4-(3-(((R)-2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile |
| | 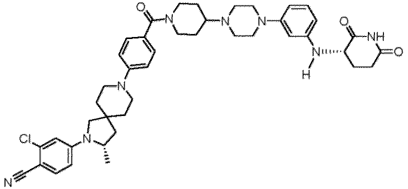 | 2-Chloro-4-((*S*)-8-(4-(4-(4-(3 -(((*S*)-2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile |
| | 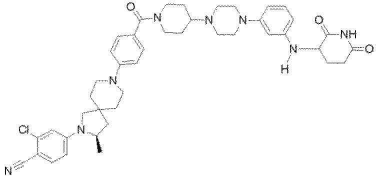 | 2-Chloro-4-((3*R*)-8-(4-(4-(4-(3-((2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile |
| | 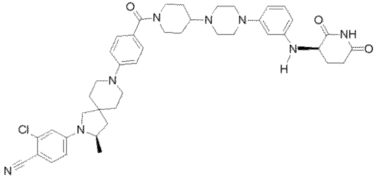 | 2-Chloro-4-((*R*)-8-(4-(4-(4-(3-(((R)-2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile |
| | 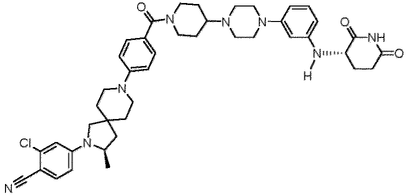 | 2-Chloro-4-((*R*)-8-(4-(4-(4-(3-(((S)-2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile |
| | 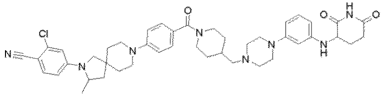 | 2-Chloro-4-(8-(4-(4-((4-(3-((2,6-dioxopiperidin-3-yl)amino)phenyl)piperazin-1-yl)methyl)piperidine-1-carboiryl)phenyl)-3-methyl-2,8-diazaspiro [4.5] decan-2-yl)benzonitrile |
| 11 | 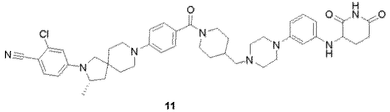 | 2-Chloro-4-((3*S*)-8-(4-(4-((4-(3-((2,6-dioxopiperidin-3-yl)amino)phenyl)piperazin-1-yl)methyl)piperidine-1-carboiryl)phenyl)-3-methyl-2,8-diazaspiro [4.5] decan-2-yl)benzonitrile **11** (a mixture of diastereomers) |
| 11-2 | 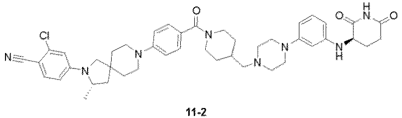 | 2-Chloro-4-((*S*)-8-(4-(4-((4-(3-(((R)-2,6-dioxopiperidin-3-yl)amino)phenyl)piperazin-1-yl)methyl)piperidine-1-carboiryl)phenyl)-3-methyl-2,8-diazaspiro [4.5] decan-2-yl)benzonitrile **11-2** |
| 11-1 | 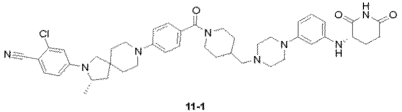 | 2-Chloro-4-((*S*)-8-(4-(4-((4-(3-(((S)-2,6-dioxopiperidin-3-yl)amino)phenyl)piperazin-1-yl)methyl)piperidine-1-carboiryl)phenyl)-3-methyl-2,8-diazaspiro [4.5] decan-2-yl)benzonitrile **11-1** |
| | 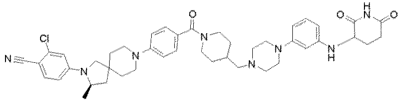 | 2-Chloro-4-((3*R*)-8-(4-(4-((4-(3-((2,6-dioxopiperidin-3-yl)amino)phenyl)piperazin-1-yl)methyl)piperidine-1-carboiryl)phenyl)-3-methyl-2,8-diazaspiro [4.5] decan-2-yl)benzonitrile |
| | 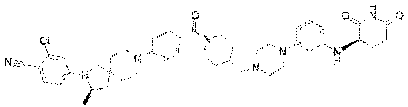 | 2-Chloro-4-((*R*)-8-(4-(4-((4-(3-(((R)-2,6-dioxopiperidin-3-yl)amino)phenyl)piperazin-1-yl)methyl)piperidine-1-carboiryl)phenyl)-3-methyl-2,8-diazaspiro[4.5]decan-2-yl)benzonitrile |
| | 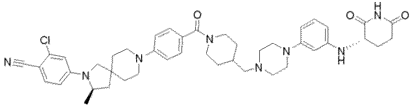 | 2-Chloro-4-((*R*)-8-(4-(4-((4-(3-(((*S*)-2,6-dioxopiperidin-3-yl)amino)phenyl)piperazin-1-yl)methyl)piperidine-1-carboiryl)phenyl)-3-methyl-2,8-diazaspiro [4.5] decan-2-yl)benzonitrile |
| | 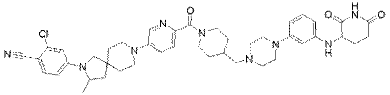 | 2-Chloro-4-(8-(6-(4-((4-(3-((2,6-dioxopiperidin-3-yl)amino)phenyl)piperazin-1-yl)methyl)piperidine-1-carboiryl)pyridin-3-yl)-3-methyl-2,8-diazaspiro[4.5]decan-2-yl)benzonitrile |
| 12 | 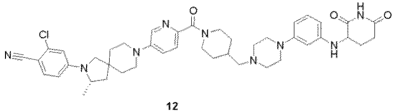 | 2-Chloro-4-((3*S*)-8-(6-(4-((4-(3-((2,6-dioxopiperidin-3-yl)amino)phenyl)piperazin-1-yl)methyl)piperidine-1-carboiryl)pyridin-3-yl)-3-methyl-2,8-diazaspiro[4.5]decan-2-yl)benzonitrile **12** (a mixture of diastereomers) |
| 12-2 | 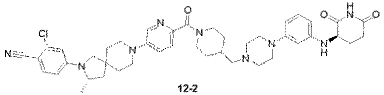 | 2-Chloro-4-((*S*)-8-(6-(4-((4-(3-(((*R*)-2,6-dioxopiperidin-3-yl)amino)phenyl)piperazin-1-yl)methyl)piperidine-1-carboiryl)pyridin-3-yl)-3-methyl-2,8-diazaspiro[4.5]decan-2-yl)benzonitrile **12-2** |
| 12-1 | 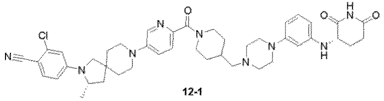 | 2-Chloro-4-((*S*)-8-(6-(4-((4-(3-(((*S*)-2,6-dioxopiperidin-3-yl)amino)phenyl)piperazin-1-yl)methyl)piperidine-1-carboiryl)pyridin-3-yl)-3-methyl-2,8-diazaspiro[4.5]decan-2-yl)benzonitrile **12-1** |
| | 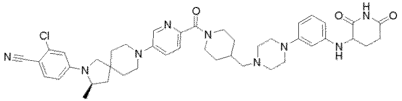 | 2-Chloro-4-((3*R*)-8-(6-(4-((4-(3-((2,6-dioxopiperidin-3-yl)amino)phenyl)piperazin-1-yl)methyl)piperidine-1-carboiryl)pyridin-3-yl)-3-methyl-2,8-diazaspiro[4.5]decan-2-yl)benzonitrile |
| | 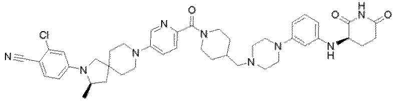 | 2-Chloro-4-((*R*)-8-(6-(4-((4-(3-(((*R*)-2,6-dioxopiperidin-3-yl)amino)phenyl)piperazin-1-yl)methyl)piperidine-1-carboir^{y}l)pyridin-3-yl)-3-methyl-2,8-diazaspiro[4.5]decan-2-yl)benzonitrile |
| | 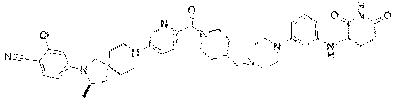 | 2-Chloro-4-((*R*)-8-(6-(4-((4-(3-(((*S*)-2,6-dioxopiperidin-3-yl)amino)phenyl)piperazin-1-yl)methyl)piperidine-1-carboiryl)pyridin-3-yl)-3-methyl-2,8-diazaspiro[4.5]decan-2-yl)benzonitrile |
| | 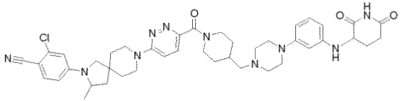 | 2-Chloro-4-(8-(6-(4-((4-(3-((2,6-dioxopiperidin-3-yl)amino)phenyl)piperazin-1-yl)methyl)piperidine-1-carboiryl)pyridazin-3-yl)-3-methyl-2,8-diazaspiro[4.5]decan-2-yl)benzonitrile |
| 13 | 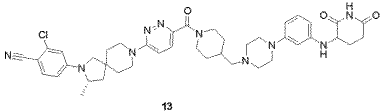 | 2-Chloro-4-((3*S*)-8-(6-(4-((4-(3-((2,6-dioxopiperidin-3-yl)amino)phenyl)piperazin-1-yl)methyl)piperidine-1-carboiryl)pyridazin-3-yl)-3-methyl-2,8-diazaspiro[4.5]decan-2-yl)benzonitrile **13** (a mixture of diastereomers) |
| | 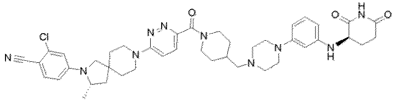 | 2-Chloro-4-((*S*)-8-(6-(4-((4-(3-(((*R*)-2,6-dioxopiperidin-3-yl)amino)phenyl)piperazin-1-yl)methyl)piperidine-1-carboiryl)pyridazin-3-yl)-3-methyl-2,8-diazaspiro[4.5]decan-2-yl)benzonitrile |
| | 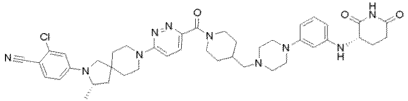 | -4-((*S*)-8-(6-(4-((4-(3-(((*S*)-2,6-dioxopiperidin-3-yl)amino)phenyl)piperazin-1-yl)methyl)piperidine-1-carbonyl)pyridazin-3-yl)-3-methyl-2,8-diazaspiro[4.5]decan-2-yl)benzonitrile |
| | 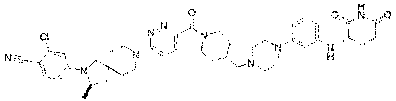 | 2-Chloro-4-((3*R*)-8-(6-(4-((4-(3-((2,6-dioxopiperidin-3-yl)amino)phenyl)piperazin-1-yl)methyl)piperidine-1-carboiryl)pyridazin-3-yl)-3-methyl-2,8-diazaspiro[4.5]decan-2-yl)benzonitrile |
| | 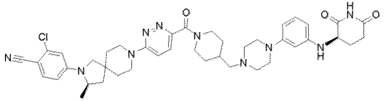 | 2-Chloro-4-((*R*)-8-(6-(4-((4-(3-(((*R*)-2,6-dioxopiperidin-3-yl)amino)phenyl)piperazin-1-yl)methyl)piperidine-1-carboiryl)pyridazin-3-yl)-3-methyl-2,8-diazaspiro[4.5]decan-2-yl)benzonitrile |
| | 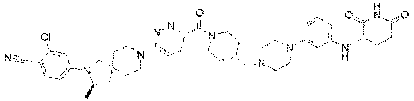 | -4-((*R*)-8-(6-(4-((4-(3-(((*S*)-2,6-dioxopiperidin-3-yl)amino)phenyl)piperazin-1-yl)methyl)piperidine-1-carboiryl)pyridazin-3-yl)-3-methyl-2,8-diazaspiro[4.5]decan-2-yl)benzonitrile |
| | 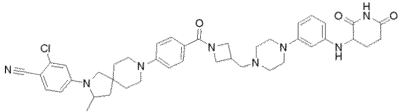 | 2-Chloro-4-(8-(4-(4-((4-(3-((2,6-dioxopiperidin-3-yl)amino)phenyl)piperazin-1-yl)methyl)azetidine-1-carbonyl)phenyl)-3-methyl-2,8-diazaspiro [4.5] decan-2-yl)benzonitrile |
| 14 | 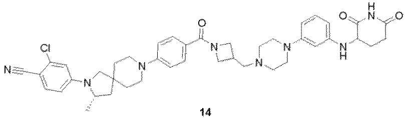 | 2-Chloro-4-((3*S*)-8-(4-(4-((4-(3-((2,6-dioxopiperidin-3-yl)amino)phenyl)piperazin-1-yl)methyl)azetidine-1-carbonyl)phenyl)-3-methyl-2,8-diazaspiro [4.5] decan-2-yl)benzonitrile **14** (a mixture of diastereomers) |
| | 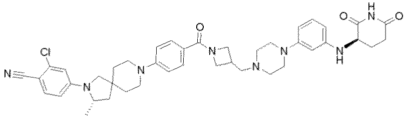 | 2-Chloro-4-((*S*)-8-(4-(4-((4-(3-(((*R*)-2,6-dioxopiperidin-3-yl)amino)phenyl)piperazin-1-yl)methyl)azetidine-1-carbonyl)phenyl)-3 - methyl-2,8-diazaspiro[4.5]decan-2-yl)benzonitrile |
| | 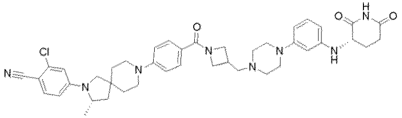 | 2-Chloro-4-((*S*)-8-(4-(4-((4-(3-(((*S*)-2,6-dioxopiperidin-3-yl)amino)phenyl)piperazin-1-yl)methyl)azetidine-1-carbonyl)phenyl)-3-methyl-2,8-diazaspiro[4.5]decan-2-yl)benzonitrile |
| | 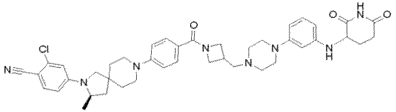 | 2-Chloro-4-((3*R*)-8-(4-(4-((4-(3-((2,6-dioxopiperidin-3-yl)amino)phenyl)piperazin-1-yl)methyl)azetidine-1-carbonyl)phenyl)-3-methyl-2,8-diazaspiro[4.5]decan-2-yl)benzonitrile |
| | 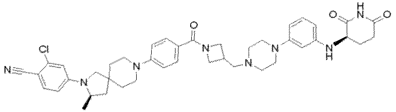 | 2-Chloro-4-((*R*)-8-(4-(4-((4-(3-(((*R*)-2,6-dioxopiperidin-3-yl)amino)phenyl)piperazin-1-yl)methyl)azetidine-1-carbonyl)phenyl)-3-methyl-2,8-diazaspiro[4.5]decan-2-yl)benzonitrile |
| | 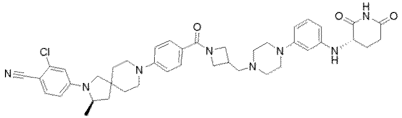 | 2-Chloro-4-((*R*)-8-(4-(4-((4-(3-(((*S*)-2,6-dioxopiperidin-3-yl)amino)phenyl)piperazin-1-yl)methyl)azetidine-1-carbonyl)phenyl)-3-methyl-2,8-diazaspiro[4.5]decan-2-yl)benzonitrile |
| | 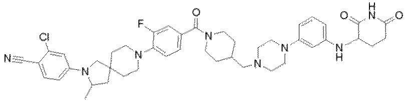 | 2-Chloro-4-(8-(4-(4-((4-(3-((2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile |
| 15 | 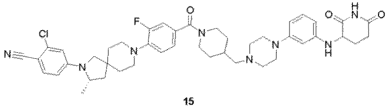 | 2-Chloro-4-((3*S*)-8-(4-(4-((4-(3-((2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile **15** (a mixture of diastereomers) |
| 15-2 | 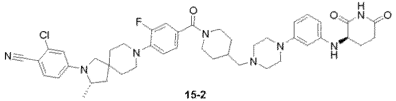 | 2-Chloro-4-((*S*)-8-(4-(4-((4-(3-(((*R*)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile **15-2** |
| 15-1 | 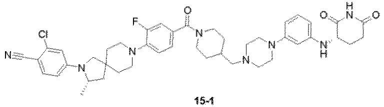 | 2-Chloro-4-((*S*)-8-(4-(4-((4-(3-(((*S*)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile **5-1** |
| | 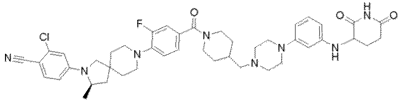 | 2-Chloro-4-((3*R*)-8-(4-(4-((4-(3-((2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile |
| | 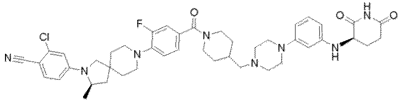 | 2-Chloro-4-((*R*)-8-(4-(4-((4-(3-(((*R*)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile |
| | 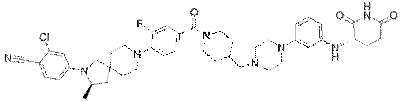 | 2-Chloro-4-((*R*)-8-(4-(4-((4-(3-(((*S*)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile |
| | 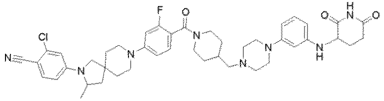 | 2-Chloro-4-(8-(4-(4-((4-(3-((2,6-dioxopiperidin-3-yl)amino)phenyl)piperazin-1-yl)methyl)piperidine-1-carbonyl)-3- fluorophenyl)-3-methyl-2,8-diazaspiro[4.5]decan-2-yl)benzonitrile |
| 16 | 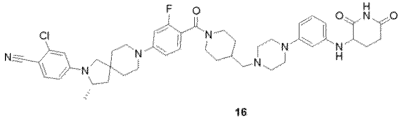 | 2-Chloro-4-((3*S*)-8-(4-(4-((4-(3-((2,6-dioxopiperidin-3-yl)amino)phenyl)piperazin-1-yl)methyl)piperidine-1-carbonyl)-3 - fluorophenyl)-3 -methyl-2,8-diazaspiro[4.5]decan-2-yl)benzonitrile **16** (a mixture of diastereomers) |
| | 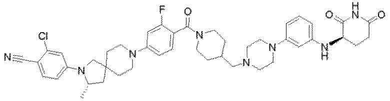 | 2-Chloro-4-((*S*)-8-(4-(4-((4-(3-(((*R*)-2,6-dioxopiperidin-3-yl)amino)phenyl)piperazin-1-yl)methyl)piperidine-1-carbonyl)-3-fluorophenyl)-3 -methyl-2,8-diazaspiro [4.5]decan-2-yl)benzonitrile |
| | 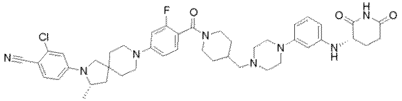 | 2-Chloro-4-((*S*)-8-(4-(4-((4-(3-(((*S*)-2,6-dioxopiperidin-3-yl)amino)phenyl)piperazin-1-yl)methyl)piperidine-1-carbonyl)-3-fluorophenyl)-3-methyl-2,8-diazaspiro[4.5]decan-2-yl)benzonitrile |
| | 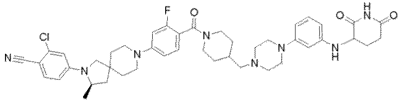 | 2-Chloro-4-((3*R*)-8-(4-(4-((4-(3-((2,6-dioxopiperidin-3-yl)amino)phenyl)piperazin-1-yl)methyl)piperidine-1-carbonyl)-3- fluorophenyl)-3-methyl-2,8-diazaspiro[4.5]decan-2-yl)benzonitrile |
| | 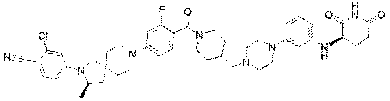 | 2-Chloro-4-((*R*)-8-(4-(4-((4-(3-(((*R*)-2,6-dioxopiperidin-3-yl)amino)phenyl)piperazin-1-yl)methyl)piperidine-1-carbonyl)-3- fluorophenyl)-3-methyl-2,8-diazaspiro[4.5]decan-2-yl)benzonitrile |
| | 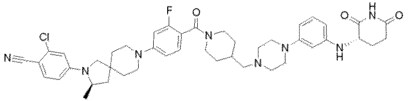 | 2-Chloro-4-((*R*)-8-(4-(4-((4-(3-(((*S*)-2,6-dioxopiperidin-3-yl)amino)phenyl)piperazin-1-yl)methyl)piperidine-1-carbonyl)-3-fluorophenyl)-3-methyl-2,8-diazaspiro[4.5]decan-2-yl)benzonitrile |
| | 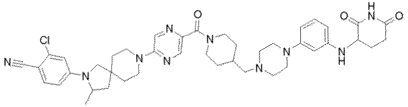 | 2-Chloro-4-(8-(5-(4-((4-(3-((2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile |
| 17 | 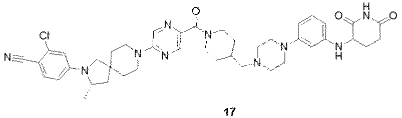 | 2-Chloro-4-((3*S*)-8-(5-(4-((4-(3-((2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile **17** (a mixture of diastereomers) |
| | 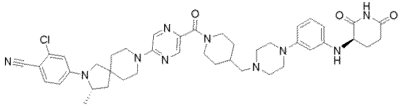 | 2-Chloro-4-((*S*)-8-(5-(4-((4-(3-(((*R*)-2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile |
| | 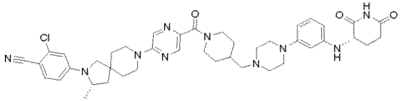 | 2-Chloro-4-((*S*)-8-(5-(4-((4-(3-(((*S*)-2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile |
| | 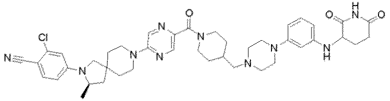 | 2-Chloro-4-((3*R*)-8-(5-(4-((4-(3-((2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile |
| | 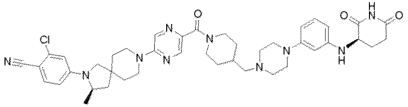 | 2-Chloro-4-((*R*)-8-(5-(4-((4-(3-(((*R*)-2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile |
| | 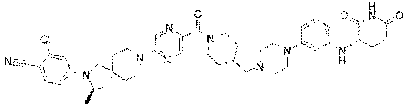 | 2-Chloro-4-((*R*)-8-(5-(4-((4-(3-(((*S*)-2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile |
| | 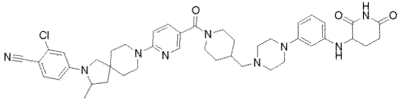 | 2-Chloro-4-(8-(5-(4-((4-(3-((2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile |
| 18 | 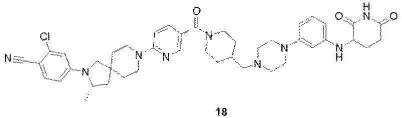 | 2-Chloro-4-((3*S*)-8-(5-(4-((4-(3-((2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile **18** (a mixture of diastereomers) |
| | 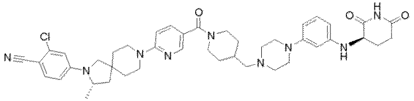 | 2-Chloro-4-((*S*)-8-(5-(4-((4-(3-(((*R*)-2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile |
| | 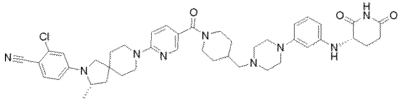 | 2-Chloro-4-((*S*)-8-(5-(4-((4-(3-(((*S*)-2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile |
| | 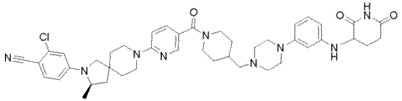 | 2-Chloro-4-((37*R*)-8-(5-(4-((4-(3-((2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile |
| | 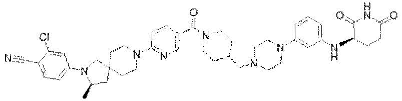 | 2-Chloro-4-((*R*)-8-(5-(4-((4-(3-(((*R*)-2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile |
| | 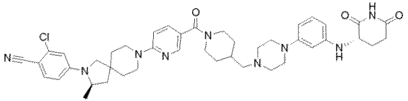 | 2-Chloro-4-((*R*)-8-(5-(4-((4-(3-(((*S*)-2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile |
| | 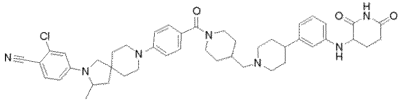 | 2-Chloro-4-(8-(4-(4-((4-(3-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| 19 | 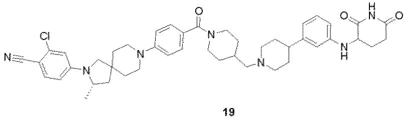 | 2-Chloro-4-((3*S*)-8-(4-(4-((4-(3-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile **19** (a mixture of diastereomers) |
| | 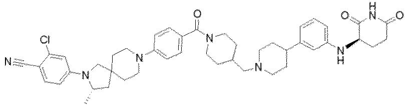 | 2-Chloro-4-((*S*)-8-(4-(4-((4-(3-(((*R*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 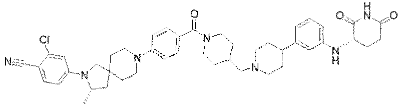 | 2-Chloro-4-((*S*)-8-(4-(4-((4-(3-(((*S*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 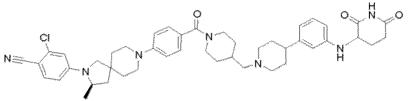 | 2-Chloro-4-((3*R*)-8-(4-(4-((4-(3-((2,6-dioxopiperidin-3-yl)amino)phenyl)piperidin-1-yl)methyl)piperidine-l-carbonyl)phenyl)-3-methyl-2,8-diazaspiro[4.5]decan-2-yl)benzonitrile |
| | 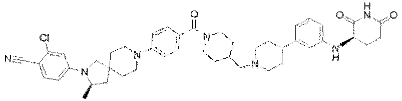 | 2-Chloro-4-((*R*)-8-(4-(4-((4-(3-(((*R*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 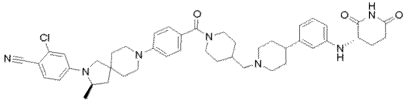 | 2-Chloro-4-((*R*)-8-(4-(4-((4-(3-(((*S*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 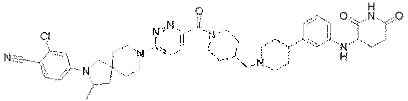 | 2-Chloro-4-(8-(6-(4-((4-(3-((2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile |
| 20 | 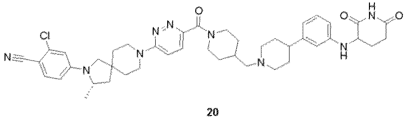 | 2-Chloro-4-((3*S*)-8-(6-(4-((4-(3-((2,6-dioxopiperidin-3-yl)amino)phenyl)piperidin-1-yl)methyl)piperidine-l-carbonyl)pyridazin-3-yl)-3-methyl-2,8-diazaspiro[4.5]decan-2-yl)benzonitrile **20** (a mixture of diastereomers) |
| | 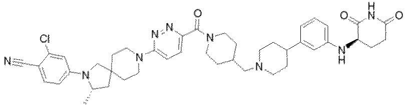 | 2-Chloro-4-((*S*)-8-(6-(4-((4-(3-(((*R*)-2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile |
| | 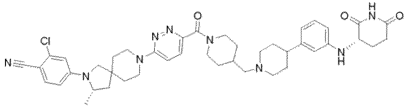 | 2-Chloro-4-((*S*)-8-(6-(4-((4-(3-(((*S*)-2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile |
| | 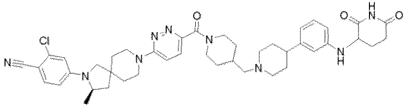 | 2-Chloro-4-((3*R*)-8-(6-(4-((4-(3-((2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile |
| | 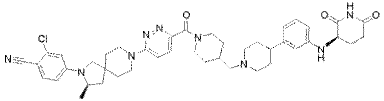 | 2-Chloro-4-((*R*)-8-(6-(4-((4-(3-(((*R*)-2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile |
| | 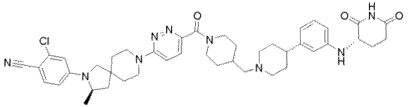 | 2-Chloro-4-((*R*)-8-(6-(4-((4-(3-(((*S*)-2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile |
| | 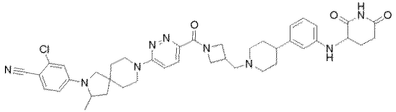 | 2-Chloro-4-(8-(6-(3-((4-(3-((2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile |
| 21 | 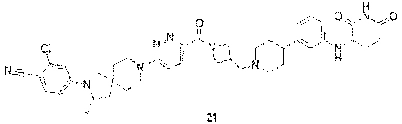 | 2-Chloro-4-((3*S*)-8-(6-(3-((4-(3-((2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile **21** (a mixture of diastereomers) |
| | 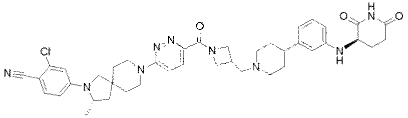 | 2-Chloro-4-((*S*)-8-(6-(3-((4-(3-(((*R*)-2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile |
| | 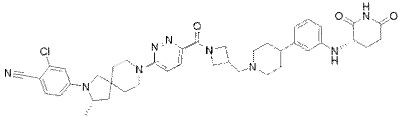 | 2-Chloro-4-((*S*)-8-(6-(3-((4-(3-(((*S*)-2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile |
| | 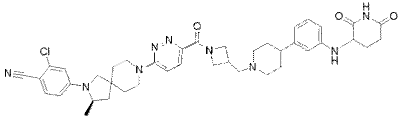 | 2-Chloro-4-((3*R*)-8-(6-(3-((4-(3-((2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile |
| | 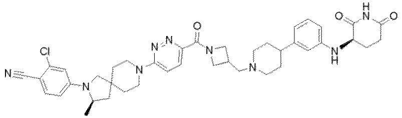 | 2-Chloro-4-((*R*)-8-(6-(3-((4-(3-(((*R*)-2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile |
| | 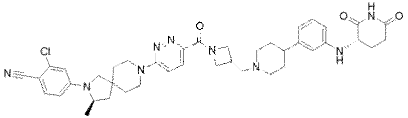 | 2-Chloro-4-((*R*)-8-(6-(3-((4-(3-(((*S*)-2,6-dioxopiperidin-3-yl)amino)phenyl)piperidin-1-yl)methyl)azetidine-1-carbonyl)pyridazin-3- yl)-3-methyl-2,8-diazaspiro[4.5]decan-2-yl)benzonitrile |
| | 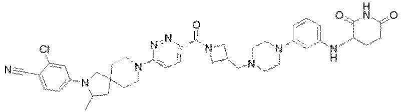 | 2-Chloro-4-(8-(6-(3-((4-(3-((2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile |
| 22 | 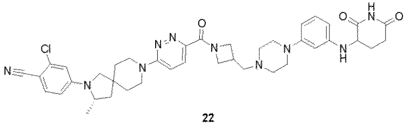 | 2-Chloro-4-((3*S*)-8-(6-(3-((4-(3-((2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile **22** (a mixture of diastereomers) |
| | 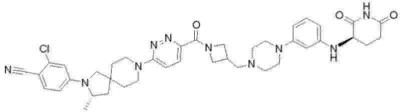 | 2-Chloro-4-((*S*)-8-(6-(3-((4-(3-(((*R*)-2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile |
| | 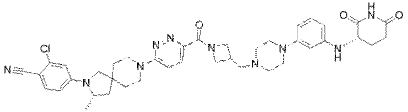 | 2-Chloro-4-((*S*)-8-(6-(3-((4-(3-(((*S*)-2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile |
| | 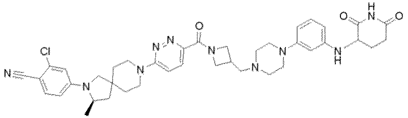 | 2-Chloro-4-((3*R*)-8-(6-(3-((4-(3-((2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile |
| | 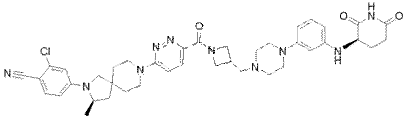 | 2-Chloro-4-((*R*)-8-(6-(3-((4-(3-(((*R*)-2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile |
| | 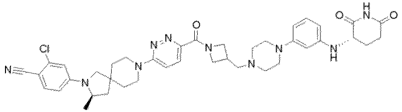 | 2-Chloro-4-((*R*)-8-(6-(3-((4-(3-(((*S*)-2,6-dioxopiperidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile |
| | 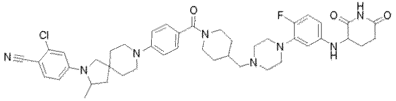 | 2-Chloro-4-(8-(4-(4-((4-(5-((2,6-dioxopiperidin-3-yl)amino)-2-fluorophenyl)piperazin-1-yl)methyl)piperidine-1-carbonyl)phenyl)-3-methyl-2,8-diazaspiro [4.5] decan-2-yl)benzonitrile |
| 23 | 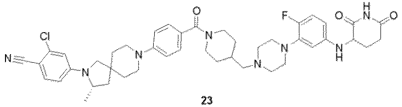 | 2-Chloro-4-((3*S*)-8-(4-(4-((4-(5-((2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile **23** (a mixture of diastereomers) |
| | 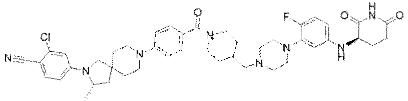 | 2-Chloro-4-((*S*)-8-(4-(4-((4-(5-(((*R*)-2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile |
| | 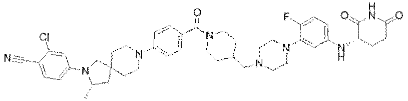 | 2-Chloro-4-((*S*)-8-(4-(4-((4-(5-(((*S*)-2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile |
| | 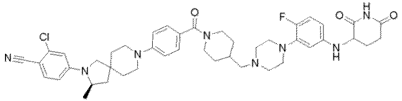 | 2-Chloro-4-((3*R*)-8-(4-(4-((4-(5-((2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile |
| | 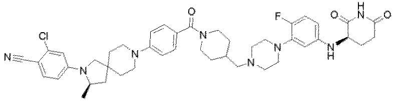 | 2-Chloro-4-((*R*)-8-(4-(4-((4-(5-(((*R*)-2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile |
| | 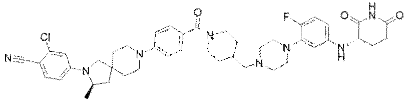 | 2-Chloro-4-((*R*)-8-(4-(4-((4-(5-(((*S*)-2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile |
| | 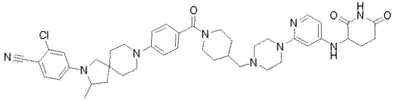 | 2-Chloro-4-(8-(4-(4-((4-(4-((2,6-dioxopiperidin-3-yl)amino)pyridin-2-yl)piperazin-1-yl)methyl)piperidine-1-carbonyl)phenyl)-3-methyl-2,8-diazaspiro [4.5]decan-2-yl)benzonitrile |
| 24 | 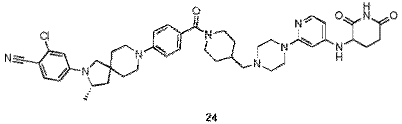 | 2-Chloro-4-((3*S*)-8-(4-(4-((4-(4-((2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile **24** (a mixture of diastereomers) |
| | 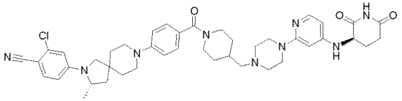 | 2-Chloro-4-((*S*)-8-(4-(4-((4-(4-(((*R*)-2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile |
| | 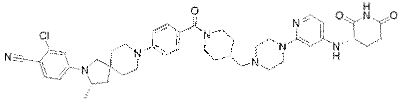 | 2-Chloro-4-((S)-8-(4-(4-((4-(4-(((S)-2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile |
| | 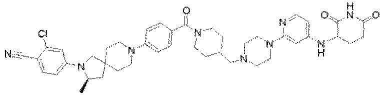 | 2-Chloro-4-((3*R*)-8-(4-(4-((4-(4-((2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile |
| | 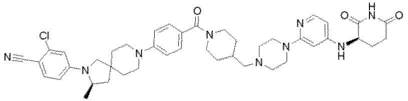 | 2-Chloro-4-((*R*)-8-(4-(4-((4-(4-(((*R*)-2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile |
| | 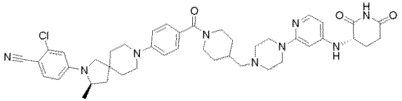 | 2-Chloro-4-((R)-8-(4-(4-((4-(4-(((*S*)-2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile |
| | 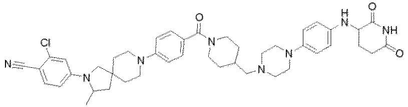 | 2-Chloro-4-(8-(4-(4-((4-(4-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| 25 | 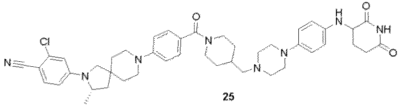 | 2-Chloro-4-((3*S*)-8-(4-(4-((4-(4-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile **25** (a mixture of diastereomers) |
| | 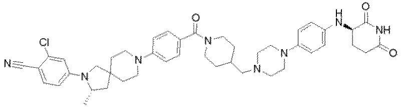 | 2-Chloro-4-((*S*)-8-(4-(4-((4-(4-(((*R*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 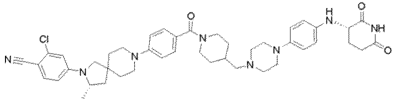 | 2-Chloro-4-((*S*)-8-(4-(4-((4-(4-(((*S*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 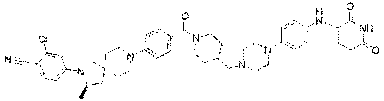 | 2-Chloro-4-((3*R*)-8-(4-(4-((4-(4-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 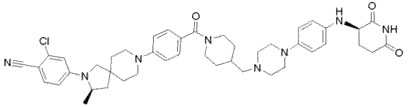 | 2-Chloro-4-((*R*)-8-(4-(4-((4-(4-(((*R*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 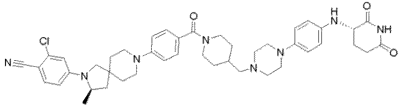 | 2-Chloro-4-((*R*)-8-(4-(4-((4-(4-(((*S*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 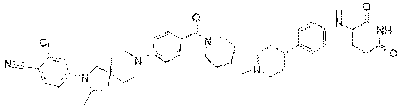 | 2-Chloro-4-(8-(4-(4-((4-(4-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| 26 | 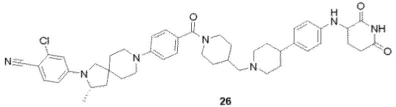 | 2-Chloro-4-((3*S*)-8-(4-(4-((4-(4-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile **26** (a mixture of diastereomers) |
| | 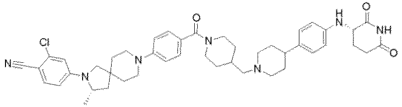 | 2-Chloro-4-((*S*)-8-(4-(4-((4-(4-(((*S*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 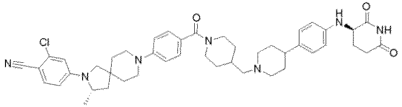 | 2-Chloro-4-((*S*)-8-(4-(4-((4-(4-(((*R*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 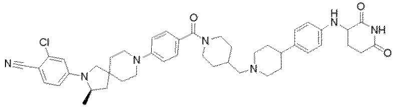 | 2-Chloro-4-((3*R*)-8-(4-(4-((4-(4-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 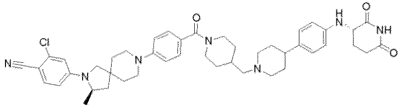 | 2-Chloro-4-((*R*)-8-(4-(4-((4-(4-(((*S*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 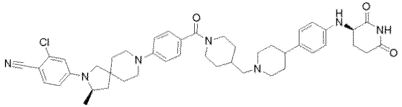 | 2-Chloro-4-((*R*)-8-(4-(4-((4-(4-(((*R*)-2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile |
| | 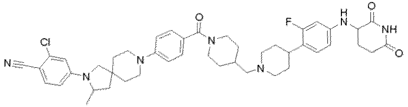 | 2-Chloro-4-(8-(4-(4-((4-(4-((2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile |
| 27 | 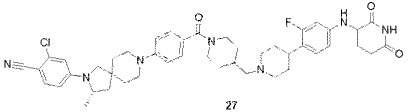 | 2-Chloro-4-((3*S*)-8-(4-(4-((4-(4-((2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile **27** (a mixture of diastereomers) |
| | 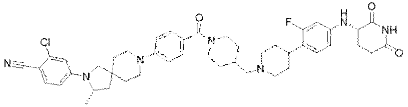 | 2-Chloro-4-((*S*)-8-(4-(4-((4-(4-(((*S*)-2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile |
| | 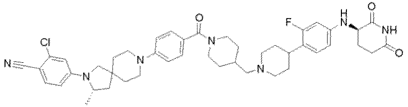 | 2-Chloro-4-((*S*)-8-(4-(4-((4-(4-(((*R*)-2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile |
| | 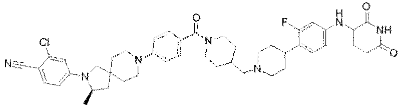 | 2-Chloro-4-((3*R*)-8-(5-(4-((4-(4-((2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile 27 (a mixture of diastereomers) |
| | 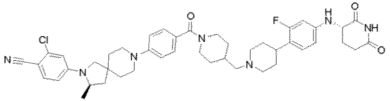 | 2-Chloro-4-((*R*)-8-(4-(4-((4-(4-(((*S*)-2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile |
| | 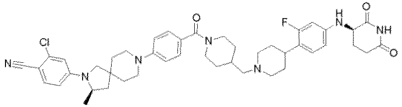 | 2-Chloro-4-((*R*)-8-(4-(4-((4-(4-(((*R*)-2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile |
| | 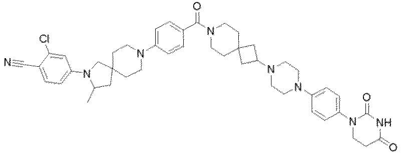 | 2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carboiryl)phenyl)-3-methyl-2,8-diazaspiro[4.5]decan-2-yl)benzonitrile |
| 28 | 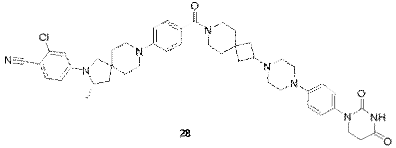 | (S)-2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)phenyl)-3-methyl-2,8-diazaspiro[4.5]decan-2-yl)benzonitrile **28** |
| | 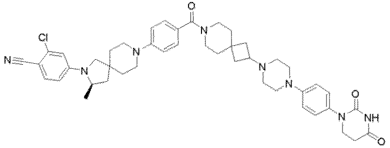 | (R)-2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carboiryl)phenyl)-3-methyl-2,8-diazaspiro[4.5]decan-2-yl)benzonitrile |
| | 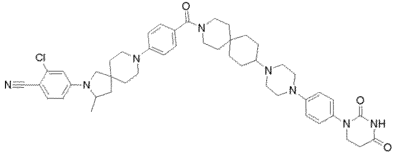 | 2-Chloro-4-(8-(4-(9-(4-(4-(2,4-dioxotetrahydropyrimidin-1(2*H*)-yl)phenyl)piperazin-1-yl)-3-azaspiro[5.5]undecane-3-carbonyl)phenyl)-3-methyl-2,8-diazaspiro[4.5]decan-2-yl)benzonitrile |
| 29 | 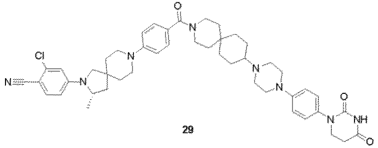 | (*S*)-2-Chloro-4-(8-(4-(9-(4-(4-(2,4-dioxotetrahydropyrimidin-1(2*H*)-yl)phenyl)piperazin-1-yl)-3-azaspiro[5.5]undecane-3-carbonyl)phenyl)-3-methyl-2,8-diazaspiro[4.5]decan-2-yl)benzonitrile **29** |
| | 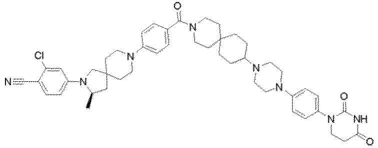 | (*R*)-2-Chloro-4-(8-(4-(9-(4-(4-(2,4-dioxotetrahydropyrimidin-1(2*H*)-yl)phenyl)piperazin-1-yl)-3-azaspiro[5.5]undecane-3-carbonyl)phenyl)-3-methyl-2,8-diazaspiro[4.5]decan-2-yl)benzonitrile |
| | 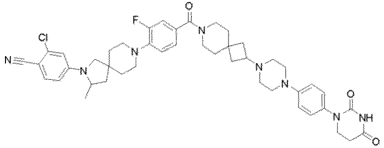 | 2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| 30 | 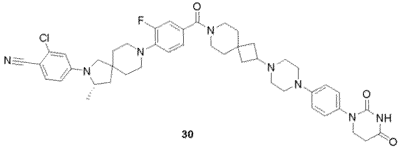 | (*S*)-2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)-2-fluorophenyl)-3-methyl-2,8-diazaspiro[4.5]decan-2-yl)benzonitrile **30** |
| | 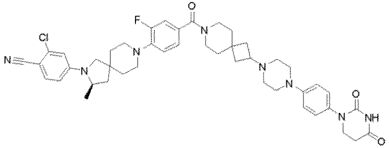 | (*R*)-2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| | 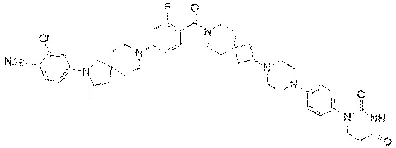 | 2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)-3-fluorophenyl)-3-methyl-2,8-diazaspiro [4.5]decan-2-yl)benzonitrile |
| 31 | 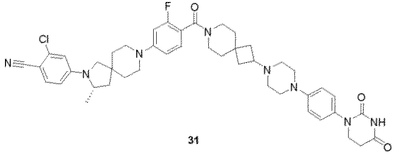 | (*S*)-2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)-3-fluorophenyl)-3-methyl-2,8-diazaspiro[4.5]decan-2-yl)benzonitrile **31** |
| | 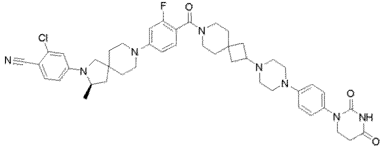 | (*R*)-2-Chloro-4-(8-(4-(2-(4-(4-(2,4-dioxotetrahydropyrimidin-1(2*H*)-yl)phenyl)piperazin-1-yl)-7-azaspiro[3.5]nonane-7-carbonyl)-3-fluorophenyl)-3-methyl-2,8-diazaspiro [4.5]decan-2-yl)benzonitrile |
| | 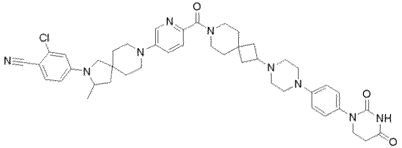 | 2-Chloro-4-(8-(6-(2-(4-(4-(2,4-dioxotetrahydropyrimidin-1(2*H*)-yl)phenyl)piperazin-1-yl)-7-azaspiro[3.5]nonane-7-carbonyl)pyridin-3-yl)-3-methyl-2,8-diazaspiro[4.5]decan-2-yl)benzonitrile |
| 32 | 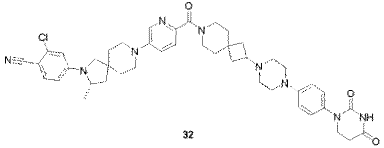 | (*S*)-2-Chloro-4-(8-(6-(2-(4-(4-(2,4-dioxotetrahydropyrimidin-1(2*H*)-yl)phenyl)piperazin-1-yl)-7-azaspiro[3.5]nonane-7-carbonyl)pyridin-3-yl)-3-methyl-2,8-diazaspiro[4.5]decan-2-yl)benzonitrile **32** |
| | 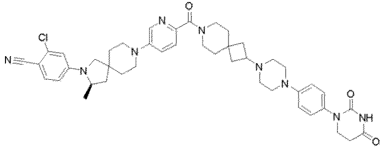 | (*R*)-2-Chloro-4-(8-(6-(2-(4-(4-(2,4-dioxotetrahydropyrimidin-1(2*H*)-yl)phenyl)piperazin-1-yl)-7-azaspiro[3.5]nonane-7-carbonyl)pyridin-3-yl)-3-methyl-2,8-diazaspiro[4.5]decan-2-yl)benzonitrile |
| | 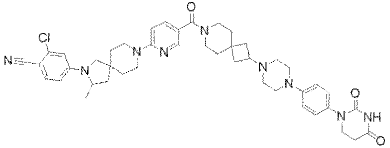 | 2-Chloro-4-(8-(5-(2-(4-(4-(2,4-dioxotetrahydropyrimidin-1(2*H*)-yl)phenyl)piperazin-1-yl)-7-azaspiro[3.5]nonane-7-carbonyl)pyridin-2-yl)-3-methyl-2,8-diazaspiro[4.5]decan-2-yl]benzonitrile |
| 33 | 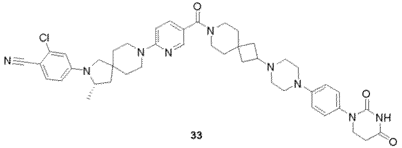 | (*S*)-2-Chloro-4-(8-(5-(2-(4-(4-(2,4-dioxotetrahydropyrimidin-1(2*H*)-yl)phenyl)piperazin-1-yl)-7-azaspiro[3.5]nonane-7-carbonyl)pyridin-2-yl)-3-methyl-2,8-diazaspiro[4.5]decan-2-yl]benzonitrile **33** |
| | 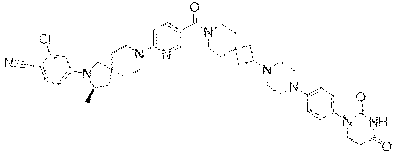 | (*R*)-2-Chloro-4-(8-(5-(2-(4-(4-(2,4-dioxotetrahydropyrimidin-1(2*H*)-yl)phenyl)piperazin-1-yl)-7-azaspiro[3.5]nonane-7-carbonyl)pyridin-2-yl)-3-methyl-2,8-diazaspiro[4.5]decan-2-yl]benzonitrile |
| | 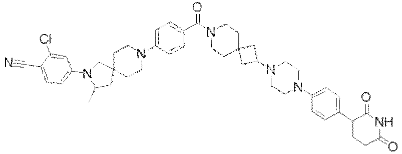 | 2-Chloro-4-(8-(4-(2-(4-(4-(2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile |
| 34 | 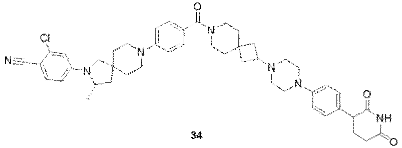 | 2-Chloro-4-((3*S*)-8-(4-(2-(4-(4-(2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile **34** (a mixture of diastereomers) |
| | 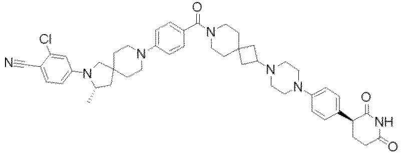 | 2-Chloro-4-((*S*)-8-(4-(2-(4-(4-((*S*)-2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile |
| | 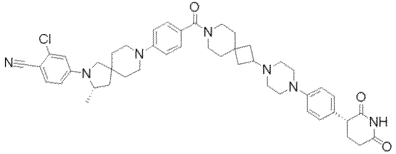 | 2-Chloro-4-((*S*)-8-(4-(2-(4-(4-((*R*)-2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile |
| | 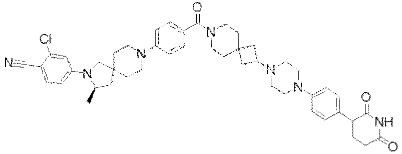 | 2-Chloro-4-((3*R*)-8-(4-(2-(4-(4-(2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile |
| | 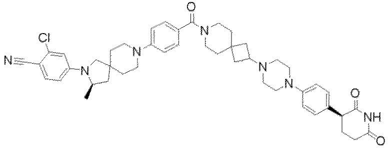 | 2-Chloro-4-((*R*)-8-(4-(2-(4-(4-((*S*)-2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile |
| | 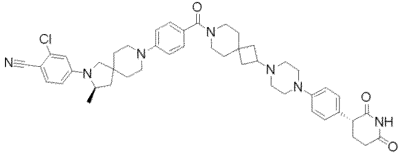 | 2-Chloro-4-((*R*)-8-(4-(2-(4-(4-((*R*)-2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile |
| | 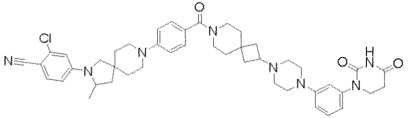 | 2-Chloro-4-(8-(4-(2-(4-(3-(2,4-dioxotetrahydropyrimidin-1(2*H*)-yl)phenyl)piperazin-1-yl)-7-azaspiro[3.5]nonane-7-carbonyl)phenyl)-3-methyl-2,8-diazaspiro[4.5]decan-2-yl)benzonitrile |
| 35 | 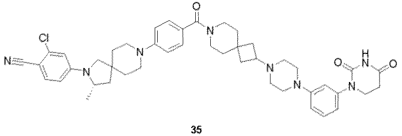 | (S)-2-Chloro-4-(8-(4-(2-(4-(3 -(2,4-dioxotetrahydropyrimidin-1(2*H*)-yl)phenyl)piperazin-1-yl)-7-azaspiro[3.5]nonane-7-carbonyl)phenyl)-3-methyl-2,8-diazaspiro[4.5]decan-2-yl)benzonitrile **35** |
| | 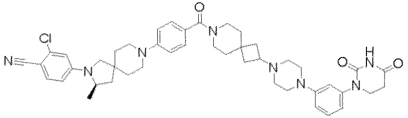 | (*R*)-2-Chloro-4-(8-(4-(2-(4-(3-(2,4-dioxotetrahydropyrimidin-1(2*H*)-yl)phenyl)piperazin-1-yl)-7-azaspiro[3.5]nonane-7-carbonyl)phenyl)-3-methyl-2,8-diazaspiro[4.5]decan-2-yl)benzonitrile |
| | 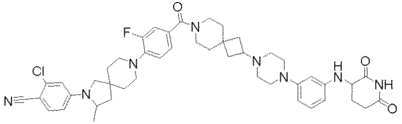 | 2-Chloro-4-(8-(4-(2-(4-(3 -((2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| 36 | 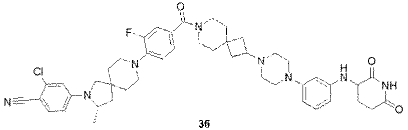 | 2-Chloro-4-((3*S*)-8-(4-(2-(4-(3-((2,6-dioxopiperidin-3-yl)amino)phenyl)piperazin-1-yl)-7-azaspiro[3.5]nonane-7-carbonyl)-2-fluorophenyl)-3 -methyl-2,8-diazaspiro[4.5]decan-2-yl)benzonitrile **36** (a mixture of diastereomers) |
| | 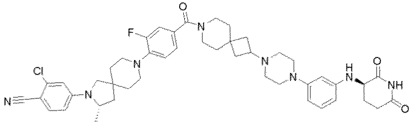 | 2-Chloro-4-((*S*)-8-(4-(2-(4-(3-(((*R*)-2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| | 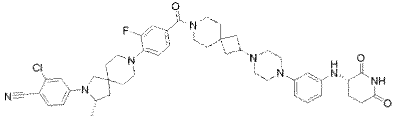 | 2-Chloro-4-((*S*)-8-(4-(2-(4-(3-(((*S*)-2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| | 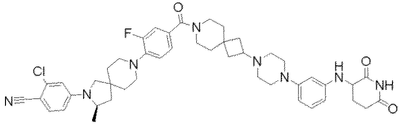 | 2-Chloro-4-((3*R*)-8-(4-(2-(4-(3-((2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| | 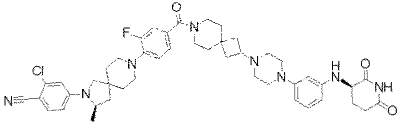 | 2-Chloro-4-((*R*)-8-(4-(2-(4-(3-(((*R*)-2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| | 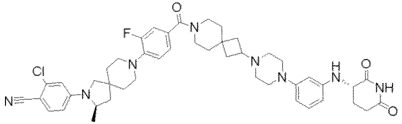 | 2-Chloro-4-((*R*)-8-(4-(2-(4-(3-(((*S*)-2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile |
| | 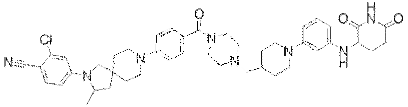 | 2-Chloro-4-(8-(4-(4-((1-(3-((2,6-dioxopiperidin-3-yl)amino)phenyl)piperidin-4-yl)methyl)piperazine-1-carbonyl)phenyl)-3- methyl-2,8-diazaspiro[4.5]decan-2-yl)benzonitrile |
| 37 | 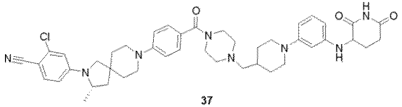 | 2-Chloro-4-((3*S*)-8-(4-(4-((1-(3-((2,6-dioxopiperidin-3-yl)amino)phenyl)piperidin-4-yl)methyl)piperazine-1-carbonyl)phenyl)-3- methyl-2,8-diazaspiro[4.5]decan-2-yl)benzonitrile **37** (a mixture of diastereomers) |
| | 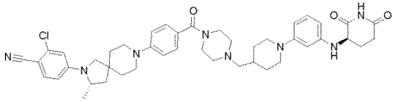 | 2-Chloro-4-((*S*)-8-(4-(4-((1-(3-(((*R*)-2,6-dioxopiperidin-3-yl)amino)phenyl)piperidin-4-yl)methyl)piperazine-1-carbonyl)phenyl)-3-methyl-2,8-diazaspiro[4.5]decan-2-yl)benzonitrile |
| | 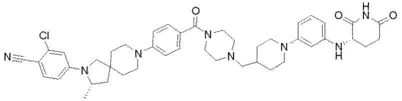 | 2-Chloro-4-((*S*)-8-(4-(4-((1-(3-(((S)-2,6-dioxopiperidin-3-yl)amino)phenyl)piperidin-4-yl)methyl)piperazine-1-carbonyl)phenyl)-3-methyl-2,8-diazaspiro[4.5]decan-2-yl)benzonitrile |
| | 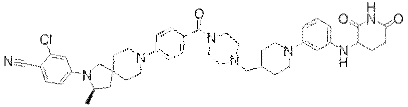 | 2-Chloro-4-((3*R*)-8-(4-(4-((1-(3-((2,6-dioxopiperidin-3-yl)amino)phenyl)piperidin-4-yl)methyl)piperazine-1-carbonyl)phenyl)-3- methyl-2,8-diazaspiro[4.5]decan-2-yl)benzonitrile |
| | 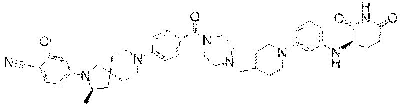 | 2-Chloro-4-((*R*)-8-(4-(4-((1-(3-(((*R*)-2,6-dioxopiperidin-3-yl)amino)phenyl)piperidin-4-yl)methyl)piperazine-1-carbonyl)phenyl)-3- methyl-2,8-diazaspiro[4.5]decan-2-yl)benzonitrile |
| | 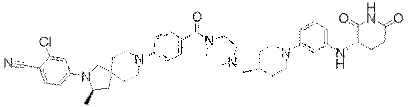 | 2-Chloro-4-((*R*)-8-(4-(4-((1-(3-(((*S*)-2,6-dioxopiperidin-3-yl)amino)phenyl)piperidin-4-yl)methyl)piperazine-1-carbonyl)phenyl)-3- methyl-2,8-diazaspiro[4.5]decan-2-yl)benzonitrile |
| 38 | 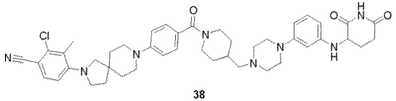 | (±)-2-Chloro-4-(8-(4-(4-((4-(3-((2,6-dioxopiperidin-3-yl)amino)phenyl)piperazin-1-yl)methyl)piperidine-1-carbonyl)phenyl)-2,8-diazaspiro[4.5]decan-2-yl)-3-methylbenzonitrile **38** (a racemate) |
| | 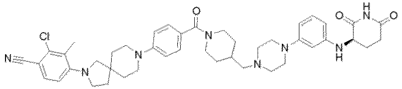 | (*R*)-2-Chloro-4-(8-(4-(4-((4-(3-((2,6-dioxopiperidin-3-yl)amino)phenyl)piperazin-1-yl)methyl)piperidine-1-carbonyl)phenyl)-2,8-diazaspiro[4.5]decan-2-yl)-3-methylbenzonitrile |
| | 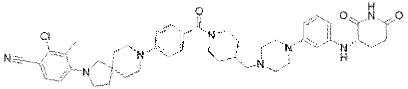 | (S)-2-Chloro-4-(8-(4-(4-((4-(3 -((2,6-dioxopiperidin-3-yl)amino)phenyl)piperazin-1-yl)methyl)piperidine-1-carbonyl)phenyl)-2,8-diazaspiro[4.5]decan-2-yl)-3-methylbenzonitrile |
| | 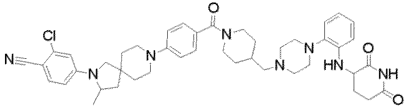 | 2-Chloro-4-(8-(4-(4-((4-(2-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| 39 | 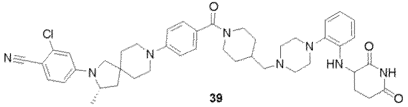 | 2-Chloro-4-((3*S*)-8-(4-(4-((4-(2-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile **39** (a mixture of diastereomers) |
| | 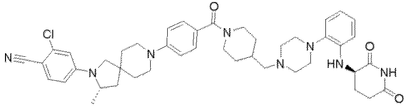 | 2-Chloro-4-((*S*)-8-(4-(4-((4-(2-(((*R*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 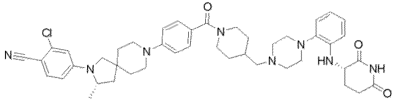 | 2-Chloro-4-((*S*)-8-(4-(4-((4-(2-(((*S*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 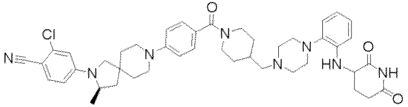 | 2-Chloro-4-((3*R*)-8-(4-(4-((4-(2-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 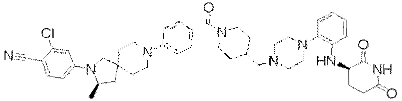 | 2-Chloro-4-((*R*)-8-(4-(4-((4-(2-(((*R*)-2,6-dioxopiperidin-3-yl)amino)phenyl)piperazin-1-yl)methyl)pipendine-1-carbonyl)phenyl)-3-methyl-2,8-diazaspiro[4.5]decan-2-yl)benzonitrile |
| | 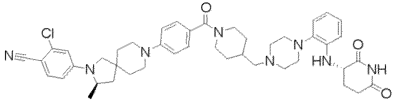 | 2-Chloro-4-((*R*)-8-(4-(4-((4-(2-(((*S*)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile |
| | 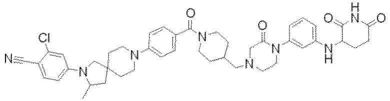 | 2-Chloro-4-(8-(4-(4-((4-(3-((2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile |
| 40 | 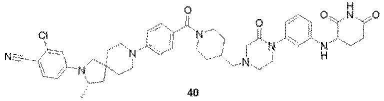 | 2-Chloro-4-((3*S*)-8-(4-(4-((4-(3-((2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile **40** (a mixture of diastereomers) |
| | 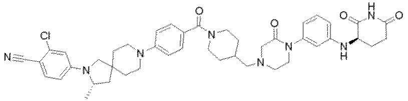 | 2-Chloro-4-((*S*)-8-(4-(4-((4-(3-(((*R*)-2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile |
| | 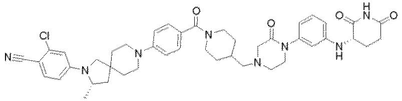 | 2-Chloro-4-((*S*)-8-(4-(4-((4-(3-(((*S*)-2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile |
| | 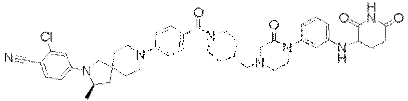 | 2-Chloro-4-((3*R*)-8-(4-(4-((4-(3-((2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile |
| | 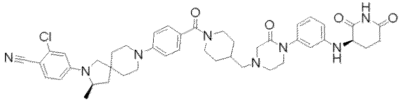 | 2-Chloro-4-((*R*)-8-(4-(4-((4-(3-(((*R*)-2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile |
| | 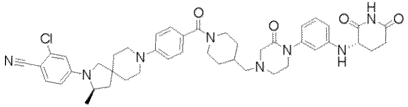 | 2-Chloro-4-((*R*)-8-(4-(4-((4-(3-(((*S*)-2,6-dioxopiperidin-3-yl)amino)phenyl)-3-oxopiperazin-1-yl)methyl)piperidine-1-carbonyl)phenyl)-3-methyl-2,8-diazaspiro[4.5]decan-2-yl)benzonitrile |
| | 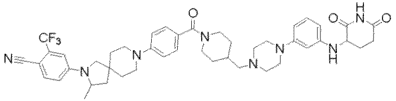 | 4-(8-(4-(4-((4-(3-((2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile |
| 41 | 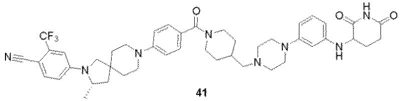 | 4-((3*S*)-8-(4-(4-((4-(3-((2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile **41** (a mixture of diastereomers) |
| | 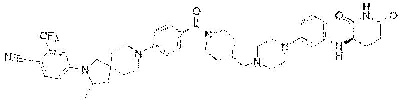 | 4-((*S*)-8-(4-(4-((4-(3-(((*R*)-2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile |
| | 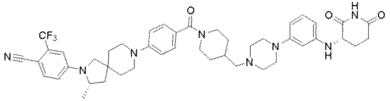 | 4-((1*S*)-8-(4-(4-((4-(3-(((*S*)-2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile |
| | 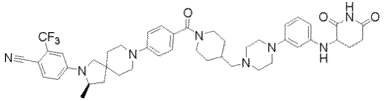 | 4-((3R)-8-(4-(4-((4-(3-((2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile |
| | 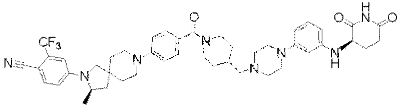 | 4-((*R*)-8-(4-(4-((4-(3-(((*R*)-2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile |
| | 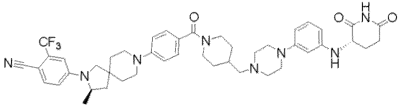 | 4-((*R*)-8-(4-(4-((4-(3-(((*S*)-2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile |
| | 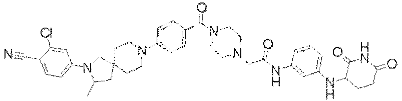 | 2-(4-(4-(2-(3-Chloro-4-cyanophenyl)-3-methyl-2,8-diazaspiro[4.5]decan-8-yl)benzoyl)piperazin-1-yl)-*N*-(3-((2,6-dioxopiperidin-3-yl)amino)phenyl)acetamide |
| 42 | 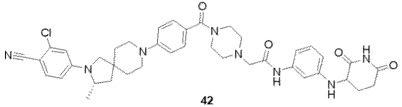 | 2-(4-(4-((*S*)-2-(3-Chloro-4-cyanophenyl)-3-methyl-2,8-diazaspiro[4.5] decan-8-yl)benzoyl)piperazin-1-yl)-*N*-(3-((2,6-dioxopiperidin-3-yl)amino)phenyl)acetamide **42** (a mixture of diastereomers) |
| | 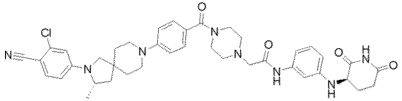 | 2-(4-(4-((*S*)-2-(3-Chloro-4-cyanophenyl)-3-methyl-2,8-diazaspiro[4.5]decan-8-yl)benzoyl)piperazin-1-yl)-*N*-(3-(((*R*)-2,6-dioxopiperidin-3-yl)amino)phenyl)acetamide |
| | 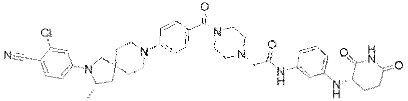 | 2-(4-(4-((*S*)-2-(3-Chloro-4-cyanophenyl)-3-methyl-2,8-diazaspiro[4.5]decan-8-yl)benzoyl)piperazin-1-yl)-*N*-(3-(((*S*)-2,6-dioxopiperidin-3-yl)amino)phenyl)acetamide |
| | 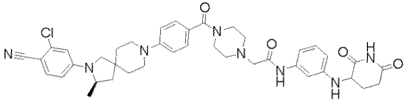 | 2-(4-(4-((*R*)-2-(3-Chloro-4-cyanophenyl)-3-methyl-2,8-diazaspiro[4.5]decan-8-yl)benzoyl)piperazin-1-yl)-*N*-(3-((2,6-dioxopiperidin-3-yl)amino)phenyl)acetamide |
| | 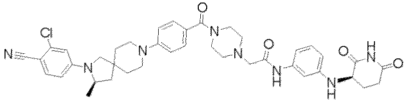 | 2-(4-(4-((*R*)-2-(3-Chloro-4-cyanophenyl)-3-methyl-2,8-diazaspiro[4.5]decan-8-yl)benzoyl)piperazin-1-yl)-*N*-(3-(((*R*)-2,6-dioxopiperidin-3-yl)amino)phenyl)acetamide |
| | 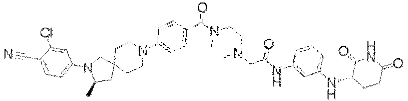 | 2-(4-(4-((*R*)-2-(3-Chloro-4-cyanophenyl)-3-methyl-2,8-diazaspiro[4.5]decan-8-yl)benzoyl)piperazin-1-yl)-*N*-(3-(((*S*)-2,6-dioxopiperidin-3-yl)amino)phenyl)acetamide |
| | 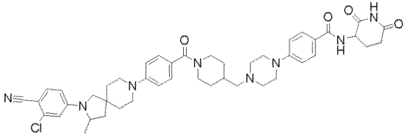 | 4-(4-((1-(4-(2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopiperidin-3-yl)benzamide |
| 43 | 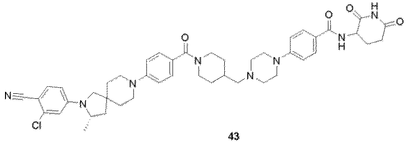 | 4-(4-((1-(4-((*S*)-2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopiperidin-3-yl)benzamide **43** (a mixture of diastereomers) |
| | 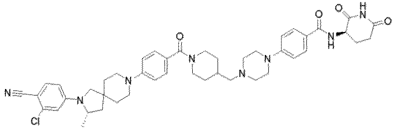 | 4-(4-((1-(4-((*S*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*R*)-2,6-dioxopiperidin-3-yl)benzamide |
| | 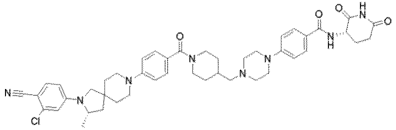 | 4-(4-((1-(4-((*S*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*S*)-2,6-dioxopiperidin-3-yl)benzamide |
| | 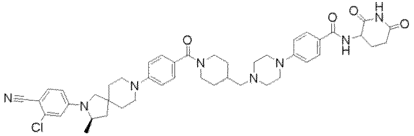 | 4-(4-((1-(4-((*R*)-2-(3-Chloro-4-cyanophenyl)-3-methyl-2,8-diazaspiro[4.5]decan-8-yl)benzoyl)piperidin-4-yl)methyl)piperazin-1-yl)-*N*-(2,6-dioxopiperidin-3-yl)benzamide |
| | 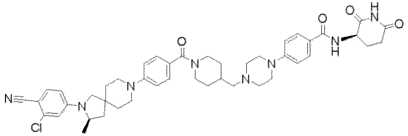 | 4-(4-((1-(4-((*R*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*R*)-2,6-dioxopiperidin-3-yl)benzamide |
| | 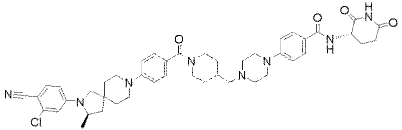 | 4-(4-((1-(4-((*R*)-2-(3-Chloro-4-cyanophenyl)-3-methyl-2,8-diazaspiro[4.5]decan-8-yl)benzoyl)piperidin-4-yl)methyl)piperazin-1-yl)-*N*-((*S*)-2,6-dioxopiperidin-3-yl)benzamide |

Further, the present disclosure provides a compound represented by general formula (XB) or a salt thereof: 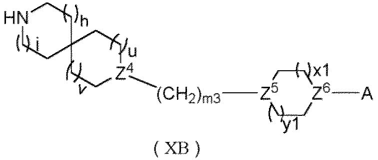 wherein:
Z⁴, Z⁵, Z⁶, h, i, u, v, x1, y1, m3, and A are as defined in general formula (X).

Further, the present disclosure provides a compound represented by general formula (MB) or a salt thereof: 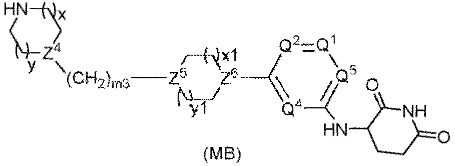 wherein:
Q¹, Q², Q⁴, Q⁵, Z⁴, Z⁵, Z⁶, x, x1, y, y1, and m3 are as defined in general formula (M).

Further, the present disclosure provides a compound represented by general formula (VA) or a salt thereof: 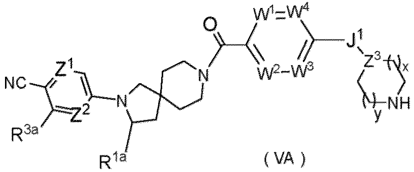 wherein:
J¹, R^{1a}, R^{3a}, W¹, W², W³, W⁴, Z¹, Z², Z³, x, and y are as defined in general formula (V).

Further, the present disclosure provides a compound represented by general formula (V-1A) or a salt thereof: 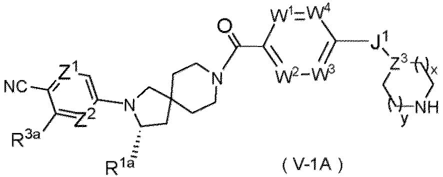 wherein:
J¹, R^{1a}, R^{3a}, W¹, W², W³, W⁴, Z¹, Z², Z³, x, and y are as defined in general formula (V-1).

Further, the present disclosure provides a compound represented by general formula (VIIA) or a salt thereof: 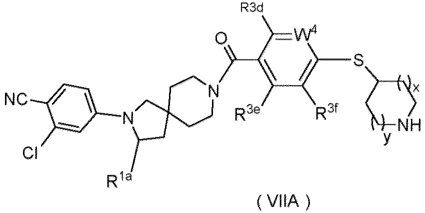 wherein:
R^{1a}, R^{3d}, R^{3e}, R^{3f}, W⁴, x, and y are as defined in general formula (VII).

Further, the present disclosure provides a compound represented by general formula (VII-1A) or a salt thereof: 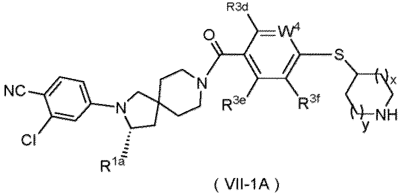 wherein:
R^{1a}, R^{3d}, R^{3e}, R^{3f}, W⁴, x, and y are as defined in general formula (VII-1).

Further, the present disclosure provides a compound represented by general formula (VB) or a salt thereof: 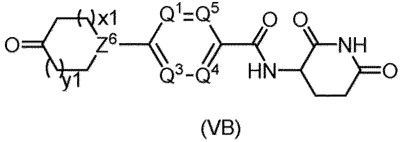 wherein:
Q¹, Q³, Q⁴, Q⁵, Z⁶, x1, and y1 are as defined in general formula (V).

Further, the present disclosure provides a compound represented by general formula (VIIB) or a salt thereof: 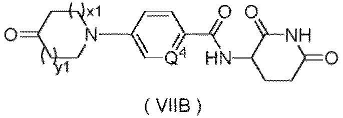 wherein:
Q⁴, x1, and y1 are as defined in general formula (VII).

**Table B. Typical intermediate compounds of the present disclosure include, but are not limited to:**

| No. | Compound structure | Name |
|---|---|---|
| **1c** | 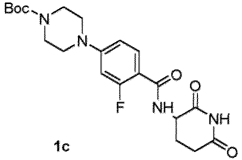 | tert-Butyl (±)-4-(4-((2,6-dioxopiperidin-3-yl)aminocarbonyl)-3-fluorophenyl)piperazine-1-carboxylate **1c** |
| **1d** | 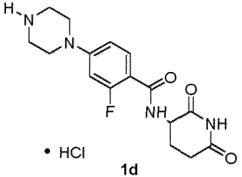 | (±)-*N*-(2,6-Dioxopiperidin-3-yl)-2-fluoro-4-(piperazin-1-yl)benzamide hydrochloride **1d** |
| | 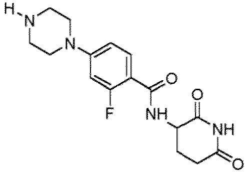 | (±)-*N*-(2,6-Dioxopiperidin-3-yl)-2-fluoro-4-(piperazin-1-yl)benzamide |
| | 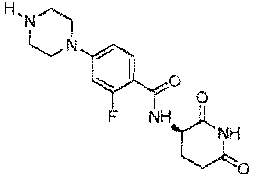 | (*R*)-*N*-(2,6-Dioxopiperidin-3-yl)-2-fluoro-4-(piperazin-1-yl)benzamide |
| | 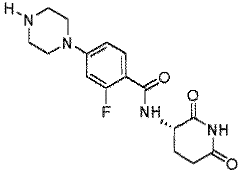 | (*S*)-*N*-(2,6-Dioxopiperidin-3-yl)-2-fluoro-4-(piperazin-1-yl)benzamide |
| **1f** | 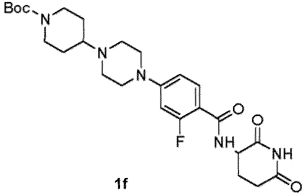 | tert-Butyl (±)-4-(4-(4-((2,6-dioxopiperidin-3 -yl)aminocarbonyl)-3 - fluorophenyl)piperazin-1-yl)piperidine-1-carboxylate **1f** |
| **1g** | 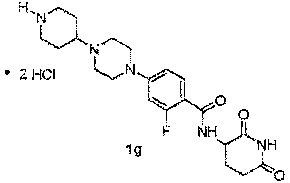 | (±)-*N*-(2,6-Dioxopiperidin-3-yl)-2-fluoro-4-(4-(piperidin-4-yl)piperazin-1-yl)benzamide dihydrochloride **1g** |
| | 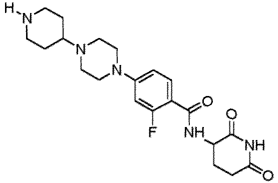 | (±)-*N*-(2,6-Dioxopiperidin-3-yl)-2-fluoro-4-(4-(piperidin-4-yl)piperazin-1-yl)benzamide |
| | 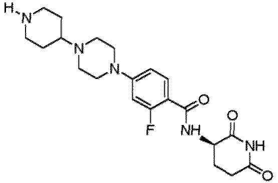 | (*R*)-*N*-(2,6-Dioxopiperidin-3-yl)-2-fluoro-4-(4-(piperidin-4-yl)piperazin-1-yl)benzamide |
| | 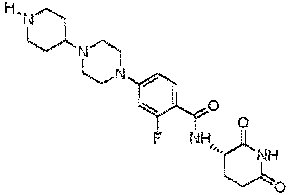 | (*S*)-*N*-(2,6-Dioxopiperidin-3-yl)-2-fluoro-4-(4-(piperidin-4-yl)piperazin-1-yl)benzamide |
| **2d** | 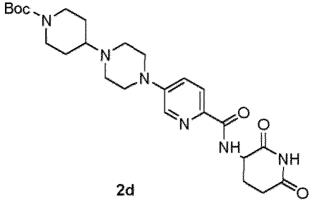 | tert-Butyl (±)-4-(4-(6-((2,6-dioxopiperidin-3-yl)aminocarbonyl)pyridin-3-yl)piperazin-1-yl)piperidine-1-carboxylate **2d** |
| **2e** | 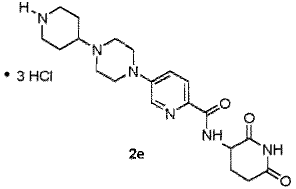 | (±)-*N*-(2,6-Dioxopiperidin-3-yl)-5-(4-(piperidin-4-yl)piperazin-1-yl)picolinamide trihydrochloride 2e |
| | 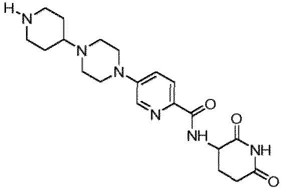 | (±)-*N*-(2,6-Dioxopiperidin-3-yl)-5-(4-(piperidin-4-yl)piperazin-1-yl)picolinamide |
| | 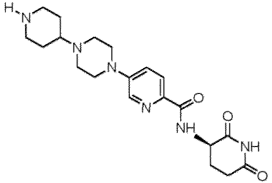 | (*R*)-*N*-(2,6-Dioxopiperidin-3-yl)-5-(4-(piperidin-4-yl)piperazin-1-yl)picolinamide |
| | 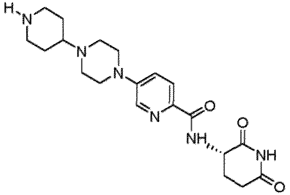 | (*S*)-*N*-(2,6-Dioxopiperidin-3-yl)-5-(4-(piperidin-4-yl)piperazin-1-yl)picolinamide |
| **3b** | 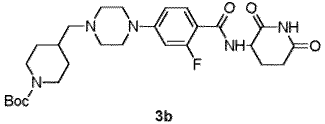 | tert-Butyl (±)-4-((4-(4-((2,6-dioxopiperidin-3 -yl)aminocarbonyl)-3 - fluorophenyl)piperazin-1-yl)methyl)piperidine-1-carboxylate **3b** |
| **3c** | 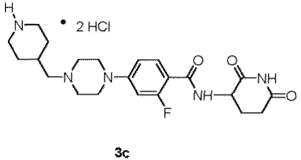 | (±)-*N*-(2,6-Dioxopiperidin-3-yl)-2-fluoro-4-(4-(piperidin-4-yl)methyl)piperazin-1-yl)benzamide dihydrochloride **3c** |
| | 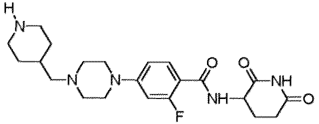 | (±)-*N*-(2,6-Dioxopiperidin-3-yl)-2-fluoro-4-(4-(piperidin-4-yl)methyl)piperazin-1-yl)benzamide |
| | 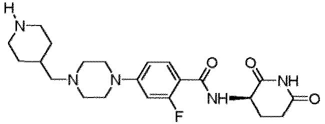 | (*R*)-*N*-(2,6-Dioxopiperidin-3-yl)-2-fluoro-4-(4-(piperidin-4-yl)methyl)piperazin-1-yl)benzamide |
| | 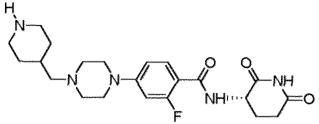 | (*S*)-*N*-(2,6-Dioxopiperidin-3-yl)-2-fluoro-4-(4-(piperidin-4-yl)methyl)piperazin-1-yl)benzamide |
| **4a** | 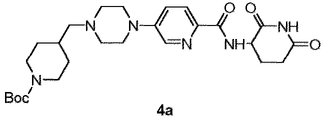 | tert-Butyl (±)-4-((4-(6-((2,6-dioxopiperidin-3-yl)aminocarbonyl)pyridin-3-yl)piperazin-1-yl)methyl)piperidine-1-carboxylate **4a** |
| **4b** | 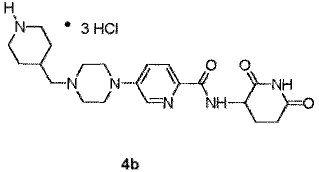 | (±)-*N*-(2,6-Dioxopiperidin-3-yl)-5-(4-(piperidin-4-yl)methyl)piperazin-1-yl)picolinamide trihydrochloride **4b** |
| | 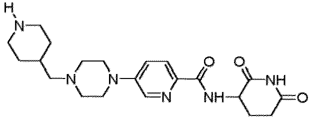 | (±)-*N*-(2,6-Dioxopiperidin-3-yl)-5-(4-(piperidin-4-yl)methyl)piperazin-1-yl)picolinamide |
| | 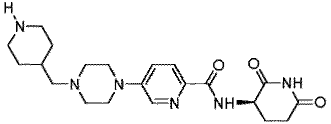 | (*R*)-*N*-(2,6-Dioxopiperidin-3-yl)-5-(4-(piperidin-4-yl)methyl)piperazin-1-yl)picolinamide |
| | 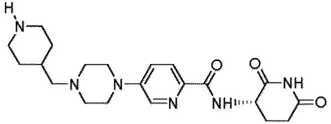 | (*S*)-*N*-(2,6-Dioxopiperidin-3-yl)-5-(4-(piperidin-4-yl)methyl)piperazin-1-yl)picolinamide |
| **5e** | 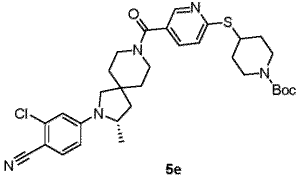 | tert-Butyl (*S*)-4-((5-(2-(3-chloro-4-cyanophenyl)-3-methyl-2,8-diazaspiro [4.5]decane-8-carbonyl)pyridin-2-yl)thio)piperidine-1-carboxylate **5e** |
| **5f** | 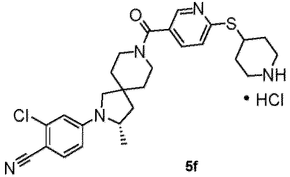 | (*S*)-2-Chloro-4-(3-methyl-8-(6-(piperidin-4-ylthio)nicotinoyl)-2,8-diazaspiro[4.5]decan-2-yl)benzonitrile hydrochloride **5f** |
| | 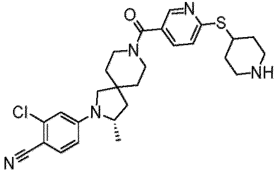 | (*S*)-2-Chloro-4-(3-methyl-8-(6-(piperidin-4-ylthio)nicotinoyl)-2,8-diazaspiro[4.5]decan-2-yl)benzonitrile |
| | 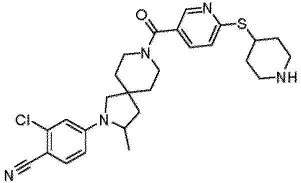 | 2-Chloro-4-(3-methyl-8-(6-(piperidin-4-ylthio)nicotinoyl)-2,8-diazaspiro[4.5]decan-2-yl)benzonitrile |
| | 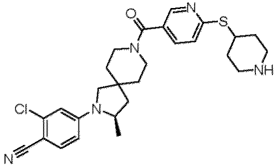 | (R)-2-Chloro-4-(3-methyl-8-(6-(piperidin-4-ylthio)nicotinoyl)-2,8-diazaspiro[4.5]decan-2-yl)benzonitrile |
| **5l** | 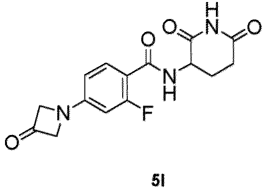 | (±)-*N*-(2,6-Dioxopiperidin-3-yl)-2-fluoro-4-(3-oxoazetidin-1-yl)benzamide **5l** |
| | 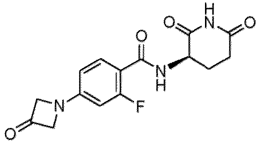 | (*R*)-*N*-(2,6-Dioxopiperidin-3-yl)-2-fluoro-4-(3-oxoazetidin-1-yl)benzamide |
| | 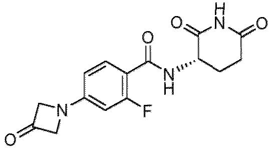 | (*S*)-*N*-(2,6-Dioxopiperidin-3-yl)-2-fluoro-4-(3-oxoazetidin-1-yl)benzamide |
| **6b** | 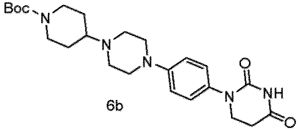 | tert-Butyl 4-(4-(4-(2,4-dioxotetrahydropyrimidin-1(2*H*)-yl)phenyl)piperazin-1-yl)piperidine-1-carboxylate **6b** |
| **6c** | 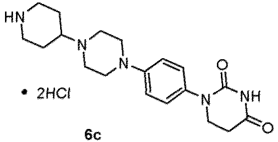 | 1-(4-(4-(Piperidin-4-yl)piperazin-1-yl)phenyl)dihydropyrimidine-2,4(1*H*,3*H*)-dione dihydrochloride **6c** |
| | 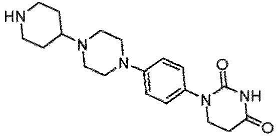 | 1-(4-(4-(Piperidin-4-yl)piperazin-1-yl)phenyl)dihydropyrimidine-2,4(1*H*,3*H*)-dione |
| **6d** | 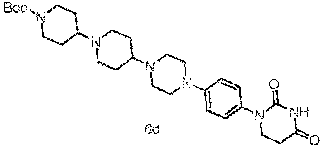 | tert-Butyl 4-(4-(4-(2,4-dioxotetrahydropyrimidin-1(2*H*)-yl)phenyl)piperazin-1-yl)-[1,4'-bipiperidine]-1'-carboxylate **6d** |
| **6e** | 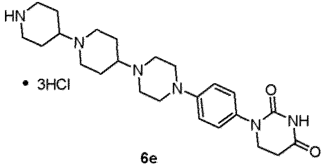 | 1-(4-(4-([1,4'-Bipiperidin]-4-yl)piperazin-1-yl)phenyl)dihydropyrimidine-2,4(1*H*,3*H*)-dione trihydrochloride **6e** |
| | 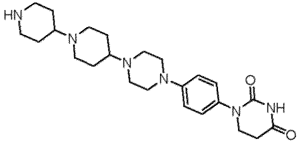 | 1-(4-(4-([1,4'-Bipiperidin]-4-yl)piperazin-1-yl)phenyl)dihydropyrimidine-2,4(1*H*,3*H*)-dione |
| **7b** | 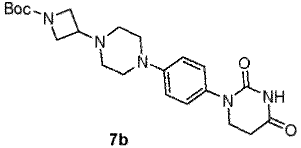 | tert-Butyl 3-(4-(4-(2,4-dioxotetrahydropyrimidin-1(2*H*)-yl)phenyl)piperazin-1-yl)azetidine-1-carboxylate **7b** |
| **7c** | 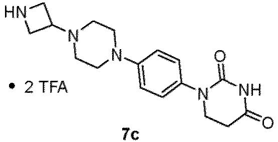 | 1-(4-(4-(Azetidin-3-yl)piperazin-1-yl)phenyl)dihydropyrimidine-2,4(1*H*,3*H*)-dione bistrifluoroacetate **7c** |
| | 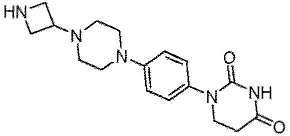 | 1-(4-(4-(Azetidin-3-yl)piperazin-1-yl)phenyl)dihydropyrimidine-2,4(1*H*,3*H*)-dione |
| **7d** | 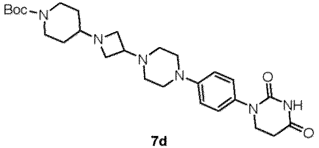 | tert-Butyl 4-(3-(4-(4-(2,4-dioxotetrahydropyrimidin-1(2*H*)-yl)phenyl)piperazin-1-yl)azetidin-1-yl)piperidine-1-carboxylate **7d** |
| **7e** | 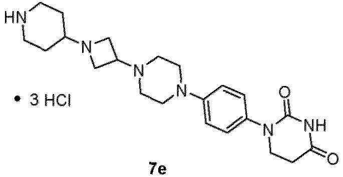 | 1-(4-(4-(1-(Piperidin-4-yl)azetidin-3-yl)piperazin-1-yl)phenyl)dihydropyrimidine-2,4(1*H*,3*H-*dione trihydrochloride **7e** |
| | 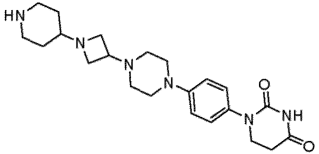 | 1-(4-(4-(1-(Piperidin-4-yl)azetidin-3-yl)piperazin-1-yl)phenyl)dihydropyrimidine-2,4(1*H*,3*H*)-dione |
| **10b** | 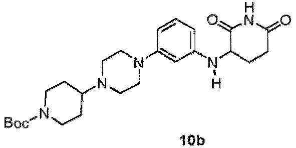 | tert-Butyl (±)-4-(4-(3-((2,6-dioxopiperidin-3-yl)amino)phenyl)piperazin-1-yl)piperidine-1-carboxylate **10b** |
| **10c** | 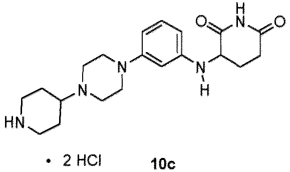 | (±)-3-((3-(4-(Piperidin-4-yl)piperazin-1-yl)phenyl)amino)piperidine-2,6-dione dihydrochloride **10c** |
| | 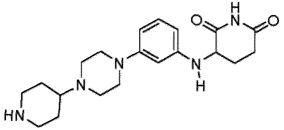 | (±)-3-((3-(4-(Piperidin-4-yl)piperazin-1-yl)phenyl)amino)piperidine-2,6-dione |
| | 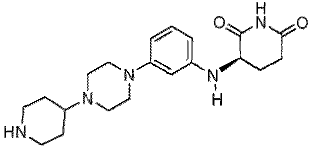 | (*R*)-3-((3-(4-(Piperidin-4-yl)piperazin-1-yl)phenyl)amino)piperidine-2,6-dione |
| | 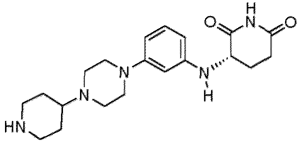 | (*S*)-3-((3-(4-(Piperidin-4-yl)piperazin-1-yl)phenyl)amino)piperidine-2,6-dione |
| **11a** | 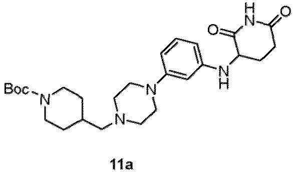 | tert-Butyl (±)-4-((4-(3-((2,6-dioxopiperidin-3-yl)amino)phenyl)piperazin-1-yl)methyl)piperidine-1-carboxylate **11a** |
| **11b** | 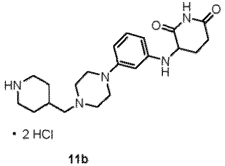 | (±)-3-((3-(4-(Piperidin-4-ylmethyl)piperazin-1-yl)phenyl)amino)piperidine-2,6-dione dihydrochloride **11b** |
| | 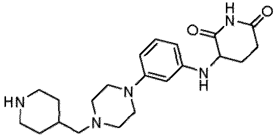 | (±)-3-((3-(4-(Piperidin-4-ylmethyl)piperazin-1-yl)phenyl)amino)piperidine-2,6-dione |
| | 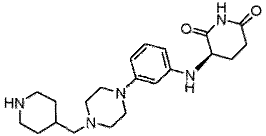 | (*R*)-3-((3-(4-(Piperidin-4-ylmethyl)piperazin-1-yl)phenyl)amino)piperidine-2,6-dione |
| | 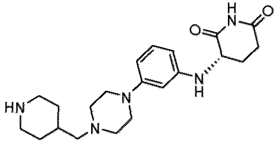 | (*S*)-3-((3-(4-(Piperidin-4-ylmethyl)piperazin-1-yl)phenyl)amino)piperidine-2,6-dione |
| **14a** | 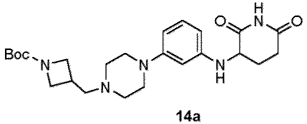 | tert-Butyl (±)-3-((4-(3-((2,6-dioxopiperidin-3-yl)amino)phenyl)piperazin-1-yl)methyl)azetidine-1-carboxylate **14a** |
| **14b** | 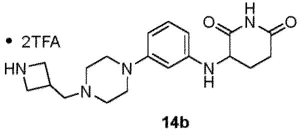 | (±)-3-((3-(4-(Azetidin-3-ylmethyl)piperazin-1-yl)phenyl)amino)piperidine-2,6-dione bistrifluoroacetate **14b** |
| | 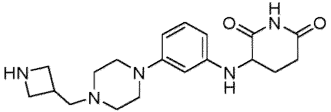 | (±)-3-((3-(4-(Azetidin-3- ylmethyl)piperazin-1-yl)phenyl)amino)piperidine-2,6-dione |
| | 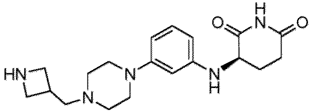 | (*R*)-3-((3-(4-(Azetidin-3-ylmethyl)piperazin-1-yl)phenyl)amino)piperidine-2,6-dione |
| | 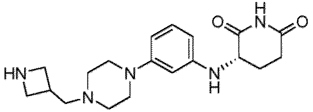 | (*S*)-3-((3-(4-(Azetidin-3-ylmethyl)piperazin-1-yl)phenyl)amino)piperidine-2,6-dione |
| **16a** | 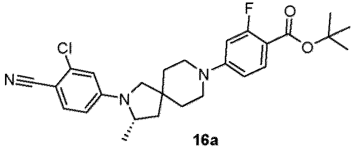 | tert-Butyl (*S*)-4-(2-(3-chloro-4-cyanophenyl)-3-methyl-2,8-diazaspiro[4.5]decan-8-yl)-2-fluorobenzoate **16a** |
| | 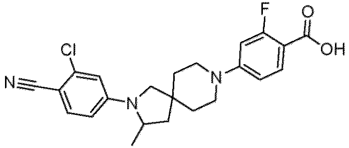 | 4-(2-(3-Chloro-4-cyanophenyl)-3-methyl-2,8-diazaspiro[4.5]decan-8-yl)-2-fluorobenzoic acid |
| **16b** | 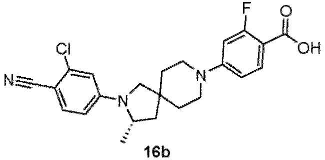 | (S)-4-(2-(3-Chloro-4-cyanophenyl)-3- methyl-2,8-diazaspiro[4.5]decan-8-yl)-2-fluorobenzoic acid **16b** |
| | 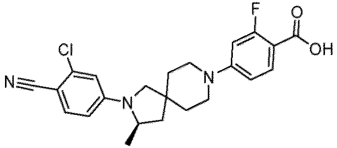 | (*R*)-4-(2-(3-Chloro-4-cyanophenyl)-3-methyl-2,8-diazaspiro [4.5] decan-8-yl)-2-fluorobenzoic acid |
| | 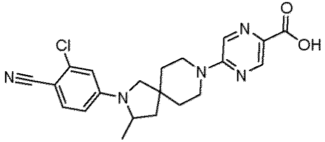 | 5-(2-(3-Chloro-4-cyanophenyl)-3-methyl-2,8-diazaspiro[4.5]decan-8-yl)pyrazine-2-carboxylic acid |
| **17b** | 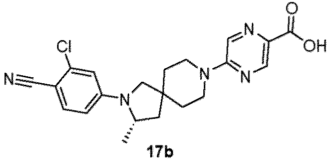 | (*S*)-5-(2-(3-Chloro-4-cyanophenyl)-3-methyl-2,8-diazaspiro[4.5]decan-8-yl)pyrazine-2-carboxylic acid **17b** |
| | 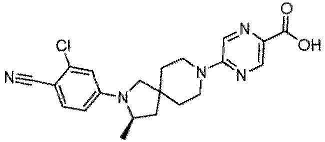 | (*R*)-5-(2-(3-Chloro-4-cyanophenyl)-3-methyl-2,8-diazaspiro[4.5]decan-8-yl)pyrazine-2-carboxylic acid |
| **19b** | 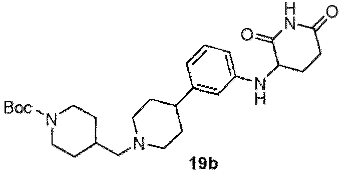 | tert-Butyl (±)-4-((4-(3-((2,6-dioxopiperidin-3-yl)amino)phenyl)piperidin-1-yl)methyl)piperidine-1-carboxylate **19b** |
| **19c** | 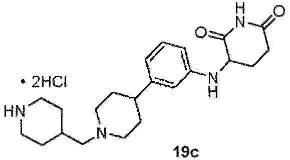 | (±)-3-((3-(1-(Piperidin-4-ylmethyl)piperidin-4-yl)phenyl)amino)piperidine-2,6-dione dihydrochloride **19c** |
| | 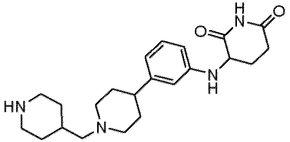 | (±)-3-((3-(1-(Piperidin-4-ylmethyl)piperidin-4-yl)phenyl)amino)piperidine-2,6-dione |
| | 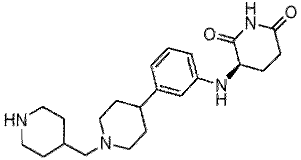 | (*R*)-3-((3-(1-(Piperidin-4-ylmethyl)piperidin-4-yl)phenyl)amino)piperidine-2,6-dione |
| | 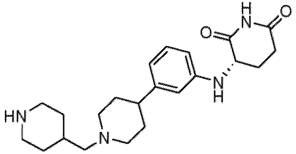 | (*S*)-3-((3-(1-(Piperidin-4-ylmethyl)piperidin-4-yl)phenyl)amino)piperidine-2,6-dione |
| **21a** | 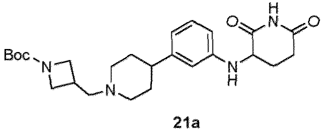 | tert-Butyl (±)-3-((4-(3-((2,6-dioxopiperidin-3-yl)amino)phenyl)piperidin-1-yl)methyl)azetidine-1 -carboxylate **21a** |
| **21b** | 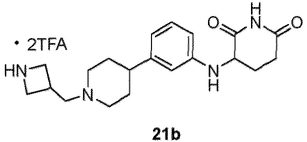 | (±)-3-((3-(1-(Azetidin-3-ylmethyl)piperidin-4-yl)phenyl)amino)piperidine-2,6-dione bistrifluoroacetate **21b** |
| | 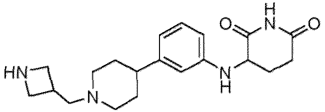 | (±)-3-((3-(1-(Azetidin-3-ylmethyl)piperidin-4-yl)phenyl)amino)piperidine-2,6-dione |
| | 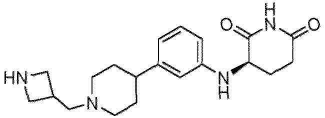 | (*R*)-3-((3-(1-(Azetidin-3-ylmethyl)piperidin-4-yl)phenyl)amino)piperidine-2,6-dione |
| | 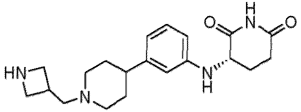 | (*S*)-3-((3-(1-(Azetidin-3-ylmethyl)piperidin-4-yl)phenyl)amino)piperidine-2,6-dione |
| **23d** | 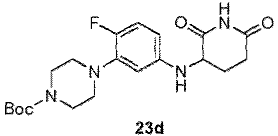 | tert-Butyl (±)-4-(5-((2,6-dioxopiperidin-3-yl)amino)-2-fluorophenyl)piperazine-1- carboxylate **23d** |
| **23e** | 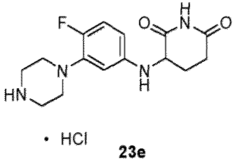 | (±)-3-((4-Fluoro-3-(piperazin-1-yl)phenyl)amino)piperidine-2,6-dione hydrochloride **23e** |
| | 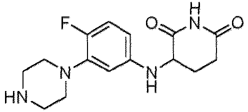 | (±)-3-((4-Fluoro-3-(piperazin-1-yl)phenyl)amino)piperidine-2,6-dione |
| | 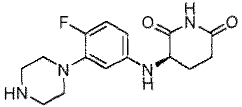 | (*R*)-3-((4-Fluoro-3-(piperazin-1-yl)phenyl)amino)piperidine-2,6-dione |
| | 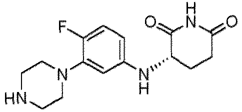 | (*S*)-3-((4-Fluoro-3-(piperazin-1-yl)phenyl)amino)piperidine-2,6-dione |
| **23f** | 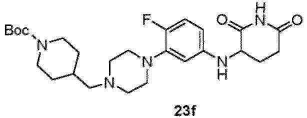 | tert-Butyl (±)-4-((4-(5-((2,6-dioxopiperidin-3 -yl)amino)-2-fluorophenyl)piperazin-1- yl)methyl)piperidine-1-carboxylate **23f** |
| **23g** | 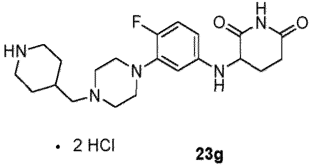 | (±)-3-((4-Fluoro-3-(4-(piperidin-4-ylmethyl)piperazin-1-yl)phenyl)amino)piperidine-2,6-dione dihydrochloride **23g** |
| | 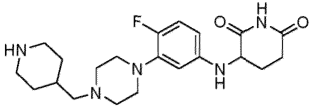 | (±)-3 -((4-Fluoro-3-(4-(piperidin-4-ylmethyl)piperazin-1-yl)phenyl)amino)piperidine-2,6-dione |
| | 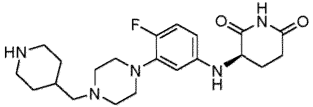 | (*R*)-3-((4-Fluoro-3-(4-(piperidin-4-ylmethyl)piperazin-1-yl)phenyl)amino)piperidine-2,6-dione |
| | 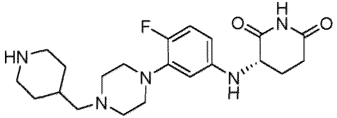 | (*S*)-3-((4-Fluoro-3-(4-(piperidin-4-ylmethyl)piperazin-1-yl)phenyl)amino)piperidine-2,6-dione |
| **24f** | 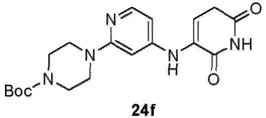 | tert-Butyl 4-(4-((2,6-dioxo-1,2,5,6-tetrahydropyridin-3-yl)amino)pyridin-2-yl)piperazine-1-carboxylate **24f** |
| **24g** | 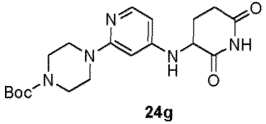 | tert-Butyl (±)-4-(4-((2,6-dioxopiperidin-3-yl)amino)pyridin-2-yl)piperazine-1-carboxylate **24g** |
| **24h** | 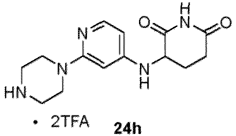 | (±)-3-((2-(Piperazin-1-yl)pyridin-4-yl)amino)piperidine-2,6-dione bistrifluoroacetate **24h** |
| | 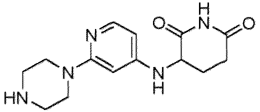 | (±)-3-((2-(Piperazin-1-yl)pyridin-4-yl)amino)piperidine-2,6-dione |
| | 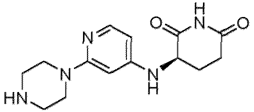 | (*R*)-3-((2-(Piperazin-1-yl)pyridin-4-yl)amino)piperidine-2,6-dione |
| | 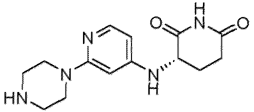 | (*S*)-3-((2-(Piperazin-1-yl)pyridin-4-yl)amino)piperidine-2,6-dione |
| **26b** | 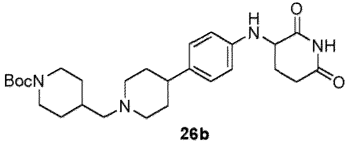 | tert-Butyl (±)-4-((4-(4-((2,6-dioxopiperidin-3-yl)amino)phenyl)piperidin-1-yl)methyl)piperidine-1-carboxylate **26b** |
| **26c** | 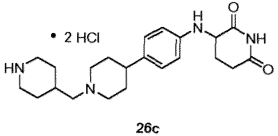 | (±)-3-((4-(1-(Piperidin-4-ylmethyl)piperidin-4-yl)phenyl)amino)piperidine-2,6-dione dihydrochloride **26c** |
| | 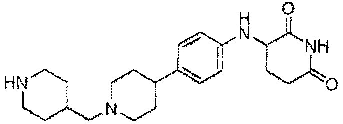 | (±)-3-((4-(1-(Piperidin-4-ylmethyl)piperidin-4-yl)phenyl)amino)piperidine-2,6-dione |
| | 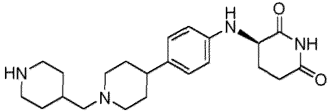 | (*R*)-3-((4-(1-(Piperidin-4-ylmethyl)piperidin-4-yl)phenyl)amino)piperidine-2,6-dione |
| | 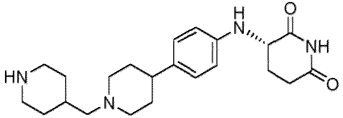 | (*S*)-3-((4-(1-(Piperidin-4-ylmethyl)piperidin-4-yl)phenyl)amino)piperidine-2,6-dione |
| **27b** | 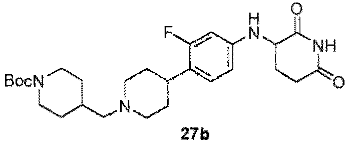 | tert-Butyl (±)-4-((4-(4-((2,6-dioxopiperidin-3-yl)amino)-2-fluorophenyl)piperidin-1-yl)methyl)piperidine-1-carboxylate **27b** |
| **27c** | 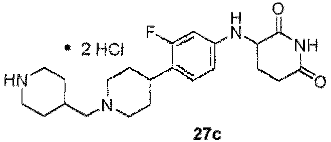 | (±)-3-((3-Fluoro-4-(1-(piperidin-4-ylmethyl)piperidin-4-yl)phenyl)amino)piperidine-2,6-dione dihydrochloride **27c** |
| | 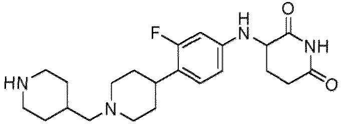 | (±)-3-((3-Fluoro-4-(1-(piperidin-4-ylmethyl)piperidin-4-yl)phenyl)amino)piperidine-2,6-dione |
| | 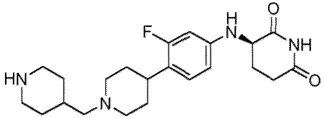 | (R)-3-((3-Fluoro-4-(1-(piperidin-4-ylmethyl)piperidin-4-yl)phenyl)amino)piperidine-2,6-dione |
| | 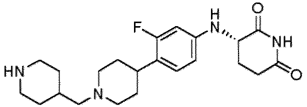 | (*S*)-3-((3-Fluoro-4-(1-(piperidin-4-ylmethyl)piperidin-4-yl)phenyl)amino)piperidine-2,6-dione |
| **28c** | 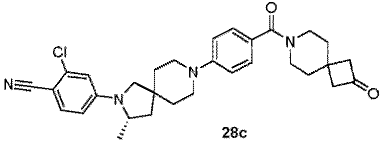 | (*S*)-2-Chloro-4-(3 -methyl-8-(4-(2-oxo-7-azaspiro[3.5]nonane-7-carbonyl)phenyl)-2,8-diazaspiro[4.5]decan-2-yl)benzonitrile **28c** |
| | 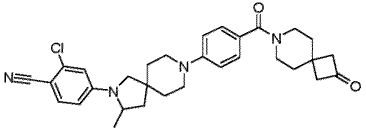 | 2-Chloro-4-(3 -methyl-8-(4-(2-oxo-7-azaspiro[3.5]nonane-7-carbonyl)phenyl)-2,8-diazaspiro[4.5]decan-2-yl)benzonitrile |
| | 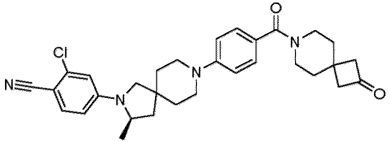 | (*R*)-2-Chloro-4-(3-methyl-8-(4-(2-oxo-7-azaspiro[3.5]nonane-7-carbonyl)phenyl)-2,8-diazaspiro[4.5]decan-2-yl)benzonitrile |
| **29b** | 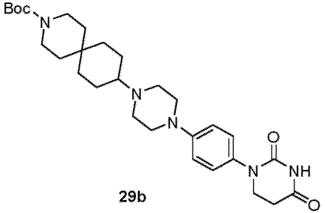 | tert-Butyl 9-(4-(4-(2,4-dioxotetrahydropyrimidin-1(2*H*)-yl)phenyl)piperazin-1-yl)-3-azaspiro[5.5]undecane-3-carboxylate **29b** |
| **29c** | 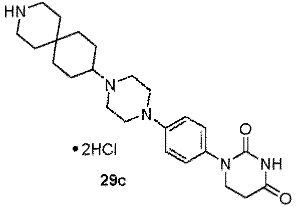 | 1-(4-(4-(3-Azaspiro[5.5]undecan-9-yl)piperazin-1-yl)phenyl)dihydropyrimidine-2,4(1*H*,3*H-*dione dihydrochloride **29c** |
| | 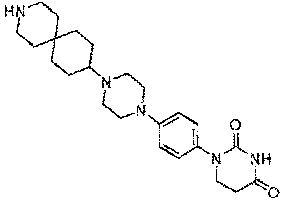 | 1-(4-(4-(3-Azaspiro[5.5]undecan-9-yl)piperazin-1-yl)pheiryl)dihydropyrimidine-2,4(1*H*,3*H-*dione |
| | 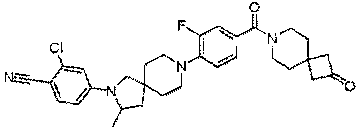 | 2-Chloro-4-(8-(2-fluoro-4-(2-oxo-7-azaspiro[3.5]nonane-7-carbonyl)phenyl)-3-methyl-2,8-diazaspiro [4.5] decan-2-yl)benzonitrile |
| **30a** | 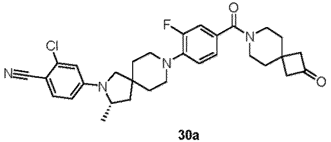 | (S)-2-Chloro-4-(8-(2-fluoro-4-(2-oxo-7-azaspiro[3.5]nonane-7-carbonyl)phenyl)-3-methyl-2,8-diazaspiro[4.5]decan-2-yl)benzonitrile **30a** |
| | 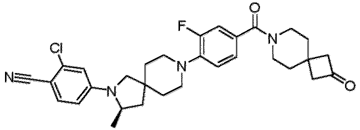 | (*R*)-2-Chloro-4-(8-(2-fluoro-4-(2-oxo-7-azaspiro[3.5]nonane-7-carbonyl)phenyl)-3-methyl-2,8-diazaspiro[4.5]decan-2-yl)benzonitrile **30a** |
| **31a** | 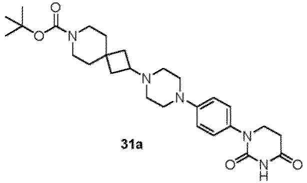 | tert-Butyl 2-(4-(4-(2,4-dioxotetrahydropyrimidin-1(2*H*)-yl)phenyl)piperazin-1-yl)-7-azaspiro[3.5]nonane-7-carboxylate **31a** |
| **31b** | 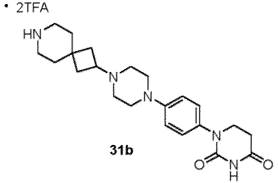 | 1-(4-(4-(7-Azaspiro[3.5]nonan-2-yl)piperazin-1-yl)phenyl)dihydropyrimidine-2,4(1*H*,3*H*)-dione bistrifluoroacetate **31b** |
| | 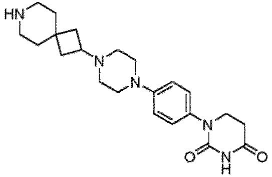 | 1-(4-(4-(7-Azaspiro[3.5]nonan-2-yl)piperazin-1-yl)phenyl)dihydropyrimidine-2,4(1*H*,3*H*)-dione |
| | 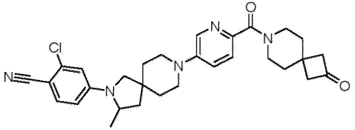 | 2-Chloro-4-(3 -methyl-8-(6-(2-oxo-7-azaspiro[3.5]nonane-7-carboiryl)pyridin-3-yl)-2,8-diazaspiro[4.5]decan-2-yl)benzonitrile |
| **32a** | 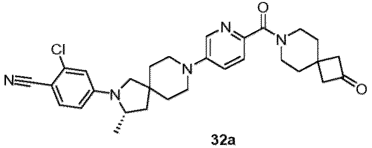 | (*S*)-2-Chloro-4-(3-methyl-8-(6-(2-oxo-7-azaspiro[3.5]nonane-7-carboiryl)pyridin-3-yl)-2,8-diazaspiro[4.5]decan-2-yl)benzonitrile **32a** |
| | 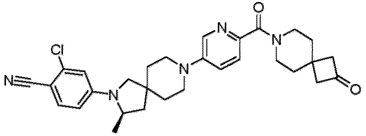 | (*R*)-2-Chloro-4-(3-methyl-8-(6-(2-oxo-7-azaspiro[3.5]nonane-7-carboiryl)pyridin-3-yl)-2,8-diazaspiro[4.5]decan-2-yl)benzonitrile |
| **35c** | 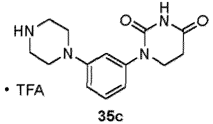 | 1-(3-(Piperazin-1-yl)phenyl)dihydropyrimidine-2,4(1*H*,3*H*)-dione trifluoroacetate **35c** |
| | 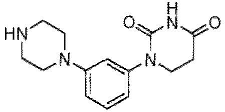 | 1-(3-(Piperazin-1-yl)phenyl)dihydropyrimidine-2,4(1*H*,3*H*)-dione |
| **39d** | 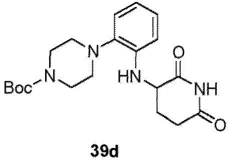 | tert-Butyl (±)-4-(2-((2,6-dioxopiperidin-3-yl)amino)phenyl)piperazine-1-carboxylate **39d** |
| **39e** | 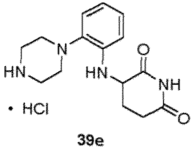 | (±)-3-((2-(Piperazin-1-yl)phenyl)amino)piperidine-2,6-dione hydrochloride 39e |
| | 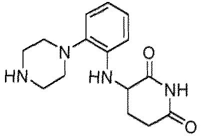 | (±)-3-((2-(Piperazin-1-yl)phenyl)amino)piperidine-2,6-dione |
| | 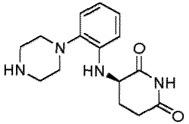 | (*R*)-3-((2-(Piperazin-1-yl)phenyl)amino)piperidine-2,6-dione |
| | 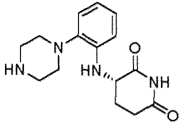 | (*S*)-3-((2-(Piperazin-1-yl)phenyl)amino)piperidine-2,6-dione |
| **40e** | 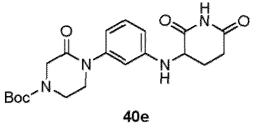 | tert-Butyl (±)-4-(3-((2,6-dioxopiperidin-3-yl)amino)phenyl)-3-oxopiperazine-1-carboxylate **40e** |
| **40f** | 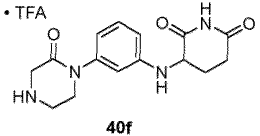 | (±)-3-((3-(2-Oxopiperazin-1-yl)phenyl)amino)piperidine-2,6-dione trifluoroacetate **40f** |
| | 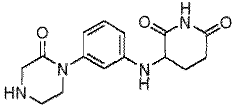 | (±)-3-((3-(2-Oxopiperazin-1-yl)phenyl)amino)piperidine-2,6-dione |
| | 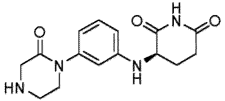 | (*R*)-3-((3-(2-Oxopiperazin-1-yl)phenyl)amino)piperidine-2,6-dione |
| | 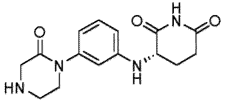 | (*S*)-3-((3-(2-Oxopiperazin-1-yl)phenyl)amino)piperidine-2,6-dione |

Another aspect of the present disclosure relates to a method for preparing a compound represented by general formula (III') or a pharmaceutically acceptable salt thereof, comprising: 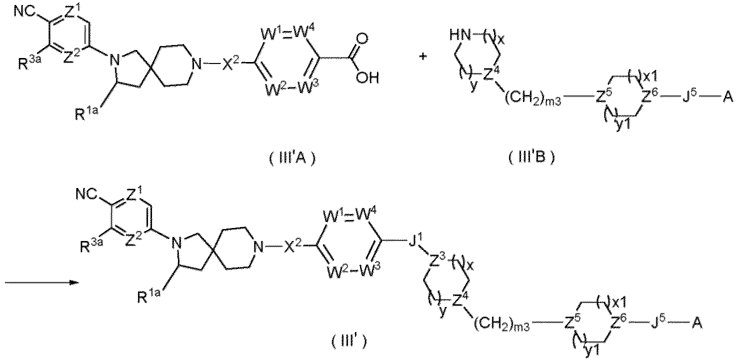 conducting a condensation reaction of a compound represented by general formula (III'A) or a salt thereof with a compound represented by general formula (III'B) or a salt thereof (preferably hydrochloride and trifluoroacetate) to give the compound represented by general formula (III') or the pharmaceutically acceptable salt thereof; wherein:
J¹ is -C(O)-;
Z³ is N;
A, R^{1a}, R^{3a}, J⁵, X², W¹, W², W³, W⁴, Z¹, Z², Z⁴, Z⁵, Z⁶, x, x1, y, y1, and m3 are as defined in general formula (III').

Another aspect of the present disclosure relates to a method for preparing a compound represented by general formula (III') or a pharmaceutically acceptable salt thereof, comprising: 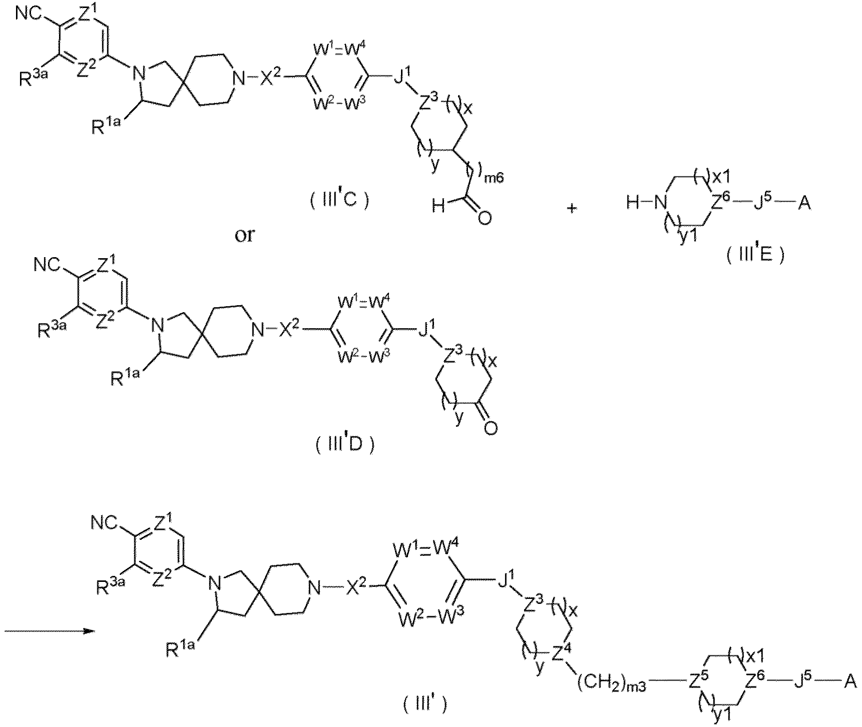
conducting a reductive amination reaction of a compound represented by general formula (III'C) or a salt thereof with a compound represented by general formula (III'E) or a salt thereof (preferably hydrochloride and trifluoroacetate) to give the compound represented by general formula (III') or the pharmaceutically acceptable salt thereof, where m3 is 1, 2, or 3;

or, conducting a reductive amination reaction of a compound represented by general formula (III'D) or a salt thereof with a compound represented by general formula (III'E) or a salt thereof (preferably hydrochloride and trifluoroacetate) to give the compound represented by general formula (III') or the pharmaceutically acceptable salt thereof, where m3 is 0;
wherein:
   m6 is 0, 1, or 2;
   Z⁴ is CH;
   Z⁵ is N;
   A, J¹, J⁵, X², R^{1a}, R^{3a}, W¹, W², W³, W⁴, Z¹, Z², Z³, Z⁶, x, x1, y, and y1 are as defined in general formula (III').

Another aspect of the present disclosure relates to a method for preparing a compound represented by general formula (III') or a pharmaceutically acceptable salt thereof, comprising: 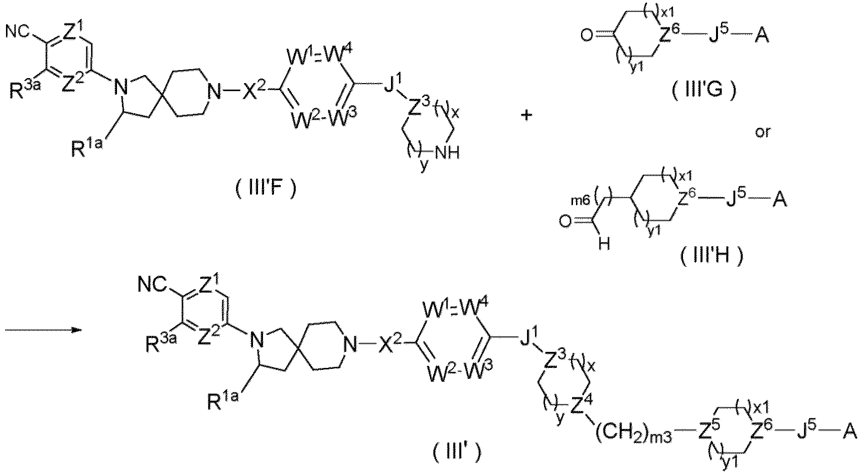 conducting a reductive amination reaction of a compound represented by general formula (III'F) or a salt thereof (preferably hydrochloride and trifluoroacetate) with a compound represented by general formula (III'G) or a salt thereof to give the compound represented by general formula (III') or the pharmaceutically acceptable salt thereof, where m3 is 0; or, conducting a reductive amination reaction of a compound represented by general formula (III'F) or a salt thereof (preferably hydrochloride and trifluoroacetate) with a compound represented by general formula (III'H) or a salt thereof to give the compound represented by general formula (III') or the pharmaceutically acceptable salt thereof, where m3 is 1, 2, or 3;
wherein:
m6 is 0, 1, or 2;
Z⁴ is N;
Z⁵ is CH;
A, J¹, J⁵, X², R^{1a}, R^{3a}, W¹, W², W³, W⁴, Z¹, Z², Z³, Z⁶, x, x1, y, and y1 are as defined in general formula (III').

Another aspect of the present disclosure relates to a method for preparing a compound represented by general formula (X) or a pharmaceutically acceptable salt thereof, comprising the following step: 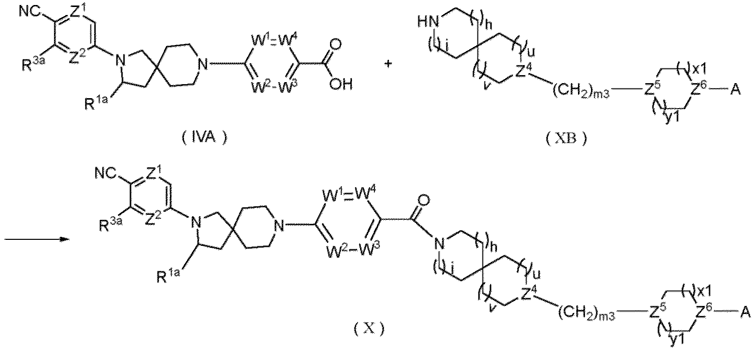 conducting a condensation reaction of a compound represented by general formula (IVA) or a salt thereof with a compound represented by general formula (XB) or a salt thereof (preferably hydrochloride and trifluoroacetate) to give the compound represented by general formula (X) or the pharmaceutically acceptable salt thereof;
wherein:
R^{1a}, R^{3a}, W¹, W², W³, W⁴, Z¹, Z², Z⁴, Z⁵, Z⁶, h, i, u, v, x1, y1, m3, and A are as defined in general formula (X).

Another aspect of the present disclosure relates to a method for preparing a compound represented by general formula (X-1) or a pharmaceutically acceptable salt thereof, comprising the following step: 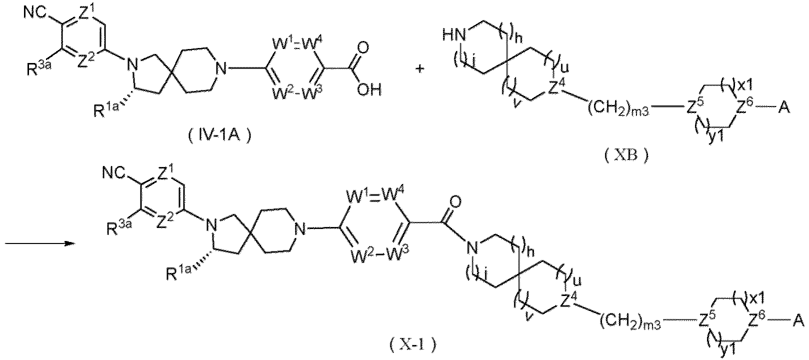 conducting a condensation reaction of a compound represented by general formula (IV-1A) or a salt thereof with a compound represented by general formula (XB) or a salt thereof (preferably hydrochloride and trifluoroacetate) to give the compound represented by general formula (X-1) or the pharmaceutically acceptable salt thereof;
wherein:
R^{1a}, R^{3a}, W¹, W², W³, W⁴, Z¹, Z², Z⁴, Z⁵, Z⁶, h, i, u, v, x1, y1, m3, and A are as defined in general formula (X-1).

Another aspect of the present disclosure relates to a method for preparing a compound represented by general formula (X) or a pharmaceutically acceptable salt thereof, comprising the following step: 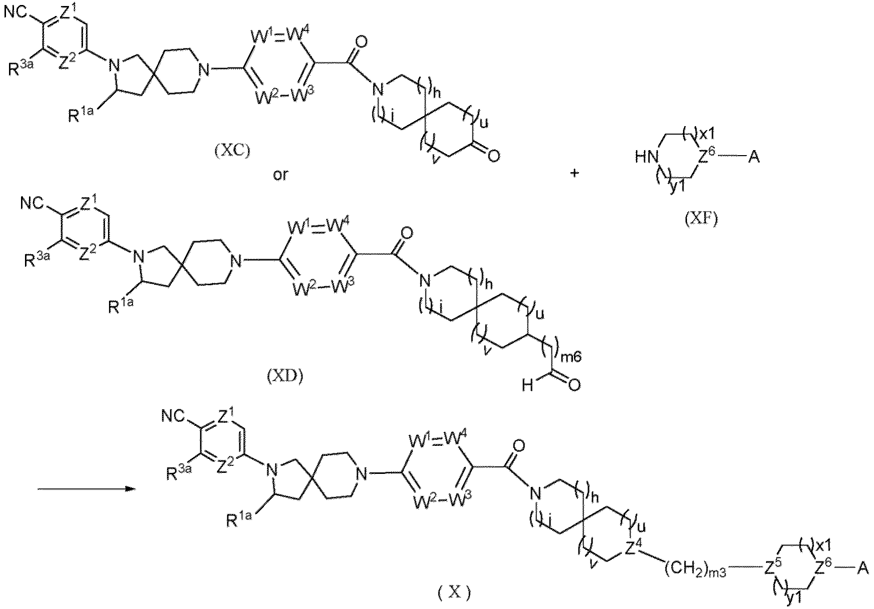 conducting a reductive amination reaction of a compound represented by general formula (XC) or a salt thereof with a compound represented by general formula (XF) or a salt thereof (preferably hydrochloride and trifluoroacetate) to give the compound represented by general formula (X) or the pharmaceutically acceptable salt thereof, where m3 is 0; or, conducting a reductive amination reaction of a compound represented by general formula (XD) or a salt thereof with a compound represented by general formula (XF) or a salt thereof (preferably hydrochloride and trifluoroacetate) to give the compound represented by general formula (X) or the pharmaceutically acceptable salt thereof, where m3 is 1, 2, or 3;
wherein:
m6 is 0, 1, or 2;
Z⁴ is CH;
Z⁵ is N;
R^{1a}, R^{3a}, W¹, W², W³, W⁴, Z¹, Z², Z⁶, h, i, u, v, x1, y1, and A are as defined in general formula (X).

Another aspect of the present disclosure relates to a method for preparing a compound represented by general formula (X-1) or a pharmaceutically acceptable salt thereof, comprising the following step: 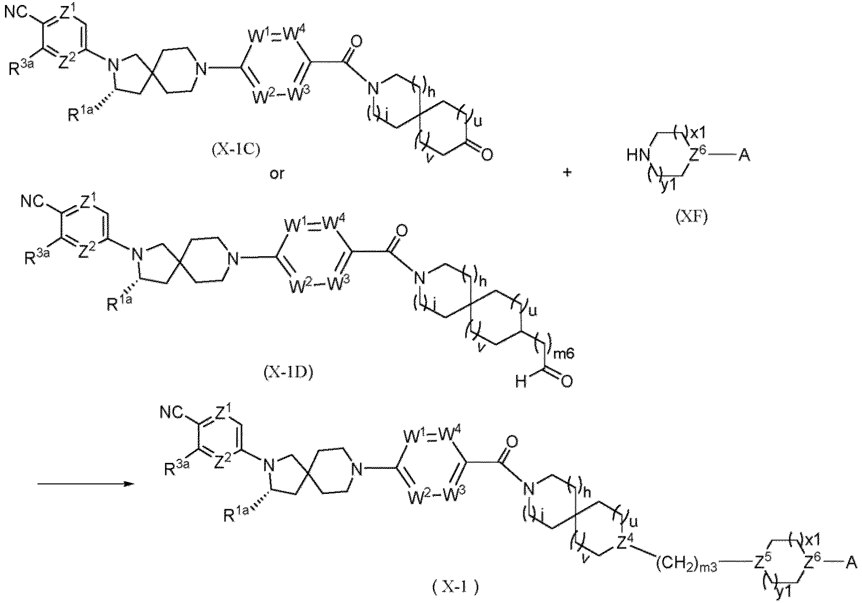 conducting a reductive amination reaction of a compound represented by general formula (X-1C) or a salt thereof with a compound represented by general formula (XF) or a salt thereof (preferably hydrochloride and trifluoroacetate) to give the compound represented by general formula (X-1) or the pharmaceutically acceptable salt thereof, where m3 is 0; or, conducting a reductive amination reaction of a compound represented by general formula (X-1D) or a salt thereof with a compound represented by general formula (XF) or a salt thereof (preferably hydrochloride and trifluoroacetate) to give the compound represented by general formula (X-1) or the pharmaceutically acceptable salt thereof, where m3 is 1, 2, or 3;
wherein:
m6 is 0, 1, or 2;
Z⁴ is CH;
Z⁵ is N;
R^{1a}, R^{3a}, W¹, W², W³, W⁴, Z¹, Z², Z⁶, h, i, u, v, x1, y1, and A are as defined in general formula (X-1).

Another aspect of the present disclosure relates to a method for preparing a compound represented by general formula (IV') or a pharmaceutically acceptable salt thereof, comprising: 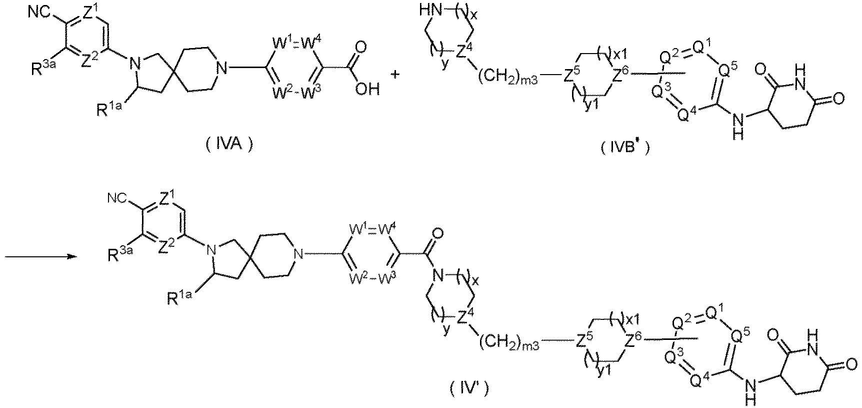 conducting a condensation reaction of a compound represented by general formula (IVA) or a salt thereof with a compound represented by general formula (IVB') or a salt thereof (preferably hydrochloride and trifluoroacetate) to give the compound represented by general formula (IV') or the pharmaceutically acceptable salt thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in general formula (IV').

Another aspect of the present disclosure relates to a method for preparing a compound represented by general formula (IV'-1) or a pharmaceutically acceptable salt thereof, comprising: 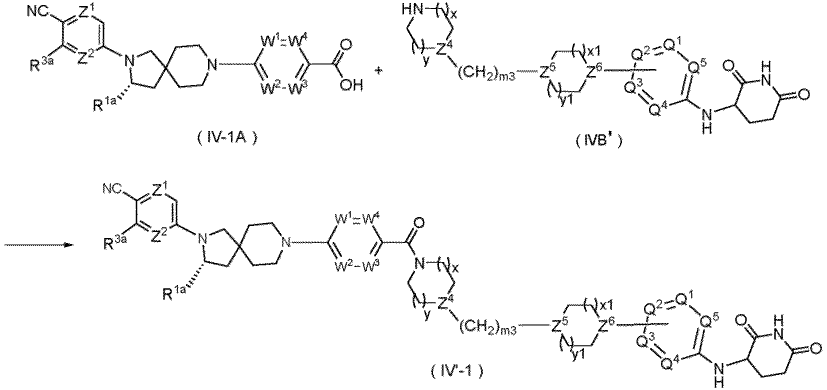 conducting a condensation reaction of a compound represented by general formula (IV-1A) or a salt thereof with a compound represented by general formula (IVB') or a salt thereof (preferably hydrochloride and trifluoroacetate) to give the compound represented by general formula (IV'-1) or the pharmaceutically acceptable salt thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in general formula (IV'-1).

Another aspect of the present disclosure relates to a method for preparing compounds represented by general formula (IV-1-1) and general formula (IV'-1-2) or pharmaceutically acceptable salts thereof, comprising: 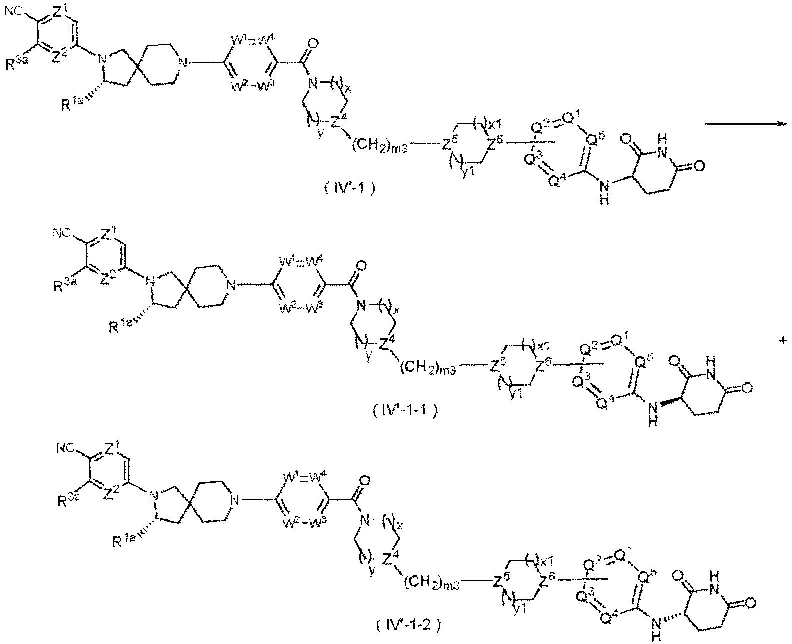 chirally resolving a compound represented by general formula (IV'-1) or a pharmaceutically acceptable salt thereof to give the compounds represented by general formula (IV'-1-1) and general formula (IV'-1-2) or the pharmaceutically acceptable salts thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in general formula (IV'-1).

Another aspect of the present disclosure relates to a method for preparing a compound represented by general formula (M) or a pharmaceutically acceptable salt thereof, comprising: 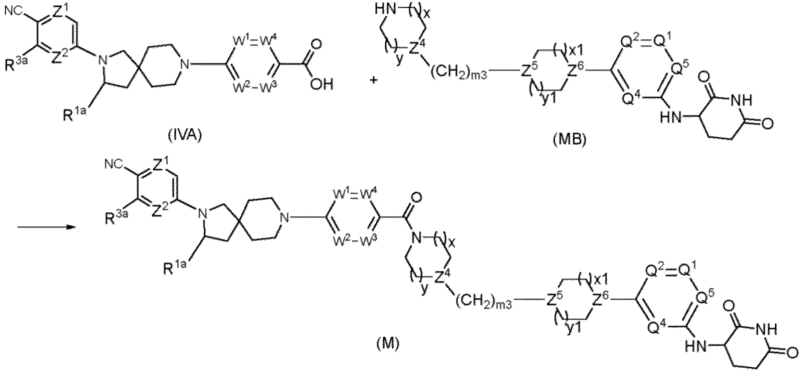 conducting a condensation reaction of a compound represented by general formula (IVA) or a salt thereof with a compound represented by general formula (MB) or a salt thereof (preferably hydrochloride and trifluoroacetate) to give the compound represented by general formula (M) or the pharmaceutically acceptable salt thereof;
wherein:
R^{1a}, Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M).

Another aspect of the present disclosure relates to a method for preparing a compound represented by general formula (M-1) or a pharmaceutically acceptable salt thereof, comprising: 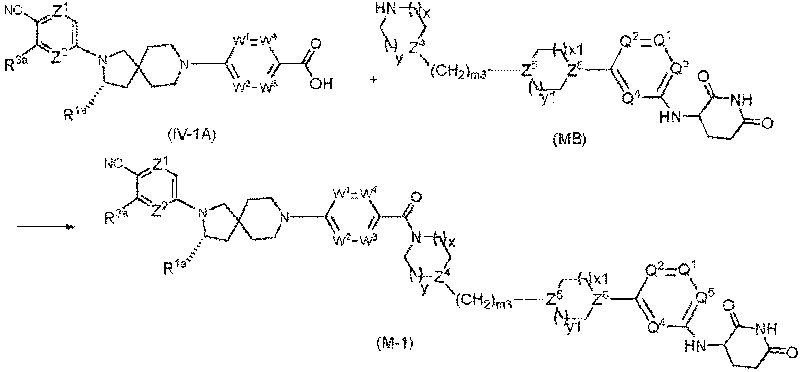 conducting a condensation reaction of a compound represented by general formula (IV-1A) or a salt thereof with a compound represented by general formula (MB) or a salt thereof (preferably hydrochloride and trifluoroacetate) to give the compound represented by general formula (M-1) or the pharmaceutically acceptable salt thereof;
wherein:
R^{1a}, Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M-1).

Another aspect of the present disclosure relates to a method for preparing compounds represented by general formula (M-1-1) and general formula (M-1-2) or pharmaceutically acceptable salts thereof, comprising: 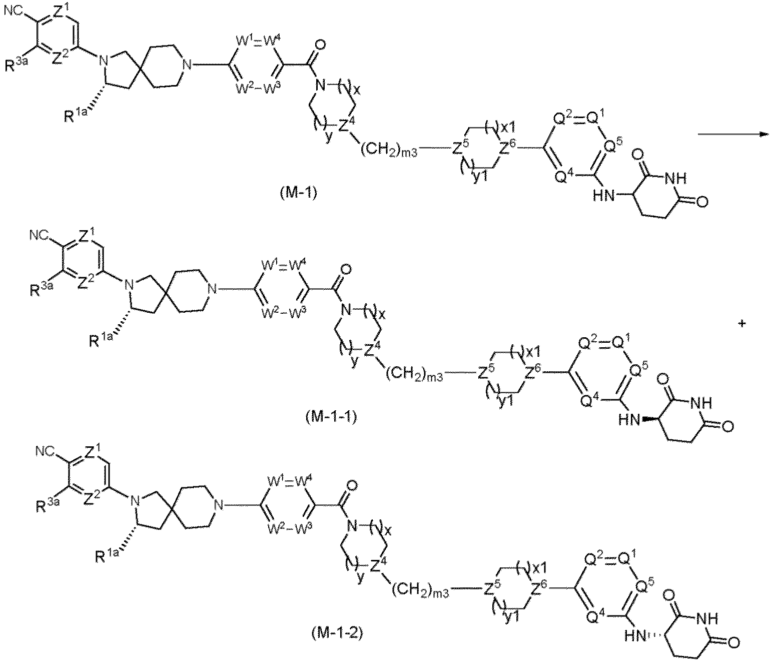 chirally resolving a compound represented by general formula (M-1) or a pharmaceutically acceptable salt thereof to give the compounds represented by general formula (M-1-1) and general formula (M-1-2) or the pharmaceutically acceptable salts thereof;
wherein:
R^{1a}, Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M-1).

Another aspect of the present disclosure relates to a method for preparing a compound represented by general formula (IV) or a pharmaceutically acceptable salt thereof, comprising: 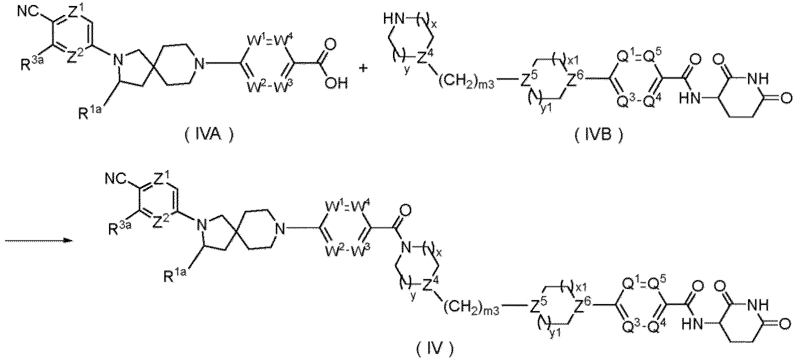 conducting a condensation reaction of a compound represented by general formula (IVA) or a salt thereof with a compound represented by general formula (IVB) or a salt thereof (preferably hydrochloride and trifluoroacetate) to give the compound represented by general formula (IV) or the pharmaceutically acceptable salt thereof;
wherein:
R^{1a}, Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV).

Another aspect of the present disclosure relates to a method for preparing a compound represented by general formula (IV-1) or a pharmaceutically acceptable salt thereof, comprising: 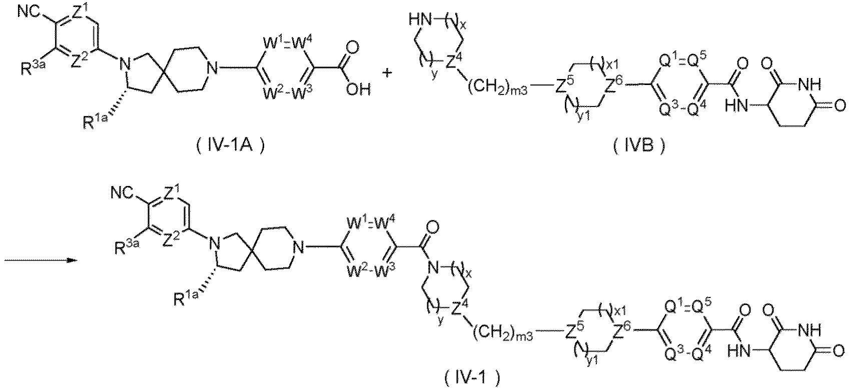 conducting a condensation reaction of a compound represented by general formula (IV-1A) or a salt thereof with a compound represented by general formula (IVB) or a salt thereof (preferably hydrochloride and trifluoroacetate) to give the compound represented by general formula (IV-1) or the pharmaceutically acceptable salt thereof;
wherein:
R^{1a}, Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV-1).

Another aspect of the present disclosure relates to a method for preparing compounds represented by general formula (IV-1-1) and general formula (IV-1-2) or pharmaceutically acceptable salts thereof, comprising: 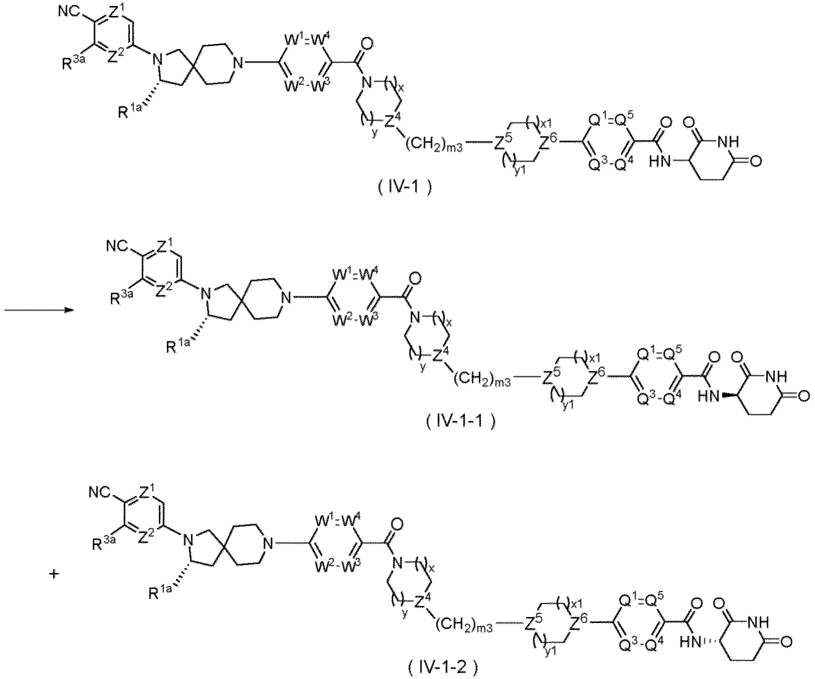 chirally resolving a compound represented by general formula (IV-1) or a pharmaceutically acceptable salt thereof to give the compounds represented by general formula (IV-1-1) and general formula (IV-1-2) or the pharmaceutically acceptable salts thereof;
wherein:
R^{1a}, Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV-1).

Another aspect of the present disclosure relates to a method for preparing a compound represented by general formula (V) or a pharmaceutically acceptable salt thereof, comprising: 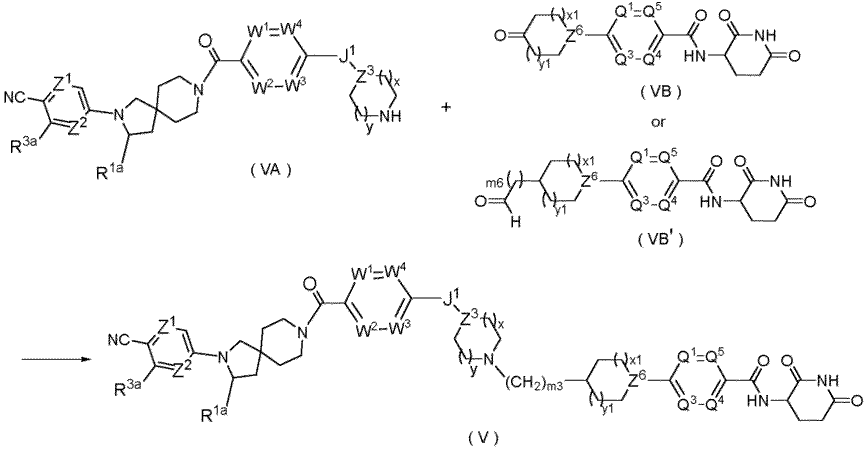 conducting a reductive amination reaction of a compound represented by general formula (VA) or a salt thereof (preferably hydrochloride and trifluoroacetate) with a compound represented by general formula (VB) or a salt thereof to give the compound represented by general formula (V) or the pharmaceutically acceptable salt thereof, where m3 is 0; or, conducting a reductive amination reaction of a compound represented by general formula (VA) or a salt thereof (preferably hydrochloride and trifluoroacetate) with a compound represented by general formula (VB') or a salt thereof to give the compound represented by general formula (V) or the pharmaceutically acceptable salt thereof, where m3 is 1, 2, or 3;
wherein:
m6 is 0, 1, or 2;
J¹, R^{1a}, Q¹, Q³, Q⁴, Q⁵, R^{3a}, W¹, W², W³, W⁴, Z¹, Z², Z³, Z⁶, x, x1, y, and y1 are as defined in general formula (V).

Another aspect of the present disclosure relates to a method for preparing a compound represented by general formula (V-1) or a pharmaceutically acceptable salt thereof, comprising: 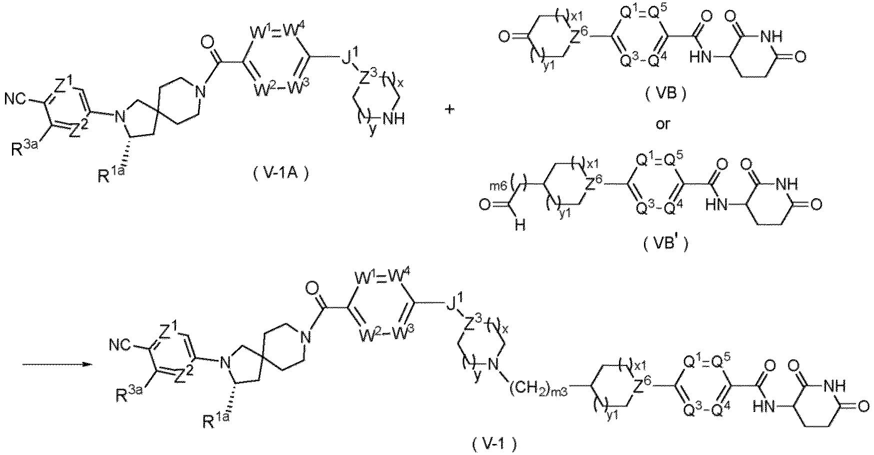 conducting a reductive amination reaction of a compound represented by general formula (V-1A) or a salt thereof (preferably hydrochloride and trifluoroacetate) with a compound represented by general formula (VB) or a salt thereof to give the compound represented by general formula (V-1) or the pharmaceutically acceptable salt thereof, where m3 is 0; or, conducting a reductive amination reaction of a compound represented by general formula (V-1A) or a salt thereof (preferably hydrochloride and trifluoroacetate) with a compound represented by general formula (VB') or a salt thereof to give the compound represented by general formula (V-1) or the pharmaceutically acceptable salt thereof, where m3 is 1, 2, or 3;
wherein:
m6 is 0, 1, or 2;
J¹, R^{1a}, Q¹, Q³, Q⁴, Q⁵, R^{3a}, W¹, W², W³, W⁴, Z¹, Z², Z³, Z⁶, x, x1, y, and y1 are as defined in general formula (V-1).

Another aspect of the present disclosure relates to a method for preparing compounds represented by general formula (V-1-1) and general formula (V-1-2) or pharmaceutically acceptable salts thereof, comprising: 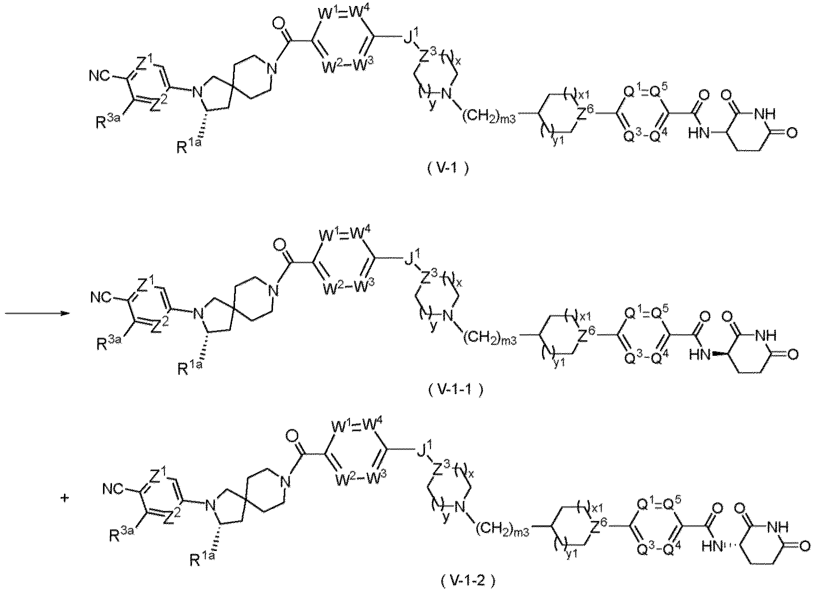 chirally resolving a compound represented by general formula (V-1) or a pharmaceutically acceptable salt thereof to give the compounds represented by general formula (V-1-1) and general formula (V-1-2) or the pharmaceutically acceptable salts thereof;
wherein:
J¹, R^{1a}, Q¹, Q³, Q⁴, Q⁵, R^{3a}, W¹, W², W³, W⁴, Z¹, Z², Z³, Z⁶, x, x1, y, y1, and m3 are as defined in general formula (V-1).

Another aspect of the present disclosure relates to a method for preparing a compound represented by general formula (VI) or a pharmaceutically acceptable salt thereof, comprising: 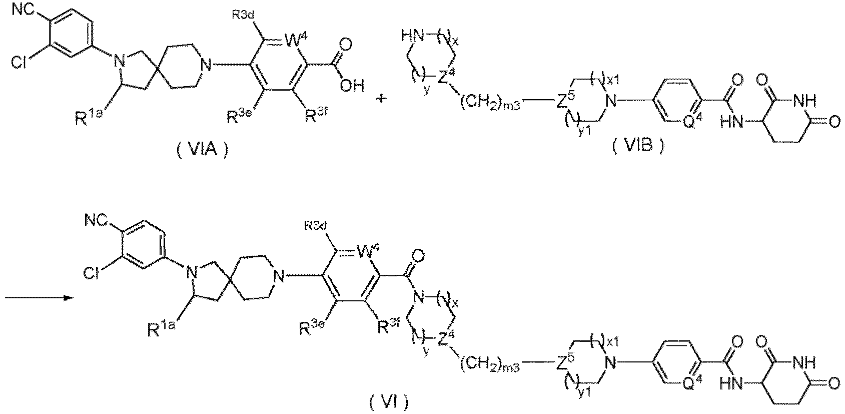 conducting a condensation reaction of a compound represented by general formula (VIA) or a salt thereof with a compound represented by general formula (VIB) or a salt thereof (preferably hydrochloride and trifluoroacetate) to give the compound represented by general formula (VI) or the pharmaceutically acceptable salt thereof;
wherein:
R^{1a}, R^{3d}, R^{3e}, R^{3f}, W⁴, Z⁴, Z⁵, x, x1, y, y1, m3, and Q⁴ are as defined in general formula (VI).

Another aspect of the present disclosure relates to a method for preparing a compound represented by general formula (VI-1) or a pharmaceutically acceptable salt thereof, comprising: 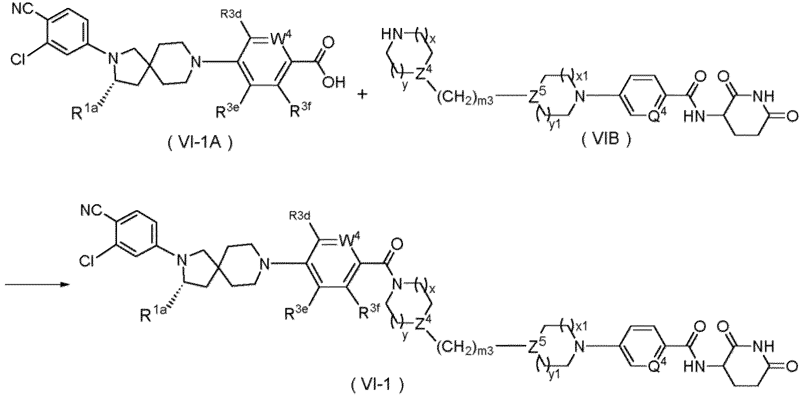 conducting a condensation reaction of a compound represented by general formula (VI-1A) or a salt thereof with a compound represented by general formula (VIB) or a salt thereof (preferably hydrochloride and trifluoroacetate) to give the compound represented by general formula (VI-1) or the pharmaceutically acceptable salt thereof;
wherein:
R^{1a}, R^{3d}, R^{3e}, R^{3f}, W⁴, Z⁴, Z⁵, x, x1, y, y1, m3, and Q⁴ are as defined in general formula (VI-1).

Another aspect of the present disclosure relates to a method for preparing compounds represented by general formula (VI-1-1) and general formula (VI-1-2) or pharmaceutically acceptable salts thereof, comprising: 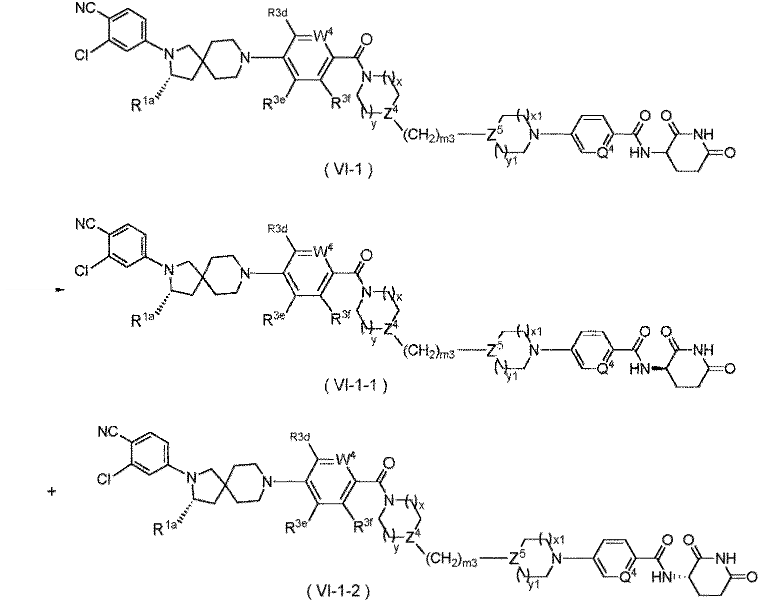 chirally resolving a compound represented by general formula (VI-1) or a pharmaceutically acceptable salt thereof to give the compounds represented by general formula (VI-1-1) and general formula (VI-1-2) or the pharmaceutically acceptable salts thereof;
wherein:
R^{1a}, R^{3d}, R^{3e}, R^{3f}, W⁴, Z⁴, Z⁵, x, x1, y, y1, m3, and Q⁴ are as defined in general formula (VI-1).

Another aspect of the present disclosure relates to a method for preparing a compound represented by general formula (VII) or a pharmaceutically acceptable salt thereof, comprising: 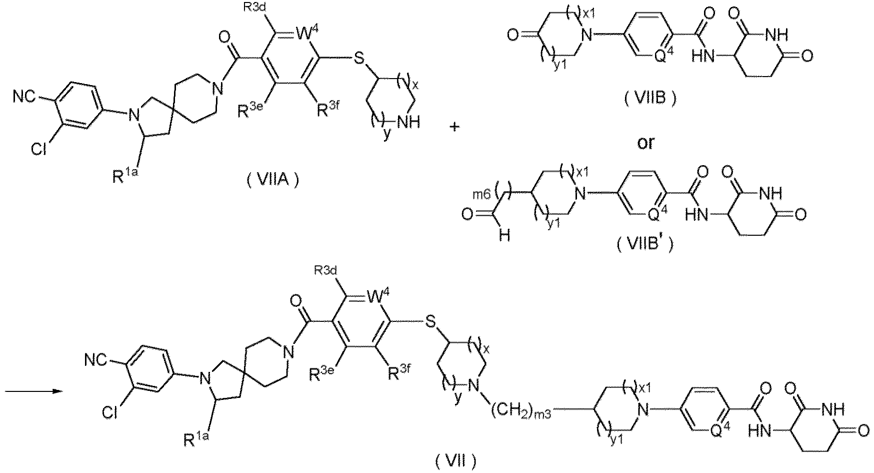 conducting a reductive amination reaction of a compound represented by general formula (VIIA) or a salt thereof (preferably hydrochloride and trifluoroacetate) with a compound represented by general formula (VIIB) or a salt thereof to give the compound represented by general formula (VII) or the pharmaceutically acceptable salt thereof, where m3 is 0; or, conducting a reductive amination reaction of a compound represented by general formula (VIIA) or a salt thereof (preferably hydrochloride and trifluoroacetate) with a compound represented by general formula (VIIB') or a salt thereof to give the compound represented by general formula (VII) or the pharmaceutically acceptable salt thereof, where m3 is 1, 2, or 3;
wherein:
m6 is 0, 1, or 2;
R^{1a}, Q⁴, R^{3d}, R^{3e}, R^{3f}, W⁴, x, x1, y, and y1 are as defined in general formula (VII).

Another aspect of the present disclosure relates to a method for preparing a compound represented by general formula (VII-1) or a pharmaceutically acceptable salt thereof, comprising: 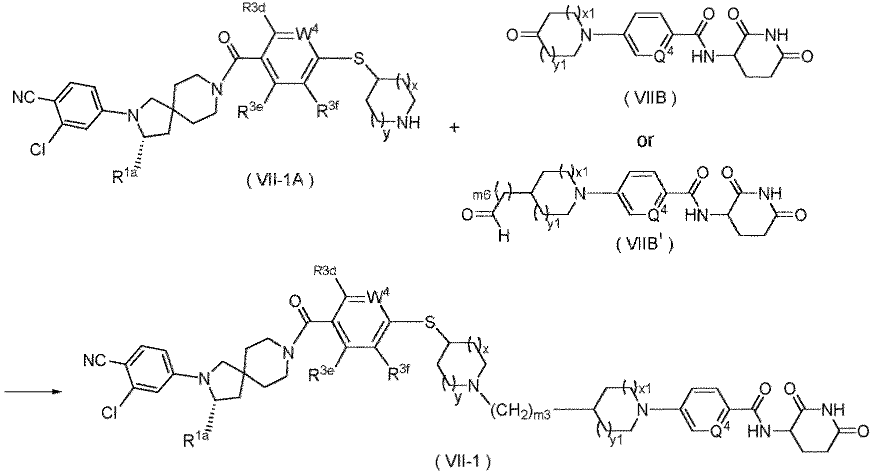
conducting a reductive amination reaction of a compound represented by general formula (VII-1A) or a salt thereof (preferably hydrochloride and trifluoroacetate) with a compound represented by general formula (VIIB) or a salt thereof to give the compound represented by general formula (VII-1) or the pharmaceutically acceptable salt thereof, where m3 is 0;
or, conducting a reductive amination reaction of a compound represented by general formula (VII-1A) or a salt thereof (preferably hydrochloride and trifluoroacetate) with a compound represented by general formula (VIIB') or a salt thereof to give the compound represented by general formula (VII-1) or the pharmaceutically acceptable salt thereof, where m3 is 1, 2, or 3;
wherein:
   m6 is 0, 1, or 2;
   R^{1a}, Q⁴, R^{3d}, R^{3e}, R^{3f}, W⁴, x, x1, y, and y1 are as defined in general formula (VII-1).

Another aspect of the present disclosure relates to a method for preparing compounds represented by general formula (VII-1-1) and general formula (VII-1-2) or pharmaceutically acceptable salts thereof, comprising: 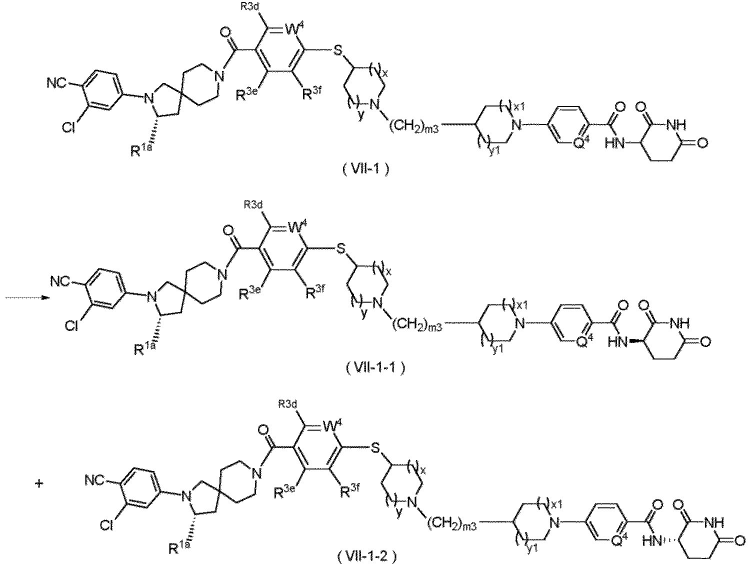 chirally resolving a compound represented by general formula (VII-1) or a pharmaceutically acceptable salt thereof to give the compounds represented by general formula (VII-1-1) and general formula (VII-1-2) or the pharmaceutically acceptable salts thereof;
wherein:
R^{1a}, Q⁴, R^{3d}, R^{3e}, R^{3f}, W⁴, m3, x, x1, y, and y1 are as defined in general formula (VII-1).

Another aspect of the present disclosure relates to a pharmaceutical composition comprising the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above in the present disclosure or the compounds shown in Table A or pharmaceutically acceptable salts thereof, and one or more pharmaceutically acceptable carriers, diluents, or excipients.

The present disclosure further relates to use of the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, in the preparation of a medicament for regulating the ubiquitination and degradation of the androgen receptor (AR) protein in a subj ect.

The present disclosure further relates to use of the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, in the preparation of a medicament for treating and/or preventing an androgen receptor-mediated or -dependent disease or disorder, wherein the androgen receptor-mediated or -dependent disease or disorder is preferably selected from the group consisting of tumors, male sexual dysfunction, and Kennedy's disease; more preferably from the group consisting of prostate cancer, prostatic hyperplasia, hirsutism, alopecia, anorexia nervosa, breast cancer, acne, male sexual dysfunction, Kennedy's disease, and AIDS; most preferably prostate cancer; and still most preferably hormone-sensitive prostate cancer or hormone-refractory prostate cancer.

The present disclosure further relates to use of the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, in the preparation of a medicament for treating and/or preventing tumors, male sexual dysfunction, and Kennedy's disease; preferably for treating and/or preventing prostate cancer, prostatic hyperplasia, hirsutism, alopecia, anorexia nervosa, breast cancer, acne, male sexual dysfunction, Kennedy's disease, and AIDS; more preferably for treating and/or preventing prostate cancer; and most preferably for treating and/or preventing hormone-sensitive prostate cancer or hormone-refractory prostate cancer.

The present disclosure also relates to a method for regulating the ubiquitination and degradation of the androgen receptor (AR) protein in a subject, comprising administering to a subject in need thereof a therapeutically effective amount of the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same.

The present disclosure also relates to a method for treating and/or preventing an androgen receptor-mediated or -dependent disease or disorder, comprising administering to a subject in need thereof a therapeutically effective amount of the compounds represented by general formula (I), general formula (II), general formula (II-1), general formula (G), general formula (G-1), general formula (III'), general formula (ΠI'-1), general formula (III), general formula (III-I), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, wherein the androgen receptor-mediated or -dependent disease or disorder is preferably selected from the group consisting of tumors, male sexual dysfunction, and Kennedy's disease; more preferably from the group consisting of prostate cancer, prostatic hyperplasia, hirsutism, alopecia, anorexia nervosa, breast cancer, acne, male sexual dysfunction, Kennedy's disease, and AIDS; most preferably prostate cancer; and still most preferably hormone-sensitive prostate cancer or hormone-refractory prostate cancer.

The present disclosure also relates to a method for treating and/or preventing tumors, male sexual dysfunction, and Kennedy's disease, preferably for treating and/or preventing prostate cancer, prostatic hyperplasia, hirsutism, alopecia, anorexia nervosa, breast cancer, acne, male sexual dysfunction, Kennedy's disease, and AIDS, more preferably for treating and/or preventing prostate cancer, and most preferably for treating and/or preventing hormone-sensitive prostate cancer or hormone-refractory prostate cancer, comprising administering to a patient in need thereof a therapeutically effective amount of the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same.

The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use as a medicament.

The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use as a medicament for regulating the ubiquitination and degradation of the androgen receptor (AR) protein in a subject.

The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use as a medicament for treating and/or preventing an androgen receptor-mediated or -dependent disease or disorder, wherein the androgen receptor-mediated or -dependent disease or disorder is preferably selected from the group consisting of tumors, male sexual dysfunction, and Kennedy's disease; more preferably from the group consisting of prostate cancer, prostatic hyperplasia, hirsutism, alopecia, anorexia nervosa, breast cancer, acne, male sexual dysfunction, Kennedy's disease, and AIDS; most preferably prostate cancer; and still most preferably hormone-sensitive prostate cancer or hormone-refractory prostate cancer.

The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use as a medicament for treating and/or preventing tumors, male sexual dysfunction, and Kennedy's disease; preferably for treating and/or preventing prostate cancer, prostatic hyperplasia, hirsutism, alopecia, anorexia nervosa, breast cancer, acne, male sexual dysfunction, Kennedy's disease, and AIDS; more preferably for treating and/or preventing prostate cancer; and most preferably for treating and/or preventing hormone-sensitive prostate cancer or hormone-refractory prostate cancer. The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use in regulating the ubiquitination and degradation of the androgen receptor (AR) protein in a subject.

The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use in treating and/or preventing an androgen receptor-mediated or -dependent disease or disorder, wherein the androgen receptor-mediated or -dependent disease or disorder is preferably selected from the group consisting of tumors, male sexual dysfunction, and Kennedy's disease; more preferably from the group consisting of prostate cancer, prostatic hyperplasia, hirsutism, alopecia, anorexia nervosa, breast cancer, acne, male sexual dysfunction, Kennedy's disease, and AIDS; most preferably prostate cancer; and still most preferably hormone-sensitive prostate cancer or hormone-refractory prostate cancer.

The present disclosure further relates to the compounds represented by general formula (I), general formula (II), general formula (II-1), general formula (G), general formula (G-1), general formula (III'), general formula (ΠI'-1), general formula (III), general formula (III-1), general formula (X), general formula (X-1), general formula (IV'), general formula (IV'-1), general formula (IV'-1-1), general formula (IV'-1-2), general formula (M), general formula (M-1), general formula (M-1-1), general formula (M-1-2), general formula (IV), general formula (IV-1), general formula (IV-1-1), general formula (IV-1-2), general formula (V), general formula (V-1), general formula (V-1-1), general formula (V-1-2), general formula (VI), general formula (VI-1), general formula (VI-1-1), general formula (VI-1-2), general formula (VII), general formula (VII-1), general formula (VII-1-1), and general formula (VII-1-2) described above or the compounds shown in Table A or pharmaceutically acceptable salts thereof, or the pharmaceutical composition comprising same, for use in treating and/or preventing tumors, male sexual dysfunction, and Kennedy's disease; preferably for use in treating and/or preventing prostate cancer, prostatic hyperplasia, hirsutism, alopecia, anorexia nervosa, breast cancer, acne, male sexual dysfunction, Kennedy's disease, and AIDS; more preferably for use in treating and/or preventing prostate cancer; and most preferably for use in treating and/or preventing hormone-sensitive prostate cancer or hormone-refractory prostate cancer.

In certain embodiments, the disease or disorder is asthma, multiple sclerosis, cancer, Kennedy's disease, ciliopathy, cleft palate, diabetes, heart disease, hypertension, inflammatory bowel disease, mental retardation, mood disorder, obesity, refractive error, infertility, Angelman syndrome, Canavan disease, coeliac disease, Charcot-Marie-Tooth disease, cystic fibrosis, Duchenne muscular dystrophy, hemochromatosis, hemophilia, Klinefelter syndrome, neurofibroma, phenylketonuria, polycystic kidney disease, (PKD1) or 4(PKD2) Prader-Willi syndrome, sickle cell disease, Tay-Sachs disease, or Turner syndrome. The cancer is squamous cell carcinoma, basal cell carcinoma, adenocarcinoma, hepatocellular carcinoma, renal cell carcinoma, bladder cancer, intestinal cancer, breast cancer, cervical cancer, colon cancer, esophageal cancer, head cancer, kidney cancer, liver cancer, lung cancer, neck cancer, ovarian cancer, pancreatic cancer, prostate cancer, gastric cancer, leukemia, benign and malignant lymphomas (particularly Burkitt's lymphoma and non-Hodgkin lymphoma), benign and malignant melanomas, myeloproliferative diseases, sarcomas (including Ewing's sarcoma, angiosarcoma, Kaposi's sarcoma, liposarcoma, myosarcoma, peripheral neuroepithelioma, synovial sarcoma, neuroglioma, astrocytoma, oligodendroglioma, ependymoma, glioblastoma, neuroblastoma, ganglioneuroma, ganglioma, medulloblastoma, pinealocyte tumors, meningioma, meningeal sarcoma, neurofibroma, and schwannomas), endometrial cancer, testicular cancer, thyroid cancer, carcinosarcoma, Hodgkin disease, Wilms' tumor, or teratocarcinoma. In certain embodiments, the disease to be treated is cancer, e.g., prostate cancer or Kennedy's disease.

The active compound may be formulated into a form suitable for administration by any suitable route, preferably in the form of a unit dose, or in the form of a single dose that can be self-administered by a patient. The unit dose of the compound or composition of the present disclosure may be expressed in the form of a tablet, capsule, cachet, vial, powder, granule, lozenge, suppository, regenerating powder, or liquid formulation.

As a general guide, a suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, or an excipient or the like. Depending on the method of administration, the composition may comprise 0.1 to 99 wt.% of the active compound.

The pharmaceutical composition comprising the active ingredient may be in a form suitable for oral administration, for example, in the form of a tablet, dragee, lozenge, aqueous or oil suspension, dispersible powder or granule, emulsion, hard or soft capsule, or syrup or elixir. An oral composition may be prepared by following any method known in the art for preparing pharmaceutical compositions, and such a composition may comprise one or more ingredients selected from the group consisting of a sweetener, a corrigent, a colorant, and a preservative, so as to provide a pharmaceutical formulation that is pleasing to the eye and palatable. The tablet comprises the active ingredient, and non-toxic pharmaceutically acceptable excipients that are used for mixing and are suitable for the preparation of the tablet. These excipients may be an inert excipient, a granulating agent, a disintegrant, a binder, and a lubricant. These tablets may be uncoated or coated using known techniques that mask the taste of the drug or delay disintegration and absorption in the gastrointestinal tract, thereby providing a sustained-release effect over an extended period of time.

An oral formulation may also be provided in the form of a soft gelatin capsule in which the active ingredient is mixed with an inert solid diluent or with a water-soluble carrier or oil vehicle.

An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant. The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using suitable dispersants or wetting agents and suspending agents. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

As is well known to those skilled in the art, the dosage of the drug depends on a variety of factors, including, but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of treatment, the daily dose of the compound, or the type of pharmaceutically acceptable salts, can be verified according to conventional treatment regimens.

### Terminology

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl is preferably an alkyl group having 1 to 12 carbon atoms (i.e., C₁₋₁₂ alkyl), and more preferably an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkenyl). The alkenyl is preferably an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkynyl). The alkynyl is preferably an alkynyl group having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e.,3- to 12-membered cycloalkyl), more preferably a cycloalkyl group having 3 to 8 ring atoms (i.e., 3- to 8-membered cycloalkyl), and most preferably a cycloalkyl group having 3 to 6 ring atoms (i.e., 3- to 6-membered cycloalkyl). Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, which may contain in the rings one or more double bonds or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one all-carbon ring is contained and the point of attachment is on the all-carbon ring; and which has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl is preferably a spirocycloalkyl group having 6 to 14 ring atoms (i.e., 6- to 14-membered spirocycloalkyl), and more preferably a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl), preferably monospirocycloalkyl or bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples include: 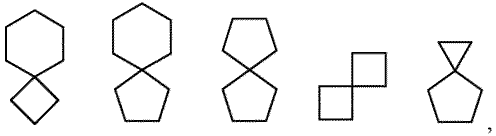 wherein the point of attachment may be at any position; 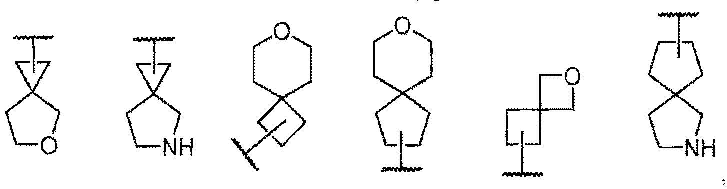 and the like.

The term "fused cycloalkyl" refers to a polycyclic system in which two adjacent carbon atoms are shared between rings, which is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group; and which may contain in the rings one or more double bonds and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl is preferably a fused cycloalkyl group having 6 to 14 ring atoms (i.e., 6- to 14-membered fused cycloalkyl), and more preferably a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples include: 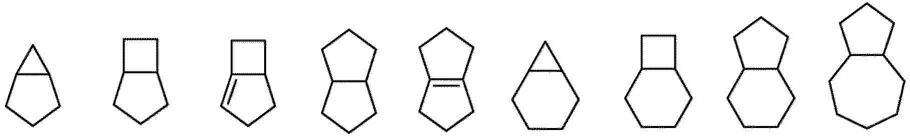 , wherein the point of attachment may be at any position; 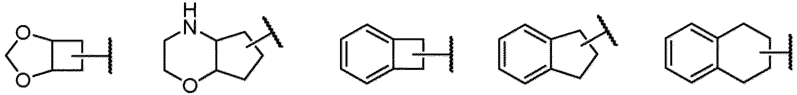 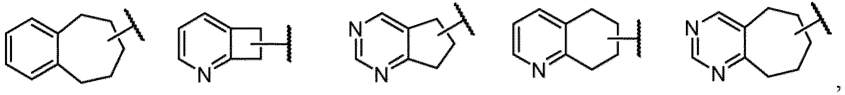 and the like.

The term "bridged cycloalkyl" refers to an all-carbon polycyclic system in which two carbon atoms that are not directly connected are shared between rings, which may contain in the rings one or more double bonds and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). The bridged cycloalkyl is preferably a bridged cycloalkyl group having 6 to 14 carbon atoms (i.e., 6- to 14-membered bridged cycloalkyl), and more preferably a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl), preferably bicyclic bridged cycloalkyl or tricyclic bridged cycloalkyl. Non-limiting examples include: 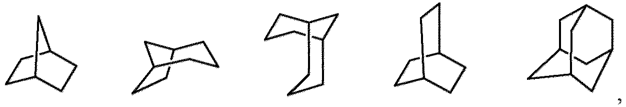 wherein the point of attachment may be at any position.

Cycloalkyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl is preferably a heterocyclyl group having 3 to 12 ring atoms (i.e., 3- to 12-membered heterocyclyl); further preferably a heterocyclyl group having 3 to 8 ring atoms (i.e., 3- to 8-membered heterocyclyl); more preferably a heterocyclyl group having 3 to 6 ring atoms (i.e., 3- to 6-membered heterocyclyl) or a heterocyclyl group having 4 to 6 ring atoms (i.e., 4- to 6-membered heterocyclyl); and most preferably a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one monocyclic heterocyclyl group is contained and the point of attachment is on the monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). The spiroheterocyclyl is preferably a spiroheterocyclyl group having 6 to 14 ring atoms (i.e., 6- to 14-membered spiroheterocyclyl), further preferably a spiroheterocyclyl group having 7 to 11 ring atoms (i.e., 7- to 11-membered spiroheterocyclyl), and more preferably a spiroheterocyclyl group having 7 to 10 ring atoms (i.e., 7- to 10-membered spiroheterocyclyl) or a spiroheterocyclyl group having 9 to 11 ring atoms (i.e., 9- to 11-membered spiroheterocyclyl). The spiroheterocyclyl includes monospiroheterocyclyl and polyspiroheterocyclyl (e.g., bispiroheterocyclyl), preferably monospiroheterocyclyl or bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples include: 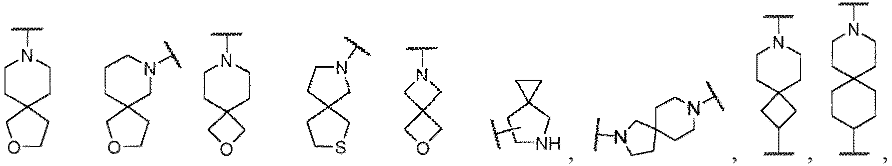 and the like.

The term "fused heterocyclyl" refers to a polycyclic heterocyclic system in which two adjacent atoms are shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); which is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl is preferably a fused heterocyclyl group having 6 to 14 ring atoms (i.e., 6- to 14-membered fused heterocyclyl), and more preferably a fused heterocyclyl group having 7 to 10 ring atoms (i.e., 7- to 10-membered fused heterocyclyl). The fused heterocyclyl includes bicyclic and polycyclic fused heterocyclyl (e.g., tricyclic fused heterocyclyl and tetracyclic fused heterocyclyl), preferably bicyclic fused heterocyclyl or tricyclic fused heterocyclyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples include: 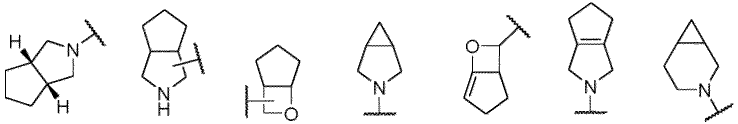 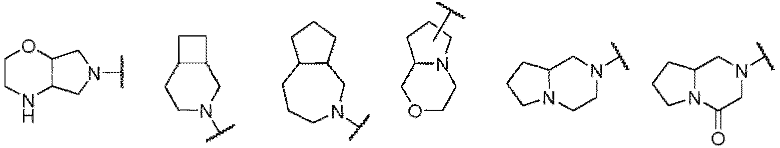 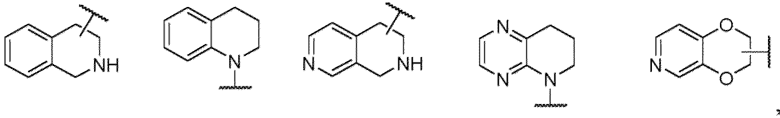 and the like.

The term "bridged heterocyclyl" refers to a polycyclic heterocyclic system in which two atoms that are not directly connected are shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); and which has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl is preferably a bridged heterocyclyl group having 6 to 14 ring atoms (i.e., 6- to 14-membered bridged heterocyclyl), and more preferably a bridged heterocyclyl group having 7 to 10 ring atoms (i.e., 7- to 10-membered bridged heterocyclyl). According to the number of constituent rings, bridged heterocyclyl can be divided into bicyclic bridged heterocyclyl and polycyclic bridged heterocyclyl (e.g., tricyclic bridged heterocyclyl and tetracyclic bridged heterocyclyl), preferably bicyclic bridged heterocyclyl or tricyclic bridged heterocyclyl. Non-limiting examples include: 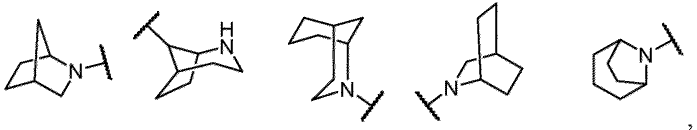 and the like.

Heterocyclyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, which has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). An example of the monocyclic aryl is phenyl. Non-limiting examples of the polycyclic aryl include: naphthyl, anthryl, phenanthryl, and the like. The polycyclic aryl also includes those formed by fusing a phenyl group with one or more of a heterocyclyl group or a cycloalkyl group or fusing a naphthyl group with one or more of a heterocyclyl group or a cycloalkyl group, wherein the point of attachment is on the phenyl group or the naphthyl group, and in the circumstances the number of ring atoms continues to represent the number of ring atoms in the polycyclic aromatic ring system; non-limiting examples include: 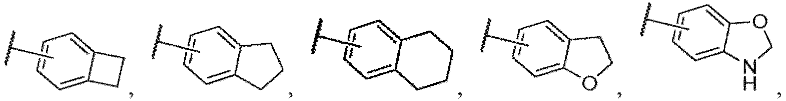 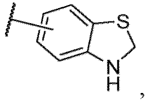 and the like.

Aryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 5 to 14 (e.g., 5, 6, 7, 8, 9, 10, 11, 12, 13, or 14) ring atoms (i.e., 5- to 14-membered heteroaryl). The heteroaryl group is preferably a heteroaryl group having 5 to 10 ring atoms (i.e., 5- to 10-membered heteroaryl), more preferably a heteroaryl group having 5 or 6 ring atoms (i.e., 5- or 6-membered heteroaryl), and most preferably a heteroaryl group having 6 ring atoms (i.e., 6-membered heteroaryl).

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridonyl, N-alkylpyridinone (e.g., 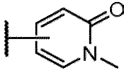 ), pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl also includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in the circumstances the number of ring atoms continues to represent the number of ring atoms in the polycyclic heteroaromatic ring system. Non-limiting examples include: 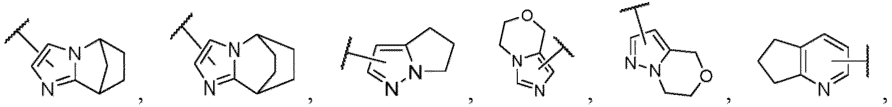 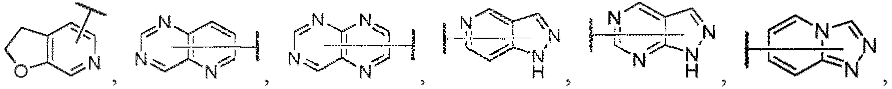 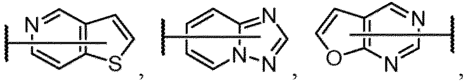 and the like.

Heteroaryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group in order for the amino group to remain unchanged when other parts of the molecule are involved in reactions. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), trifluoroacetyl (Tfa), trityl (Trt), 2,4-dimethoxybenzyl (DMB), acetyl, benzyl, allyl, p-methoxybenzyl, and the like.

The term "hydroxy protecting group" refers to an easily removable group that is introduced onto a hydroxy group to block or protect the hydroxy group so that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "spirocyclyl" refers to a spirocycloalkyl or spiroheterocyclyl group, wherein the spirocycloalkyl or spiroheterocyclyl group is as defined above.

The term "cycloalkylalkyl" refers to an alkyl group substituted with one or more cycloalkyl groups, wherein the cycloalkyl and alkyl groups are as defined above.

The term "heterocyclylalkyl" refers to an alkyl group substituted with one or more heterocyclyl groups, wherein the heterocyclyl and alkyl groups are as defined above. The term "aminoalkyl" refers to an alkyl group substituted with one or more amino groups, wherein the alkyl group is as defined above.

The term "alkoxyalkyl" refers to an alkyl group substituted with one or more alkoxy groups, wherein the alkoxy and alkyl groups are as defined above.

The term "haloalkyl" refers to an alkyl group substituted with one or more halogens, wherein the alkyl group is as defined above.

The term "haloalkoxy" refers to an alkoxy group substituted with one or more halogens, wherein the alkoxy group is as defined above.

The term "deuterated alkyl" refers to an alkyl group substituted with one or more deuterium atoms, wherein the alkyl group is as defined above.

The term "hydroxyalkyl" refers to an alkyl group substituted with one or more hydroxy groups, wherein the alkyl group is as defined above.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

In the present disclosure, when m1 is 0, X² is a bond.

In the present disclosure, when m2 is 0, J¹ is a bond.

In the present disclosure, when m3 is 0, J³ is a bond.

In the present disclosure, when m4 is 0, J⁵ is a bond.

The term "ubiquitin ligase" refers to a family of proteins that facilitate the transfer of ubiquitin to a specific substrate protein, targeting the substrate protein for degradation. For example, cereblon is an E3 ubiquitin ligase protein that alone or in combination with an E2 ubiquitin conjugating enzyme causes the attachment of ubiquitin to a lysine on a target protein and subsequently targets the specific protein substrate for degradation by the proteasome. Thus, E3 ubiquitin ligase alone or in complex with an E2 ubiquitin conjugating enzyme is responsible for the transfer of ubiquitin to target proteins. In general, the ubiquitin ligase is involved in polyubiquitination such that a second ubiquitin is attached to the first ubiquitin, a third ubiquitin is attached to the second ubiquitin, and so forth. Polyubiquitination marks proteins for degradation by the proteasome. However, there are some ubiquitination events that are limited to mono-ubiquitination, in which only a single ubiquitin is added by the ubiquitin ligase to a substrate molecule. Mono-ubiquitinated proteins are not targeted to the proteasome for degradation, but may instead be altered in their cellular location or function, for example, via binding other proteins that have domains capable of binding ubiquitin. To complicate matters further, different lysines on ubiquitin can be targeted by E3 to prepare chains. The most common lysine is Lys48 on the ubiquitin chain. This is the lysine used to prepare polyubiquitin, which is recognized by the proteasome.

The term "target proteins" refers to proteins and peptides having any biological function or activity (including structural, regulatory, hormonal, enzymatic, genetic, immunological, contractile, storage, transportation, and signal transduction). In some embodiments, target proteins include structural proteins, receptors, enzymes, cell surface proteins, and proteins associated with the integrated function of a cell, including proteins involved in: catalytic activity, aromatase activity, motor activity, helicase activity, metabolic processes (anabolism and catrabolism), antioxidant activity, proteolysis, biosynthesis, proteins with kinase activity, oxidoreductase activity, transferase activity, hydrolase activity, lyase activity, isomerase activity, ligase activity, enzyme regulator activity, signal transducer activity, structural molecule activity, binding activity (protein and lipid carbohydrate), receptor activity, cell motility, membrane fusion, cell communication, regulation of biological processes, development, cell differentiation, response to stimulus, behavioral proteins, cell adhesion proteins, proteins involved in cell death, proteins involved in transport (including protein transporter activity, nuclear transport, ion transporter activity, channel transporter activity, and carrier activity), permease activity, secretion activity, electron transporter activity, pathogenesis, chaperone regulator activity, nucleic acid binding activity, transcription regulator activity, extracellular organization and biogenesis activity, and translation regulator activity. The proteins include proteins derived from eukaryotes and prokaryotes, including microbes, viruses, fungi and parasites, among numerous others; including humans, microbes, viruses, fungi, and parasites as targets for drug therapy, other animals including domestic animals), microbes for determining targets for antibiotics and other antimicrobials and plants and even viruses, among numerous others.

The compounds of the present disclosure may exist in specific stereoisomeric forms. The term "stereoisomer" refers to isomers that are structurally identical but differ in the arrangement of the atoms in space. It includes cis and trans (or Z and E) isomers, (-)- and (+)-isomers, (R)- and (*S*)-enantiomers, diastereomers, (D)- and (L)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (R)- and (*S*)-enantiomers, and (D)- and (L)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. In addition, separation of enantiomers and diastereomers is generally accomplished by chromatography.

In the chemical structures of the compounds of the present disclosure, a bond " 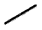 " represents an unspecified configuration; that is, if chiral isomers exist in the chemical structures, the bond " 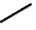 " may be " 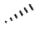 " or " 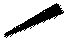 ", or both the configurations of " 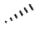 " and " 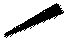 " are included simultaneously. For all carbon-carbon double bonds, both Z- and E-forms are included, even if only one configuration is named.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

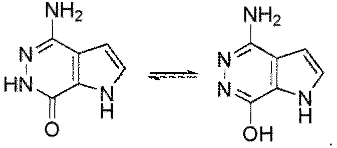

For example, reference to pyrazolyl is understood to include any one of the following two structures or a mixture of the two tautomers:

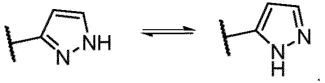

All tautomeric forms fall within the scope of the present disclosure, and the nomenclature of the compounds does not exclude any tautomer.

The compound of the present disclosure may include atropisomers. The term "atropisomers" refers to conformational stereoisomers that result from hindered or greatly slowed rotation about a single bond in a molecule (as a result of the steric interactions with other parts of the molecule and the asymmetry of the substituents at both ends of the single bond), which interconvert sufficiently slowly to allow separation and isolation under predetermined conditions. For example, certain compounds of the present disclosure may exist in the form of a mixture of atropisomers (e.g., an equal ratio mixture, a mixture enriched in one atropisomer) or a purified atropisomer.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium , T), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶C1, ⁸²Br, ¹²³I, ¹²⁴I, ¹²⁵I, ¹²⁹I, and ¹³¹I, respectively; deuterium is preferred.

Compared to non-deuterated drugs, deuterated drugs have the advantages of reduced toxic and side effects, increased drug stability, enhanced efficacy, prolonged biological half-lives, and the like. All isotopic variations of the compounds of the present disclosure, whether radioactive or not, are intended to be included within the scope of the present disclosure. Each available hydrogen atom connected to a carbon atom may be independently replaced with a deuterium atom, wherein replacement of deuterium may be partial or complete, and replacement of partial deuterium refers to replacement of at least one hydrogen atom with at least one deuterium atom.

When a position is specifically assigned deuterium (D), the position should be understood as deuterium with an abundance that is at least 1000 times greater than the natural abundance of deuterium (which is 0.015%) (i.e., at least 15% deuterium incorporation). The deuterium of the compounds in the examples with an abundance greater than the natural abundance of deuterium may be deuterium with an abundance that is at least 1000 times greater (i.e., at least 15% deuterium incorporation), at least 2000 times greater (i.e., at least 30% deuterium incorporation), at least 3000 times greater (i.e., at least 45% deuterium incorporation), at least 3340 times greater (i.e., at least 50.1% deuterium incorporation), at least 3500 times greater (i.e., at least 52.5% deuterium incorporation), at least 4000 times greater (i.e., at least 60% deuterium incorporation), at least 4500 times greater (i.e., at least 67.5% deuterium incorporation), at least 5000 times greater (i.e., at least 75% deuterium incorporation), at least 5500 times greater (i.e., at least 82.5% deuterium incorporation), at least 6000 times greater (i.e., at least 90% deuterium incorporation), at least 6333.3 times greater (i.e., at least 95% deuterium incorporation), at least 6466.7 times greater (i.e., at least 97% deuterium incorporation), at least 6600 times greater (i.e., at least 99% deuterium incorporation), or at least 6633.3 times greater (i.e., at least 99.5% deuterium incorporation), or deuterium with a higher abundance.

"Optional" or "optionally" means that the event or circumstance subsequently described may, but does not necessarily, occur and includes an instance where the event or circumstance occurs and an instance where it does not. For example, "C₁₋₆ alkyl that is optionally substituted with halogen or cyano" includes an instance where the alkyl is substituted with halogen or cyano and an instance where the alkyl is not substituted with halogen or cyano.

"Substitution" or "substituted" means that one or more, preferably 1 to 6, and more preferably 1 to 3, hydrogen atoms in the group are independently substituted with a corresponding number of substituents. Those skilled in the art can determine (experimentally or theoretically) possible or impossible substitutions without undue effort. For example, it may be unstable when an amino or hydroxy group having free hydrogen binds to a carbon atom having an unsaturated bond (e.g., an olefin). "Pharmaceutical composition" refers to a mixture containing one or more of the compounds or the pharmaceutically acceptable salts thereof described herein, and other chemical components, and other components, for example, pharmaceutically acceptable carriers and excipients. The pharmaceutical composition is intended to promote the administration to an organism, so as to facilitate the absorption of the active ingredient, thereby exerting biological activity.

"Pharmaceutically acceptable salt" refers to a salt of the compound of the present disclosure, which may be selected from the group consisting of inorganic or organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the therapeutically effective amount varies from person to person. It depends on the age and general condition of a subject, as well as the specific active substance used. The appropriate therapeutically effective amount in a case may be determined by those skilled in the art in the light of routine tests.

The term "pharmaceutically acceptable" as used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be understood by those skilled in the art, when the parameters are not critical, the numbers are generally given for illustrative purposes only and are not intended to be limiting.

### Synthetic methods of the compounds of the present disclosure

To achieve the purposes of the present disclosure, the following technical solutions are adopted in the present disclosure:

### Scheme 1

A method for preparing the compound represented by general formula (III') or the pharmaceutically acceptable salt thereof of the present disclosure, comprising the following step: 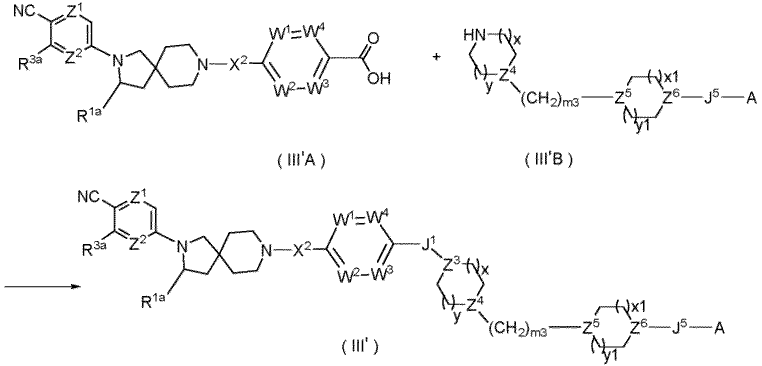 conducting a condensation reaction of a compound represented by general formula (III'A) or a salt thereof with a compound represented by general formula (III'B) or a salt thereof (preferably hydrochloride and trifluoroacetate) under alkaline conditions in the presence of a condensing agent to give the compound represented by general formula (III') or the pharmaceutically acceptable salt thereof;
wherein:
J¹ is -C(O)-;
Z³ is N;
A, R^{1a}, R^{3a}, J⁵, X², W¹, W², W³, W⁴, Z¹, Z², Z⁴, Z⁵, Z⁶, x, x1, y, y1, and m3 are as defined in general formula (III').

### Scheme 2

A method for preparing the compound represented by general formula (III') or the pharmaceutically acceptable salt thereof of the present disclosure, comprising the following step: 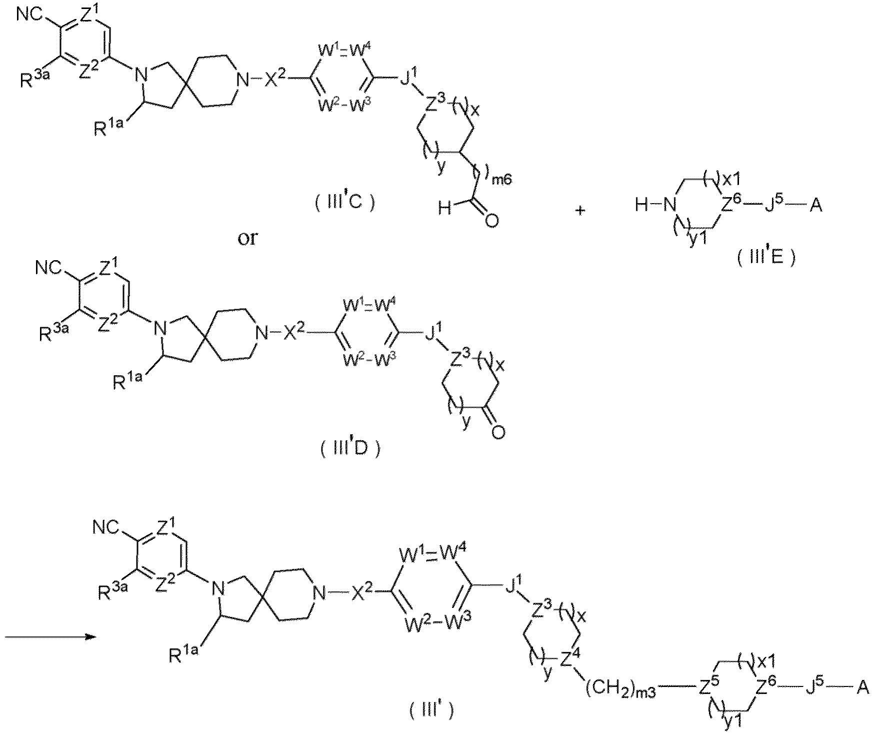 conducting a reductive amination reaction of a compound represented by general formula (III'C) or a salt thereof with a compound represented by general formula (III'E) or a salt thereof (preferably hydrochloride and trifluoroacetate) under weakly acidic conditions in the presence of a reductant to give the compound represented by general formula (III') or the pharmaceutically acceptable salt thereof, where m3 is 1, 2, or 3; or, conducting a reductive amination reaction of a compound represented by general formula (III'D) or a salt thereof with a compound represented by general formula (III'E) or a salt thereof (preferably hydrochloride and trifluoroacetate) under weakly acidic conditions in the presence of a reductant to give the compound represented by general formula (III') or the pharmaceutically acceptable salt thereof, where m3 is 0;
wherein:
m6 is 0, 1, or 2;
Z⁴ is CH;
Z⁵ is N;
A, J¹, J⁵, X², R^{1a}, R^{3a}, W¹, W², W³, W⁴, Z¹, Z², Z³, Z⁶, x, x1, y, and y1 are as defined in general formula (III').

### Scheme 3

A method for preparing the compound represented by general formula (III') or the pharmaceutically acceptable salt thereof of the present disclosure, comprising the following step: 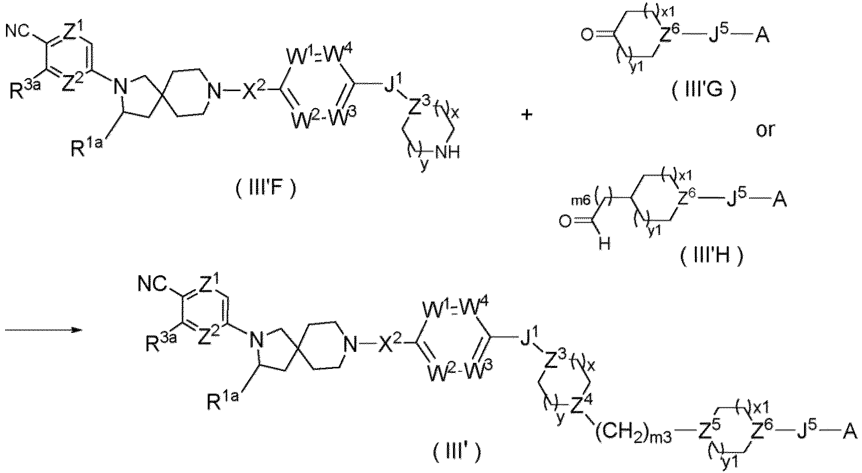 conducting a reductive amination reaction of a compound represented by general formula (III'F) or a salt thereof (preferably hydrochloride and trifluoroacetate) with a compound represented by general formula (III'G) or a salt thereof under weakly acidic conditions in the presence of a reductant to give the compound represented by general formula (III') or the pharmaceutically acceptable salt thereof, where m3 is 0; or, conducting a reductive amination reaction of a compound represented by general formula (III'F) or a salt thereof (preferably hydrochloride and trifluoroacetate) with a compound represented by general formula (III'H) or a salt thereof under weakly acidic conditions in the presence of a reductant to give the compound represented by general formula (III') or the pharmaceutically acceptable salt thereof, where m3 is 1, 2, or 3;
wherein:
m6 is 0, 1, or 2;
Z⁴ is N;
Z⁵ is CH;
A, J¹, J⁵, X², R^{1a}, R^{3a}, W¹, W², W³, W⁴, Z¹, Z², Z³, Z⁶, x, x1, y, and y1 are as defined in general formula (III').

### Scheme 4

A method for preparing the compound represented by general formula (X) or the pharmaceutically acceptable salt thereof of the present disclosure, comprising the following step: 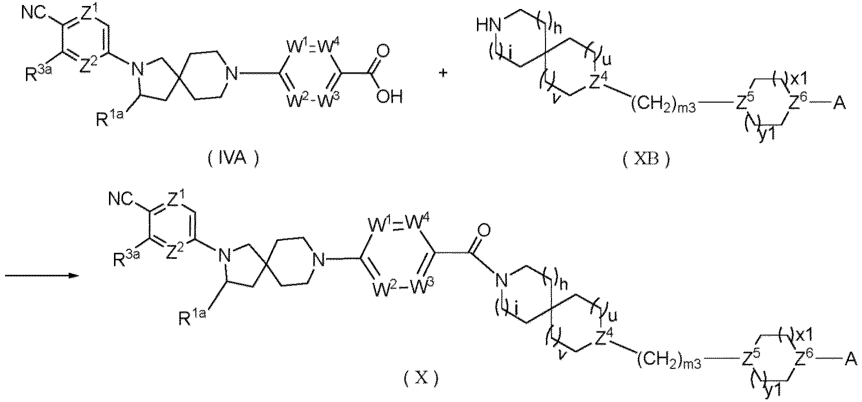 conducting a condensation reaction of a compound represented by general formula (IVA) or a salt thereof with a compound represented by general formula (XB) or a salt thereof (preferably hydrochloride and trifluoroacetate) under alkaline conditions in the presence of a condensing agent to give the compound represented by general formula (X) or the pharmaceutically acceptable salt thereof;
wherein:
R^{1a}, R^{3a}, W¹, W², W³, W⁴, Z¹, Z², Z⁴, Z⁵, Z⁶, h, i, u, v, x1, y1, m3, and A are as defined in general formula (X).

### Scheme 5

A method for preparing the compound represented by general formula (X-1) or the pharmaceutically acceptable salt thereof of the present disclosure, comprising the following step: 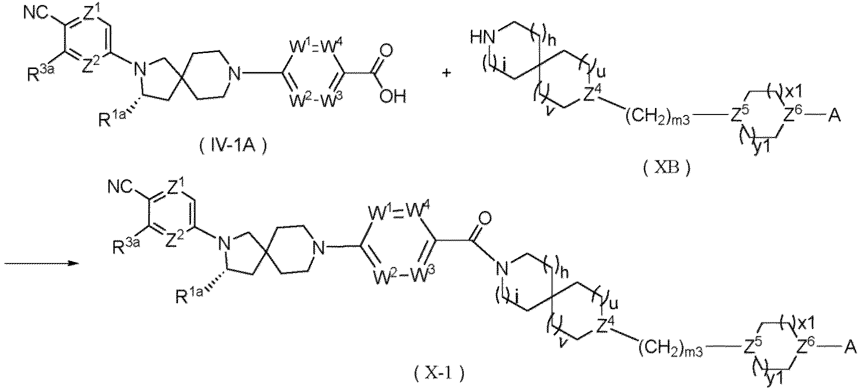 conducting a condensation reaction of a compound represented by general formula (IV-1A) or a salt thereof with a compound represented by general formula (XB) or a salt thereof (preferably hydrochloride and trifluoroacetate) under alkaline conditions in the presence of a condensing agent to give the compound represented by general formula (X-1) or the pharmaceutically acceptable salt thereof; wherein:
R^{1a}, R^{3a}, W¹, W², W³, W⁴, Z¹, Z², Z⁴, Z⁵, Z⁶, h, i, u, v, x1, y1, m3, and A are as defined in general formula (X-1).

### Scheme 6

A method for preparing the compound represented by general formula (X) or the pharmaceutically acceptable salt thereof of the present disclosure, comprising the following step: 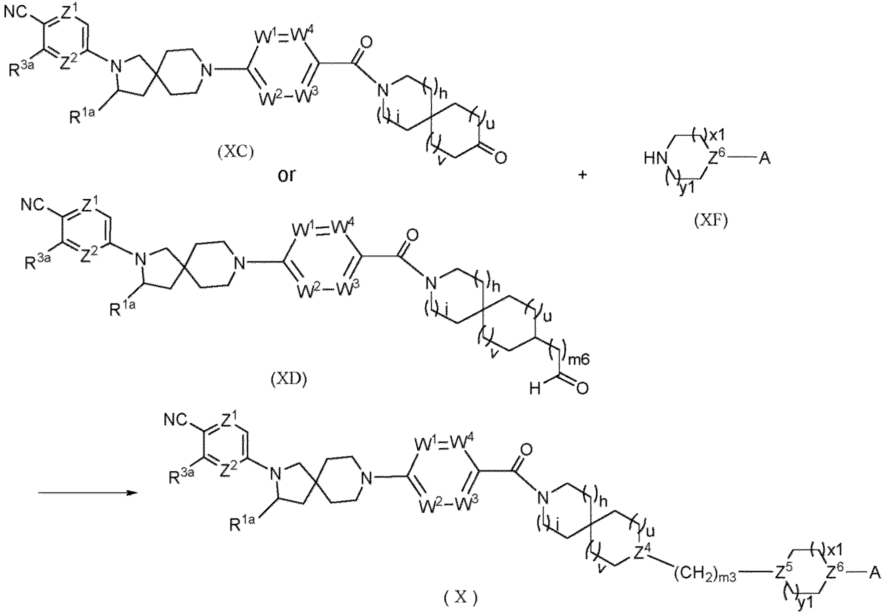
conducting a reductive amination reaction of a compound represented by general formula (XC) or a salt thereof with a compound represented by general formula (XF) or a salt thereof (preferably hydrochloride and trifluoroacetate) under weakly acidic conditions in the presence of a reductant to give the compound represented by general formula (X) or the pharmaceutically acceptable salt thereof, where m3 is 0;
or, conducting a reductive amination reaction of a compound represented by general formula (XD) or a salt thereof with a compound represented by general formula (XF) or a salt thereof (preferably hydrochloride and trifluoroacetate) under weakly acidic conditions in the presence of a reductant to give the compound represented by general formula (X) or the pharmaceutically acceptable salt thereof, where m3 is 1, 2, or 3;
wherein:
   m6 is 0, 1, or 2;

   Z⁴ is CH;
   Z⁵ is N;
   R^{1a}, R^{3a}, W¹, W², W³, W⁴, Z¹, Z², Z⁶, h, i, u, v, x1, y1, and A are as defined in general formula (X).

### Scheme 7

A method for preparing the compound represented by general formula (X-1) or the pharmaceutically acceptable salt thereof of the present disclosure, comprising the following step: 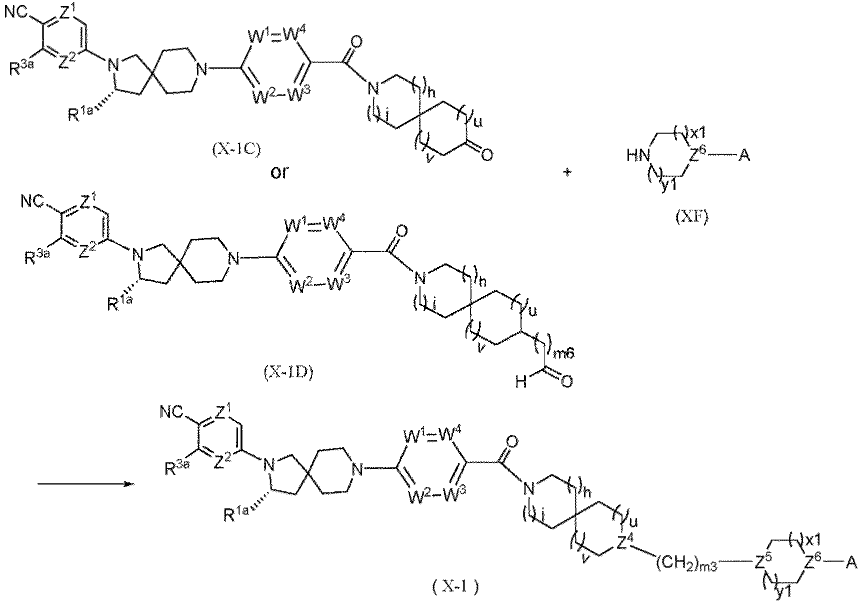
conducting a reductive amination reaction of a compound represented by general formula (X-1C) or a salt thereof with a compound represented by general formula (XF) or a salt thereof (preferably hydrochloride and trifluoroacetate) under weakly acidic conditions in the presence of a reductant to give the compound represented by general formula (X-1) or the pharmaceutically acceptable salt thereof, where m3 is 0;
or, conducting a reductive amination reaction of a compound represented by general formula (X-1D) or a salt thereof with a compound represented by general formula (XF) or a salt thereof (preferably hydrochloride and trifluoroacetate) under weakly acidic conditions in the presence of a reductant to give the compound represented by general formula (X-1) or the pharmaceutically acceptable salt thereof, where m3 is 1, 2, or 3;
wherein:
   m6 is 0, 1, or 2;

   Z⁴ is CH;
   Z⁵ is N;
   R^{1a}, R^{3a}, W¹, W², W³, W⁴, Z¹, Z², Z⁶, h, i, u, v, x1, y1, and A are as defined in general formula (X-1).

### Scheme 8

A method for preparing the compound represented by general formula (IV') or the pharmaceutically acceptable salt thereof of the present disclosure, comprising the following step: 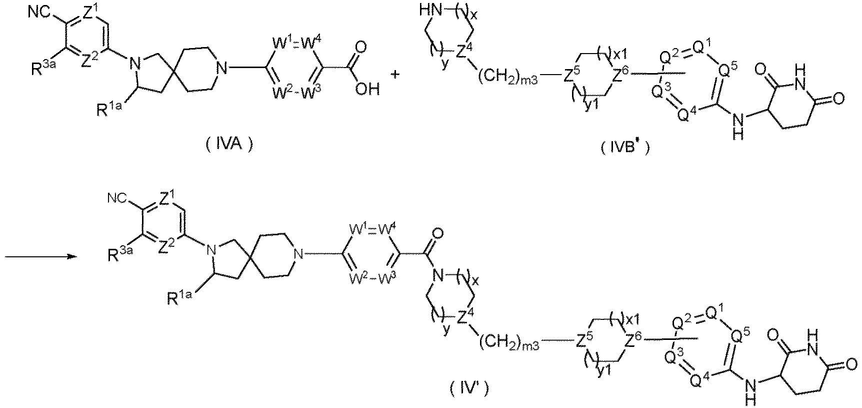 conducting a condensation reaction of a compound represented by general formula (IVA) or a salt thereof with a compound represented by general formula (IVB') or a salt thereof (preferably hydrochloride and trifluoroacetate) under alkaline conditions in the presence of a condensing agent to give the compound represented by general formula (IV') or the pharmaceutically acceptable salt thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in general formula (IV').

### Scheme 9

A method for preparing the compound represented by general formula (IV-1) or the pharmaceutically acceptable salt thereof of the present disclosure, comprising the following step: 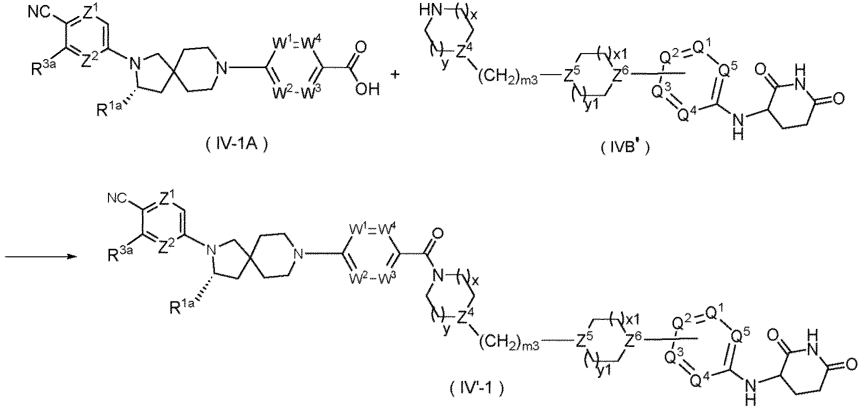 conducting a condensation reaction of a compound represented by general formula (IV-1A) or a salt thereof with a compound represented by general formula (IVB') or a salt thereof (preferably hydrochloride and trifluoroacetate) under alkaline conditions in the presence of a condensing agent to give the compound represented by general formula (IV'-1) or the pharmaceutically acceptable salt thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y 1, and m3 are as defined in general formula (IV'-1).

### Scheme 10

A method for preparing the compounds represented by general formula (IV-1-1) and general formula (IV-1-2) or the pharmaceutically acceptable salts thereof of the present disclosure, comprising the following step: 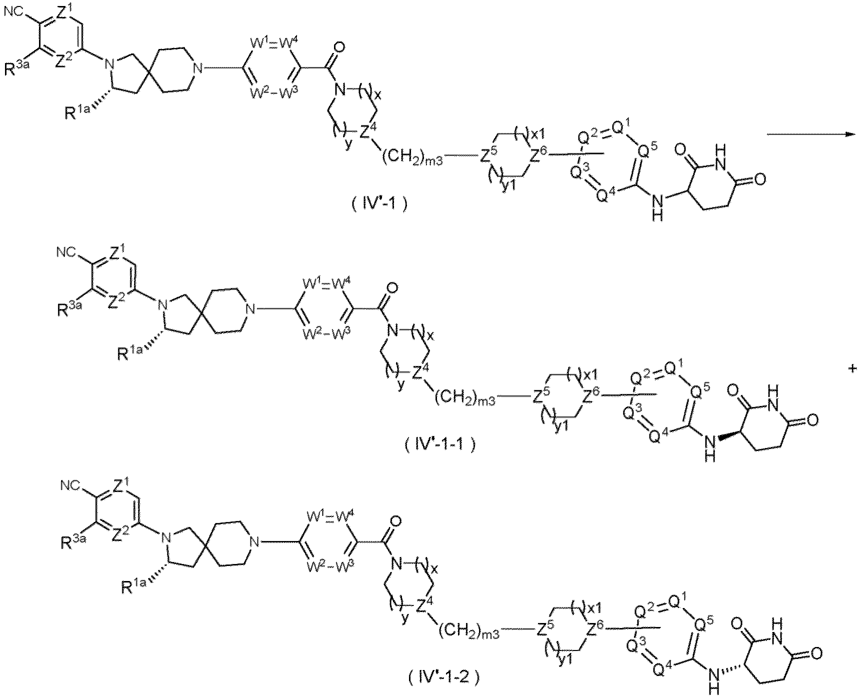 chirally resolving a compound represented by general formula (IV-1) or a pharmaceutically acceptable salt thereof to give the compounds represented by general formula (IV'-1-1) and general formula (IV-1-2) or the pharmaceutically acceptable salts thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in general formula (IV'-1).

### Scheme 11

A method for preparing the compound represented by general formula (M) or the pharmaceutically acceptable salt thereof of the present disclosure, comprising the following step: 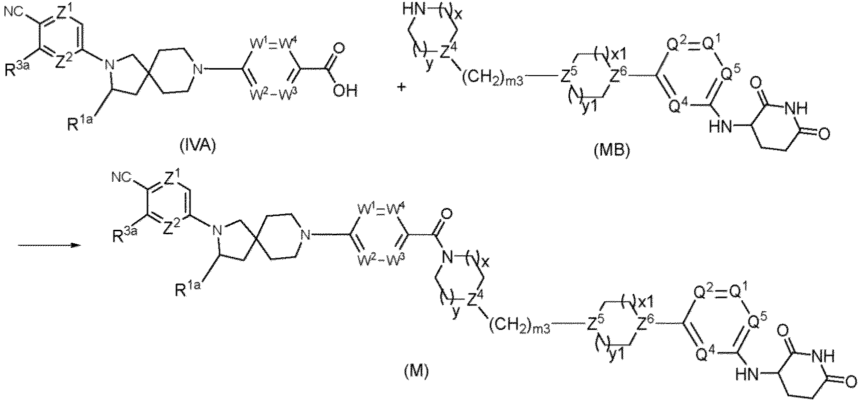 conducting a condensation reaction of a compound represented by general formula (IVA) or a salt thereof with a compound represented by general formula (MB) or a salt thereof (preferably hydrochloride and trifluoroacetate) under alkaline conditions in the presence of a condensing agent to give the compound represented by general formula (M) or the pharmaceutically acceptable salt thereof;
wherein:
R^{1a}, Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M).

### Scheme 12

A method for preparing the compound represented by general formula (M-1) or the pharmaceutically acceptable salt thereof of the present disclosure, comprising the following step: 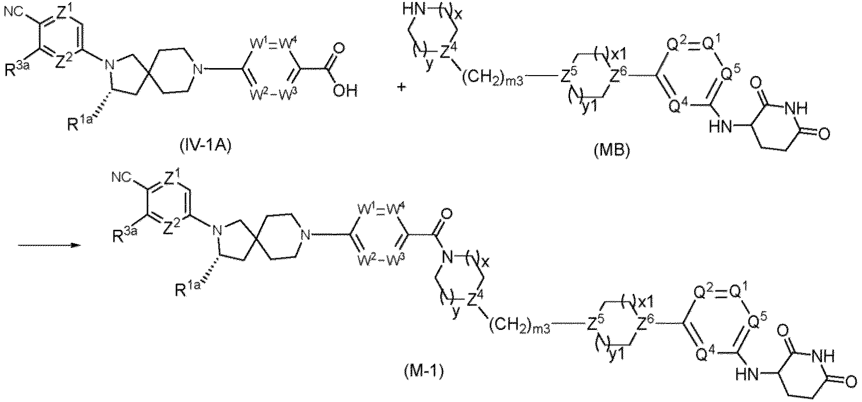 conducting a condensation reaction of a compound represented by general formula (IV-1A) or a salt thereof with a compound represented by general formula (MB) or a salt thereof (preferably hydrochloride and trifluoroacetate) under alkaline conditions in the presence of a condensing agent to give the compound represented by general formula (M-1) or the pharmaceutically acceptable salt thereof;
wherein:
R^{1a}, Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M-1).

### Scheme 13

A method for preparing the compounds represented by general formula (M-1-1) and general formula (M-1-2) or the pharmaceutically acceptable salts thereof of the present disclosure, comprising the following step: 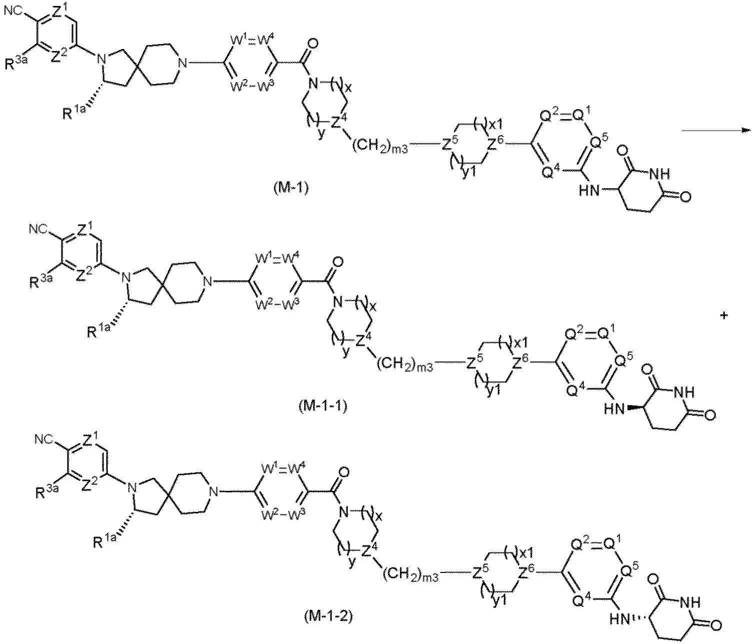 chirally resolving a compound represented by general formula (M-1) or a pharmaceutically acceptable salt thereof to give the compounds represented by general formula (M-1-1) and general formula (M-1-2) or the pharmaceutically acceptable salts thereof;
wherein:
R^{1a}, Q¹, Q², Q⁴, Q⁵, R^{3a}, W¹, W², W³, W⁴, Z¹, Z², Z⁴, Z⁵, Z⁶, x, x1, y, y1, and m3 are as defined in general formula (M-1).

### Scheme 14

A method for preparing the compound represented by general formula (IV) or the pharmaceutically acceptable salt thereof of the present disclosure, comprising the following step: 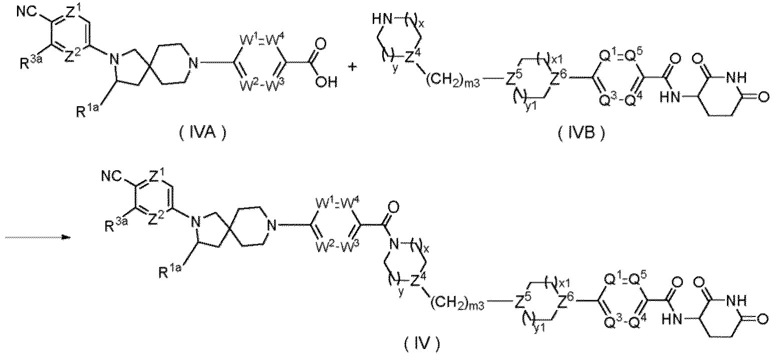 conducting a condensation reaction of a compound represented by general formula (IVA) or a salt thereof with a compound represented by general formula (IVB) or a salt thereof (preferably hydrochloride and trifluoroacetate) under alkaline conditions in the presence of a condensing agent to give the compound of general formula (IV) or the pharmaceutically acceptable salt thereof;
wherein:
R^{1a}, Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV).

### Scheme 15

A method for preparing the compound represented by general formula (IV-1) or the pharmaceutically acceptable salt thereof of the present disclosure, comprising the following step: 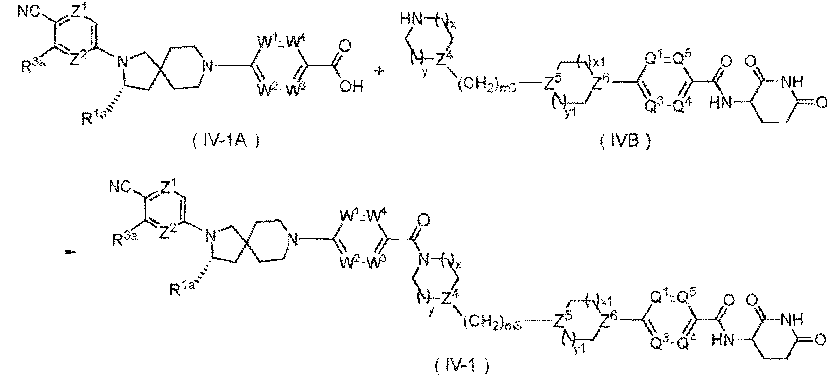 conducting a condensation reaction of a compound represented by general formula (IV-1A) or a salt thereof with a compound represented by general formula (IVB) or a salt thereof (preferably hydrochloride and trifluoroacetate) under alkaline conditions in the presence of a condensing agent to give the compound represented by general formula (IV-1) or the pharmaceutically acceptable salt thereof;
wherein:
R^{1a}, Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV-1).

### Scheme 16

A method for preparing the compounds represented by general formula (IV-1-1) and general formula (IV-1-2) or the pharmaceutically acceptable salts thereof of the present disclosure, comprising the following step: 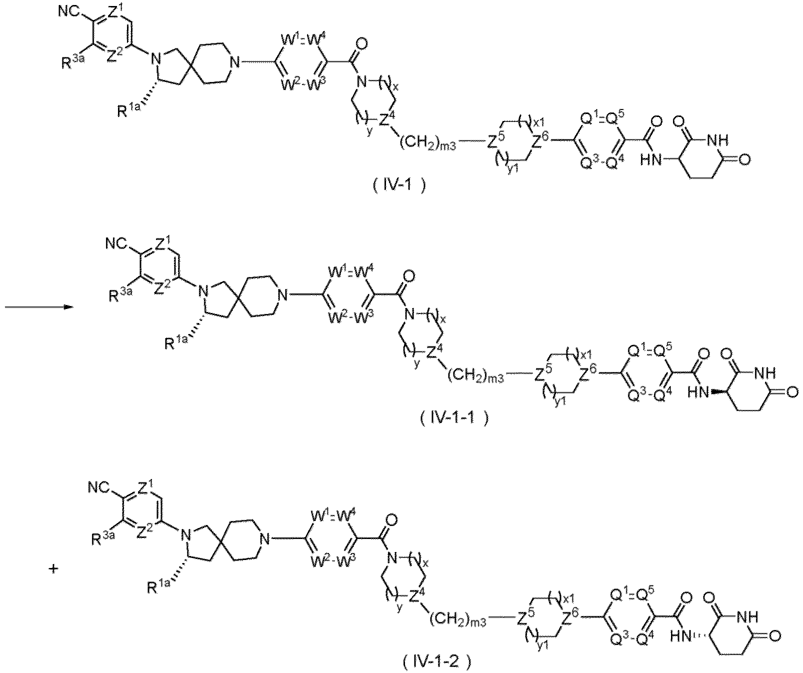 chirally resolving a compound represented by general formula (IV-1) or a pharmaceutically acceptable salt thereof to give the compounds represented by general formula (IV-1-1) and general formula (IV-1-2) or the pharmaceutically acceptable salts thereof;
wherein:
R^{1a}, Q¹, Q³, Q⁴, Q⁵, R^{3a}, W¹, W², W³, W⁴, Z¹, Z², Z⁴, Z⁵, Z⁶, x, x1, y, y1, and m3 are as defined in general formula (IV-1).

### Scheme 17

A method for preparing the compound represented by general formula (V) or the pharmaceutically acceptable salt thereof of the present disclosure, comprising the following step: 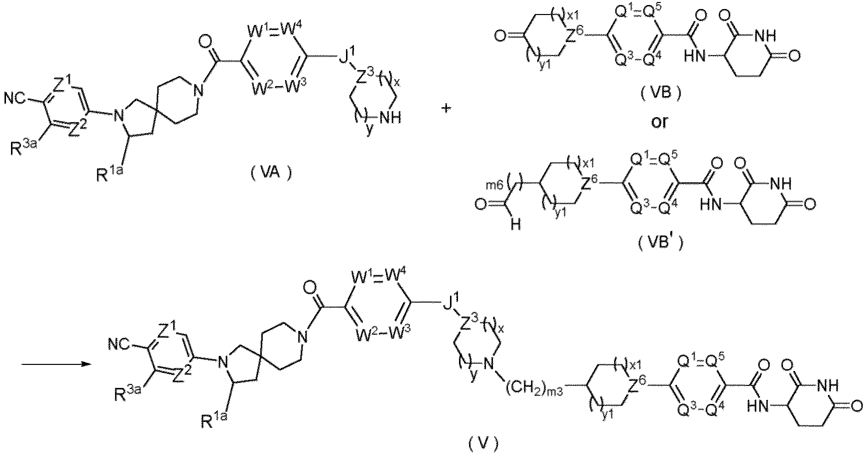
conducting a reductive amination reaction of a compound represented by general formula (VA) or a salt thereof (preferably hydrochloride and trifluoroacetate) with a compound represented by general formula (VB) or a salt thereof under weakly acidic conditions in the presence of a reductant to give the compound represented by general formula (V) or the pharmaceutically acceptable salt thereof, where m3 is 0;
or, conducting a reductive amination reaction of a compound represented by general formula (VA) or a salt thereof (preferably hydrochloride and trifluoroacetate) with a compound represented by general formula (VB') or a salt thereof under weakly acidic conditions in the presence of a reductant to give the compound represented by general formula (V) or the pharmaceutically acceptable salt thereof, where m3 is 1, 2, or 3;
wherein:
   m6 is 0, 1, or 2;
   J¹, R^{1a}, Q¹, Q³, Q⁴, Q⁵, R^{3a}, W¹, W², W³, W⁴, Z¹, Z², Z³, Z⁶, x, x1, y, and y1 are as defined in general formula (V).

### Scheme 18

A method for preparing the compound represented by general formula (V-1) or the pharmaceutically acceptable salt thereof of the present disclosure, comprising the following step: 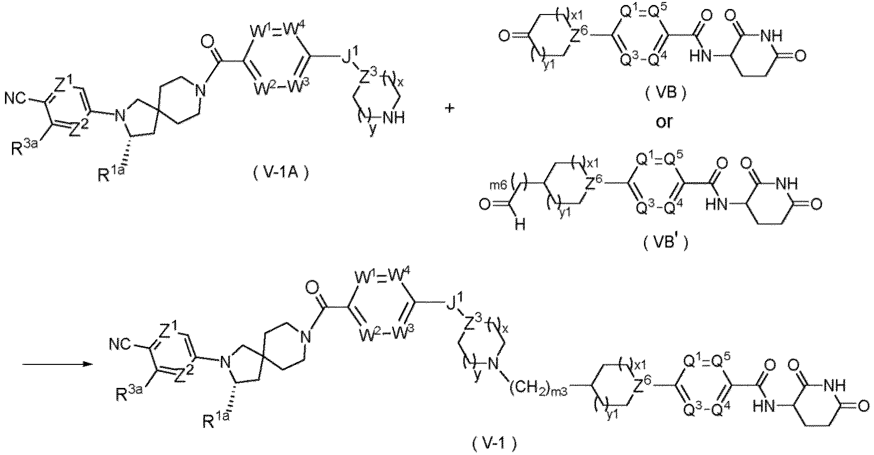
conducting a reductive amination reaction of a compound represented by general formula (V-1A) or a salt thereof (preferably hydrochloride and trifluoroacetate) with a compound represented by general formula (VB) or a salt thereof under weakly acidic conditions in the presence of a reductant to give the compound represented by general formula (V-1) or the pharmaceutically acceptable salt thereof, where m3 is 0;
or, conducting a reductive amination reaction of a compound represented by general formula (V-1A) or a salt thereof (preferably hydrochloride and trifluoroacetate) with a compound represented by general formula (VB') or a salt thereof under weakly acidic conditions in the presence of a reductant to give the compound represented by general formula (V-1) or the pharmaceutically acceptable salt thereof, where m3 is 1, 2, or 3;
wherein:
   m6 is 0, 1, or 2;
   J¹, R^{1a}, Q¹, Q³, Q⁴, Q⁵, R^{3a}, W¹, W², W³, W⁴, Z¹, Z², Z³, Z⁶, x, x1, y, and y1 are as defined in general formula (V-1).

### Scheme 19

A method for preparing the compounds represented by general formula (V-1-1) and general formula (V-1-2) or the pharmaceutically acceptable salts thereof of the present disclosure, comprising the following step: 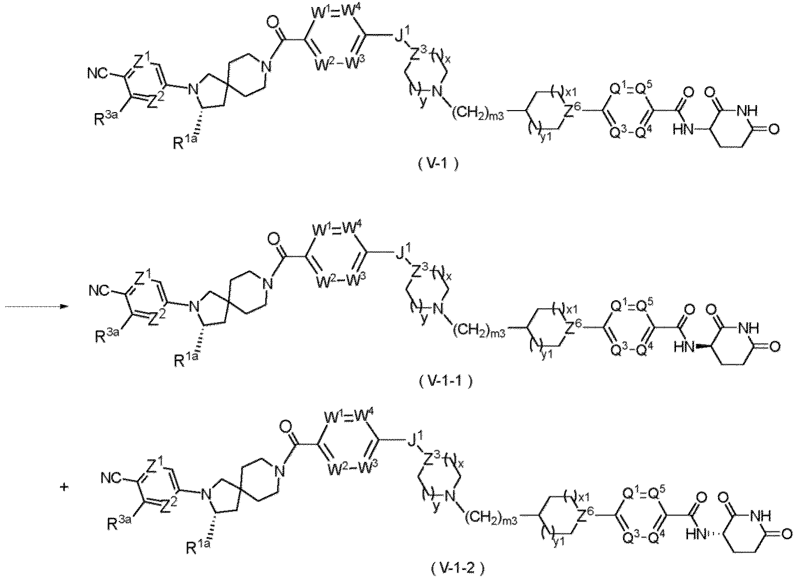 chirally resolving a compound represented by general formula (V-1) or a pharmaceutically acceptable salt thereof to give the compounds represented by general formula (V-1-1) and general formula (V-1-2) or the pharmaceutically acceptable salts thereof;
wherein:
J¹, R^{1a}, Q¹, Q³, Q⁴, Q⁵, R^{3a}, W¹, W², W³, W⁴, Z¹, Z², Z³, Z⁶, x, x1, y, y1, and m3 are as defined in general formula (V-1).

### Scheme 20

A method for preparing the compound represented by general formula (VI) or the pharmaceutically acceptable salt thereof of the present disclosure, comprising the following step: 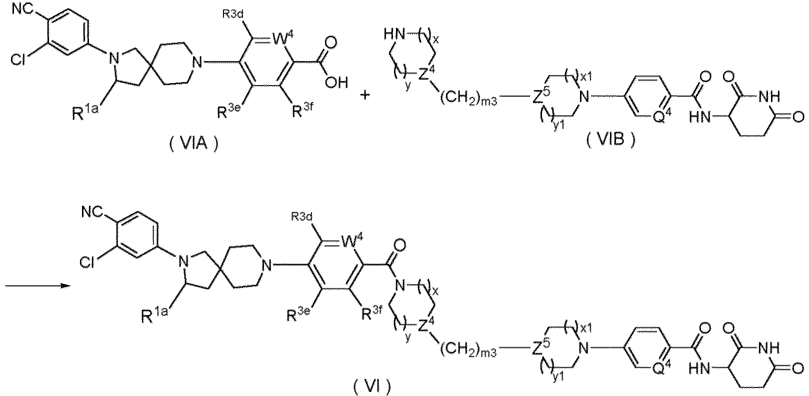 conducting a condensation reaction of a compound represented by general formula (VIA) or a salt thereof with a compound represented by general formula (VIB) or a salt thereof (preferably hydrochloride and trifluoroacetate) under alkaline conditions in the presence of a condensing agent to give the compound represented by general formula (VI) or the pharmaceutically acceptable salt thereof;
wherein:
R^{1a}, R^{3d}, R^{3e}, R^{3f}, W⁴, Z⁴, Z⁵, x, x1, y, y1, m3, and Q⁴ are as defined in general formula (VI).

### Scheme 21

A method for preparing the compound represented by general formula (VI-1) or the pharmaceutically acceptable salt thereof of the present disclosure, comprising the following step: 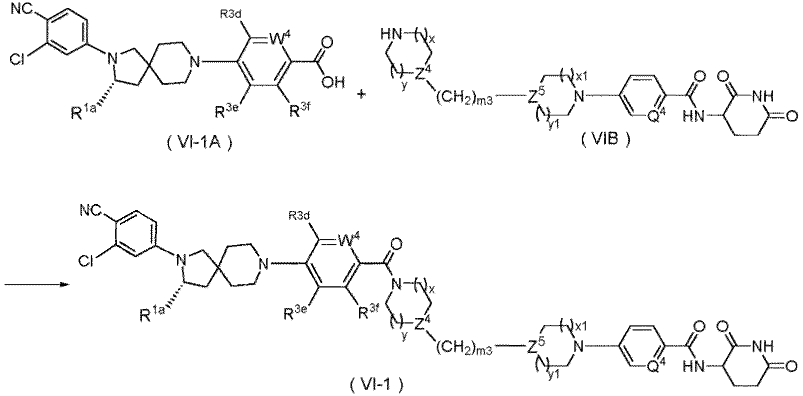 conducting a condensation reaction of a compound represented by general formula (VI-1A) or a salt thereof with a compound represented by general formula (VIB) or a salt thereof (preferably hydrochloride and trifluoroacetate) under alkaline conditions in the presence of a condensing agent to give the compound represented by general formula (VI-1) or the pharmaceutically acceptable salt thereof;
wherein:
R^{1a}, R^{3d}, R^{3e}, R^{3f}, W⁴, Z⁴, Z⁵, x, x1, y, y1, m3, and Q⁴ are as defined in general formula (VI-1).

### Scheme 22

A method for preparing the compounds represented by general formula (VI-1-1) and general formula (VI-1-2) or the pharmaceutically acceptable salts thereof of the present disclosure, comprising the following step: 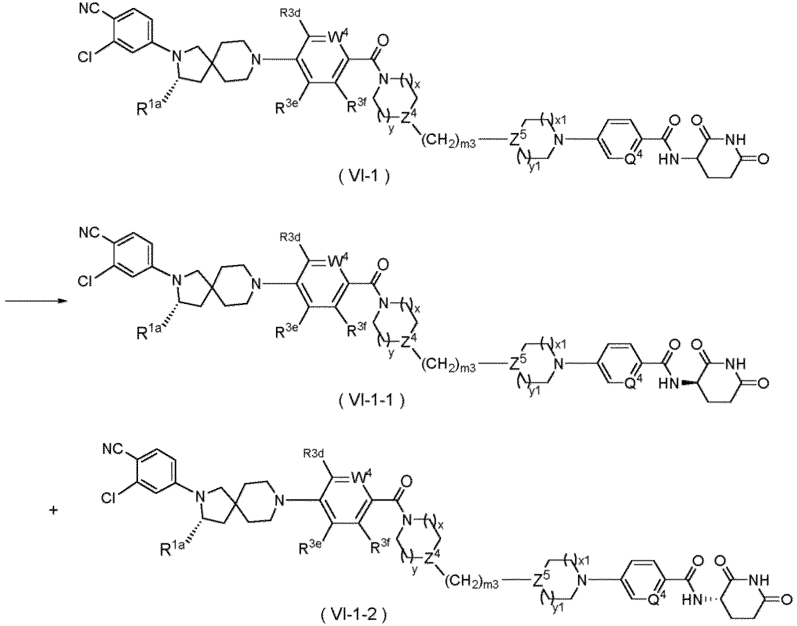 chirally resolving a compound represented by general formula (VI-1) or a pharmaceutically acceptable salt thereof to give the compounds represented by general formula (VI-1-1) and general formula (VI-1-2) or the pharmaceutically acceptable salts thereof;
wherein:
R^{1a}, R^{3d}, R^{3e}, R^{3f}, W⁴, Z⁴, Z⁵, x, x1, y, y1, m3, and Q⁴ are as defined in general formula (VI-1).

### Scheme 23

A method for preparing the compound represented by general formula (VII) and or the pharmaceutically acceptable salt thereof of the present disclosure, comprising the following step: 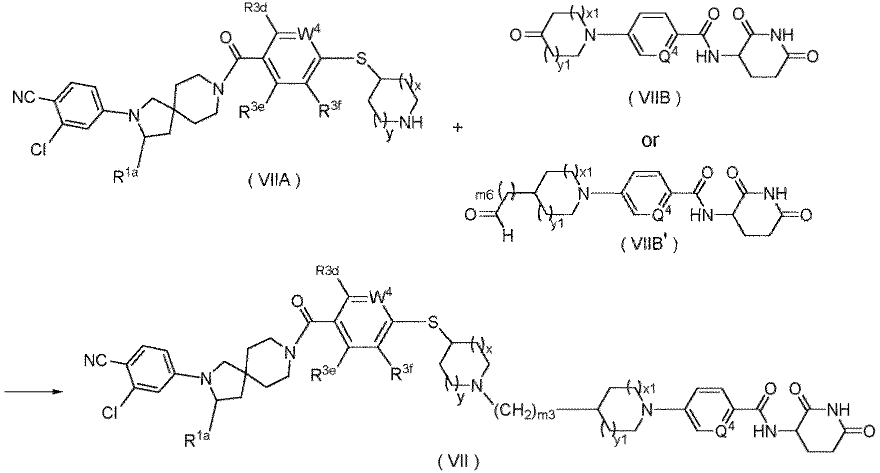 conducting a reductive amination reaction of a compound represented by general formula (VIIA) or a salt thereof (preferably hydrochloride and trifluoroacetate) with a compound represented by general formula (VIIB) or a salt thereof under weakly acidic conditions in the presence of a reductant to give the compound represented by general formula (VII) or the pharmaceutically acceptable salt thereof, where m3 is 0;
or, conducting a reductive amination reaction of a compound represented by general formula (VIIA) or a salt thereof (preferably hydrochloride and trifluoroacetate) with a compound represented by general formula (VIIB') or a salt thereof under weakly acidic conditions in the presence of a reductant to give the compound represented by general formula (VII) or the pharmaceutically acceptable salt thereof, where m3 is 1, 2, or 3;
wherein:
m6 is 0, 1, or 2;
R^{1a}, Q⁴, R^{3d}, R^{3e}, R^{3f}, W⁴, x, x1, y, and y1 are as defined in general formula (VII).

### Scheme 24

A method for preparing the compound represented by general formula (VII-1) or the pharmaceutically acceptable salt thereof of the present disclosure, comprising the following step: 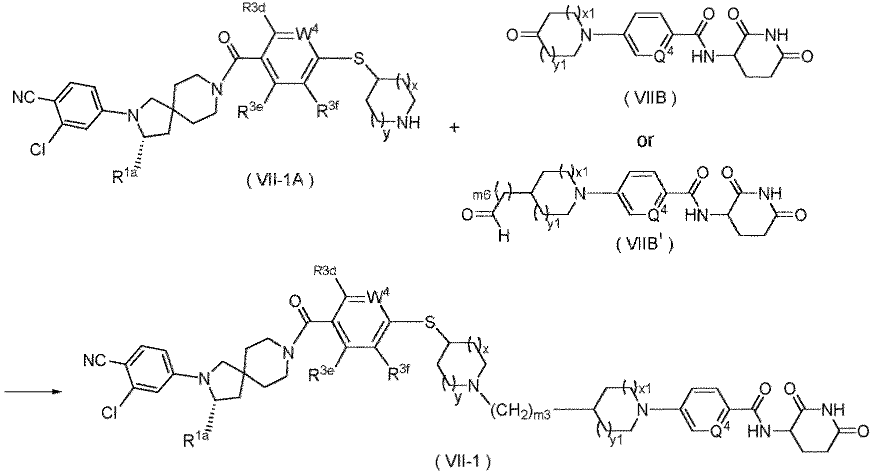
conducting a reductive amination reaction of a compound represented by general formula (VII-1A) or a salt thereof (preferably hydrochloride and trifluoroacetate) with a compound represented by general formula (VIIB) or a salt thereof under weakly acidic conditions in the presence of a reductant to give the compound represented by general formula (VII-1) or the pharmaceutically acceptable salt thereof, where m3 is 0;
or, conducting a reductive amination reaction of a compound represented by general formula (VII-1A) or a salt thereof (preferably hydrochloride and trifluoroacetate) with a compound represented by general formula (VIIB') or a salt thereof under weakly acidic conditions in the presence of a reductant to give the compound represented by general formula (VII-1) or the pharmaceutically acceptable salt thereof, where m3 is 1, 2, or 3;
wherein:
   m6 is 0, 1, or 2;
   R^{1a}, Q⁴, R^{3d}, R^{3e}, R^{3f}, W⁴, x, x1, y, and y1 are as defined in general formula (VII-1).

### Scheme 25

A method for preparing the compounds represented by general formula (VII-1-1) and general formula (VII-1-2) or the pharmaceutically acceptable salts thereof of the present disclosure, comprising the following step: 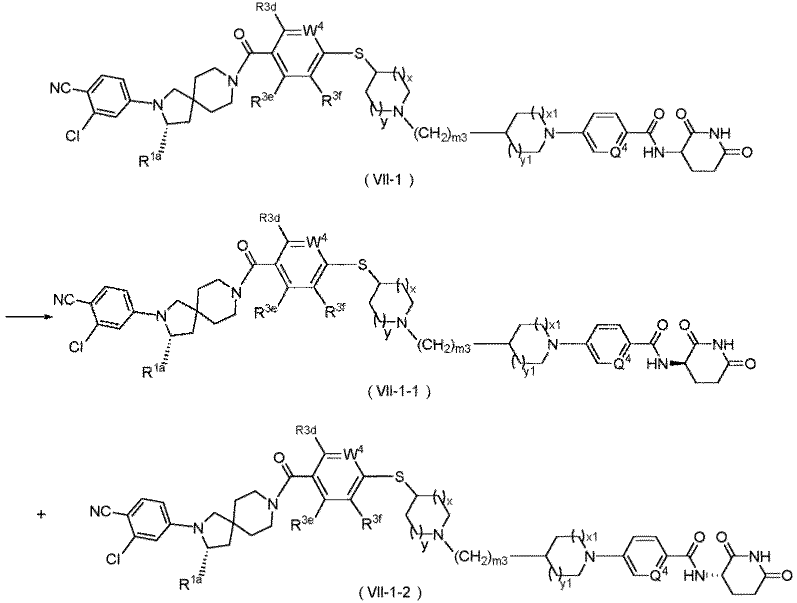 chirally resolving a compound represented by general formula (VII-1) or a pharmaceutically acceptable salt thereof to give the compounds represented by general formula (VII-1-1) and general formula (VII-1-2) or the pharmaceutically acceptable salts thereof;
wherein:
R^{1a}, Q⁴, R^{3d}, R^{3e}, R^{3f}, W⁴, m3, x, x1, y, and y1 are as defined in general formula (VII-1).

In the above synthesis schemes, the condensing agents include, but are not limited to, 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azabenzotriazole, O-benzotriazol-N,N,N',N'-tetramethyluronium hexafluorophosphate, *O*-(7-azabenzotriazol-1-yl)-*N*,*N*,*N'*,*N'*-tetramethyluronium hexafluorophosphate (HATU), 2-(7-oxybenzotriazole oxide)-N,N,N',N'-tetramethyluronium hexafluorophosphate, benzotriazol-1-yloxytris(dimethylamino)phosphonium hexafluorophosphate, or benzotriazol-1-yloxytripyrrolidinophosphonium hexafluorophosphate; preferably, the condensing agent is *O*-(7-azabenzotriazol-1-yl)-*N,N,N',N'*-tetramethyluronium hexafluorophosphate (HATU).

In the above synthesis schemes, reagents that provide the alkaline conditions include organic bases and inorganic bases; the organic bases include, but are not limited to, triethylamine, N,N-diisopropylethylamine, n-butyllithium, lithium diisopropylamide, potassium acetate, sodium acetate, sodium ethoxide, sodium tert-butoxide, or potassium tert-butoxide; the inorganic bases include, but are not limited to, sodium hydride, potassium phosphate, sodium carbonate, potassium carbonate, cesium carbonate, sodium hydroxide, lithium hydroxide monohydrate, lithium hydroxide, and potassium hydroxide; preferably, the reagent that provides the alkaline conditions is N,N-diisopropylethylamine.

In the above synthesis schemes, reagents that provide the weakly acidic conditions include, but are not limited to, acetic acid, Ti(i-PrO)₃, and BF₃·Et₂O; preferably, the agent that provides the weakly acidic conditions is acetic acid; or the weakly acidic conditions are provided by acids produced in the reaction, including, but not limited to, acetic acid. In the above synthesis schemes, the reductants include, but are not limited to, sodium triacetoxyborohydride, sodium borohydride, lithium borohydride, sodium cyanoborohydride, sodium acetylborohydride, and the like; sodium cyanoborohydride and sodium triacetoxyborohydride are preferred.

The above synthesis schemes are preferably carried out in solvents, including, but not limited to: ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylacetamide, N,N-dimethylformamide, and mixtures thereof.

### BRIEF DESCRIPTION OF THE DRAWINGS

FIG. 1 shows the efficacy of Example 15-1 and 11-2 or 11-1 (longer retention time) compounds against LNCap-FGC xenograft tumors in castrated CB17-SCID mice.
FIG. 2 shows the effects of Example 15-1 and 11-2 or 11-1 (longer retention time) compounds on the body weight of CB17-SCID mice.

### DETAILED DESCRIPTION

The present disclosure is further described below with reference to examples, but these examples are not intended to limit the scope of the present disclosure.

### Examples

The structures of the compounds were determined by nuclear magnetic resonance (NMR) spectroscopy or/and mass spectrometry (MS). NMR shifts (δ) are given in a unit of 10⁻⁶ (ppm). The NMR analyses were performed on a Bruker AVANCE-400 nuclear magnetic resonance instrument or Bruker AVANCE NEO 500M, with dimethyl sulfoxide-D6 (DMSO-*d₆*), chloroform-D (CDCl₃), and methanol-D4 (CD₃OD) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and
THERMO Ultimate 3000-Q Exactive (manufacturer: THERMO; MS model: THERMO Q Exactive).

The high performance liquid chromatography (HPLC) analyses were performed on Agilent HPLC 1200DAD, Agilent HPLC 1200VWD, and Waters HPLC e2695-2489 high performance liquid chromatographs.

The chiral HPLC analyses were performed on an Agilent 1260 DAD high performance liquid chromatograph.

The preparative high performance liquid chromatography steps were performed on Waters 2545-2767, Waters 2767-SQ Detecor2, Shimadzu LC-20AP, and Gilson GX-281 preparative chromatographs.

The preparative chiral chromatography steps were performed on a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) analyses had a layer thickness of 0.15 mm-0.2 mm, and those used in the thin-layer chromatography separation and purification had a layer thickness of 0.4 mm-0.5 mm.

In the silica gel column chromatography steps, a 200-300 mesh silica gel (Huanghai, Yantai) was generally used as the carrier.

The kinase mean inhibition rates and IC₅₀ values were measured using a NovoStar microplate reader (BMG, Germany).

The known starting materials in the present disclosure may be synthesized using or by following methods known in the art, or may be purchased from companies such as ABCR GmbH & Co. KG, Acros Organics, Aldrich Chemical Company, Accela ChemBio Inc., Chembee Chemicals, and the like.

In the examples, the reactions can all be performed under an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions refer to aqueous solutions unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvent used in the reactions, the eluent systems used in the column chromatography purification, and the developing solvent systems used in the thin-layer chromatography analyses include: A: a dichloromethane/methanol system, and B: a n-hexane/ethyl acetate system. The volume ratio of the solvents was adjusted according to the polarity of the compound, or by adding a small amount of basic or acidic reagents such as triethylamine and acetic acid.

### Example 1 4-(4-(1-(4-((S)-2-(3-Chloro-4-cyanophenyl)-3-methyl-2,8-diazaspiro[4.5]decan-8-yl)benzoyl)piperidin-4-yl)piperazin-1-yl)-N (2,6-dioxopiperidin-3-yl)-2-fluorobenzamide 1 (a mixture of diastereomers)

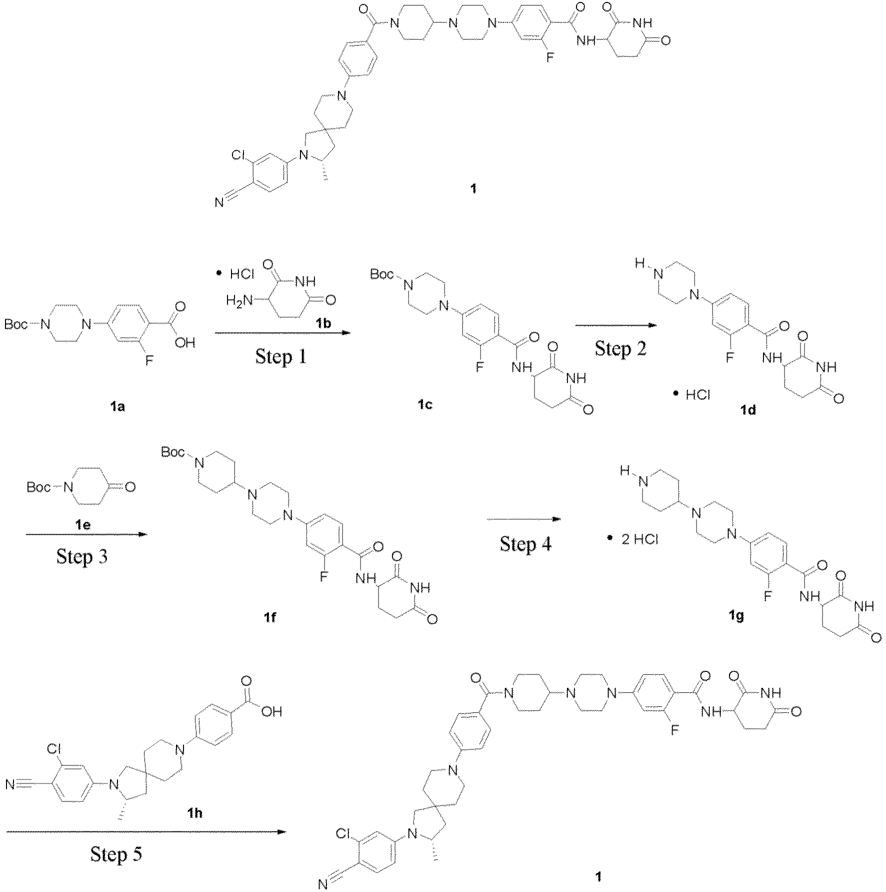

### Step 1 tert-Butyl (±)-4-(4-((2,6-dioxopiperidin-3-yl)aminocarbonyl)-3-fluorophenyl)piperazine-1-carboxylate 1c

The compound 4-(4-(tert-butoxycarbonyl)piperazin-1-yl)-2-fluorobenzoic acid **1a** (3.0 g, 9.25 mmol, prepared using the well-known method *"*ChemBioChem, 2014, 15(8), 1111-1120") was dissolved in *N,N-*dimethylformamide (25 mL), and *O*-(7-azabenzotriazol-1-yl)-N,N,N,N-tetramethyluronium hexafluorophosphate (5.28 g, 13.87 mmol) and *N,N-*diisopropylethylamine (3.59 g, 27.75 mmol, 4.6 mL) were then added. After 10 minutes of stirring, (±)-3-aminopiperidine-2,6-dione hydrochloride **1b** (1.83 g, 11.10 mmol, Bide Pharmatech) was added, and the mixture was stirred for another 2 hours. The reaction mixture was added dropwise to water (30 mL), and saturated brine (about 20 mL) was then added to the water. The mixture was filtered, and the filter cake was washed with water (40 mL), collected, and dried *in vacuo* to give the title product **1c** (3.8 g, yield: 95%).

### Step 2 (±)-N-(2,6-Dioxopiperidin-3-yl)-2-fluoro-4-(piperazin-1-yl)benzamide hydrochloride 1d

Compound **1c** (3.8 g, 8.75 mmol) was dissolved in dichloromethane (40 mL), and a 4 M solution of hydrogen chloride in 1,4-dioxane (17.5 mL) was added. The mixture was stirred for 2 hours and filtered, and the filter cake was stirred in 50 mL of a mixed solvent of ethyl acetate and methanol (V/V = 20:1). The filter cake was repeatedly treated twice to give the title compound **1d** (2.53 g, yield: 78%).

MS m/z (ESI): 335.1 [M+1].

### Step 3 tert-Butyl (±)-4-(4-(4-((2,6-dioxopiperidin-3-yl)aminocarbonyl)-3-fluorophenyl)piperazin-1-yl)piperidine-1-carboxylate 1f

To a 25 mL three-necked flask were sequentially added dichloromethane (5 mL), methanol (1 mL), and compound **1d** (80 mg, 0.21 mmol), followed by anhydrous sodium acetate (89 mg, 1.08 mmol). After 15 minutes of stirring, acetic acid (26 mg, 0.43 mmol) and tert-butyl 4-oxopiperi dine-1-carboxylate **1e** (86 mg, 0.43 mmol, Bide Pharmatech) were added. After 30 minutes of stirring at room temperature, sodium triacetoxyborohydride (92 mg, 0.43 mmol) was slowly added. After 16 hours of reaction, compound **1e** (344 mg, 1.73 mmol) and sodium triacetoxyborohydride (92 mg, 0.43 mmol) were added again, and the mixture was heated to 40 °C and left to react for another 12 hours. 10 mL of a saturated sodium bicarbonate solution was added to the system under ice bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system A to give the title compound **1f** (65 mg, yield: 58%).

MS m/z (ESI): 518.8 [M+1].

### Step 4 (±)-N-(2,6-Dioxopiperidin-3-yl)-2-fluoro-4-(4-(piperidin-4-yl)piperazin-1-yl)benzamide dihydrochloride 1g

Compound **1f** (80 mg, 0.15 mmol) was dissolved in dichloromethane (3 mL), and a 4 M solution of hydrogen chloride in 1,4-dioxane (1.1 mL) was slowly added under an ice bath. The mixture was left to react for 3 hours. The reaction mixture was concentrated under reduced pressure and dried *in vacuo* to give the title compound **1g** as a crude product (75 mg, yield: 100%). The crude product was directly used in the next step without purification.

MS m/z (ESI): 418.3 [M+1].

### Step 5 4-(4-(1-(4-((S)-2-(3-Chloro-4-cyanophenyl)-3-methyl-2,8-diazaspiro[4.5]decan-8-yl)benzoyl)piperidin-4-yl)piperazin-1-yl)-N (2,6-dioxopiperidin-3-yl)-2-fluorobenzamide 1 (a mixture of diastereomers)

To a 25 mL three-necked flask were sequentially added *N,N*-dimethylformamide (2 mL), compound **1g** (50 mg, 0.10 mmol), and the compound (5)-4-(2-(3-chloro-4-cyanophenyl)-3-methyl-2,8-diazaspiro[4.5]decan-8-yl)benzoic acid **1h** (50 mg, 0.078 mmol, prepared using the method disclosed in "Example 10 on page 211 of the specification in the patent application WO2021055756A1"). *N,N-*Diisopropylethylamine (102 mg, 0.79 mmol) and *O*-(7-azabenzotriazol-1-yl)-*N,N,N',N'*-tetramethyluronium hexafluorophosphate (60 mg, 0.16 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 36%-51%, flow rate: 30 mL/min) to give the title compound **1** (a mixture of diastereomers, a 1:1 ratio, 26 mg, yield: 41%).

MS m/z (ESI): 809.3 [M+1].

¹H NMR (500 MHz, DMSO-*d₆*): δ 10.84 (s, 1H), 8.04 (t, 1H), 7.72-7.52 (m, 2H), 7.26 (d, 2H), 6.95 (d, 2H), 6.87-6.72 (m, 3H), 6.66 (d, 1H), 4.78-4.64 (m, 1H), 4.34-3.92 (m, 3H), 3.52-3.42 (m, 2H), 3.40-3.34 (m, 2H), 3.29-3.12 (m, 5H), 3.02-2.71 (m, 5H), 2.68-2.54 (m, 4H), 2.28-2.18 (m, 1H), 2.10-2.05 (m, 1H), 2.04-1.96 (m, 1H), 1.90-1.66 (m, 4H), 1.64-1.55 (m, 1H), 1.53-1.44 (m, 2H), 1.42-1.32 (m, 2H), 1.30-1.13 (m, 4H).

### Example 2 5-(4-(1-(4-((S)-2-(3-Chloro-4-cyanophenyl)-3-methyl-2,8-diazaspiro[4.5]decan-8-yl)benzoyl)piperidin-4-yl)piperazin-1-yl)-N (2,6-dioxopiperidin-3-yl)picolinamide 2 (a mixture of diastereomers)

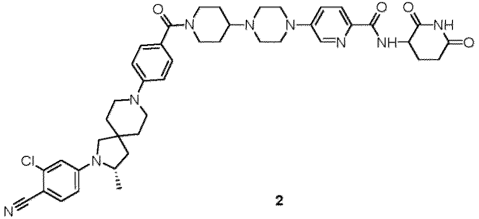

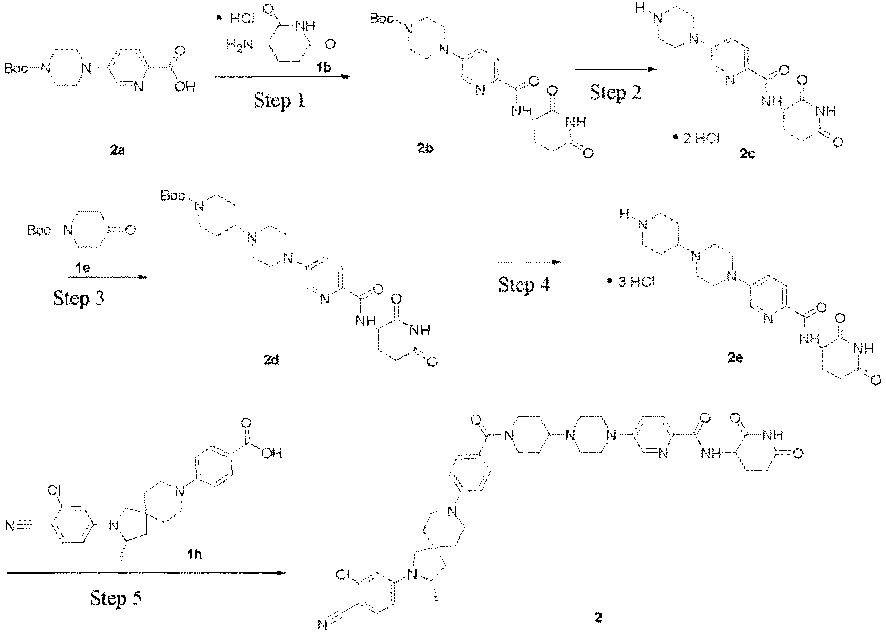

### Step 1 tert-Butyl (±)-4-(6-((2,6-dioxopiperidin-3-yl)aminocarbonyl)pyridin-3-yl)piperazine-1-carboxylate 2b

To a 100 mL three-necked flask were sequentially added *N,N-*dimethylformamide (30 mL), 5-(4-(tert-butoxycarbonyl)piperazin-1-yl)picolinic acid **2a** (2.9 g, 9.44 mmol, prepared using the method disclosed in "Example 21 on page 95 of the specification in the patent application WO2012003189A1"), compound **1b** (1.86 g, 11.32 mmol), O-(7-azabenzotriazol-1-yl)-*N,N,N',N'*-tetramethyluronium hexafluorophosphate (5.38 g, 14.14 mmol), and triethylamine (2.86 g, 28.31 mmol), and the mixture was stirred at room temperature for 1 hour. Water (20 mL) was added to the system, and extraction was performed with ethyl acetate (50 mL × 3). The organic phases were combined, washed with a saturated sodium chloride solution (20 mL × 2), dried over anhydrous sodium sulfate, and filtered. The filtrate was concentrated under reduced pressure, and the resulting residue was stirred in 50 mL of a mixed solvent of n-heptane and ethyl acetate (V/V = 20:1). The mixture was filtered, and the filter cake was collected to give the title compound **2b** (2.9 g, yield: 74%).

### Step 2 (±)-N-(2,6-Dioxopiperidin-3-yl)-5-(piperazin-1-yl)picolinamide dihydrochloride 2c

Compound **2b** (2.9 g, 6.95 mmol) was dissolved in dichloromethane (60 mL), and a 4 M solution of hydrogen chloride in 1,4-dioxane (30 mL) was added. The mixture was stirred at room temperature for 3 hours. The reaction mixture was filtered, and the filter cake was washed sequentially with dichloromethane (5 mL) and ethyl acetate (5 mL) and dried *in vacuo* to give the title compound **2c** (2.7 g, yield: 100%).

MS m/z (ESI): 318.1 [M+1].

### Step 3 tert-Butyl (±)-4-(4-(6-((2,6-dioxopiperidin-3-yl)aminocarbonyl)pyridin-3-yl)piperazin-1-yl)piperidine-1-carboxylate 2d

To a 25 mL three-necked flask were sequentially added dichloromethane (5 mL), methanol (1 mL), and compound **2c** (80 mg, 0.20 mmol), followed by anhydrous sodium acetate (93 mg, 1.13 mmol). After 15 minutes of stirring, acetic acid (28 mg, 0.47 mmol) and compound **1e** (90 mg, 0.45 mmol) were added. After 30 minutes of stirring at room temperature, sodium triacetoxyborohydride (96 mg, 0.45 mmol) was slowly added. After 16 hours of reaction, compound **1e** (360 mg, 1.80 mmol) and sodium triacetoxyborohydride (96 mg, 0.45 mmol) were added again, and the mixture was heated to 40 °C and left to react for another 12 hours. 10 mL of a saturated sodium bicarbonate solution was added to the system under ice bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system A to give the title compound **2d** (63 mg, yield: 63%).

MS m/z (ESI): 501.5 [M+1].

### Step 4 (±)-N-(2,6-Dioxopiperidin-3-yl)-5-(4-(piperidin-4-yl)piperazin-1-yl)picolinamide trihydrochloride 2e

Compound **2d** (60 mg, 0.12 mmol) was dissolved in dichloromethane (3 mL), and a 4 M solution of hydrogen chloride in 1,4-dioxane (0.9 mL) was slowly added under an ice bath. The mixture was left to react for 2 hours. The reaction mixture was concentrated under reduced pressure and dried *in vacuo* to give the title compound **2e** as a crude product (61 mg, yield: 100%). The crude product was directly used in the next step without purification.

MS m/z (ESI): 401.4 [M+1].

### Step 5 5-(4-(1-(4-((S)-2-(3-Chloro-4-cyanophenyl)-3-methyl-2,8-diazaspiro[4.5]decan-8-yl)benzoyl)piperidin-4-yl)piperazin-1-yl)-N (2,6-dioxopiperidin-3-yl)picolinamide 2 (a mixture of diastereomers)

To a 25 mL three-necked flask were sequentially added *N,N-*dimethylformamide (2 mL), compound **2e** (52 mg, 0.10 mmol), and compound **1h** (50 mg, 0.078 mmol). *N,N-*Diisopropylethylamine (102 mg, 0.79 mmol) and O-(7-azabenzotriazol-1-yl)-N,N,N,N-tetramethyluronium hexafluorophosphate (60 mg, 0.16 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 36%-51%, flow rate: 30 mL/min) to give the title compound **2** (a mixture of diastereomers, a 1:1 ratio, 32 mg, yield: 51%).

MS m/z (ESI): 792.3 [M+1].

¹H NMR (500 MHz, DMSO-*d₆*): δ 10.84 (s, 1H), 8.72 (d, 1H), 8.31 (s, 1H), 7.86 (d, 1H), 7.59 (d, 1H), 7.41 (d, 1H), 7.26 (d, 2H), 6.95 (d, 2H), 6.80 (s, 1H), 6.66 (d, 1H), 4.84-4.61 (m, 1H), 4.40-3.88 (m, 3H), 3.60-3.32 (m, 7H), 3.28-3.06 (m, 2H), 3.02-2.72 (m, 3H), 2.71-2.57 (m, 6H), 2.33-2.09 (m, 2H), 2.07-1.94 (m, 1H), 1.91-1.65 (m, 4H), 1.62-1.31 (m, 5H), 1.30-0.98 (m, 4H).

### Example 3 4-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl)-N-(2,6-dioxopiperidin-3-yl)-2-fluorobenzamide 3 (a mixture of diastereomers)

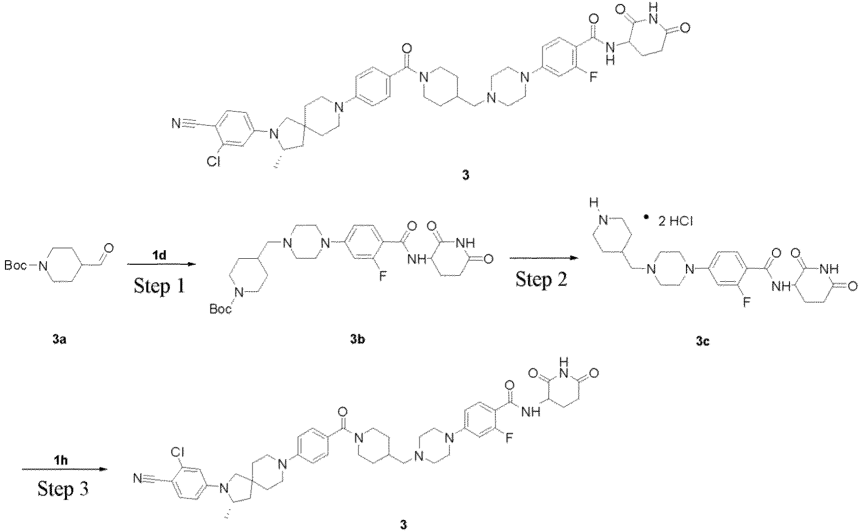

### Step 1 tert-Butyl (±)-4-((4-(4-((2,6-dioxopiperidin-3-yl)aminocarbonyl)-3-fluorophenyl)piperazin-1-yl)methyl)piperidine-1-carboxylate 3b

To a 25 mL three-necked flask were sequentially added dichloromethane (4 mL), methanol (1 mL), and compound **1d** (80 mg, 0.22 mmol), followed by anhydrous sodium acetate (54 mg, 0.66 mmol). After 15 minutes of stirring, tert-butyl 4-formylpiperidine-1-carboxylate **3a** (65 mg, 0.30 mmol, Bide Pharmatech) was added. After 30 minutes of stirring at room temperature, sodium triacetoxyborohydride (92 mg, 0.43 mmol) was slowly added, and the mixture was left to react for 16 hours. 10 mL of a saturated sodium bicarbonate solution was added to the system under ice bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system A to give the title compound **3b** (82 mg, yield: 71%). MS m/z (ESI): 530.2 [M-1].

### Step 2 (±)-N-(2,6-Dioxopiperidin-3-yl)-2-fluoro-4-(4-(piperidin-4-yl)methyl)piperazin-1-yl)benzamide dihydrochloride 3c

Compound **3b** (82 mg, 0.15 mmol) was dissolved in dichloromethane (3 mL), and a 4 M solution of hydrogen chloride in 1,4-dioxane (0.8 mL) was slowly added under an ice bath. The mixture was left to react for 3 hours. The reaction mixture was concentrated under reduced pressure and dried *in vacuo* to give the title compound 3c as a crude product (77 mg, yield: 100%). The crude product was directly used in the next step without purification.

MS m/z (ESI): 432.4 [M+1].

### Step 3 4-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl)-N-(2,6-dioxopiperidin-3-yl)-2-fluorobenzamide 3 (a mixture of diastereomers)

To a 25 mL three-necked flask were sequentially added *N,N*-dimethylformamide (2 mL), compound **3c** (51 mg, 0.10 mmol), and compound **1h** (50 mg, 0.078 mmol). *N,N-*Diisopropylethylamine (152 mg, 1.18 mmol) and O-(7-azabenzotriazol-1-yl)-N,N,N,N-tetramethyluronium hexafluorophosphate (60 mg, 0.16 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 36%-51%, flow rate: 30 mL/min) to give the title compound **3** (a mixture of diastereomers, a 1:1 ratio, 48 mg, yield: 74%).

MS m/z (ESI): 823.6 [M+1].

¹H NMR (500 MHz, DMSO-*d₆*): δ 10.85 (s, 1H), 8.05 (t, 1H), 7.72-7.52 (m, 2H), 7.25 (d, 2H), 6.95 (d, 2H), 6.88-6.72 (m, 3H), 6.66 (d, 1H), 4.80-4.64 (m, 1H), 4.34-3.82 (m, 3H), 3.44 (d, 1H), 3.32-3.05 (m, 7H), 3.03-2.66 (m, 4H), 2.48-2.35 (m, 4H), 2.32-2.07 (m, 4H), 2.05-1.94 (m, 1H), 1.92-1.66 (m, 5H), 1.62-1.37 (m, 4H), 1.35-1.17 (m, 4H), 1.14-0.95 (m, 2H).

### Example 4 5-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl) N (2,6-dioxopiperidin-3-yl)picolinamide 4 (a mixture of diastereomers)

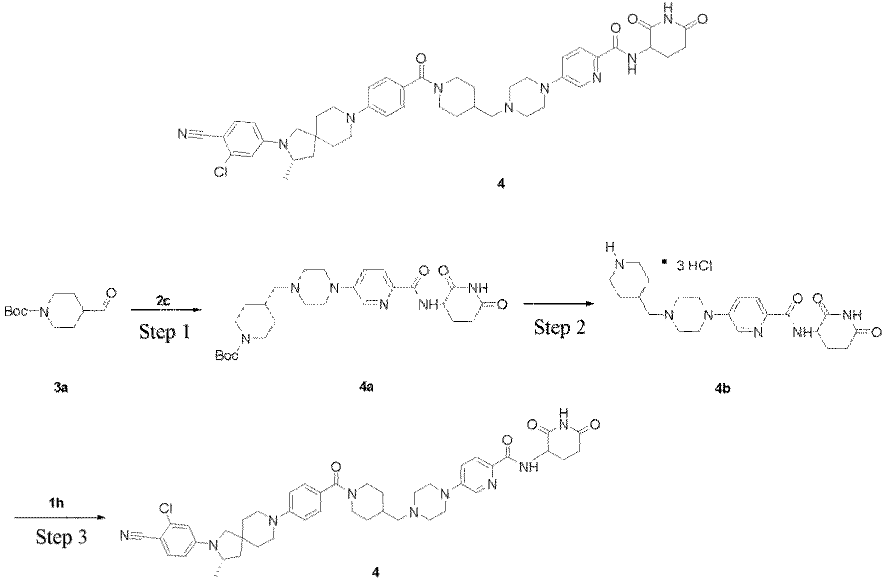

### Step 1 tert-Butyl (±)-4-((4-(6-((2,6-dioxopiperidin-3-yl)aminocarbonyl)pyridin-3-yl)piperazin-1-yl)methyl)piperidine-1-carboxylate 4a

To a 25 mL three-necked flask were sequentially added dichloromethane (4 mL), methanol (1 mL), and compound **2c** (100 mg, 0.28 mmol), followed by anhydrous sodium acetate (70 mg, 0.85 mmol). After 15 minutes of stirring, compound **3a** (80 mg, 0.38 mmol) was added. After 30 minutes of stirring at room temperature, sodium triacetoxyborohydride (120 mg, 0.57 mmol) was slowly added, and the mixture was left to react for 16 hours. 10 mL of a saturated sodium bicarbonate solution was added to the system under ice bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system A to give the title compound **4a** (125 mg, yield: 85%).

MS m/z (ESI): 515.4 [M+1].

### Step 2 (±)-N-(2,6-Dioxopiperidin-3-yl)-5-(4-(piperidin-4-yl)methyl)piperazin-1-yl)picolinamide trihydrochloride 4b

Compound **4a** (125 mg, 0.24 mmol) was dissolved in dichloromethane (3 mL), and a 4 M solution of hydrogen chloride in 1,4-dioxane (1.2 mL) was slowly added under an ice bath. The mixture was left to react for 4 hours. The reaction mixture was concentrated under reduced pressure and dried *in vacuo* to give the title compound **4b** as a crude product (126 mg, yield: 100%). The crude product was directly used in the next step without purification.

MS m/z (ESI): 415.5 [M+1].

### Step 3 5-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl) N (2,6-dioxopiperidin-3-yl)picolinamide 4 (a mixture of diastereomers)

To a 25 mL three-necked flask were sequentially added *N,N*-dimethylformamide (2 mL), compound **4b** (51 mg, 0.10 mmol), and compound **1h** (50 mg, 0.078 mmol). *N,N-*Diisopropylethylamine (152 mg, 1.18 mmol) and *O*-(7-azabenzotriazol-1-yl)-*N,N,N',N'-*tetramethyluronium hexafluorophosphate (60 mg, 0.16 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 36%-51%, flow rate: 30 mL/min) to give the title compound **4** (a mixture of diastereomers, a 1:1 ratio, 35 mg, yield: 55%).

MS m/z (ESI): 806.2 [M+1].

¹H NMR (500 MHz, DMSO-*d₆*): δ 10.85 (s, 1H), 8.73 (d, 1H), 8.32 (s, 1H), 7.87 (d, 1H), 7.60 (d, 1H), 7.42 (d, 1H), 7.25 (d, 2H), 6.95 (d, 2H), 6.80 (s, 1H), 6.66 (d, 1H), 4.86-4.63 (m, 1H), 4.28-3.82 (m, 3H), 3.44 (d, 1H), 3.41-3.32 (m, 7H), 3.26-3.10 (m, 2H), 2.98-2.72 (m, 3H), 2.64-2.53 (m, 4H), 2.32-2.14 (m, 4H), 2.07-1.94 (m, 1H), 1.92-1.66 (m, 5H), 1.62-1.56 (m, 1H), 1.52-1.40 (m, 2H), 1.33-1.17 (m, 4H), 1.14-0.98 (m, 2H).

### Example 5 4-(3-(4-((5-((S)-2-(3-Chloro-4-cyanophenyl)-3-methyl-2,8-diazaspiro[4.5]decane-8-carbonyl)pyridin-2-yl)thio)piperidin-1-yl)azetidin-1-yl)-N-(2,6-dioxopiperidin-3-yl)-2-fluorobenzamide 5 (a mixture of diastereomers)

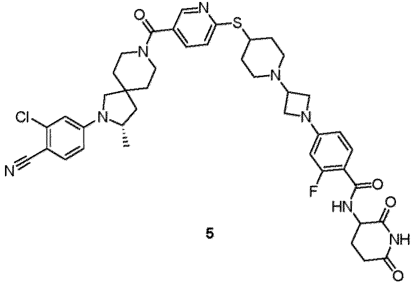

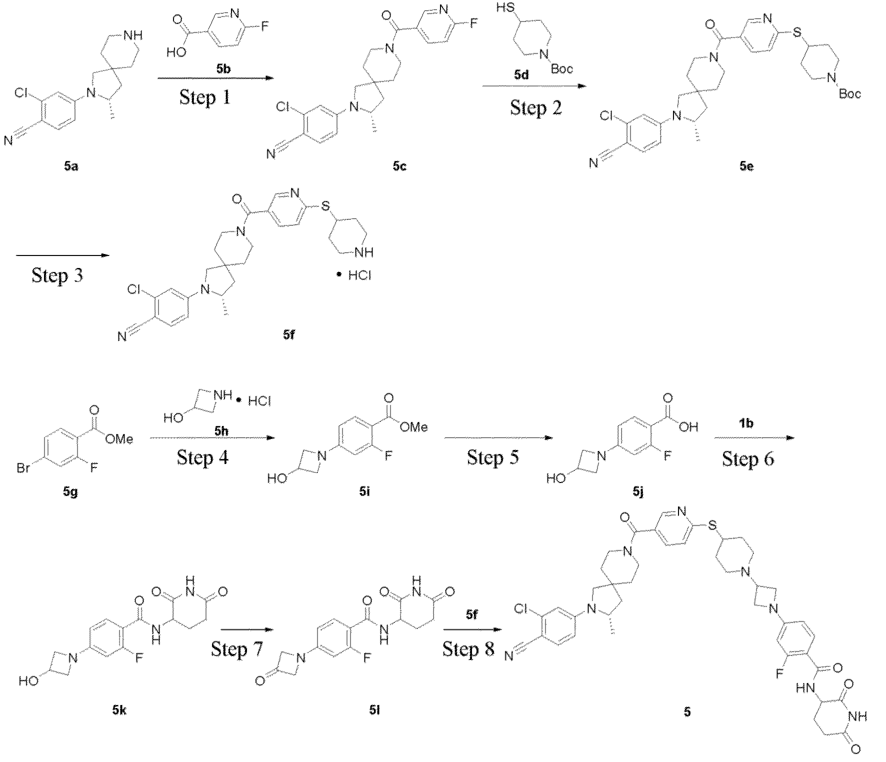

### Step 1

### (S)-2-Chloro-4-(8-(6-fluoronicotinoyl)-3-methyl-2,8-diazaspiro[4.5]decan-2-yl)benzonitrile 5c

6-Fluoronicotinic acid **5b** (139 mg, 0.98 mmol, Bide Pharmatech) was weighed into a 50 mL eggplant-shaped flask. *N,N*-Dimethylformamide (3 mL) was added, and *N,N-*diisopropylethylamine (446 mg, 3.45 mmol, 0.86 mL) and *O*-(7-azabenzotriazole-1-yl)-*N,N,N',N*'-tetramethyluronium hexafluorophosphate (563 mg, 1.48 mmol) were then added. After 15 minutes of stirring, the compound (S)-2-chloro-4-(3-methyl-2,8-diazaspiro[4.5]decan-2-yl)benzonitrile **5a** (284 mg, 0.98 mmol, prepared using the method disclosed in "Example 10 on page 211 of the specification in the patent application WO2021055756A1") was added, and the mixture was left to react for 2 hours. Water (5 mL) was added to the system, and extraction was performed with ethyl acetate (10 mL × 3). The organic phases were combined, washed with a saturated sodium chloride solution (10 mL), dried over anhydrous sodium sulfate, filtered, and concentrated under reduced pressure. The resulting residue was purified by silica gel column chromatography with eluent system B to give the title compound **5c** (407 mg, yield: 100%). MS m/z (ESI): 413.2 [M+1].

### Step 2 tert-Butyl (S)-4-((5-(2-(3-chloro-4-cyanophenyl)-3-methyl-2,8-diazaspiro[4.5]decane-8-carbonyl)pyridin-2-yl)thio)piperidine-1-carboxylate 5e

Compound **5c** (407 mg, 0.98 mmol) and tert-butyl 4-mercaptopiperidine-1-carboxylate **5d** (428 mg, 1.96 mmol) were weighed into a 50 mL eggplant-shaped flask. *N,N-*Dimethylformamide (5 mL) and cesium carbonate (481 mg, 1.48 mmol) were added, and the mixture was then left to react at 110 °C for 5 hours. The heating was stopped. Water (8 mL) was added to the system, and extraction was performed with ethyl acetate (15 mL × 3). The organic phases were combined, washed with a saturated sodium chloride solution (20 mL), dried over anhydrous sodium sulfate, filtered, and concentrated under reduced pressure. The resulting residue was purified by silica gel column chromatography with eluent system B to give the title compound **5e** (596 mg, yield: 99%).

MS m/z (ESI): 510.1 [M-99].

### Step 3 (S)-2-Chloro-4-(3-methyl-8-(6-(piperidin-4-ylthio)nicotinoyl)-2,8-diazaspiro[4.5]decan-2-yl)benzonitrile hydrochloride 5f

Compound **5e** (596 mg, 0.98 mmol) was weighed into a 25 mL eggplant-shaped flask, and a 4 M solution of hydrogen chloride in 1,4-dioxane (5 mL) was added. After 2 hours of reaction, the system was concentrated and dried to give the title compound **5f** (528 mg, yield: 99%). The crude product was directly used in the next step without purification. MS m/z (ESI): 510.1 [M+1].

### Step 4 Methyl 2-fluoro-4-(3-hydroxyazetidin-1-yl)benzoate 5i

Methyl 2-fluoro-4-bromobenzoate **5g** (3 g, 12.9 mmol, Bide Pharmatech) and azetidin-3-ol hydrochloride **5h** (1.41 g, 12.87 mmol, Bide Pharmatech) were weighed into a 100 mL eggplant-shaped flask, and palladium acetate (289 mg, 1.289 mmol), 2,2'-bis(diphenylphosphino)-1,1'-binaphthyl (962 mg, 1.55 mmol), and cesium carbonate (12.6 g, 38.67 mmol) were sequentially added. 1,4-Dioxane (30 mL) was added, and the mixture was then left to react at 105 °C for 5 hours. After being cooled to room temperature, the reaction mixture was filtered using celite, and the filter cake was washed with ethyl acetate (15 mL × 3). The filtrate was concentrated under reduced pressure, and the resulting residue was purified by silica gel column chromatography with eluent system B to give the title compound **5i** (1.8 g, yield: 62%).

MS m/z (ESI): 226.1 [M+1].

### Step 5 2-Fluoro-4-(3-hydroxyazetidin-1-yl)benzoic acid 5j

Compound **5i** (600 mg, 2.66 mmol) was weighed into a 50 mL eggplant-shaped flask and dissolved in tetrahydrofuran (10 mL), and a solution of sodium hydroxide (533 mg, 13.32 mmol) in water (2 mL) was then added. The mixture was left to react at 80 °C for 4 hours. After the reaction mixture was cooled to room temperature, 1 M hydrochloric acid was added to the system to adjust the pH to 3-4. The reaction mixture was concentrated under reduced pressure to remove the organic solvent and filtered, and the filter cake was collected to give the title compound **5j** (560 mg, 99%).

MS m/z (ESI): 212.3 [M+1].

### Step 6 (±)-N-(2,6-Dioxopiperidin-3-yl)-2-fluoro-4-(3-hydroxyazetidin-1-yl)benzamide 5k

Compound **5j** (653 mg, 3.09 mmol) was weighed into a 50 mL eggplant-shaped flask. N,N-Dimethylformamide (5 mL) was added, and *N,N-*diisopropylethylamine (1.2 g, 9.28 mmol, 1.61 mL) and *O*-(7-azabenzotriazole-1-yl)-*N,N,N',N*'-tetramethyluronium hexafluorophosphate (1.76 g, 4.63 mmol) were then added. After 15 minutes of stirring, compound **1b** (763 mg, 4.63 mmol) was added. After 2 hours of reaction, water (8 mL) was added to the system, and extraction was performed with ethyl acetate (15 mL × 3). The organic phases were combined, washed with a saturated sodium chloride solution (20 mL), dried over anhydrous sodium sulfate, filtered, and concentrated under reduced pressure. The resulting residue was purified by silica gel column chromatography with eluent system A to give the title compound **5k** (260 mg, 26%).

MS m/z (ESI): 322.3 [M+1].

### Step 7 (±)-N-(2,6-Dioxopiperidin-3-yl)-2-fluoro-4-(3-oxoazetidin-1-yl)benzamide 51

Compound **5k** (50 mg, 0.16 mmol) was weighed into a 50 mL eggplant-shaped flask, and dichloromethane (10 mL) and sodium bicarbonate (26 mg, 0.31 mmol) were added. Under conditions of an ice-water bath, Dess-Martin oxidant (73 mg, 0.172 mmol) was added, and the system was naturally warmed to room temperature and left to react for 12 hours. A saturated sodium thiosulfate solution (5 mL) and water (5 mL) were added to the system, and extraction was performed with dichloromethane (10 mL × 3). The organic phases were combined, washed with a saturated sodium chloride solution (15 mL), dried over anhydrous sodium sulfate, filtered, and concentrated under reduced pressure. The resulting residue was purified by silica gel column chromatography with eluent system A to give the title compound **51** (35 mg, 70%).

MS m/z (ESI): 320.1 [M+1].

### Step 8 4-(3-(4-((5-((S)-2-(3-Chloro-4-cyanophenyl)-3-methyl-2,8-diazaspiro[4.5]decane-8-carbonyl)pyridin-2-yl)thio)piperidin-1-yl)azetidin-1-yl)-N (2,6-dioxopiperidin-3-yl)-2-fluorobenzamide 5 (a mixture of diastereomers)

Compound **5f** (18 mg, 0.033 mmol) was weighed into a 25 mL eggplant-shaped flask. Methanol (2 mL) and sodium acetate (13.8 mg, 0.16 mmol) were added, and the mixture was then left to react at 50 °C for 30 minutes. To the reaction system were added compound **51** (32 mg, 0.099 mmol) and acetic acid (4 mg, 0.066 mmol). After 1 hour of reaction, sodium cyanoborohydride (12 mg, 0.02 mmol) was added, and the mixture was left to react for 12 hours. The reaction mixture was filtered and separated and purified by preparative high performance liquid chromatography (Waters-2545, elution system: 10 mmol/L aqueous ammonium bicarbonate solution and acetonitrile, gradient of acetonitrile: 45%-60%, flow rate: 30 mL/min) to give the title compound 5 (a mixture of diastereomers, a 1:1 ratio, 10 mg, yield: 37%).

MS m/z (ESI): 813.2 [M+1].

¹H NMR (500 MHz, DMSO-*d₆*): δ 10.83 (s, 1H), 8.45 (s, 1H), 7.98 (t, 1H), 7.78-7.50 (m, 3H), 7.33 (d, 1H), 6.77 (s, 1H), 6.64 (d, 1H), 6.28 (d, 1H), 6.23 (d, 1H), 4.80-4.66 (m, 1H), 4.14-3.95 (m, 3H), 3.93-3.64 (m, 4H), 3.57-3.38 (m, 3H), 2.93-2.60 (m, 4H), 2.32-1.95 (m, 8H), 1.84-1.35 (m, 9H), 1.19 (d, 3H).

### Example 6 (S)-2-Chloro-4-(8-(4-(4-(4-(4-(2,4-dioxotetrahydropyrimidin-1(2H)-yl)phenyl)piperazin-1-yl)-[1,4'-bipiperidine]-1'-carbonyl)phenyl)-3-methyl-2,8-diazaspiro[4.5]decan-2-yl)benzonitrile 6

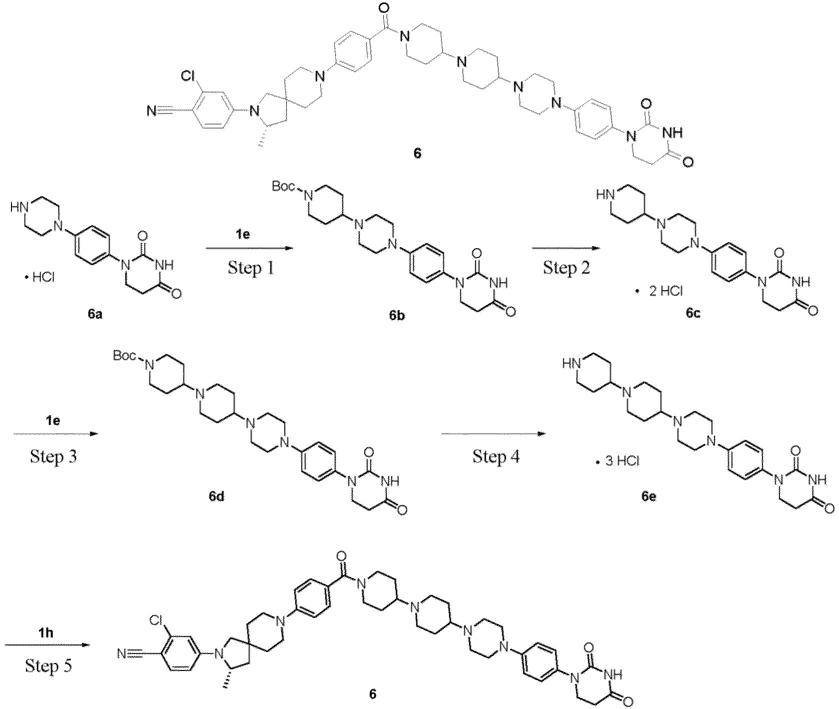

### Step 1 tert-Butyl 4-(4-(4-(2,4-dioxotetrahydropyrimidin-1(2H-yl)phenyl)piperazin-1-yl)piperidine-1-carboxylate 6b

To a 50 mL reaction flask was added 6 mL of a mixed solvent of 1,2-dichloroethane and methanol (V/V = 5/1), followed by 1-(4-(piperazin-1-yl)phenyl)dihydropyrimidine-2,4(1*H*,3*H*)-dione hydrochloride **6a** (500 mg, 1.61 mmol, prepared using the method disclosed in "Scheme 15 on page 345 of the specification in the patent application WO2020132561 A1") and anhydrous sodium acetate (660 mg, 8.05 mmol). After 10 minutes of stirring at room temperature, compound **1e** (960 mg, 4.82 mmol) was added. After 1 hour of stirring, sodium triacetoxyborohydride (685 mg, 3.23 mmol) was added. After 3 hours of reaction, sodium cyanoborohydride (200 mg, 3.34 mmol) was added. After 3 hours of reaction, compound **1e** (960 mg, 4.82 mmol) was added again. After another 12 hours of reaction, a saturated sodium bicarbonate solution (20 mL) was added to quench the reaction, and extraction was performed with 1,2-dichloroethane (50 mL × 3). The organic phases were combined, dried over anhydrous sodium sulfate, filtered, and concentrated under reduced pressure. The resulting residue was purified by silica gel column chromatography with eluent system A to give the title compound **6b** (736 mg, yield: 99%).

MS m/z (ESI): 458.6 [M+1].

### Step 2 1-(4-(4-(Piperidin-4-yl)piperazin-1-yl)phenyl)dihydropyrimidine-2,4(1H,3H)-dione dihydrochloride 6c

Compound **6b** (736 mg, 1.61 mmol) was dissolved in 5 mL of dichloromethane, and a 4 M solution of hydrogen chloride in 1,4-dioxane (5 mL) was slowly added under an ice bath. The mixture was left to react for 1 hour. The reaction mixture was concentrated under reduced pressure and dried *in vacuo* to give the title compound **6c** as a crude product (692 mg, yield: 99%). The crude product was directly used in the next step without purification.

MS m/z (ESI): 358.3 [M+1].

### Step 3 tert-Butyl 4-(4-(4-(2,4-dioxotetrahydropyrimidin-1(2H)-yl)phenyl)piperazin-1-yl)-[1,4'-bipiperidine]-1'-carboxylate 6d

To a 50 mL reaction flask were added 10 mL of a mixed solution of 1,2-dichloromethane and methanol (V/V = 7/3), compound **6c** (300 mg, 0.70 mmol), and anhydrous sodium acetate (350 mg, 4.27 mmol). The mixture was stirred for 30 minutes. Compound **1e** (400 mg, 2.01 mmol) was added, and the mixture was stirred for another 10 minutes. Sodium cyanoborohydride (84 mg, 1.40 mmol) was added. After 1 hour of reaction, compound **1e** (400 mg, 2.01 mmol) was added again, and the mixture was heated to 50 °C and stirred overnight. The reaction mixture was concentrated under reduced pressure and separated and purified by preparative high performance liquid chromatography (Gilson GX-281, elution system: 10 mmol/L aqueous ammonium bicarbonate solution and acetonitrile, gradient of acetonitrile: 28%-48%, flow rate: 30 mL/min) to give the title compound **6d** (138 mg, yield: 37%).

MS m/z (ESI): 541.4 [M+1].

### Step 4 1-(4-(4-([1,4'-Bipiperidin]-4-yl)piperazin-1-yl)phenyl)dihydropyrimidine-2,4(1H,3H)-dione trihydrochloride 6e

Compound **6d** (36 mg, 0.066 mmol) was dissolved in 1 mL of dichloromethane, and a 4 M solution of hydrogen chloride in 1,4-dioxane (1 mL) was slowly added under an ice bath. The mixture was left to react for 1 hour. The reaction mixture was concentrated under reduced pressure and dried *in vacuo* to give the title compound **6e** as a crude product (36 mg, yield: 99%). The crude product was directly used in the next step without purification.

MS m/z (ESI): 441.4 [M+1].

### Step 5 (S)-2-Chloro-4-(8-(4-(4-(4-(4-(2,4-dioxotetrahydropyrimidin-1(2H)-yl)phenyl)piperazin-1-yl)-[1,4'-bipiperidine]-1'-carbonyl)phenyl)-3-methyl-2,8-diazaspiro[4.5]decan-2-yl)benzonitrile 6

To a 25 mL reaction flask were sequentially added 2 mL of *N,N-*dimethylformamide, compound **1h** (35 mg, 0.085 mmol), and compound **6e** (42 mg, 0.076 mmol), followed by *O*-(7-azabenzotriazol-1-yl)-*N,N,N',N'*-tetramethyluronium hexafluorophosphate (36 mg, 0.095 mmol) and *N,N-*diisopropylethylamine (60 mg, 0.464 mmol). After 1 hour of reaction, the reaction mixture was filtered and separated and purified by preparative high performance liquid chromatography (Waters 2767-SQ Detecor2, elution system: 10 mmol/L aqueous ammonium bicarbonate solution and acetonitrile, gradient of acetonitrile: 70%-90%, flow rate: 30 mL/min) to give the title compound **6** (4 mg, yield: 6%).

MS m/z (ESI): 832.4 [M+1].

¹H NMR (500 MHz, DMSO-*d₆*): δ 10.26 (s, 1H), 7.60 (d, 1H), 7.27 (d, 2H), 7.15 (d, 2H), 7.01-6.85 (m, 4H), 6.80 (d, 1H), 6.66 (dd, 1H), 4.08-3.96 (m, 1H), 3.69 (t, 2H), 3.58-3.35 (m, 6H), 3.28-3.04 (m, 7H), 3.01-2.55 (m, 12H), 2.28-2.18 (m, 1H), 2.10-1.91 (m, 3H), 1.83-1.36 (m, 9H), 1.32-1.12 (m, 5H).

### Example 7 (S)-2-Chloro-4-(8-(4-(4-(3-(4-(4-(2,4-dioxotetrahydropyrimidin-1(2H)-yl)phenyl)piperazin-1-yl)azetidin-1-yl)piperidine-1-carbonyl)phenyl)-3-methyl-2,8-diazaspiro[4.5]decan-2-yl)benzonitrile 7

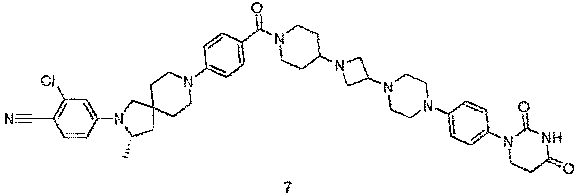

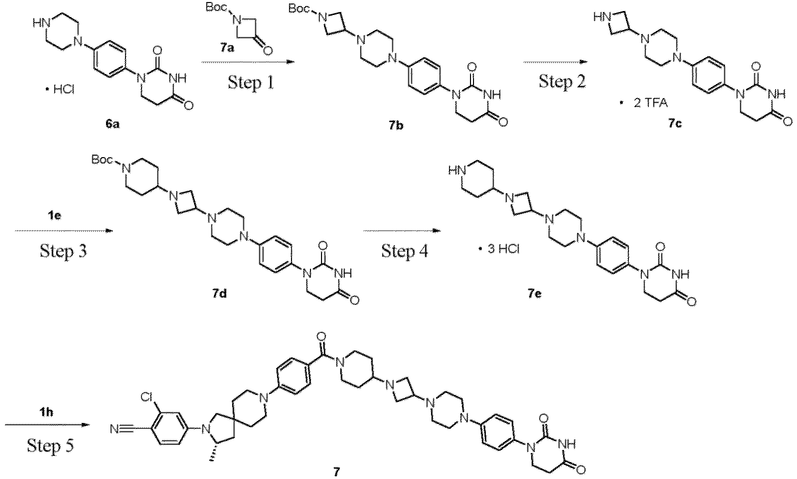

### Step 1 tert-Butyl 3-(4-(4-(2,4-dioxotetrahydropyrimidin-1(2H)-yl)phenyl)piperazin-1-yl)azetidine-1-carboxylate 7b

Compound **6a** (500 mg, 1.82 mmol) and tert-butyl 3-oxoazetidine-1-carboxylate **7a** (343 mg, 2.0 mmol, Bide Pharmatech) were dissolved in 45 mL of a mixed solvent of dichloromethane and methanol (V/V = 2/1). Anhydrous sodium acetate (897 mg, 10.94 mmol) was added, and acetic acid (0.1 mL) was added. The mixture was heated to 60 °C and left to react for 0.5 hours. Sodium triacetoxyborohydride (848 mg, 4.0 mmol) was added, and the mixture was heated to 60 °C and left to react for 12 hours. The reaction mixture was filtered and concentrated under reduced pressure. The resulting residue was purified by silica gel column chromatography with eluent system A to give the title compound **7b** (440 mg, yield: 56%).

MS m/z (ESI): 430.3 [M+1].

### Step 2 1-(4-(4-(Azetidin-3-yl)piperazin-1-yl)phenyl)dihydropyrimidine-2,4(1H,3H)-dione bistrifluoroacetate 7c

Compound **7b** (440 mg, 1.02 mmol) was dissolved in 15 mL of dichloromethane, and trifluoroacetic acid (3 mL) was added dropwise. After 2 hours of reaction, the reaction mixture was concentrated under reduced pressure and dried *in vacuo* to give the title compound **7c** (570 mg, yield: 100%). The crude product was directly used in the next step without purification.

### Step 3 tert-Butyl 4-(3-(4-(4-(2,4-dioxotetrahydropyrimidin-1(2H-yl)phenyl)piperazin-1-yl)azetidin-1-yl)piperidine-1-carboxylate 7d

To a 50 mL reaction flask were sequentially added 6.5 mL of a mixed solution of 1,2-dichloroethane and methanol (V/V = 10/3), compound **7c** (100 mg, 0.18 mmol), and anhydrous sodium acetate (150 mg, 1.83 mmol). After 30 minutes of stirring, compound **1e** (400 mg, 2.01 mmol) was added, and the mixture was heated to 50 °C. After 15 minutes of reaction, sodium cyanoborohydride (40 mg, 0.67 mmol) was added, and the mixture was left to react for 12 hours. The solvent was removed by concentration under reduced pressure, and the resulting residue was purified by silica gel column chromatography with eluent system A to give the title compound **7d** (50 mg, yield: 54%).

MS m/z (ESI): 513.6 [M+1].

### Step 4 1-(4-(4-(1-(Piperidin-4-yl)azetidin-3-yl)piperazin-1-yl)phenyl)dihydropyrimidine-2,4(1H,3H)-dione trihydrochloride 7e

Compound **7d** (50 mg, 0.098 mmol) was dissolved in 1 mL of dichloromethane, and a 4 M solution of hydrogen chloride in 1,4-dioxane (2 mL) was slowly added under an ice bath. The mixture was left to react for 1 hour. The reaction mixture was concentrated and dried *in vacuo* to give the title compound **7e** as a crude product (50 mg, yield: 98%). The crude product was directly used in the next step without purification.

MS m/z (ESI): 413.3 [M+1].

### Step 5 (S)-2-Chloro-4-(8-(4-(4-(3-(4-(4-(2,4-dioxotetrahydropyrimidin-1(2H)-yl)phenyl)piperazin-1 -yl)azetidin-1 -yl)piperidine-1 -carbonyl)phenyl)-3-methyl-2,8-diazaspiro[4.5]decan-2-yl)benzonitrile 7

To a 25 mL reaction flask were sequentially added 2 mL of *N,N-*dimethylformamide, compound **1h** (50 mg, 0.12 mmol), and compound **7e** (50 mg, 0.096 mmol), followed by *O*-(7-azabenzotriazol-1-yl)-*N,N,N',N'*-tetramethyluronium hexafluorophosphate (50 mg, 0.13 mmol) and *N,N-*diisopropylethylamine (100 mg, 0.77 mmol). After 1 hour of reaction at room temperature, the reaction mixture was filtered and separated and purified by preparative high performance liquid chromatography (Waters 2767-SQ Detecor2, elution system: 10 mmol/L aqueous ammonium bicarbonate solution and acetonitrile, gradient of acetonitrile: 70%-90%, flow rate: 30 mL/min) to give the title compound 7 (10 mg, yield: 13%).

MS m/z (ESI): 804.4 [M+1].

¹H NMR (500 MHz, DMSO-*d₆*): δ 10.26 (s, 1H), 7.59 (d, 1H), 7.23 (d, 2H), 7.14 (d, 2H), 7.01-6.85 (m, 4H), 6.80 (d, 1H), 6.65 (dd, 1H), 4.17-3.96 (m, 1H), 3.94-3.60 (m, 4H), 3.55-3.35 (m, 5H), 3.28-2.98 (m, 8H), 2.94-2.74 (m, 3H), 2.72-2.60 (m, 2H), 2.47-2.31 (m, 4H), 2.30-2.12 (m, 2H), 1.88-1.39 (m, 7H), 1.36-0.98 (m, 6H).

### Example 8 (S)-2-Chloro-4-(8-(4-(4-((3-(4-(4-(2,4-dioxotetrahydropyrimidin-1(2H)-yl)phenyl)piperazin-1 -yl)azetidin-1 -yl)methyl)piperidine-1 -carbonyl)phenyl)-3-methyl-2,8-diazaspiro[4.5]decan-2-yl)benzonitrile 8

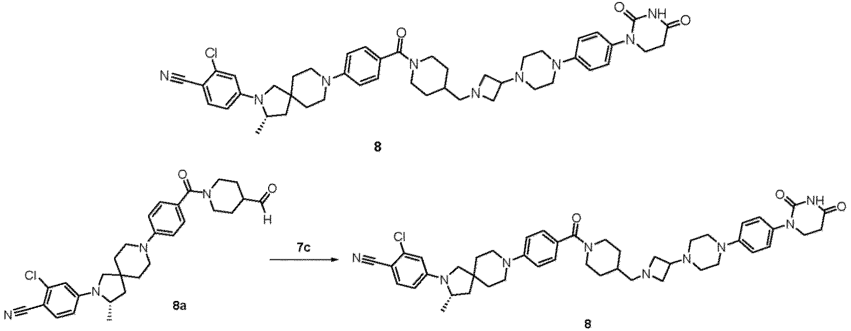

### Step 1

Compound **7c** (44 mg, 0.079 mmol) was weighed into a 25 mL eggplant-shaped flask, and 8 mL of a mixed solvent of dichloromethane and methanol (V/V = 3/1) and anhydrous sodium acetate (81 mg, 0.99 mmol) were added. After 15 minutes of reaction, (*S*)-2-chloro-4-(8-(4-(4-formylpiperidine-1-carbonyl)phenyl)-3-methyl-2,8-diazaspiro[4.5]decan-2-yl)benzonitrile **8a** (50 mg, 0.099 mmol, prepared using the method disclosed in "Example 47 on page 265 of the specification in patent application WO2021055756 A1") and acetic acid (4 mg, 0.066 mmol) were added to the system. After 1 hour of reaction, sodium cyanoborohydride (12 mg, 0.19 mmol) was added, and the mixture was left to react for 12 hours. The reaction mixture was filtered and separated and purified by preparative high performance liquid chromatography (Waters-2545, elution system: 10 mmol/L aqueous ammonium bicarbonate solution and acetonitrile, gradient of acetonitrile: 45%-60%, flow rate: 30 mL/min) to give the title compound **8** (20 mg, yield: 31%).

MS m/z (ESI): 818.7 [M+1].

¹H NMR (500 MHz, DMSO-*d₆*): δ 10.26 (s, 1H), 7.60 (d, 1H), 7.23 (d, 2H), 7.15 (d, 2H), 7.01-6.85 (m, 4H), 6.81 (d, 1H), 6.66 (dd, 1H), 4.07-3.98 (m, 1H), 3.70 (t, 2H), 3.56-3.35 (m, 6H), 3.28-2.98 (m, 7H), 2.94-2.74 (m, 3H), 2.72-2.64 (m, 2H), 2.62-2.52 (m, 4H), 2.42-2.20 (m, 6H), 1.88-1.40 (m, 8H), 1.21 (d, 3H), 1.13-0.96 (m, 2H).

### Example 9 (S)-2-Chloro-4-(8-(4-(4-((4-(4-(4-(2,4-dioxotetrahydropyrimidin-1(2H)-yl)phenyl)piperazin-1 -yl)piperidin-1 -yl)methyl)piperidine-1 -carbonyl)phenyl)-3 - methyl-2,8-diazaspiro[4.5]decan-2-yl)benzonitrile 9

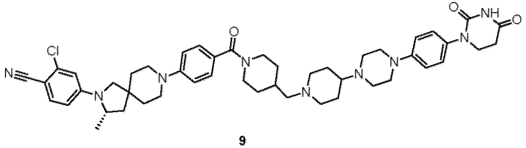

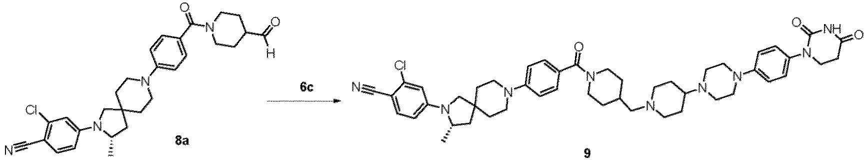

### Step 1

Compound 6c (44 mg, 0.10 mmol) was weighed into a 25 mL eggplant-shaped flask, and 8 mL of a mixed solvent of dichloromethane and methanol (V/V = 3/1) and anhydrous sodium acetate (92 mg, 1.12 mmol) were added. After 15 minutes of reaction, compound **8a** (53 mg, 0.10 mmol) and acetic acid (4 mg, 0.066 mmol) were added. After 1 hour of reaction, sodium triacetoxyborohydride (47 mg, 0.22 mmol) was added, and the mixture was left to react for 2 hours. The reaction mixture was filtered and separated and purified by preparative high performance liquid chromatography (Waters-2545, elution system: 10 mmol/L aqueous ammonium bicarbonate solution and acetonitrile, gradient of acetonitrile: 45%-60%, flow rate: 30 mL/min) to give the title compound 9 (20 mg, yield: 23%).

MS m/z (ESI): 846.5 [M+1].

¹H NMR (500 MHz, DMSO-*d₆*): δ 10.25 (s, 1H), 7.59 (d, 1H), 7.23 (d, 2H), 7.14 (d, 2H), 7.01-6.85 (m, 4H), 6.80 (d, 1H), 6.66 (dd, 1H), 4.06-3.98 (m, 1H), 3.69 (t, 2H), 3.51-3.38 (m, 2H), 3.26-2.99 (m, 7H), 2.94-2.74 (m, 4H), 2.71-2.66 (m, 2H), 2.71-2.66 (m, 4H), 2.64-2.57 (m, 5H), 1.92-1.62 (m, 10H), 1.61-1.36 (m, 6H), 1.20 (d, 3H), 1.08-0.96 (m, 2H).

### Example 10 2-Chloro-4-((3S)-8-(4-(4-(4-(3-((2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile 10 (a mixture of diastereomers)

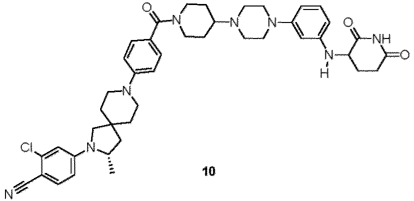

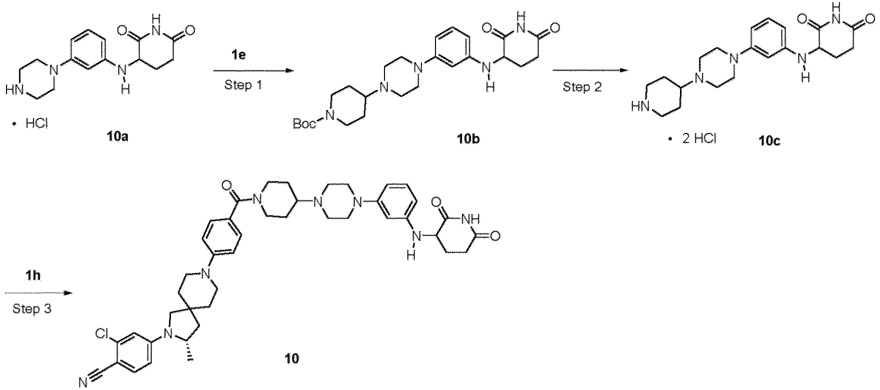

### Step 1

### tert-Butyl (±)-4-(4-(3-((2,6-dioxopiperidin-3-yl)amino)phenyl)piperazin-1-yl)piperidine-1-carboxylate 10b

To a 25 mL three-necked flask was added 3 mL of a mixed solution of dichloromethane and methanol (V/V = 2/1), followed by (±)-3-((3-(piperazin-1-yl)phenyl)amino)piperidine-2,6-dione hydrochloride **10a** (90 mg, 0.25 mmol, prepared using the method disclosed for "Compound 86 on page 266 of the specification in the patent application WO 2018237026 A1") and anhydrous sodium acetate (103 mg, 1.25 mmol). After 15 minutes of stirring, acetic acid (30 mg, 0.49 mmol) and compound **1e** (148 mg, 0.74 mmol) were added. After 30 minutes of stirring at room temperature, sodium cyanoborohydride (38 mg, 0.60 mmol) was slowly added, and the mixture was left to react for 16 hours. 10 mL of a saturated sodium bicarbonate solution was added to the system under ice bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system A to give the title compound 10b (95 mg, yield: 80%).

MS m/z (ESI): 472.8 [M+1].

### Step 2 (±)-3-((3-(4-(Piperidin-4-yl)piperazin-1-yl)phenyl)amino)piperidine-2,6-dione dihydrochloride 10c

Compound **10b** (90 mg, 0.19 mmol) was dissolved in 2 mL of dichloromethane, and a 4 M solution of hydrogen chloride in 1,4-dioxane (0.5 mL) was slowly added under an ice bath. The mixture was left to react for 4 hours. The reaction mixture was concentrated and dried *in vacuo* to give the title compound **10c** as a crude product (84 mg, yield: 99%). The crude product was directly used in the next step without purification.

MS m/z (ESI): 372.4 [M+1].

### Step 3 2-Chloro-4-((3S)-8-(4-(4-(4-(3-((2,6-dioxopiperidin-3-yl)amino)phenyl)piperazin-1-yl)piperidine-1-carbonyl)phenyl)-3-methyl-2,8-diazaspiro[4.5]decan-2-yl)benzonitrile 10 (a mixture of diastereomers)

To a 25 mL three-necked flask were sequentially added 2 mL of *N,N*-dimethylformamide, compound **10c** (38 mg, 0.085 mmol), and compound **1h** (29 mg, 0.071 mmol). *N,N-*Diisopropylethylamine (136 mg, 1.05 mmol) and *O*-(7-azabenzotriazol-1-yl)-*N,N,N*',*N*'-tetramethyluronium hexafluorophosphate (53 mg, 0.14 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 45%-95%, flow rate: 30 mL/min) to give the title compound **10** (a mixture of diastereomers, a 1:1 ratio, 24 mg, yield: 44%).

MS m/z (ESI): 763.3 [M+1].

¹H NMR (500 MHz, DMSO-*d₆*): δ 10.76 (s, 1H), 7.59 (d, 1H), 7.26 (d, 2H), 6.95 (d, 2H), 6.90 (t, 1H), 6.80 (d, 1H), 6.66 (dd, 1H), 6.25 (s, 1H), 6.16 (dd, 2H), 5.62 (d, 1H), 4.41-4.24 (m, 1H), 4.19-3.72 (m, 3H), 3.56-3.32 (m, 4H), 3.27-3.12 (m, 2H), 3.10-2.97 (m, 3H), 2.95-2.80 (m, 2H), 2.78-2.69 (m, 1H), 2.67-2.54 (m, 4H), 2.48-2.32 (m, 4H), 2.31-2.16 (m, 1H), 2.15-2.03 (m, 1H), 1.94-1.62 (m, 4H), 1.61-1.32 (m, 4H), 1.30-1.04 (m, 4H).

### Example 11 2-Chloro-4-((3S)-8-(4-(4-((4-(3-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 11 (a mixture of diastereomers)

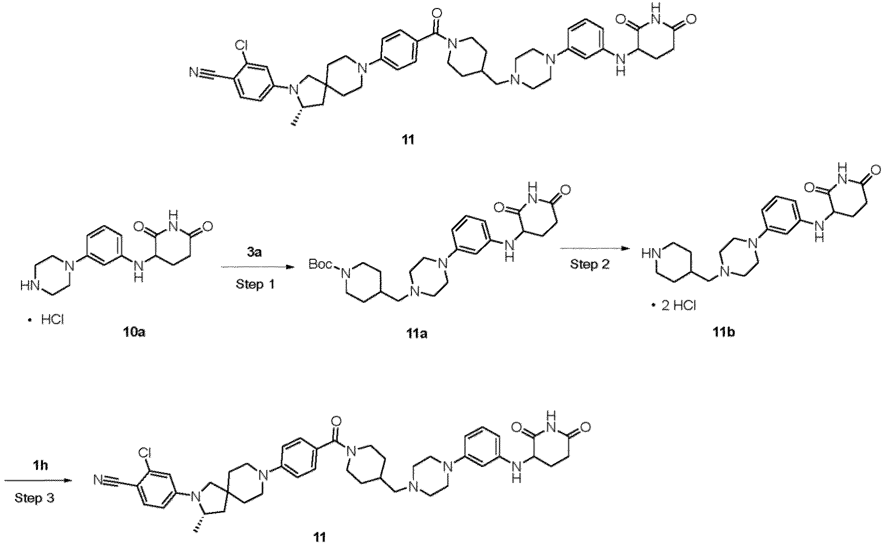

### Step 1 tert-Butyl (±)-4-((4-(3-((2,6-dioxopiperidin-3-yl)amino)phenyl)piperazin-1-yl)methyl)piperidine-1-carboxylate 11a

To a 25 mL three-necked flask was added 3 mL of a mixed solution of dichloromethane and methanol (V/V = 2/1), followed by (±)-3-((3-(piperazin-1-yl)phenyl)amino)piperidine-2,6-dione **10a** (90 mg, 0.25 mmol) and anhydrous sodium acetate (102 mg, 1.24 mmol). After 15 minutes of reaction, compound 3a (85 mg, 0.40 mmol) was added. After 30 minutes of reaction, sodium triacetoxyborohydride (106 mg, 0.50 mmol) was slowly added, and the mixture was left to react for 16 hours. 10 mL of a saturated sodium bicarbonate solution was added to the system under ice bath cooling to quench the reaction, and extraction was performed with dichloromethane (20 mL × 3). The organic phases were combined, washed with a saturated sodium chloride solution (20 mL × 3), dried over anhydrous sodium sulfate, and filtered. The filtrate was concentrated under reduced pressure, and the resulting residue was purified by silica gel column chromatography with eluent system A to give the title compound **11a** (110 mg, yield: 90%). MS m/z (ESI): 486.6 [M+1].

### Step 2 (±)-3-((3-(4-(Piperidin-4-ylmethyl)piperazin-1-yl)phenyl)amino)piperidine-2,6-dione dihydrochloride 11b

Compound **11a** (105 mg, 0.22 mmol) was dissolved in 3 mL of dichloromethane, and a 4 M solution of hydrogen chloride in 1,4-dioxane (0.53 mL) was slowly added under an ice bath. The mixture was left to react for 4 hours. The reaction mixture was concentrated and dried *in vacuo* to give the title compound **11b** as a crude product (99 mg, yield: 98%). The crude product was directly used in the next step without purification. MS m/z (ESI): 386.5 [M+1].

### Step 3 2-Chloro-4-((3S)-8-(4-(4-((4-(3-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 11 (a mixture of diastereomers)

To a 25 mL three-necked flask were sequentially added 2 mL of *N,N*-dimethylformamide, compound **11b** (42 mg, 0.092 mmol), and compound **1h** (29 mg, 0.071 mmol). *N,N-*Diisopropylethylamine (136 mg, 1.05 mmol) and O-(7-azabenzotriazol-1-yl)-N,N,N,N-tetramethyluronium hexafluorophosphate (53 mg, 0.14 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **11** (a mixture of diastereomers, a 1:1 ratio, 24 mg, yield: 43%).

MS m/z (ESI): 777.3 [M+1].

¹H NMR (500 MHz, DMSO-*d₆*): δ 10.76 (s, 1H), 7.59 (d, 1H), 7.26 (d, 2H), 6.94 (d, 2H), 6.90 (t, 1H), 6.80 (d, 1H), 6.66 (dd, 1H), 6.25 (s, 1H), 6.16 (dd, 2H), 5.61 (d, 1H), 4.42-4.25 (m, 1H), 4.19-3.74 (m, 3H), 3.56-3.33 (m, 4H), 3.26-3.14 (m, 2H), 3.12-2.97 (m, 4H), 2.95-2.80 (m, 2H), 2.78-2.68 (m, 1H), 2.66-2.52 (m, 2H), 2.48-2.38 (m, 3H), 2.29-1.96 (m, 4H), 1.96-1.66 (m, 5H), 1.62-1.38 (m, 3H), 1.36-1.16 (m, 4H), 1.11-0.97 (m, 2H).

### Example 11-1 and Example 11-2 2-Chloro-4-((S)-8-(4-(4-((4-(3-(((S)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 11-1 2-Chloro-4-((S)-8-(4-(4-((4-(3-(((R)-2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 11-2

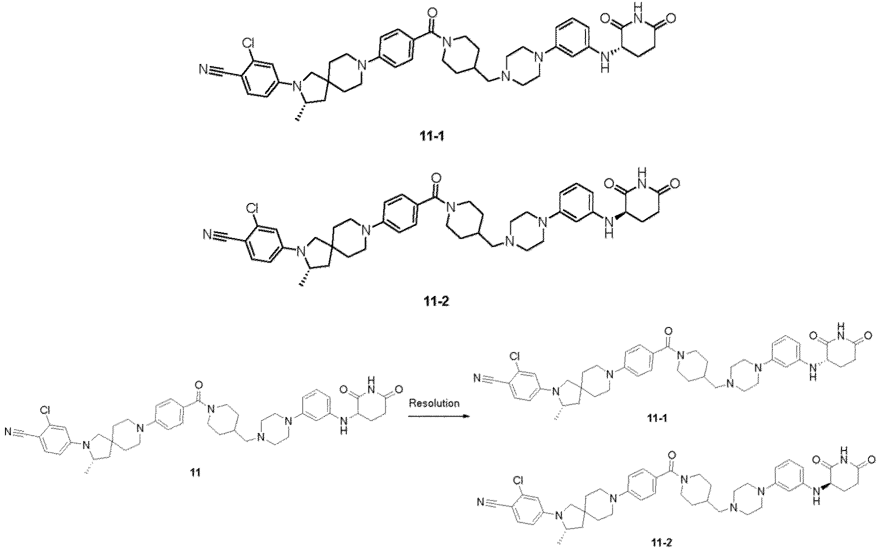

Compound 11 (100 mg) was resolved by preparative chiral chromatography (separation conditions: Gilson-281, column: Phenomenex Amylose-2, 5 µm, 21.2 mm × 250 mm, mobile phase: acetonitrile (0.5% 7 M ammonia (NH₃) in methanol)/ethanol (0.5% 7 M ammonia (NH₃) in methanol) = 40/60 (v/v)), flow rate 20 mL/min) to give eluates of 2 compounds. Each of the two eluates was well mixed with formic acid (500 mg), and the solvent was removed by concentration under reduced pressure to give crude products (46 mg, 45 mg). The crude products of the 2 compounds (46 mg, 45 mg) were then separated and purified by preparative high performance liquid chromatography (Waters 2545, elution system: 10 mmol/L aqueous ammonium bicarbonate solution and acetonitrile, gradient of acetonitrile: 60%-85%, flow rate: 30 mL/min) to give the title compounds (30 mg, 27 mg).

Single-configuration compound (shorter retention time) (30 mg, yield: 30%): MS m/z (ESI): 777.3 [M+1]. Chiral HPLC analysis method: retention time 4.27 minutes (Agilent1260 DAD, column: Phenomenex LUX Amylose-2, (4.6 × 150 mM), 5 µm; mobile phase: acetonitrile/ethanol/diethylamine) = 60/40/0.1 (v/v/v)), flow rate 1 mL/min).

¹H NMR (500 MHz, DMSO-*d₆*): δ 10.76 (s, 1H), 7.59 (d, 1H), 7.26 (d, 2H), 6.94 (d, 2H), 6.90 (t, 1H), 6.80 (s, 1H), 6.66 (d, 1H), 6.25 (s, 1H), 6.18 (d, 1H), 6.13 (d, 1H), 5.61 (d, 1H), 4.42-4.25 (m, 1H), 4.19-3.74 (m, 3H), 3.56-3.33 (m, 4H), 3.26-3.14 (m, 2H), 3.12-2.97 (m, 4H), 2.95-2.80 (m, 2H), 2.78-2.68 (m, 1H), 2.66-2.52 (m, 1H), 2.48-2.38 (m, 4H), 2.29-1.96 (m, 4H), 1.96-1.66 (m, 5H), 1.62-1.38 (m, 3H), 1.36-1.16 (m, 4H), 1.11-0.97 (m, 2H).

Single-configuration compound (longer retention time) (27 mg, yield: 27%): MS m/z (ESI): 777.3 [M+1].

Chiral HPLC analysis method: retention time 5.24 minutes (Agilent1260 DAD, column: Phenomenex LUX Amylose-2, (4.6 × 150 mM), 5 µm; mobile phase: acetonitrile/ethanol/diethylamine) = 60/40/0.1 (v/v/v)), flow rate 1 mL/min).

¹H NMR (500 MHz, DMSO-*d₆*): δ 10.76 (s, 1H), 7.59 (d, 1H), 7.24 (d, 2H), 6.94 (d, 2H), 6.90 (t, 1H), 6.80 (s, 1H), 6.66 (d, 1H), 6.25 (s, 1H), 6.18 (d, 1H), 6.13 (d, 1H), 5.61 (d, 1H), 4.42-4.25 (m, 1H), 4.19-3.74 (m, 3H), 3.56-3.33 (m, 4H), 3.26-3.14 (m, 2H), 3.12-2.97 (m, 4H), 2.95-2.80 (m, 2H), 2.78-2.68 (m, 1H), 2.66-2.52 (m, 1H), 2.48-2.38 (m, 4H), 2.29-1.96 (m, 4H), 1.96-1.66 (m, 5H), 1.62-1.38 (m, 3H), 1.36-1.16 (m, 4H), 1.11-0.97 (m, 2H).

### Example 12 2-Chloro-4-((3S)-8-(6-(4-((4-(3-((2,6-dioxopiperidin-3-yl)amino)phenyl)piperazin-1-yl)methyl)piperidine-1-carbonyl)pyridin-3-yl)-3-methyl-2,8-diazaspiro[4.5]decan-2-yl)benzonitrile 12 (a mixture of diastereomers)

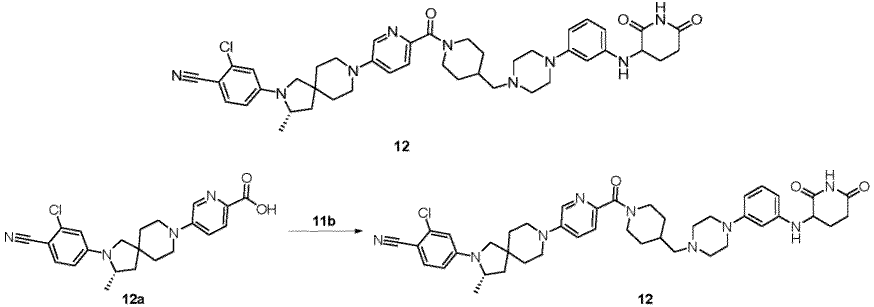

To a 25 mL single-necked flask were sequentially added 2 mL of *N,N-*dimethylformamide, compound **11b** (64 mg, 0.12 mmol), and (5)-5-(2-(3-chloro-4-cyanophenyl)-3-methyl-2,8-diazaspiro[4.5]decan-8-yl)picolinic acid 12a (45 mg, 0.11 mmol, prepared using the method disclosed in "Example 35 on page 245 of the specification in the patent application WO2021055756A1"). *N,N-*Diisopropylethylamine (84 mg, 0.65 mmol) and *O*-(7-azabenzotriazol-1-yl)-*N,N,N*',*N*'-tetramethyluronium hexafluorophosphate (83 mg, 0.22 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **12** (a mixture of diastereomers, a 1:1 ratio, 48 mg, yield: 56%).

MS m/z (ESI): 778.3 [M+1].

¹H NMR (500 MHz, DMSO-*d₆*):δ 10.75 (s, 1H), 8.24 (d, 1H), 7.58 (d, 1H), 7.42 (d, 1H), 7.36 (dd, 1H), 6.89 (t, 1H), 6.79 (d, 1H), 6.65 (dd, 1H), 6.24 (s, 1H), 6.17 (d, 1H), 6.12 (d, 1H), 5.60 (d, 1H), 4.51-4.39 (m, 1H), 4.33-4.25 (m, 1H), 4.09-3.97 (m, 2H), 3.48-3.32 (m, 4H), 3.30-3.21 (m, 2H), 3.09-2.96 (m, 4H), 2.79-2.67 (m, 2H), 2.64-2.50 (m, 3H), 2.47-2.41 (m, 3H), 2.28-1.94 (m, 4H), 1.90-1.62 (m, 5H), 1.61-1.42 (m, 3H), 1.31-1.16 (m, 4H), 1.14-0.99 (m, 2H).

### Example 12-1 and Example 12-2 2-Chloro-4-((S)-8-(6-(4-((4-(3-(((S)-2,6-dioxopiperidin-3-yl)amino)phenyl)piperazin-1-yl)methyl)piperidine-1-carbonyl)pyridin-3-yl)-3-methyl-2,8-diazaspiro[4.5]decan-2-yl)benzonitrile 12-1 2-Chloro-4-((S)-8-(6-(4-((4-(3-(((R)-2,6-dioxopipeiidin-3-yl)amino)phenyl)piperazin-1-yl)methyl)piperidine-1-carbonyl)pyridin-3-yl)-3-methyl-2,8-diazaspiro[4.5]decan-2-yl)benzonitrile 12-2

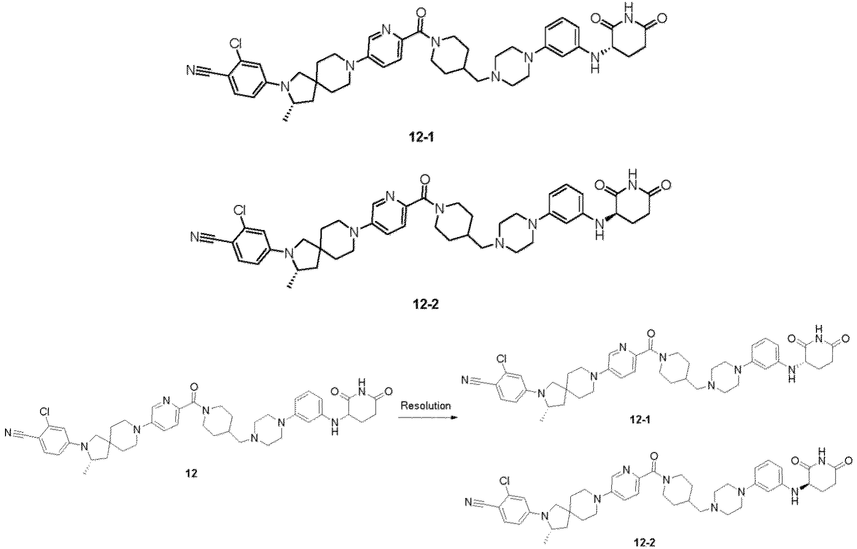

Compound **12** (500 mg) was resolved by preparative chiral chromatography (separation conditions: Gilson-281, column: Phenomenex Amylose-2, 5 µm, 21.2 mm × 250 mm, mobile phase: acetonitrile (0.5% 7 M ammonia (NH₃) in methanol)/ethanol (0.5% 7 M ammonia (NH₃) in methanol) = 40/60 (v/v)), flow rate 20 mL/min) to give eluates of two compounds. Each of the two eluates was well mixed with formic acid (1.0 g), and the solvent was removed by concentration under reduced pressure to give crude products (160 mg, 140 mg). The crude products of the two compounds (160 mg, 140 mg) were then separated and purified by preparative high performance liquid chromatography (Waters 2545, elution system: 10 mmol/L aqueous ammonium bicarbonate solution and acetonitrile, gradient of acetonitrile: 50%-67%, flow rate: 30 mL/min) to give the title compounds (80 mg, 70 mg).

Single-configuration compound (shorter retention time) (80 mg, yield: 16%): MS m/z (ESI): 778.3 [M+1].

Chiral HPLC analysis method: retention time 3.92 minutes (Agilent1260 DAD, column: Phenomenex LUX Amylose-2, (4.6 × 150 mM), 5 µm; mobile phase: acetonitrile/ethanol/diethylamine = 40/60/0.06 (v/v/v), flow rate 1 mL/min).

¹H NMR (500 MHz, DMSO-*d₆*):δ 10.75 (s, 1H), 8.25 (d, 1H), 7.59 (d, 1H), 7.43 (d, 1H), 7.37 (dd, 1H), 6.90 (t, 1H), 6.80 (d, 1H), 6.66 (d, 1H), 6.25 (s, 1H), 6.18 (d, 1H), 6.13 (d, 1H), 5.61 (d, 1H), 4.51-4.39 (m, 1H), 4.33-4.25 (m, 1H), 4.09-3.97 (m, 2H), 3.48-3.32 (m, 4H), 3.30-3.21 (m, 2H), 3.09-2.96 (m, 4H), 2.79-2.67 (m, 2H), 2.64-2.50 (m, 3H), 2.47-2.41 (m, 3H), 2.28-1.94 (m, 4H), 1.90-1.62 (m, 5H), 1.61-1.42 (m, 3H), 1.38-1.16 (m, 4H), 1.14-0.99 (m, 2H). Single-configuration compound (longer retention time) (70 mg, yield: 14%):

MS m/z (ESI): 778.3 [M+1].

Chiral HPLC analysis method: retention time 4.74 minutes (Agilent1260 DAD, column: Phenomenex LUX Amylose-2, (4.6 × 150 mM), 5 µm; mobile phase: acetonitrile/ethanol/diethylamine = 40/60/0.06 (v/v/v), flow rate 1 mL/min).

¹H NMR (500 MHz, DMSO-*d₆*):δ 10.75 (s, 1H), 8.25 (d, 1H), 7.59 (d, 1H), 7.43 (d, 1H), 7.37 (dd, 1H), 6.90 (t, 1H), 6.80 (d, 1H), 6.66 (d, 1H), 6.25 (s, 1H), 6.18 (d, 1H), 6.13 (d, 1H), 5.61 (d, 1H), 4.51-4.39 (m, 1H), 4.33-4.25 (m, 1H), 4.09-3.94 (m, 2H), 3.52-3.32 (m, 4H), 3.30-3.21 (m, 2H), 3.09-2.96 (m, 4H), 2.79-2.67 (m, 2H), 2.64-2.50 (m, 3H), 2.47-2.41 (m, 3H), 2.28-1.94 (m, 4H), 1.90-1.62 (m, 5H), 1.61-1.42 (m, 3H), 1.38-1.16 (m, 4H), 1.14-0.99 (m, 2H).

### Example 13 2-Chloro-4-((3S)-8-(6-(4-((4-(3-((2,6-dioxopiperidin-3-yl)amino)phenyl)piperazin-1-yl)methyl)piperidine-1-carbonyl)pyridazin-3-yl)-3-methyl-2,8-diazaspiro[4.5]decan-2-yl)benzonitrile 13 (a mixture of diastereomers)

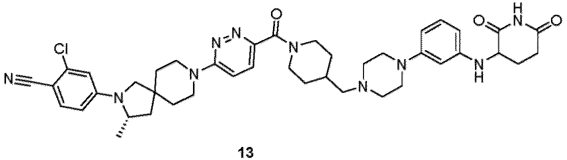

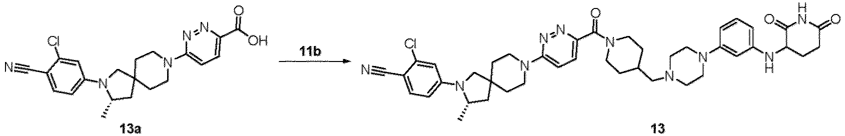

To a 25 mL single-necked flask were sequentially added 2 mL of *N,N-*dimethylformamide, compound **11b** (47 mg, 0.088 mmol), and (*S*)-6-(2-(3-chloro-4-cyanophenyl)-3-methyl-2,8-diazaspiro[4.5]decan-8-yl)pyridazine-3-carboxylic acid **13a** (36 mg, 0.087 mmol, prepared using the method disclosed in "Example 54 on page 273 of the specification in the patent application WO2021055756A1"). *N,N-*Diisopropylethylamine (90 mg, 0.69 mmol) and *O*-(7-azabenzotriazol-1-yl)-N,N,N,N-tetramethyluronium hexafluorophosphate (66 mg, 0.17 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **13** (a mixture of diastereomers, a 1:1 ratio, 45 mg, yield: 66%).

MS m/z (ESI): 779.3 [M+1].

¹H NMR (500 MHz, DMSO-*d₆*):δ 10.74 (s, 1H), 7.59 (d, 1H), 7.50 (d, 1H), 7.32 (d, 1H), 6.89 (t, 1H), 6.79 (s, 1H), 6.65 (d, 1H), 6.24 (s, 1H), 6.17 (d, 1H), 6.12 (d, 1H), 5.60 (d, 1H), 4.48 (d, 1H), 4.34-4.25 (m, 1H), 4.10-3.95 (m, 2H), 3.87-3.74 (m, 2H), 3.66-3.55 (m, 2H), 3.48 (d, 1H), 3.38-3.32 (m, 1H), 3.13-2.99 (m, 4H), 2.85-2.67 (m, 2H), 2.64-2.50 (m, 3H), 2.48-2.42 (m, 3H), 2.31-1.93 (m, 4H), 1.92-1.63 (m, 5H), 1.62-1.40 (m, 3H), 1.31-1.17 (m, 4H), 1.17-1.02(m, 2H).

### Example 14 2-Chloro-4-((3S)-8-(4-(3-((4-(3-((2,6-dioxopiperidin-3-yl)amino)phenyl)piperazin-1-yl)methyl)azetidine-1-carbonyl)phenyl)-3-methyl-2,8-diazaspiro[4.5]decan-2-yl)benzonitrile 14 (a mixture of diastereomers)

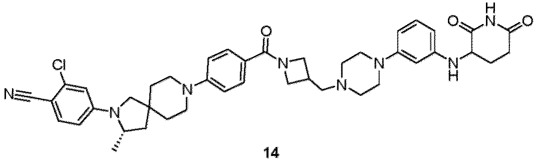

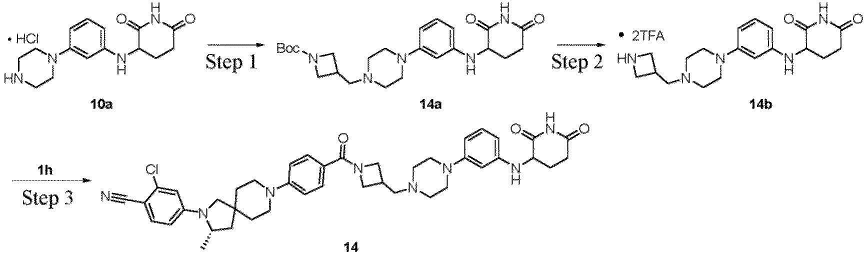

### Step 1 tert-Butyl (±)-3-((4-(3-((2,6-dioxopiperidin-3-yl)amino)phenyl)piperazin-1-yl)methyl)azetidine-1-carboxylate 14a

To a 50 mL single-necked flask was added 4 mL of a mixed solution of dichloromethane and methanol (V/V = 3/1), followed by compound **10a** (250 mg, 0.77 mmol) and anhydrous sodium acetate (729 mg, 8.67 mmol). After 15 minutes of reaction, tert-butyl 3-formylazetidine-1-carboxylate (324 mg, 1.54 mmol) was added. After 30 minutes of reaction, sodium triacetoxyborohydride (326 mg, 1.54 mmol) was slowly added, and the mixture was left to react for 1 hour. Dichloromethane and methanol were removed by concentration under reduced pressure, and the resulting residue was purified by silica gel column chromatography with eluent system A to give the title compound **14a** (205 mg, yield: 58%).

MS m/z (ESI): 458.8 [M+1].

### Step 2 (±)-3-((3-(4-(Azetidin-3-ylmethyl)piperazin-1-yl)phenyl)amino)piperidine-2,6-dione bistrifluoroacetate 14b

Compound **14a** (100 mg, 0.22 mmol) was dissolved in 3 mL of dichloromethane, and trifluoroacetic acid (1 mL) was slowly added. The mixture was left to react for 1 hour. Dichloromethane and trifluoroacetic acid were removed by concentration under reduced pressure, and the residue was dried *in vacuo* to give the title compound **14b** as a crude product (120 mg, yield: 94%). The crude product was directly used in the next step without purification.

MS m/z (ESI): 358.3 [M+1].

### Step 3 2-Chloro-4-((3S)-8-(4-(3-((4-(3-((2,6-dioxopiperidin-3-yl)amino)phenyl)piperazin-1-yl)methyl)azetidine-1-carbonyl)phenyl)-3-methyl-2,8-diazaspiro[4.5]decan-2-yl)benzonitrile 14 (a mixture of diastereomers)

To a 50 mL single-necked flask were sequentially added 2 mL of *N,N-*dimethylformamide, compound **1h** (75 mg, 0.18 mmol), *N,N-*diisopropylethylamine (118 mg, 0.91 mmol), and *O*-(7-azabenzotriazol-1-yl)-N,N,N,N-tetramethyluronium hexafluorophosphate (104 mg, 0.27 mmol). After 15 minutes of reaction, compound **14b** (103 mg, 0.18 mmol) was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 48%-63%, flow rate: 30 mL/min) to give the title compound **14** (a mixture of diastereomers, a 1:1 ratio, 70 mg, yield: 63%).

MS m/z (ESI): 749.3 [M+1].

¹H NMR (500 MHz, DMSO-*d₆*): δ 10.76 (s, 1H), 7.59 (d, 1H), 7.50 (d, 2H), 7.00-6.85 (m, 3H), 6.80 (s, 1H), 6.65 (d, 1H), 6.24 (s, 1H), 6.15 (dd, 2H), 5.62 (s, 1H), 4.49-4.23 (m, 2H), 4.17-3.89 (m, 4H), 3.71-3.58 (m, 1H), 3.52-3.16 (m, 5H), 3.03 (s, 4H), 2.92-2.81 (m, 1H), 2.78-2.33 (m, 6H), 2.28-2.18 (m, 1H), 2.14-1.95 (m, 2H), 1.92-1.79 (m, 2H), 1.78-1.65 (m, 2H), 1.62-1.41 (m, 3H), 1.20 (d, 3H).

### Example 15 2-Chloro-4-((3S)-8-(4-(4-((4-(3-((2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile 15 (a mixture of diastereomers)

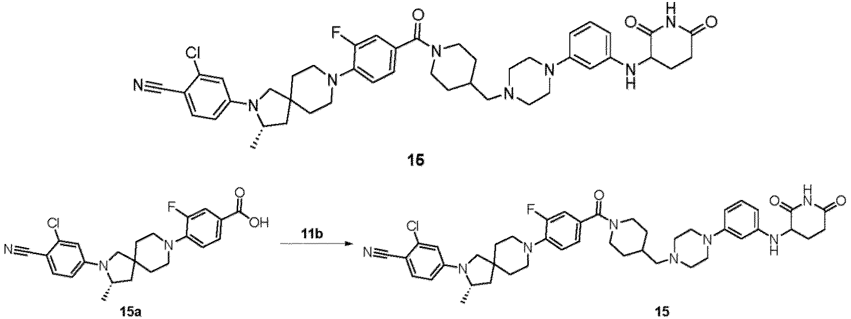

To a 25 mL three-necked flask were sequentially added 2 mL of *N,N*-dimethylformamide, compound **11b** (50 mg, 0.094 mmol), and (*S*)-4-(2-(3-chloro-4-cyanophenyl)-3-methyl-2,8-diazaspiro[4.5]decan-8-yl)-3-fluorobenzoic acid **15a** (45 mg, 0.083 mmol, prepared using the method disclosed in "Example 52 on page 271 of the specification in the patent application WO2021055756A1"). *N,N-*Diisopropylethylamine (85 mg, 0.66 mmol) and *O*-(7-azabenzotriazol-1-yl)-*N,N,N*',*N*'-tetramethyluronium hexafluorophosphate (63 mg, 0.16 mmol) were slowly added under an ice bath. The cooling bath was removed, and the mixture was left to react for 16 hours. The reaction mixture was filtered and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **15** (a mixture of diastereomers, a 1:1 ratio, 36 mg, yield: 54%).

MS m/z (ESI): 795.3 [M+1].

¹H NMR (500 MHz, DMSO-*d₆*): δ 10.75 (s, 1H), 7.59 (d, 1H), 7.13 (d, 1H), 7.11 (s, 1H), 7.06 (t, 1H), 6.89 (t, 1H), 6.79 (d, 1H), 6.65 (dd, 1H), 6.24 (s, 1H), 6.17 (d, 1H), 6.12 (d, 1H), 5.60 (d, 1H), 4.35-4.25 (m, 1H), 4.07-3.97 (m, 1H), 3.43 (d, 1H), 3.34-3.30 (m, 3H), 3.17-2.92(m, 8H), 2.78-2.68 (m, 1H), 2.64-2.50 (m, 3H), 2.48-2.40 (m, 4H), 2.28-1.93 (m, 4H), 1.90-1.67 (m, 5H), 1.62-1.48 (m, 3H), 1.27-1.15 (m, 4H), 1.12-0.99 (m, 2H).

### Example 15-1 and Example 15-2 2-Chloro-4-((S)-8-(4-(4-((4-(3-(((S)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile 15-1 2-Chloro-4-((S)-8-(4-(4-((4-(3-(((R)-2,6-dioxopiperidin-3-yl)amino)phenyl)piperazin-1-yl)methyl)piperidine-1-carbonyl)-2-fluorophenyl)-3-methyl-2,8-diazaspiro[4.5]decan-2-yl)benzonitrile 15-2

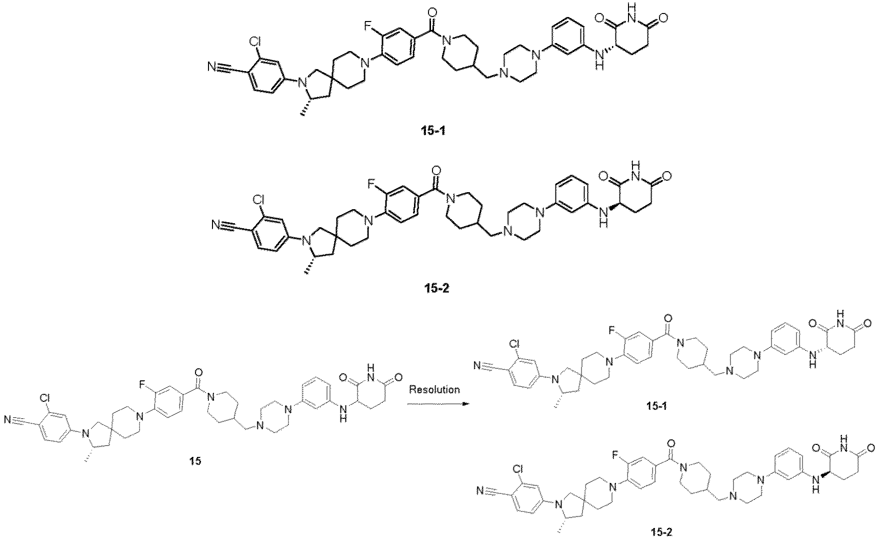

Compound **15** (21 mg) was resolved by preparative chiral chromatography (separation conditions: Gilson-281, column: Phenomenex Amylose-2, 5 µm, 21.2 mm × 250 mm, mobile phase: acetonitrile (0.5% 7 M ammonia (NH₃) in methanol)/ethanol (0.5% 7 M ammonia (NH₃) in methanol) = 40/60 (v/v)), flow rate 20 mL/min) to give the title compound 15-2 (5 mg) and compound **15-1** (8 mg).

Compound **15-2** (5 mg, yield: 24%), with a shorter retention time:
MS m/z (ESI): 795.3 [M+1].

Chiral HPLC analysis method: retention time 3.53 minutes (Agilent1260 DAD, column: Phenomenex LUX Amylose-2, (4.6 × 150 mM), 5 µm; mobile phase: acetonitrile/ethanol/diethylamine = 40/60/0.06 (v/v/v), flow rate 1 mL/min). ¹H NMR (500 MHz, DMSO-*d₆*): δ 10.75 (s, 1H), 7.59 (d, 1H), 7.14 (d, 1H), 7.12 (s, 1H), 7.06 (t, 1H), 6.90 (t, 1H), 6.79 (d, 1H), 6.65 (dd, 1H), 6.24 (s, 1H), 6.17 (d, 1H), 6.13 (d, 1H), 5.61 (d, 1H), 4.35-4.25 (m, 1H), 4.07-3.97 (m, 1H), 3.43 (d, 1H), 3.37-3.30 (m, 3H), 3.17-2.92 (m, 8H), 2.78-2.68 (m, 1H), 2.64-2.50 (m, 3H), 2.48-2.40 (m, 4H), 2.28-1.93 (m, 4H), 1.90-1.67 (m, 5H), 1.62-1.48 (m, 3H), 1.27-1.15 (m, 4H), 1.12-0.99 (m, 2H).

Compound **15-1** (8 mg, yield: 38%), with a longer retention time:
MS m/z (ESI): 795.3 [M+1].

Chiral HPLC analysis method: retention time 4.19 minutes (Agilent1260 DAD, column: Phenomenex LUX Amylose-2, (4.6 × 150 mM), 5 µm; mobile phase: acetonitrile/ethanol/diethylamine = 40/60/0.06 (v/v/v), flow rate 1 mL/min).

¹H NMR (500 MHz, DMSO-*d₆*): δ 10.76 (s, 1H), 7.59 (d, 1H), 7.13 (d, 1H), 7.11 (s, 1H), 7.06 (t, 1H), 6.90 (t, 1H), 6.80 (d, 1H), 6.65 (dd, 1H), 6.24 (s, 1H), 6.17 (d, 1H), 6.12 (d, 1H), 5.61 (d, 1H), 4.35-4.25 (m, 1H), 4.07-3.97 (m, 1H), 3.43 (d, 1H), 3.34-3.30 (m, 3H), 3.17-2.92(m, 8H), 2.78-2.68 (m, 1H), 2.64-2.50 (m, 3H), 2.48-2.40 (m, 4H), 2.28-1.93 (m, 4H), 1.90-1.67 (m, 5H), 1.62-1.48 (m, 3H), 1.27-1.15 (m, 4H), 1.12-0.99 (m, 2H).

### Example 16 2-Chloro-4-((3S)-8-(4-(4-((4-(3-((2,6-dioxopiperidin-3-yl)amino)phenyl)piperazin-1-yl)methyl)piperidine-1-carbonyl)-3-fluorophenyl)-3-methyl-2,8-diazaspiro[4.5]decan-2-yl)benzonitrile 16 (a mixture of diastereomers)

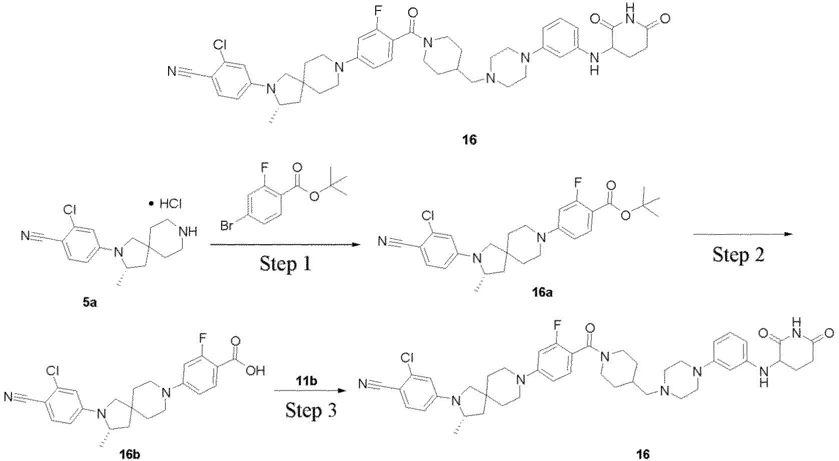

### Step 1 tert-Butyl (S)-4-(2-(3-chloro-4-cyanophenyl)-3-methyl-2,8-diazaspiro[4.5]decan-8-yl)-2-fluorobenzoate 16a

To a 25 mL single-necked flask were sequentially added 10 mL of toluene, compound **5a** (100 mg, 0.31 mmol), tert-butyl 4-bromo-2-fluorobenzoate (168 mg, 0.61 mmol, Bide Pharmatech), palladium acetate (13 mg, 0.057 mmol), 4,5-bis(diphenylphosphino)-9,9-dimethylxanthene (35 mg, 0.061 mmol), and anhydrous cesium carbonate (299 mg, 0.92 mmol). The system was purged with nitrogen gas 3 times, and the reaction mixture was heated to 85 °C and left to react for 10 hours. The reaction mixture was filtered. The filtrate was concentrated under reduced pressure, and the resulting residue was purified by silica gel column chromatography with eluent system B to give the title compound **16a** (100 mg, yield: 67%).

MS m/z (ESI):484.4 [M+1].

### Step 2 (S)-4-(2-(3-Chloro-4-cyanophenyl)-3-methyl-2,8-diazaspiro[4.5]decan-8-yl)-2-fluorobenzoic acid 16b

Compound **16a** (98 mg, 0.20 mmol) was dissolved in 3 mL of dichloromethane, and trifluoroacetic acid (461 mg, 4.04 mmol) was slowly added under an ice bath. The mixture was left to react for 2 hours. The reaction mixture was concentrated under reduced pressure and dried *in vacuo* to give the title compound **16b** as a crude product (110 mg, yield: 99%). The crude product was directly used in the next step without purification.

MS m/z (ESI): 428.2 [M+1].

### Step 3 2-Chloro-4-((3S)-8-(4-(4-((4-(3-((2,6-dioxopiperidin-3-yl)amino)phenyl)piperazin-1-yl)methyl)piperidine-1-carbonyl)-3-fluorophenyl)-3-methyl-2,8-diazaspiro[4.5]decan-2-yl)benzonitrile 16 (a mixture of diastereomers)

To a 25 mL single-necked flask were sequentially added 2 mL of *N,N-*dimethylformamide, compound **11b** (53 mg, 0.099 mmol), and (*S*)-4-(2-(3-chloro-4-cyanophenyl)-3-methyl-2,8-diazaspiro[4.5]decan-8-yl)-2-fluorobenzoic acid **16b** (50 mg, 0.092 mmol). *N,N-*Diisopropylethylamine (95 mg, 0.73 mmol) and *O*-(7-azabenzotriazol-1-yl)-*N*,*N*,*N*',*N*'-tetramethyluronium hexafluorophosphate (70 mg, 0.18 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **16** (a mixture of diastereomers, a 1:1 ratio, 38 mg, yield: 51%).

MS m/z (ESI): 795.8 [M+1].

¹H NMR (500 MHz, DMSO-*d₆*): δ 10.75 (s, 1H), 7.58 (d, 1H), 7.14 (t, 1H), 6.89 (t, 1H), 6.81-6.70 (m, 3H), 6.64 (dd, 1H), 6.24 (s, 1H), 6.17 (d, 1H), 6.12 (d, 1H), 5.60 (d, 1H), 4.49-4.38 (m, 1H), 4.32-4.24 (m, 1H), 4.06-3.98 (m, 1H), 3.54-3.32 (m, 4H), 3.30-3.16 (m, 3H), 3.09-2.94 (m, 4H), 2.78-2.67 (m, 2H), 2.64-2.50 (m, 3H), 2.48-2.41 (m, 3H), 2.26-1.95 (m, 4H), 1.90-1.62 (m, 5H), 1.60-1.52 (m, 1H), 1.50-1.40 (m, 2H), 1.30-1.15 (m, 4H), 1.08-0.92 (m, 2H).

### Example 17 2-Chloro-4-((3S)-8-(5-(4-((4-(3-((2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile 17 (a mixture of diastereomers)

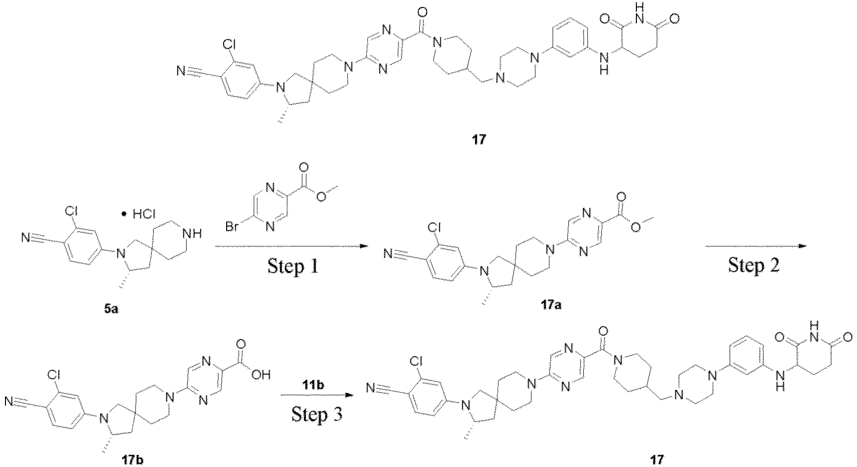

### Step 1 Methyl (S)-5-(2-(3-chloro-4-cyanophenyl)-3-methyl-2,8-diazaspiro[4.5]decan-8-yl)pyrazine-2-carboxylate 17a

To a 25 mL single-necked flask were sequentially added 10 mL of 1,4-dioxane, compound **5a** (100 mg, 0.34 mmol), methyl 5-bromopyrazine-2-carboxylate (134 mg, 0.62 mmol, Bide Pharmatech), tris(dibenzylideneacetone)dipalladium(0) (31 mg, 0.033 mmol), 4,5-bis(diphenylphosphino)-9,9-dimethylxanthene (19 mg, 0.033 mmol), and anhydrous cesium carbonate (281 mg, 0.86 mmol). The system was purged with nitrogen gas 3 times, and the reaction mixture was heated to 100 °C and left to react for 8 hours. The reaction mixture was filtered. The filtrate was concentrated under reduced pressure, and the resulting residue was purified by silica gel column chromatography with eluent system B to give the title compound **17a** (130 mg, yield: 88%).

MS m/z (ESI):426.2 [M+1].

### Step 2 (S)-5-(2-(3-Chloro-4-cyanophenyl)-3-methyl-2,8-diazaspiro[4.5]decan-8-yl)pyrazine-2-carboxylic acid 17b

Compound **17a** (120 mg, 0.28 mmol) was dissolved in 2 mL of methanol, and a 1 M solution of sodium hydroxide (0.55 mL, 0.55 mmol) was slowly added under an ice bath. The mixture was left to react for 3 hours. The reaction mixture was concentrated under reduced pressure, and the pH of the reaction mixture was adjusted to about 4 with 1 M dilute hydrochloric acid. Filtration was performed, and the filter cake was collected and dried *in vacuo* to give the title compound **17b** as a crude product (115 mg, yield: 99%). The crude product was directly used in the next step without purification.

MS m/z (ESI): 412.1 [M+1].

### Step 3 2-Chloro-4-((3S)-8-(5-(4-((4-(3-((2,6-dioxopiperidin-3-yl)amino)phenyl)piperazin-1-yl)methyl)piperidine-1-carbonyl)pyrazin-2-yl)-3-methyl-2,8-diazaspiro[4.5]decan-2-yl)benzonitrile 17 (a mixture of diastereomers)

To a 25 mL single-necked flask were sequentially added 2 mL of *N,N-*dimethylformamide, compound **11b** (51 mg, 0.096 mmol), and compound **17b** (50 mg, 0.097 mmol). *N,N-*Diisopropylethylamine (100 mg, 0.77 mmol) and *O*-(7-azabenzotriazol-1-yl)-*N*,*N*,*N*',*N*'-tetramethyluronium hexafluorophosphate (73 mg, 0.19 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **17** (a mixture of diastereomers, a 1:1 ratio, 40 mg, yield: 52%).

MS m/z (ESI): 779.3 [M+1].

¹H NMR (500 MHz, DMSO-*d₆*): δ 10.75 (s, 1H), 8.29 (s, 1H), 8.26 (s, 1H), 7.59 (d, 1H), 6.89 (t, 1H), 6.78 (s, 1H), 6.64 (d, 1H), 6.24 (s, 1H), 6.17 (d, 1H), 6.12 (d, 1H), 5.60 (d, 1H), 4.50-4.36 (m, 1H), 4.34-4.25 (m, 1H), 4.11-3.99 (m, 2H), 3.84-3.74 (m, 2H), 3.65-3.53 (m, 2H), 3.47 (d, 1H), 3.33 (d, 1H), 3.11-2.96 (m, 4H), 2.81-2.67 (m, 2H), 2.64-2.50 (m, 3H), 2.47-2.42 (m, 3H), 2.30-1.93 (m, 4H), 1.91-1.62 (m, 5H), 1.62-1.53 (m, 1H), 1.50-1.39 (m, 2H), 1.32-1.16 (m, 4H), 1.15-0.99 (m, 2H).

### Example 18 2-Chloro-4-((3S)-8-(5-(4-((4-(3-((2,6-dioxopiperidin-3-yl)amino)phenyl)piperazin-1-yl)methyl)piperidine-1-carbonyl)pyridin-2-yl)-3-methyl-2,8-diazaspiro[4.5]decan-2-yl)benzonitrile 18 (a mixture of diastereomers)

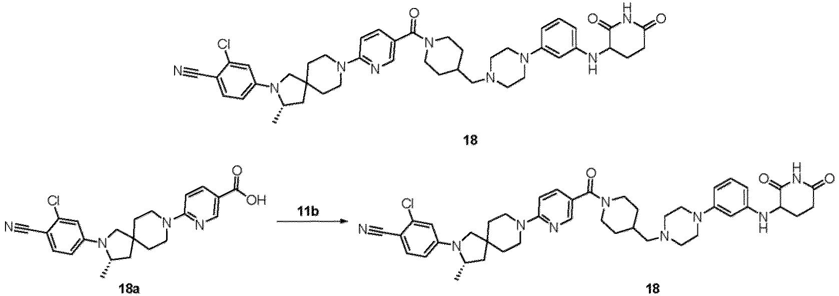

To a 25 mL single-necked flask were sequentially added 2 mL of *N,N-*dimethylformamide, compound **11b** (59 mg, 0.11 mmol), and (*S*)-6-(2-(3-chloro-4-cyanophenyl)-3-methyl-2,8-diazaspiro[4.5]decan-8-yl)nicotinic acid **18a** (65 mg, 0.10 mmol, prepared using the method disclosed in "Example 51 on page 268 of the specification in the patent application WO2021055756A1"). *N,N-*Diisopropylethylamine (131 mg, 1.01 mmol) and *O*-(7-azabenzotriazol-1-yl)-*N,N,N*',*N*'-tetramethyluronium hexafluorophosphate (77 mg, 0.20 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **18** (a mixture of diastereomers, a 1:1 ratio, 40 mg, yield: 50%).

MS m/z (ESI): 778.8 [M+1].

¹H NMR (500 MHz, DMSO-*d₆*):δ 10.75 (s, 1H), 8.14 (d, 1H), 7.59 (d, 1H), 7.53 (d, 1H), 6.89 (t, 1H), 6.83 (d, 1H), 6.78 (d, 1H), 6.64 (dd, 1H), 6.24 (s, 1H),6.17 (d, 1H), 6.12 (d, 1H), 5.60 (d, 1H), 4.33-4.26 (m, 1H), 4.08-3.99 (m, 1H), 3.77-3.66 (m, 2H), 3.57-3.47 (m, 2H), 3.45 (d, 1H), 3.32 (d, 1H), 3.30-3.26 (m, 2H), 3.10-2.96 (m, 4H), 2.95-2.82 (m, 1H), 2.78-2.67 (m, 1H), 2.64-2.50 (m, 2H), 2.47-2.41 (m, 4H), 2.28-1.94 (m, 4H), 1.89-1.61 (m, 5H),1.61-1.52 (m, 1H), 1.46-1.37 (m, 2H), 1.26-1.15 (m, 4H), 1.12-0.99 (m, 2H).

### Example 19 2-Chloro-4-((3S)-8-(4-(4-((4-(3-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile 19 (a mixture of diastereomers)

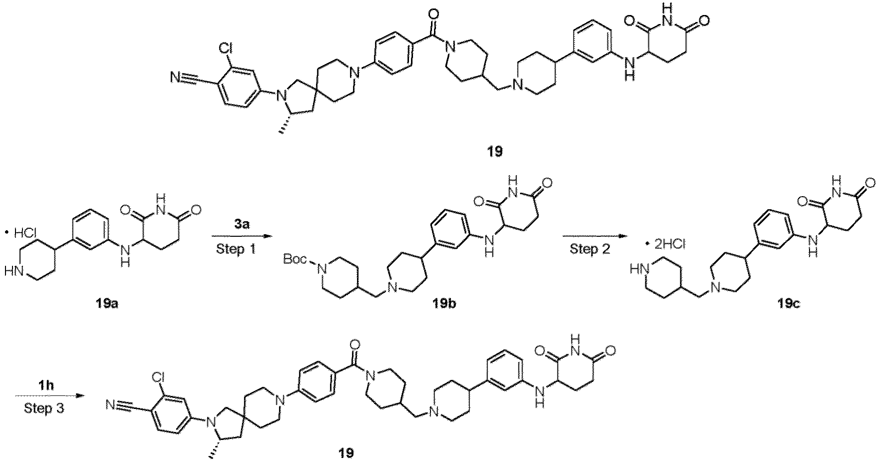

### Step 1 tert-Butyl (±)-4-((4-(3-((2,6-dioxopiperidin-3-yl)amino)phenyl)piperidin-1-yl)methyl)piperidine-1-carboxylate 19b

To a 50 mL single-necked flask was added 4 mL of a mixed solution of dichloromethane and methanol (V/V = 3/1), followed by (±)-3-((3-(piperidin-4-yl)phenyl)amino)piperidine-2,6-dione **19a** (500 mg, 1.54 mmol, prepared using the method disclosed for "compound 2-2 on page 348 of the specification in the patent application WO2021127561A1") and anhydrous sodium acetate (1.30 mg, 15.4 mmol). After 15 minutes of reaction, compound **3a** (659 mg, 3.09 mmol) was added. After 30 minutes of reaction, sodium triacetoxyborohydride (654 mg, 3.09 mmol) was slowly added, and the mixture was left to react for 1 hour. Dichloromethane and methanol were removed by concentration under reduced pressure, and the resulting residue was purified by silica gel column chromatography with eluent system A to give the title compound **19b** (130 mg, yield: 17%).

MS m/z (ESI): 485.2 [M+1].

### Step 2 (±)-3-((3-(1-(Piperidin-4-ylmethyl)piperidin-4-yl)phenyl)amino)piperidine-2,6-dione dihydrochloride 19c

Compound **19b** (100 mg, 0.22 mmol) was dissolved in 3 mL of dichloromethane, and a 4 M solution of hydrogen chloride in 1,4-dioxane (1 mL) was slowly added. The mixture was left to react for 1 hour. The reaction mixture was concentrated under reduced pressure and dried *in vacuo* to give the title compound **19c** as a crude product (170 mg, yield: 97%). The crude product was directly used in the next step without purification.

MS m/z (ESI): 385.3 [M+1].

### Step 3 2-Chloro-4-((3S)-8-(4-(4-((4-(3-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile 19 (a mixture of diastereomers)

To a 50 mL single-necked flask were sequentially added 2 mL of *N,N-*dimethylformamide, compound **1h** (50 mg, 0.12 mmol), *N,N-*diisopropylethylamine (79 mg, 0.61 mmol), and O-(7-azabenzotriazol-1-yl)-*N*,*N*,*N*,*N*-tetramethyluronium hexafluorophosphate (70 mg, 0.18 mmol). After 15 minutes of reaction, compound **19c** (130 mg, 0.28 mmol) was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 60%-76%, flow rate: 30 mL/min) to give the title compound **19** (a mixture of diastereomers, a 1:1 ratio, 30 mg, yield: 32%).

MS m/z (ESI): 776.6 [M+1].

¹H NMR (500 MHz, DMSO-*d₆*):δ 10.76 (s, 1H), 7.59 (d, 1H), 7.24 (d, 2H), 6.98 (t, 1H), 6.94 (d, 2H), 6.80 (s, 1H), 6.65 (d, 1H), 6.55 (s, 1H), 6.49 (d, 1H), 6.44 (d, 1H), 5.71 (d, 1H), 4.37-4.28 (m, 1H), 4.08-3.98 (m, 1H), 3.51-3.13 (m, 5H), 3.00-2.69 (m, 5H), 2.62-2.53 (m, 1H), 2.38-2.28 (m, 1H), 2.07-2.04 (m, 4H), 2.02-1.42 (m, 18H), 1.20 (d, 3H), 1.12-0.98 (m, 2H).

### Example 20 2-Chloro-4-((3S)-8-(6-(4-((4-(3-((2,6-dioxopiperidin-3-yl)amino)phenyl)piperidin-1-yl)methyl)piperidine-1-carbonyl)pyridazin-3-yl)-3-methyl-2,8-diazaspiro[4.5]decan-2-yl)benzonitrile 20 (a mixture of diastereomers)

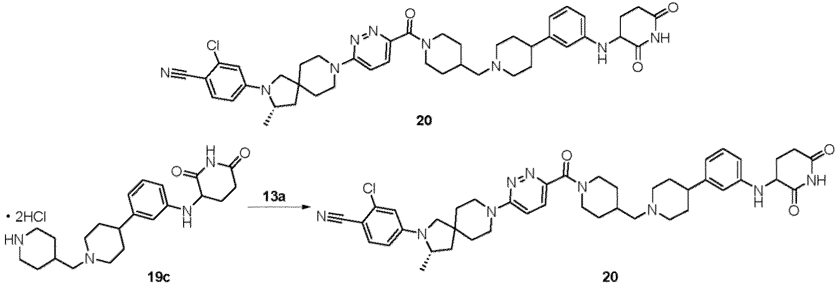

To a 50 mL single-necked flask were sequentially added 2 mL of *N,N-*dimethylformamide, compound **13a** (30 mg, 0.072 mmol), *N,N*-diisopropylethylamine (47 mg, 0.36 mmol), and *O*-(7-azabenzotriazol-1-yl)-*N,N,N*',*N*'-tetramethyluronium hexafluorophosphate (42 mg, 0.093 mmol). After 15 minutes of reaction, compound **19c** (43 mg, 0.08 mmol) was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-67%, flow rate: 30 mL/min) to give the title compound **20** (a mixture of diastereomers, a 1:1 ratio, 20 mg, yield: 35%).

MS m/z (ESI): 778.3 [M+1].

¹H NMR (500 MHz, DMSO-*d₆*):δ 10.76 (s, 1H), 7.60 (d, 1H), 7.51 (d, 1H), 7.33 (d, 1H), 6.98 (t, 1H), 6.80 (s, 1H), 6.66 (d, 1H), 6.55 (s, 1H), 6.48 (d, 1H), 6.45 (d, 1H), 5.71 (d, 1H), 4.52-4.46 (m, 1H), 4.36-4.28 (m, 1H), 4.10-3.96 (m, 2H), 3.86-3.76 (m, 2H), 3.67-3.57 (m, 2H), 3.49 (d, 1H), 3.40-3.26 (m, 2H), 3.08 (t, 1H), 2.99-2.88 (m, 2H), 2.84-2.70 (m, 2H), 2.65-2.53 (m, 2H), 2.37-2.25 (m, 2H), 2.20-2.08 (m, 2H), 2.03-1.80 (m, 5H), 1.77-1.55 (m, 7H), 1.51-1.42 (m, 2H), 1.21 (d, 3H), 1.17-1.03 (m, 2H).

### Example 21 2-Chloro-4-((3S)-8-(6-(3-((4-(3-((2,6-dioxopiperidin-3-yl)amino)phenyl)piperidin-1-yl)methyl)azetidine-1-carbonyl)pyridazin-3-yl)-3-methyl-2,8-diazaspiro[4.5]decan-2-yl)benzonitrile 21 (a mixture of diastereomers)

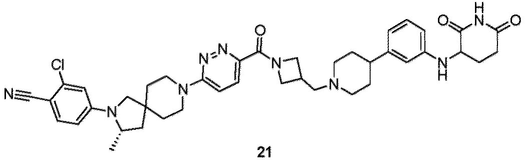

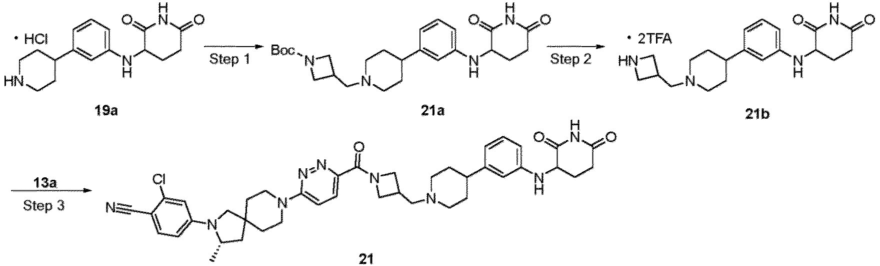

### Step 1 tert-Butyl (±)-3-((4-(3-((2,6-dioxopiperidin-3-yl)amino)phenyl)piperidin-1-yl)methyl)azetidine-1-carboxylate 21a

To a 50 mL single-necked flask was added 4 mL of a mixed solution of dichloromethane and methanol (V/V = 3/1), followed by compound **19a** (390 mg, 1.20 mmol) and anhydrous sodium acetate (505 mg, 6.02 mmol). After 15 minutes of reaction, tert-butyl 3-formylazetidine-1-carboxylate (446 mg, 2.41 mmol, Bide Pharmatech) was added. After 30 minutes of reaction, sodium triacetoxyborohydride (508 mg, 2.41 mmol) was slowly added, and the mixture was left to react for 1 hour. Dichloromethane and methanol were removed by concentration under reduced pressure, and the resulting residue was purified by silica gel column chromatography with eluent system A to give the title compound **21a** (100 mg, yield: 22%).

MS m/z (ESI): 457.3 [M+1].

### Step 2 (±)-3-((3-(1-(Azetidin-3-ylmethyl)piperidin-4-yl)phenyl)amino)piperidine-2,6-dione bistrifluoroacetate 21b

Compound **21a** (95 mg, 0.21 mmol) was dissolved in 3 mL of dichloromethane, and trifluoroacetic acid (0.5 mL) was slowly added. The mixture was left to react for 1 hour. Dichloromethane and trifluoroacetic acid were removed by concentration under reduced pressure, and the residue was dried *in vacuo* to give the title compound **21b** as a crude product (120 mg, yield: 99%). The crude product was directly used in the next step without purification.

MS m/z (ESI): 357.5 [M+1].

### Step 3 2-Chloro-4-((3S)-8-(6-(3-((4-(3-((2,6-dioxopiperidin-3-yl)amino)phenyl)piperidin-1-yl)methyl)azetidine-1-carbonyl)pyridazin-3-yl)-3-methyl-2,8-diazaspiro[4.5]decan-2-yl)benzonitrile 21 (a mixture of diastereomers)

To a 50 mL single-necked flask were sequentially added 2 mL of *N,N-*dimethylformamide, compound **13a** (30 mg, 0.073 mmol), *N,N*-diisopropylethylamine (47 mg, 0.36 mmol), and *O*-(7-azabenzotriazol-1-yl)-N,N,N,N-tetramethyluronium hexafluorophosphate (30 mg, 0.11 mmol). After 15 minutes of reaction, compound **21b** (59 mg, 0.10 mmol) was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-67%, flow rate: 30 mL/min) to give the title compound **21** (a mixture of diastereomers, a 1:1 ratio, 20 mg, yield: 37%).

MS m/z (ESI): 750.5 [M+1].

¹H NMR (500 MHz, DMSO-*d₆*):δ 10.76 (s, 1H), 7.75 (d, 1H), 7.60 (d, 1H), 7.32 (d, 1H), 6.98 (t, 1H), 6.80 (s, 1H), 6.66 (d, 1H), 6.56 (s, 1H), 6.48 (d, 1H), 6.45 (d, 1H), 5.71 (d, 1H), 4.64 (t, 1H), 4.37-4.29 (m, 1H), 4.27-4.21 (m, 1H), 4.15 (t, 1H), 4.09-4.00 (m, 1H), 3.91-3.80 (m, 2H), 3.74-3.59 (m, 3H), 3.49 (d, 1H), 3.35 (d, 1H), 2.95-2.84 (m, 2H), 2.80-2.70 (m, 1H), 2.62-2.53 (m, 3H), 2.38-2.28 (m, 2H), 2.14-1.96 (m, 4H), 1.92-1.81 (m, 1H), 1.77-1.56 (m, 7H), 1.50-1.42 (m, 2H), 1.21 (d, 3H).

### Example 22 2-Chloro-4-((3S)-8-(6-(3-((4-(3-((2,6-dioxopipeiidin-3-yl)amino)phenyl)piperazin-1-yl)methyl)azetidine-1-carbonyl)pyridazin-3-yl)-3-methyl-2,8-diazaspiro[4.5]decan-2-yl)benzonitrile 22 (a mixture of diastereomers)

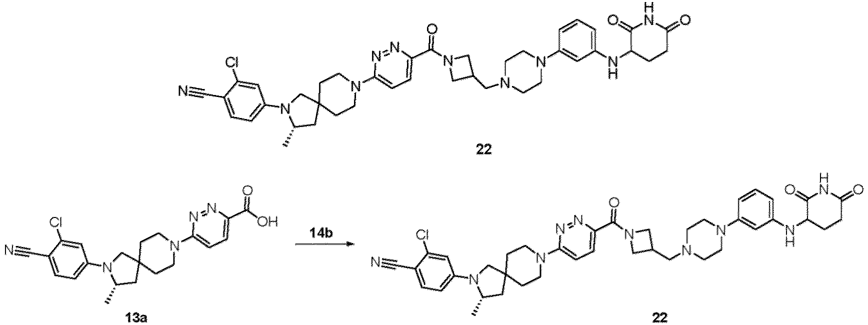

To a 50 mL single-necked flask were sequentially added 2 mL of *N,N-*dimethylformamide, compound **14b** (55 mg, 0.079 mmol), and compound **13a** (35 mg, 0.068 mmol). *N,N-*Diisopropylethylamine (70 mg, 0.54 mmol) and *O*-(7-azabenzotriazol-1-yl)-*N,N,N*'*,N*'-tetramethyluronium hexafluorophosphate (47 mg, 0.12 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **22** (a mixture of diastereomers, a 1:1 ratio, 20 mg, yield: 39%).

MS m/z (ESI): 751.3 [M+1].

¹H NMR (500 MHz, DMSO-*d₆*): δ 10.74 (s, 1H), 7.74 (d, 1H), 7.59 (d, 1H), 7.31 (d, 1H), 6.89 (t, 1H), 6.79 (s, 1H), 6.65 (d, 1H), 6.24 (s, 1H), 6.17 (d, 1H), 6.13 (d, 1H), 5.61(d, 1H), 4.64 (t, 1H), 4.34-4.20 (m, 2H), 4.18-4.12 (m, 1H), 4.07-3.99 (m, 1H), 3.89-3.79 (m, 2H), 3.75-3.58 (m, 3H), 3.48 (d, 1H), 3.34 (d, 1H), 3.11-2.99 (m, 4H), 2.94-2.83 (m, 1H), 2.78-2.68 (m, 1H), 2.66-2.50 (m, 5H), 2.30-2.21 (m, 1H), 2.12-2.04 (m, 2H), 2.89-2.79 (m, 1H), 1.75-1.62 (m, 2H), 1.61-1.55 (m, 1H), 1.50-1.41 (m, 2H), 1.28-1.16 (m, 4H).

### Example 23 2-Chloro-4-((3S)-8-(4-(4-((4-(5-((2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile 23 (a mixture of diastereomers)

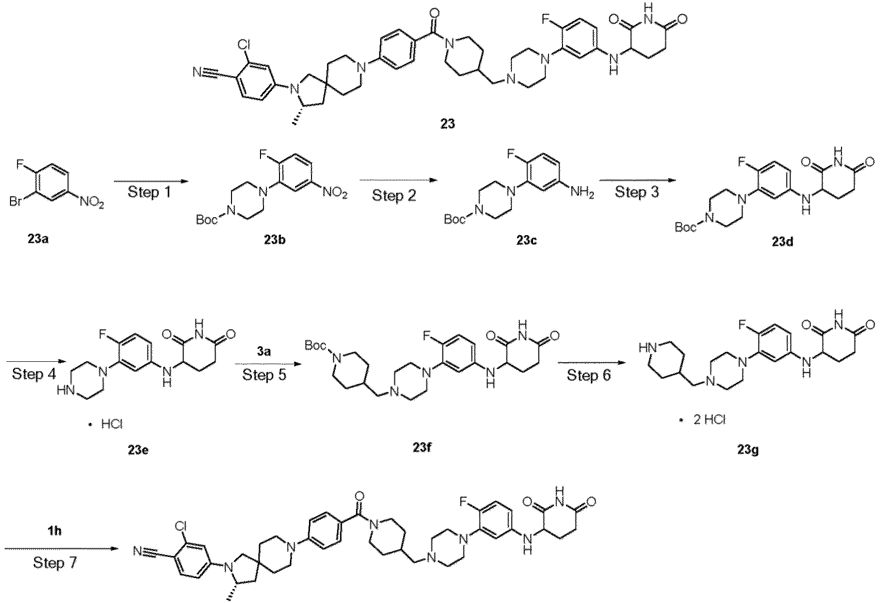

### Step 1

tert-Butyl 4-(2-fluoro-5-nitrophenyl)piperazine-1-carboxylate **23b** 2-Bromo-1-fluoro-4-nitrobenzene **23a** (1.0 g, 4.55 mmol) and tert-butyl piperazine-1-carboxylate (847 mg, 4.55 mmol, Bide Pharmatech) were dissolved in 15 mL of 1,4-dioxane, and cesium carbonate (4.44 g, 13.64 mmol) and methanesulfonato(2-dicyclohexylphosphino-2',6'-diisopropoxy-1,1'-biphenyl)(2-amino-1,1'-biphenyl-2-yl)palladium(II) (381 mg, 0.454 mmol, Bide Pharmatech) were added. The system was purged with nitrogen gas 3 times, heated to 110 °C, and left to react for 12 hours. 10 mL of water was added, and extraction was performed with ethyl acetate (20 mL × 3). The organic phase was washed with a saturated sodium chloride solution (10 mL × 3), dried over anhydrous sodium sulfate, and filtered. The filtrate was concentrated under reduced pressure, and the resulting residue was purified by silica gel column chromatography with eluent system B to give the title compound **23b** (1.0 g, yield: 68%).

MS m/z (ESI): 270.1 [M-55].

### Step 2

tert-Butyl 4-(5-amino-2-fluorophenyl)piperazine-1-carboxylate **23c** Compound **23b** (1.0 g, 3.07 mmol) was dissolved in 12 mL of methanol, and 10% wet

palladium on carbon (containing 50% water, 327 mg, Accela ChemBio (Shanghai) Co., Ltd.) was then added. The system was purged with hydrogen gas 3 times and left to react for 12 hours under a hydrogen balloon atmosphere. The reaction mixture was filtered using celite. The resulting filtrate was concentrated under reduced pressure, and the resulting residue was purified by silica gel column chromatography with eluent system B to give the title compound **23c** (600 mg, yield: 66%).

MS m/z (ESI): 296.2 [M+1].

### Step 3 tert-Butyl (±)-4-(5-((2,6-dioxopiperidin-3-yl)amino)-2-fluorophenyl)piperazine-1-carboxylate 23d

Compound **23c** (400 mg, 1.35 mmol) was dissolved in 10 mL of acetonitrile, and (±)-3-bromopiperidine-2,6-dione (312 mg, 1.63 mmol) and sodium bicarbonate (455 mg, 5.42 mmol) were then added. After 4 hours of reaction at 85 °C, 3-bromopiperidine-2,6-dione (312 mg, 1.63 mmol) was added again. After another 4 hours of reaction, 3-bromopiperidine-2,6-dione (312 mg, 1.63 mmol) was added again, and the mixture was left to react for 4 hours. The reaction mixture was concentrated under reduced pressure, and the resulting residue was purified by silica gel column chromatography with eluent system B to give the title compound **23d** (400 mg, yield: 73%).

MS m/z (ESI): 407.4 [M+1].

### Step 4 (±)-3-((4-Fluoro-3-(piperazin-1-yl)phenyl)amino)piperidine-2,6-dione hydrochloride 23e

Compound **23d** (200 mg, 0.49 mmol) was weighed into a 50 mL eggplant-shaped flask, and a 4 M solution of hydrogen chloride in 1,4-dioxane (3 mL) was added. After 2 hours of reaction, the system was concentrated under reduced pressure and dried *in vacuo* to give the title compound **23e** as a crude product (168 mg, yield: 99%). The crude product was directly used in the next step without purification.

MS m/z (ESI): 307.2 [M+1].

### Step 5 tert-Butyl (±)-4-((4-(5-((2,6-dioxopiperidin-3-yl)amino)-2-fluorophenyl)piperazin-1-yl)methyl)piperidine-1-carboxylate 23f

To a 25 mL single-necked flask was added 3 mL of a mixed solution of dichloromethane and methanol (V/V = 2/1), followed by compound **23e** (100 mg, 0.29 mmol) and anhydrous sodium acetate (216 mg, 2.6 mmol). After 15 minutes of reaction, compound **3a** (112 mg, 0.53 mmol) was added. After 0.5 hours of reaction, sodium triacetoxyborohydride (112 mg, 0.53 mmol) was slowly added, and the mixture was left to react for 1 hour. The reaction mixture was concentrated under reduced pressure, and the resulting residue was purified by silica gel column chromatography with eluent system A to give the title compound **23f** (130 mg, yield: 98%).

MS m/z (ESI): 504.9 [M+1].

### Step 6 (±)-3-((4-Fluoro-3-(4-(piperidin-4-ylmethyl)piperazin-1-yl)phenyl)amino)piperidine-2,6-dione dihydrochloride 23g

Compound **23f** (130 mg, 0.26 mmol) was weighed into a 50 mL eggplant-shaped flask, and a 4 M solution of hydrogen chloride in 1,4-dioxane (3 mL) was added. After 2 hours of reaction, the system was concentrated under reduced pressure and dried *in vacuo* to give the title compound **23g** as a crude product (122 mg, yield: 99%). The crude product was directly used in the next step without purification.

### Step 7 2-Chloro-4-((3S)-8-(4-(4-((4-(5-((2,6-dioxopiperidin-3-yl)amino)-2-fluorophenyl)piperazin-1-yl)methyl)piperidine-1-carbonyl)phenyl)-3-methyl-2,8-diazaspiro[4.5]decan-2-yl)benzonitrile 23 (a mixture of diastereomers)

To a 25 mL single-necked flask were sequentially added 2 mL of *N,N-*dimethylformamide, compound **23g** (80 mg, 0.17 mmol), and compound **1h** (69 mg, 0.17 mmol). *N,N-*Diisopropylethylamine (130 mg, 1.0 mmol) and *O*-(7-azabenzotriazol-1-yl)-*N*,*N*,*N*,*N*-tetramethyluronium hexafluorophosphate (96 mg, 0.25 mmol) were slowly added under an ice bath. The cooling bath was removed, and the mixture was left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 60%-80%, flow rate: 30 mL/min) to give the title compound **23** (a mixture of diastereomers, a 1:1 ratio, 35 mg, yield: 26%).

MS m/z (ESI):795.3 [M+1].

¹H NMR (500 MHz, DMSO-*d₆*):δ 10.75 (s, 1H), 7.58 (d, 1H), 7.23 (d, 2H), 6.93 (d, 2H), 6.85-6.76 (m, 2H), 6.65 (dd, 1H), 6.33 (dd, 1H), 6.19 (dt, 1H), 5.66 (d, 1H), 4.28-4.21 (m, 1H), 4.07-3.99 (m, 1H), 3.42 (d, 1H), 3.38-3.31 (m, 5H),3.25-3.14 (m, 2H), 3.00-2.80 (m, 5H), 2.76-2.67 (m, 1H), 2.64-2.50 (m, 3H), 2.48-2.44 (m, 3H), 2.26-2.16 (m, 2H),2.12-1.94 (m, 2H), 1.90-1.65 (m, 5H),1.60-1.54 (m, 1H), 1.53-1.40 (m, 2H),1.29-1.16 (m, 4H), 1. 11 -0.98(m, 2H).

### Example 24 2-Chloro-4-((3S)-8-(4-(4-((4-(4-((2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile 24 (a mixture of diastereomers)

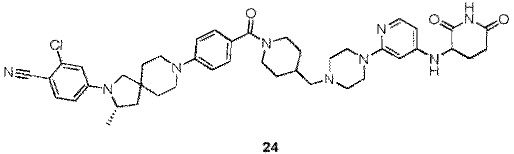

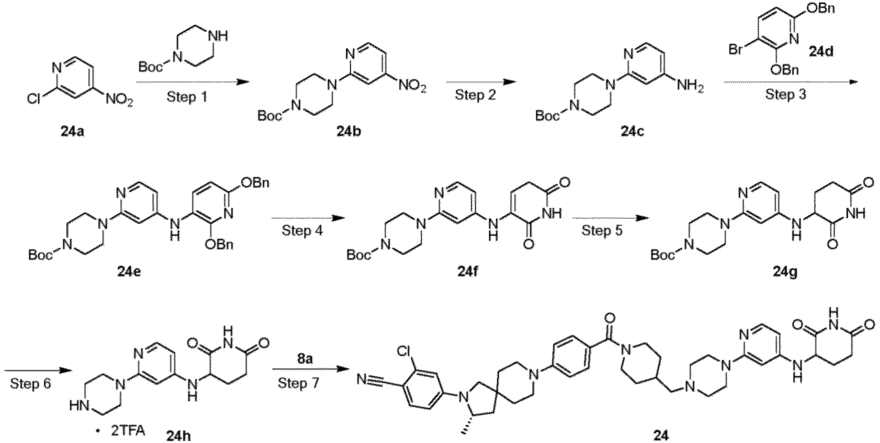

### Step 1 tert-Butyl 4-(4-nitropyridin-2-yl)piperazine-1-carboxylate 24b

To a 50 mL single-necked flask were sequentially added 25 mL of *N,N-*dimethylformamide, 2-chloro-4-nitropyridine **24a** (2.84 g, 17.90 mmol, Bide Pharmatech), and tert-butyl piperazine-1-carboxylate (3.34 g, 19.9 mmol, Bide Pharmatech). *N,N-*Diisopropylethylamine (4.63 mg, 35.8 mmol) was slowly added, and the system was then heated to 100 °C and left to react for 16 hours. 50 mL of a saturated sodium bicarbonate solution was added to the reaction system under ice bath cooling, and extraction was performed with dichloromethane (50 mL × 3). The organic phases were combined, washed with a saturated sodium chloride solution (50 mL × 3), dried over anhydrous sodium sulfate, and filtered. The filtrate was concentrated under reduced pressure, and the resulting residue was purified by silica gel column chromatography with eluent system B to give the title compound **24b** (1.3 g, yield: 23%).

MS m/z (ESI): 253.1 [M-55].

### Step 2 tert-Butyl 4-(4-aminopyridin-2-yl)piperazine-1-carboxylate 24c

To a 100 mL single-necked flask were sequentially added 30 mL of a mixed solution of ethyl acetate and methanol (V/V = 1/1), compound **24b** (1.0 g, 3.24 mmol), and 10% palladium on carbon (containing 50% water, 300 mg). The system was then purged with hydrogen gas 3 times and left to react for 2 hours under a hydrogen atmosphere. The reaction mixture was filtered, and the filtrate was concentrated under reduced pressure to give the title compound **24c** (902 mg, yield: 99%). The crude product was directly used in the next step without purification.

MS m/z (ESI): 279.2 [M+1].

### Step 3 tert-Butyl 4-(4-((2,6-bis(benzyloxy)pyridin-3-yl)amino)pyridin-2-yl)piperazine-1-carboxylate 24e

To a 50 mL single-necked flask were sequentially added 25 mL of toluene, 2,6-bis(benzyloxy)-3-bromopyridine **24d** (554 mg, 1.49 mmol, Bide Pharmatech), and compound **24c** (278 mg, 1.00 mmol), followed by methanesulfonato(9,9-dimethyl-4,5-bis(diphenylphosphino)xanthene)(2'-amino-1,1'-biphenyl-2-yl)palladium(II) (135 mg, 0.14 mmol) and sodium tert-butoxide (191 mg, 1.99 mmol). The system was heated to 100 °C and left to react for 16 hours under a nitrogen atmosphere. 50 mL of a saturated sodium bicarbonate solution was added to the system under ice bath cooling to quench the reaction, and extraction was performed with dichloromethane (50 mL × 3). The organic phases were combined, washed with a saturated sodium chloride solution (50 mL × 3), dried over anhydrous sodium sulfate, and filtered. The filtrate was concentrated under reduced pressure, and the resulting residue was purified by silica gel column chromatography with eluent system A to give the title compound **24e** (480 mg, yield: 84%).

MS m/z (ESI): 568.2 [M+1].

### Step 4 tert-Butyl 4-(4-((2,6-dioxo-1,2,5,6-tetrahydropyridin-3-yl)amino)pyridin-2-yl)piperazine-1-carboxylate 24f

To a 100 mL single-necked flask were sequentially added 20 mL of a mixed solution of ethyl acetate and ethanol (V/V = 3/1), compound **24e** (480 mg, 0.84 mmol), and 10% palladium on carbon (containing 50% water, 480 mg, 3.91 mmol). The mixture was left to react at room temperature for 16 hours under a hydrogen atmosphere. The filtrate was concentrated under reduced pressure to give the title compound **24f** as a crude product (327 mg, yield: 99%). The crude product was directly used in the next step without purification.

MS m/z (ESI): 388.2 [M+1].

### Step 5 tert-Butyl (±)-4-(4-((2,6-dioxopiperidin-3-yl)amino)pyridin-2-yl)piperazine-1-carboxylate 24g

To a 100 mL single-necked flask were sequentially added 48 mL of a mixed solution of tetrahydrofuran and ethanol (V/V = 5/1), compound **24g** (400 mg, 1.03 mmol), and platinum dioxide (152 mg, 0.67 mmol). The system was purged with hydrogen gas 3 times, heated to 45 °C, and left to react for 8 hours under a hydrogen atmosphere. The system was purged with hydrogen gas 3 times and left to react at 45 °C for 8 hours under a hydrogen atmosphere. The filtrate was concentrated under reduced pressure to give the title compound **24g** as a crude product (30 mg, yield: 7.6%). The crude product was directly used in the next step without purification.

MS m/z (ESI): 390.2 [M+1].

### Step 6 (±)-3-((2-(Piperazin-1-yl)pyridin-4-yl)amino)piperidine-2,6-dione bistrifluoroacetate 24h

To a 50 mL single-necked flask were sequentially added 4 mL of a mixed solution of trifluoroacetic acid and dichloromethane (V/V = 1/4) and compound **24g** (50 mg, 0.13 mmol). The mixture was left to react for 1 hour. The reaction mixture was concentrated under reduced pressure to give the title compound **24h** as a crude product (66 mg, yield: 99%). The crude product was directly used in the next step without purification.

MS m/z (ESI): 290.3 [M+1].

### Step 7 2-Chloro-4-((3S)-8-(4-(4-((4-(4-((2,6-dioxopiperidin-3-yl)amino)pyridin-2-yl)piperazin-1-yl)methyl)piperidine-1-carbonyl)phenyl)-3-methyl-2,8-diazaspiro[4.5]decan-2-yl)benzonitrile 24 (a mixture of diastereomers)

To a 25 mL single-necked flask was added 3 mL of a mixed solution of dichloromethane and methanol (V/V = 2/1), followed by compound **24h** (39 mg, 0.07 mmol) and anhydrous sodium acetate (49 mg, 0.64 mmol). After 15 minutes of reaction, compound **8a** (49 mg, 0.09 mmol) was added. After 30 minutes of reaction, sodium triacetoxyborohydride (32 mg, 0.15 mmol) was slowly added, and the mixture was left to react for 16 hours. 10 mL of a saturated sodium bicarbonate solution was added to the reaction system under ice bath cooling, and extraction was performed with ethyl acetate (50 mL × 3). The organic phases were combined, washed with a saturated sodium chloride solution (20 mL × 3), dried over anhydrous sodium sulfate, and filtered. The filtrate was concentrated under reduced pressure and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **24** (a mixture of diastereomers, a 1:1 ratio, 10 mg, yield: 17%).

MS m/z (ESI): 778.4 [M+1].

¹H NMR (500 MHz, DMSO-*d₆*):δ 10.97 (s, 1H), 8.22-7.90 (m, 1H), 7.80-7.48 (m, 2H), 7.24 (d, 2H), 6.95 (d, 2H), 6.80 (s, 1H), 6.66 (d, 1H), 6.40 (s, 1H), 6.14 (s, 1H), 4.85-4.62 (m, 1H), 4.35-3.92 (m, 3H), 3.72-3.40 (m, 4H), 3.39-3.32 (m, 6H), 3.22-3.13 (m, 2H), 3.10-2.58 (m, 5H), 2.48-2.35 (m, 2H), 2.30-1.95 (m, 4H), 1.92-1.65 (m, 4H), 1.62-1.40 (m, 3H), 1.34-1.16 (m, 4H),1.14-0.95 (m, 2H).

### Example 25 2-Chloro-4-((3S)-8-(4-(4-((4-(4-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 25 (a mixture of diastereomers)

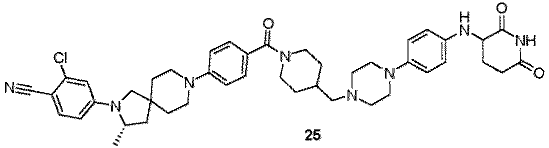

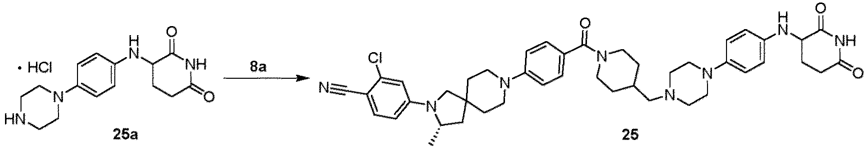

The compound (±)-3-((4-(piperazin-1-yl)phenyl)amino)piperidine-2,6-dione hydrochloride **25a** (25 mg, 0.069 mmol, prepared using the method disclosed in "Example 89 on page 268 of the specification in the patent application WO2018237026A1") was dissolved in 5 mL of a mixed solvent of methanol and dichloromethane (V/V = 4/1), and sodium acetate (25 mg, 0.30 mmol) was added. After 30 minutes of reaction, compound **8a** (30 mg, 0.059 mmol) was added. After 15 minutes of reaction, sodium triacetoxyborohydride (26 mg, 0.12 mmol) was added, and the mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 50%-95%, flow rate: 30 mL/min) to give the title compound **25** (a mixture of diastereomers, a 1:1 ratio, 16 mg, yield: 35%).

MS m/z (ESI): 777.3 [M+1].

¹H NMR (500 MHz, DMSO-*d₆*): δ 10.74 (s, 1H), 7.58 (d, 1H), 7.26-7.20 (m, 2H), 6.96-6.90 (m, 2H), 6.79 (s, 1H), 6.76-6.70 (m, 2H), 6.65 (d, 1H), 6.62-6.55 (m, 2H), 5.36 (d, 1H), 4.21-4.14 (m, 1H), 4.06-3.98 (m, 1H), 3.46-3.33 (m, 4H), 3.30-3.24 (m, 2H), 3.24-3.13 (m, 2H), 2.98-2.84 (m, 4H), 2.77-2.65 (m, 1H), 2.65-2.52 (m, 2H), 2.48-2.42 (m, 5H), 2.27-1.94 (m, 4H),1.86-1.66 (m, 5H),1.61-1.53 (m, 1H), 1.52-1.41 (m, 2H), 1.25-1.16 (m, 4H), 1.10-0.97 (m, 2H).

### Example 26 2-Chloro-4-((3S)-8-(4-(4-((4-(4-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile 26 (a mixture of diastereomers)

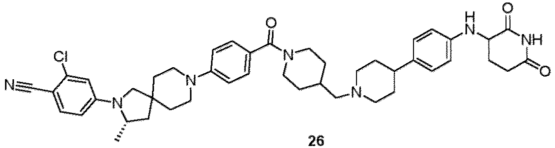

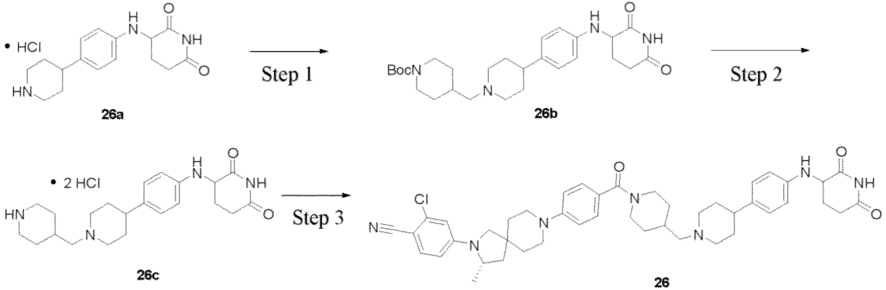

### Step 1 tert-Butyl (±)-4-((4-(4-((2,6-dioxopiperidin-3-yl)amino)phenyl)piperidin-1-yl)methyl)piperidine-1-carboxylate 26b

To a 50 mL single-necked flask was added 8 mL of a mixed solution of dichloromethane and methanol (V/V = 4/1), followed by (±)-3-((4-(piperidin-4-yl)phenyl)amino)piperidine-2,6-dione hydrochloride **26a** (830 mg, 2.89 mmol, prepared using the method disclosed for "compound 2-2 on page 348 of the specification in the patent application WO2021127561A1") and anhydrous sodium acetate (731 mg, 8.7 mmol). After 15 minutes of reaction, compound **3a** (742 mg, 3.48 mmol) was added. After 30 minutes of reaction, sodium triacetoxyborohydride (738 mg, 3.48 mmol) was slowly added, and the mixture was left to react for 1 hour. Dichloromethane and methanol were removed by concentration under reduced pressure, and the resulting residue was purified by silica gel column chromatography with eluent system A to give the title compound **26b** (500 mg, yield: 59%).

MS m/z (ESI): 485.8 [M+1].

### Step 2 (±)-3-((4-(1-(Piperidin-4-ylmethyl)piperidin-4-yl)phenyl)amino)piperidine-2,6-dione dihydrochloride 26c

Compound **26b** (500 mg, 1.03 mmol) was dissolved in 10 mL of dichloromethane, and a 4 M solution of hydrogen chloride in 1,4-dioxane (2 mL) was slowly added. The mixture was left to react for 1 hour. The reaction mixture was concentrated under reduced pressure and dried *in vacuo* to give the title compound **26c** as a crude product (450 mg, yield: 95%). The crude product was directly used in the next step without purification.

MS m/z (ESI): 385.3 [M+1].

### Step 3 2-Chloro-4-((3S)-8-(4-(4-((4-(4-((2,6-dioxopiperidin-3-yl)amino)phenyl)piperidin-1-yl)methyl)piperidine-1-carbonyl)phenyl)-3-methyl-2,8-diazaspiro[4.5]decan-2-yl)benzonitrile 26 (a mixture of diastereomers)

To a 50 mL single-necked flask were sequentially added 2 mL of *N,N-*dimethylformamide, compound **1h** (40 mg, 0.10 mmol), *N,N-*diisopropylethylamine (63 mg, 0.49 mmol), and *O*-(7-azabenzotriazol-1-yl)-N,N,N,N-tetramethyluronium hexafluorophosphate (55 mg, 0.14 mmol). After 15 minutes of reaction, compound **26c**

(53 mg, 0.12 mmol) was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 60%-85%, flow rate: 30 mL/min) to give the title compound **26** (a mixture of diastereomers, a 1:1 ratio, 25 mg, yield: 33%).

MS m/z (ESI): 776.3 [M+1].

¹H NMR (500 MHz, DMSO-*d₆*): δ 10.76 (s, 1H), 7.59 (d, 1H), 7.24 (d, 2H), 6.94 (d, 4H), 6.80 (d, 1H), 6.66 (d, 1H), 6.60 (d, 2H), 5.64 (d, 1H), 4.31-4.21 (m, 1H), 4.08-4.00 (m, 1H), 3.44 (d, 1H), 3.26-3.15 (m, 2H), 3.00-2.68 (m, 5H), 2.64-2.54 (m, 2H), 2.38-2.06 (m, 6H), 2.03-1.91 (m, 3H), 1.87-1.43 (m, 15H), 1.20 (d, 3H), 1.10-1.00 (m, 2H).

### Example 27 2-Chloro-4-((3S)-8-(4-(4-((4-(4-((2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile 27 (a mixture of diastereomers)

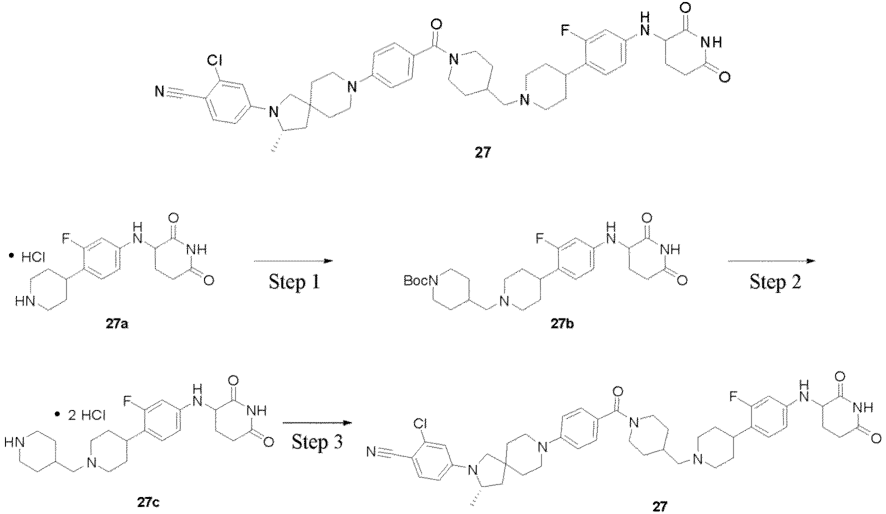

### Step 1 tert-Butyl (±)-4-((4-(4-((2,6-dioxopiperidin-3-yl)amino)-2-fluorophenyl)piperidin-1-yl)methyl)piperidine-1-carboxylate 27b

To a 50 mL single-necked flask was added 4 mL of a mixed solution of dichloromethane and methanol (V/V = 3/1), followed by (±)-3-((3-fluoro-4-(piperidin-4-yl)phenyl)amino)piperidine-2,6-dione hydrochloride **27a** (106 mg, 0.35 mmol, prepared using the method disclosed for "compound 6 on page 353 of the specification in the patent application WO2021127561A1") and anhydrous sodium acetate (146 mg, 1.74 mmol). After 15 minutes of reaction, compound **3a** (148 mg, 0.70 mmol) was added. After 30 minutes of reaction, sodium triacetoxyborohydride (148 mg, 0.70 mmol) was slowly added, and the mixture was left to react for 1 hour. Dichloromethane and methanol were removed by concentration under reduced pressure, and the resulting residue was purified by silica gel column chromatography with eluent system A to give the title compound **27b** (150 mg, yield: 86%).

MS m/z (ESI): 503.9 [M+1].

### Step 2 (±)-3-((3-Fluoro-4-(1-(piperidin-4-ylmethyl)piperidin-4-yl)phenyl)amino)piperidine-2,6-dione dihydrochloride 27c

Compound **27b** (150 mg, 0.30 mmol) was dissolved in 3 mL of dichloromethane, and a 4 M solution of hydrogen chloride in 1,4-dioxane (0.5 mL) was slowly added. The mixture was left to react for 1 hour. The reaction mixture was concentrated under reduced pressure and dried *in vacuo* to give the title compound **27c** as a crude product (140 mg, yield: 99%). The crude product was directly used in the next step without purification.

MS m/z (ESI): 403.4 [M+1].

### Step 3 2-Chloro-4-((3S)-8-(4-(4-((4-(4-((2,6-dioxopiperidin-3-yl)amino)-2-fluorophenyl)piperidin-1-yl)methyl)piperidine-1-carbonyl)phenyl)-3-methyl-2,8-diazaspiro[4.5]decan-2-yl)benzonitrile 27 (a mixture of diastereomers)

To a 50 mL single-necked flask were sequentially added 2 mL of *N,N-*dimethylformamide, compound **1h** (50 mg, 0.12 mmol), *N,N-*diisopropylethylamine (79 mg, 0.61 mmol), and O-(7-azabenzotriazol-1-yl)-*N*,*N*,*N*,*N*-tetramethyluronium hexafluorophosphate (70 mg, 0.18 mmol). After 15 minutes of reaction, compound **27c** (70 mg, 0.15 mmol) was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 64%-82%, flow rate: 30 mL/min) to give the title compound 27 (a mixture of diastereomers, a 1:1 ratio, 40 mg, yield: 41%).

MS m/z (ESI): 794.3 [M+1].

¹H NMR (500 MHz, DMSO-*d₆*) δ 10.78 (s, 1H), 7.59 (d, 1H), 7.24 (d, 2H), 6.98 (t, 1H), 6.94 (d, 2H), 6.80 (s, 1H), 6.66 (d, 1H), 6.43 (t, 2H), 5.99 (d, 1H), 4.33-4.25 (m, 1H), 4.07-4.00 (m, 1H), 3.49-3.14 (m, 11H), 2.96-2.80 (m, 3H), 2.78-2.68 (m, 1H), 2.61-2.55 (m, 1H), 2.27-2.10 (m, 1H), 2.16 (d, 2H), 2.11-2.04 (m, 1H), 2.03-1.91 (m, 2H), 1.88-1.67 (m, 6H), 1.65-1.55 (m, 4H), 1.52-1.43 (m, 2H), 1.20 (d, 3H), 1.10-0.97 (m, 2H).

### Example 28 (S)-2-Chloro-4-(8-(4-(2-(4-(4-(2,4-dioxotetrahydropyrimidin-1(2H)-yl)phenyl)piperazin-1-yl)-7-azaspiro[3.5]nonane-7-carbonyl)phenyl)-3-methyl-2,8-diazaspiro[4.5]decan-2-yl)benzonitrile 28

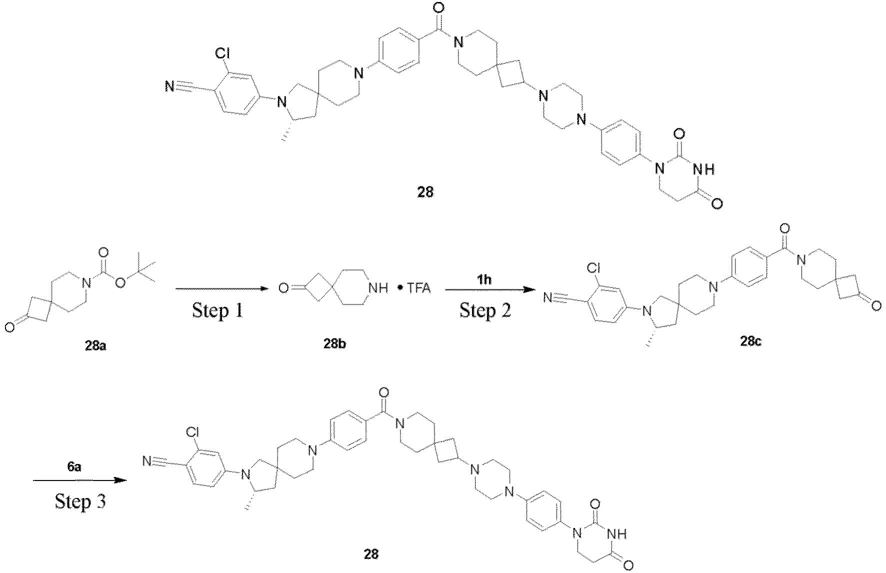

### Step 1 7-Azaspiro[3.5]nonan-2-one trifluoroacetate 28b

tert-Butyl 2-oxo-7-azaspiro[3.5]nonane-7-carboxylate **28a** (323 mg, 1.35 mmol, Bide Pharmatech) was dissolved in dichloromethane (3 mL), and 1 mL of trifluoroacetic acid was added. The mixture was left to react for 1 hour. The reaction mixture was concentrated under reduced pressure and dried *in vacuo* to give the title compound **28b** as a crude product (237 mg, yield: 99%). The crude product was directly used in the next step without purification.

MS m/z (ESI): 140.2 [M+1].

### Step 2 (S)-2-Chloro-4-(3-methyl-8-(4-(2-oxo-7-azaspiro[3.5]nonane-7-carbonyl)phenyl)-2,8-diazaspiro[4.5]decan-2-yl)benzonitrile 28c

To a 25 mL reaction flask were sequentially added **1h** (200 mg, 0.49 mmol), compound **28b** (190 mg, 0.75 mmol), and *N,N-*dimethylformamide (2 mL), followed by O-(7-azabenzotriazol-1-yl)-*N,N,N*',*N*'-tetramethyluronium hexafluorophosphate (222 mg, 0.54 mmol) and *N,N-*diisopropylethylamine (252 mg, 1.95 mmol). After 1 hour of reaction, the reaction mixture was concentrated under reduced pressure, and the resulting residue was purified by silica gel column chromatography with eluent system A to give the title compound **28c** (200 mg, yield: 77%).

MS m/z (ESI): 531.2 [M+1].

### Step 3 (S)-2-Chloro-4-(8-(4-(2-(4-(4-(2,4-dioxotetrahydropyrimidin-1(2H)-yl)phenyl)piperazin-1-yl)-7-azaspiro[3.5]nonane-7-carbonyl)phenyl)-3-methyl-2,8-diazaspiro[4.5]decan-2-yl)benzonitrile 28

Compound **6a** (65 mg, 0.21 mmol) was weighed into a 25 mL eggplant-shaped flask, and 8 mL of a mixed solvent of dichloromethane and methanol (V/V = 3/1) and anhydrous sodium acetate (93 mg, 1.14 mmol) were added. After 15 minutes of reaction, compound **28c** (100 mg, 0.19 mmol) was added to the system. After 1 hour of reaction, sodium triacetoxyborohydride (80 mg, 0.377 mmol) was added, and the mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 50%-70%, flow rate: 30 mL/min) to give the title compound **28** (100 mg, yield: 67%).

MS m/z (ESI): 789.5 [M+1].

¹H NMR (500 MHz, DMSO-*d₆*) δ 10.25 (s, 1H), 7.59 (d, 1H), 7.23 (d, 2H), 7.14 (d, 2H), 6.93 (t, 4H), 6.80 (s, 1H), 6.66 (d, 1H), 4.07-4.00 (m, 1H), 3.69 (t, 2H), 3.43 (d, 2H), 3.39-3.33 (m, 3H), 3.26-3.16 (m, 2H),3.12 (s, 4H) 2.76-2.62 (m, 4H), 2.38 (s, 4H), 2.23 (dd, 1H), 2.05-1.95 (m, 3H), 1.78-1.66 (m, 2H), 1.64-1.42 (m, 10H), 1.20 (d, 3H).

### Example 29 (S)-2-Chloro-4-(8-(4-(9-(4-(4-(2,4-dioxotetrahydropyrimidin-1(2H)-yl)phenyl)piperazin-1-yl)-3-azaspiro[5.5]undecane-3-carbonyl)phenyl)-3-methyl-2,8-diazaspiro[4.5]decan-2-yl)benzonitrile 29

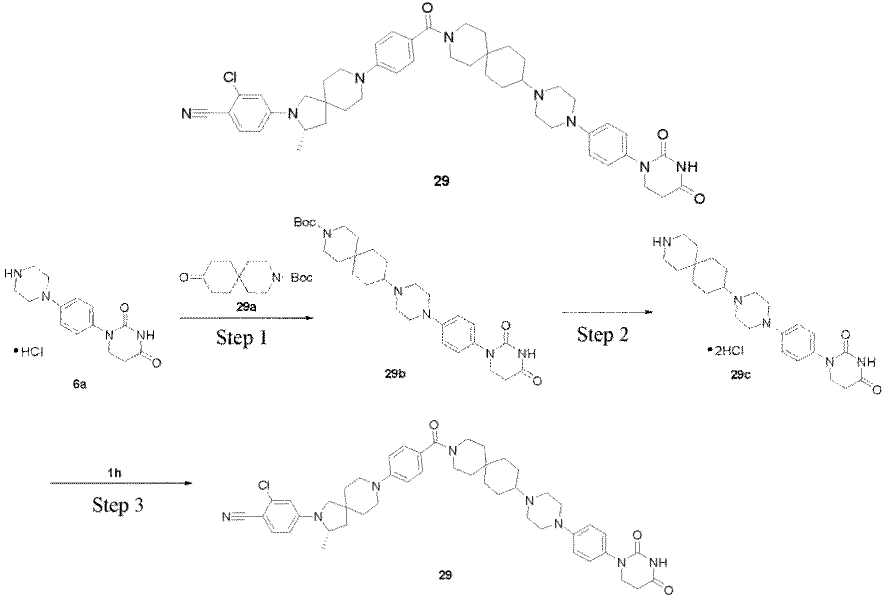

### Step 1 tert-Butyl 9-(4-(4-(2,4-dioxotetrahydropyrimidin-1(2H)-yl)phenyl)piperazin-1-yl)-3-azaspiro[5.5]undecane-3-carboxylate 29b

To a 50 mL reaction flask was added 6 mL of a mixed solvent of 1,2-dichloroethane and methanol (V/V = 5/1), followed by compound **6a** (100 mg, 0.32 mmol) and anhydrous sodium acetate (158 mg, 1.93 mmol). After 10 minutes of reaction, tert-butyl 9-oxo-3-azaspiro[5.5]undecane-3-carboxylate **29a** (215 mg, 0.80 mmol) was added. After 0.5 hours of reaction, sodium triacetoxyborohydride (137 mg, 0.65 mmol) was added. After 1 hour of reaction, the reaction mixture was concentrated under reduced pressure, and the resulting residue was purified by silica gel column chromatography with eluent system A to give the title compound **29b** (100 mg, yield: 59%).

MS m/z (ESI): 526.3 [M+1].

### Step 2 1-(4-(4-(3-Azaspiro[5.5]undecan-9-yl)piperazin-1-yl)phenyl)dihydropyrimidine-2,4(1H,3H)-dione dihydrochloride 29c

Compound **29b** (100 mg, 0.19 mmol) was dissolved in a 4 M solution of hydrogen chloride in 1,4-dioxane (3 mL), and the mixture was left to react for 3 hours. The reaction mixture was concentrated and dried *in vacuo* to give the title compound **29c** as a crude product (94 mg, yield: 99%). The crude product was directly used in the next step without purification.

MS m/z (ESI): 426.2 [M+1].

### Step 3 (S)-2-Chloro-4-(8-(4-(9-(4-(4-(2,4-dioxotetrahydropyrimidin-1(2H)-yl)phenyl)piperazin-1-yl)-3-azaspiro[5.5]undecane-3-carbonyl)phenyl)-3-methyl-2, 8-diazaspiro[4.5]decan-2-yl)benzonitrile 29

To a 25 mL reaction flask were sequentially added 3 mL of *N,N-*dimethylformamide, compound **1h** (30 mg, 0.073 mmol), and compound **29c** (44 mg, 0.09 mmol), followed by *O*-(7-azabenzotriazol-1-yl)-*N,N,N',N'*-tetramethyluronium hexafluorophosphate (42 mg, 0.11 mmol) and *N,N-*diisopropylethylamine (38 mg, 0.29 mmol). After 1 hour of reaction, the reaction mixture was filtered and separated and purified by preparative high performance liquid chromatography (Waters 2767-SQ Detecor2, elution system: 10 mmol/L aqueous ammonium bicarbonate solution and acetonitrile, gradient of acetonitrile: 50%-70%, flow rate: 30 mL/min) to give the title compound **29** (15 mg, yield: 25%).

MS m/z (ESI): 817.3 [M+1].

¹H NMR (500 MHz, DMSO-*d₆*): δ 10.24 (s, 1H), 7.58 (d, 1H), 7.23 (d, 2H), 7.13 (d, 2H), 6.96-6.86 (m, 4H), 6.79 (d, 1H), 6.65 (dd, 1H), 4.07-3.97 (m, 1H), 3.73-3.64 (m, 2H), 3.51-3.34 (m, 6H), 3.29-3.04 (m, 7H), 2.92-2.83 (m, 1H), 2.71-2.52 (m, 7H), 2.29-2.17 (m, 2H), 2.05-1.94 (m, 1H), 1.79-1.54 (m, 6H), 1.52-1.25 (m, 7H), 1.19 (d, 3H), 1.16-1.05 (m, 2H).

### Example 30 (S)-2-Chloro-4-(8-(4-(2-(4-(4-(2,4-dioxotetrahydropyrimidin-1(2H)-yl)phenyl)piperazin-1-yl)-7-azaspiro[3.5]nonane-7-carbonyl)-2-fluorophenyl)-3-methyl-2,8-diazaspiro[4.5]decan-2-yl)benzonitrile 30

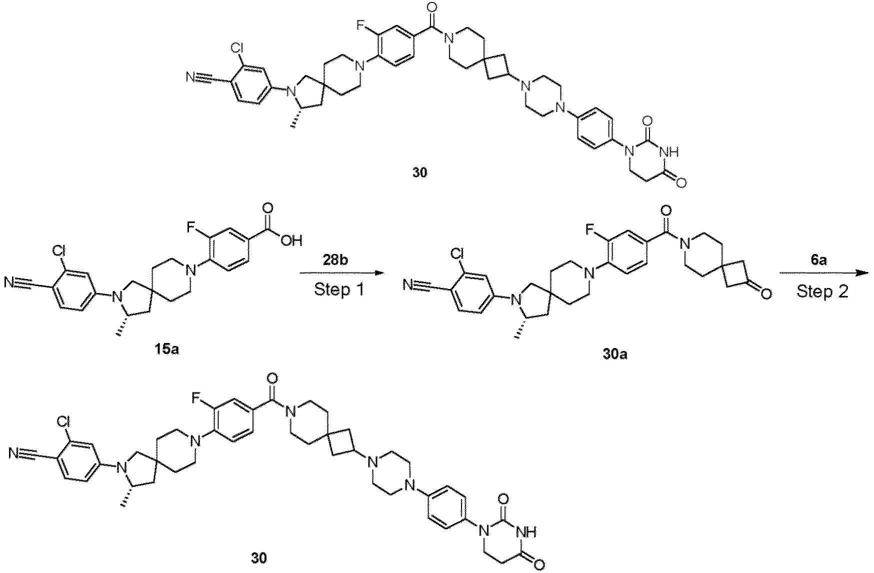

### Step 1 (S)-2-Chloro-4-(8-(2-fluoro-4-(2-oxo-7-azaspiro[3.5]nonane-7-carbonyl)phenyl)-3-methyl-2,8-diazaspiro[4.5]decan-2-yl)benzonitrile 30a

Compound **15a** (77 mg, 0.18 mmol) was dissolved in *N,N-*dimethylformamide (2 mL). Compound **28b** (71 mg, 0.28 mmol) was added, and *O*-(7-azabenzotriazol-1-yl)-*N,N,N*',*N*'-tetramethyluronium hexafluorophosphate (82 mg, 0.215 mmol) and *N,N-*diisopropylethylamine (116 mg, 0.90 mmol) were then added. After 1 hour of reaction, the reaction mixture was concentrated under reduced pressure, and the resulting residue was purified by silica gel column chromatography with eluent system A to give the title compound **30a** (80 mg, yield: 81%).

### Step 2 (S)-2-Chloro-4-(8-(4-(2-(4-(4-(2,4-dioxotetrahydropyrimidin-1(2H)-yl)phenyl)piperazin-1-yl)-7-azaspiro[3.5]nonane-7-carbonyl)-2-fluorophenyl)-3-methyl-2,8-diazaspiro[4.5]decan-2-yl)benzonitrile 30

Compound **6a** (53 mg, 0.17 mmol) was weighed into a 25 mL eggplant-shaped flask, and 8 mL of a mixed solvent of dichloromethane and methanol (V/V = 3/1) and anhydrous sodium acetate (65 mg, 0.79 mmol) were added. After 15 minutes of reaction, compound **30a** (72 mg, 0.13 mmol) was added to the system. After 1 hour of reaction, sodium cyanoborohydride (16 mg, 0.26 mmol) was added, and the mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 47%-67%, flow rate: 30 mL/min) to give the title compound **30** (25 mg, yield: 24%).

MS m/z (ESI): 807.3 [M+1].

¹H NMR (500 MHz, DMSO-*d₆*): δ 10.26 (s, 1H), 7.60 (d, 1H), 7.20-7.12 (m, 4H), 7.07 (t, 1H), 6.93 (d, 2H), 6.80 (s, 1H), 6.66 (d, 1H), 4.09-3.97 (m, 1H), 3.69 (t, 2H), 3.54-3.40 (m, 3H), 3.34-3.32 (m, 3H), 3.16-3.09 (m, 6H), 3.06-3.00 (m, 2H), 2.68 (t, 2H), 2.45-2.30 (m, 3H), 2.28-2.22 (m, 1H), 2.05-1.92 (m, 3H), 1.85-1.72 (m, 2H), 1.68-1.39 (m, 10H), 1.21 (d, 3H).

### Example 31 (S)-2-Chloro-4-(8-(4-(2-(4-(4-(2,4-dioxotetrahydropyrimidin-1(2H)-yl)phenyl)piperazin-1-yl)-7-azaspiro[3.5]nonane-7-carbonyl)-3-fluorophenyl)-3-methyl-2,8-diazaspiro[4.5]decan-2-yl)benzonitrile 31

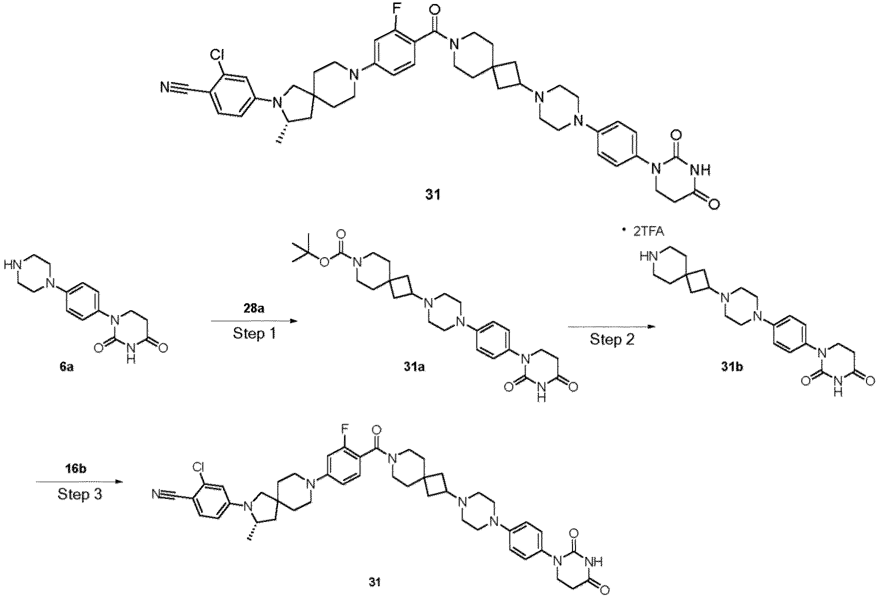

### Step 1 tert-Butyl 2-(4-(4-(2,4-dioxotetrahydropyrimidin-1(2H)-yl)phenyl)piperazin-1-yl)-7-azaspiro[3.5]nonane-7-carboxylate 31a

Compound **6a** (277 mg, 0.89 mmol) was weighed into a 25 mL eggplant-shaped flask, and 25 mL of a mixed solvent of dichloromethane and methanol (V/V = 4/1) and anhydrous sodium acetate (439 mg, 5.35 mmol) were added. After 15 minutes of reaction, compound **28a** (257 mg, 1.07 mmol) was added to the system. After 1 hour of reaction, sodium cyanoborohydride (107 mg, 1.79 mmol) was added, and the mixture was left to react for 12 hours. The reaction mixture was concentrated under reduced pressure, and the resulting residue was purified by silica gel column chromatography with eluent system A to give the title compound **31a** (330 mg, yield: 74%).

MS m/z (ESI): 498.8 [M+1].

### Step 2 1-(4-(4-(7-Azaspiro[3.5]nonan-2-yl)piperazin-1-yl)phenyl)dihydropyrimidine-2,4(1H,3H)-dione bistrifluoroacetate 31b

Compound **31a** (330 mg, 0.66 mmol) was added to a 25 mL eggplant-shaped flask. 10 mL of dichloromethane was added, and 2 mL of trifluoroacetic acid was added. The mixture was left to react for 1 hour. The reaction mixture was concentrated under reduced pressure to give the title compound **31b** as a crude product (300 mg, yield: 72%). The crude product was directly used in the next step without purification.

MS m/z (ESI): 398.4 [M+1].

### Step 3 (S)-2-Chloro-4-(8-(4-(2-(4-(4-(2,4-dioxotetrahydropyrimidin-1(2H)-yl)phenyl)piperazin-1-yl)-7-azaspiro[3.5]nonane-7-carbonyl)-3-fluorophenyl)-3-methyl-2,8-diazaspiro[4.5]decan-2-yl)benzonitrile 31

Compound **31b** (43 mg, 0.069 mmol) was dissolved in *N*,*N*-dimethylformamide (3 mL). Compound **16b** (30 mg, 0.70 mmol) was added, and *O*-(7-azabenzotriazol-1-yl)-*N,N,N*',*N*'-tetramethyluronium hexafluorophosphate (35 mg, 0.091 mmol) and *N,N-*diisopropylethylamine (36 mg, 0.28 mmol) were then added. The mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 47%-67%, flow rate: 30 mL/min) to give the title compound **31** (15 mg, yield: 27%).

MS m/z (ESI): 807.3 [M+1].

¹H NMR (500 MHz, DMSO-*d₆*): δ 10.26 (s, 1H), 7.60 (d, 1H), 7.22-7.10 (m, 3H), 6.93 (d, 2H), 6.84-6.72 (m, 3H), 6.66 (d, 1H), 4.09-3.98 (m, 1H), 3.69 (t, 2H), 3.57-3.32 (m, 6H), 3.27-3.19 (m, 3H), 3.17-3.02 (m, 4H), 2.80-2.62 (m, 3H), 2.45-2.35 (m, 4H), 2.26-2.20 (m, 1H), 2.10-1.94 (m, 2H), 1.80-1.38 (m, 12H), 1.21 (d, 3H).

### Example 32 (S)-2-Chloro-4-(8-(6-(2-(4-(4-(2,4-dioxotetrahydropyrimidin-1(2H)-yl)phenyl)piperazin-1-yl)-7-azaspiro[3.5]nonane-7-carbonyl)pyridin-3-yl)-3-methyl-2,8-diazaspiro[4.5]decan-2-yl)benzonitrile 32

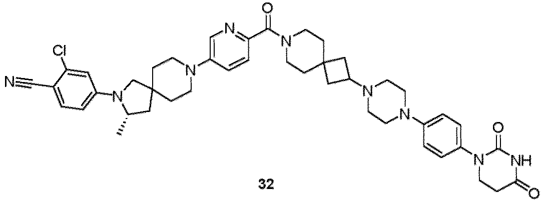

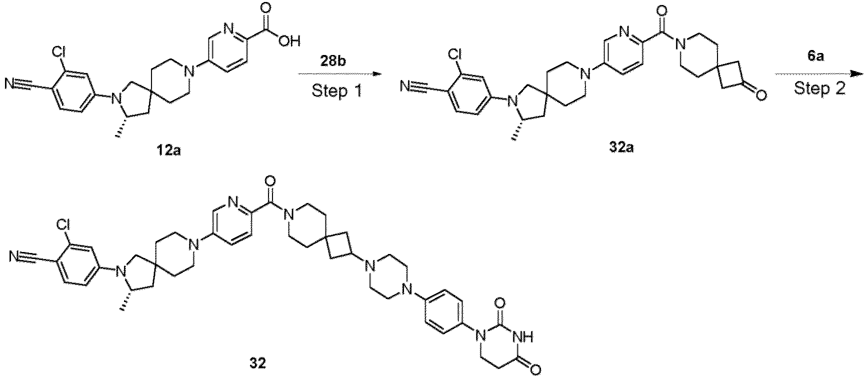

### Step 1 (S)-2-Chloro-4-(3-methyl-8-(6-(2-oxo-7-azaspiro[3.5]nonane-7-carbonyl)pyridin-3-yl)-2,8-diazaspiro[4.5]decan-2-yl)benzonitrile 32a

Compound **12a** (100 mg, 0.24 mmol) was dissolved in *N*,*N*-dimethylformamide (4 mL). Compound **28b** (92 mg, 0.36 mmol) was added, and *O*-(7-azabenzotriazol-1-yl)-*N,N*,*N*',*N*'-tetramethyluronium hexafluorophosphate (111 mg, 0.29 mmol) and *N,N-*diisopropylethylamine (135 mg, 0.97 mmol) were then added. After 1 hour of reaction, the reaction mixture was concentrated under reduced pressure, and the resulting residue was purified by silica gel column chromatography with eluent system A to give the title compound **32a** (100 mg, yield: 77%).

### Step 2 (S)-2-Chloro-4-(8-(6-(2-(4-(4-(2,4-dioxotetrahydropyrimidin-1(2H)-yl)phenyl)piperazin-1-yl)-7-azaspiro[3.5]nonane-7-carbonyl)pyridin-3-yl)-3-methyl-2,8-diazaspiro[4.5]decan-2-yl)benzonitrile 32

Compound 6a (35 mg, 0.11 mmol) was weighed into a 25 mL eggplant-shaped flask, and 4 mL of a mixed solvent of dichloromethane and methanol (V/V = 3/1) and anhydrous sodium acetate (46 mg, 0.56 mmol) were added. After 15 minutes of reaction, compound **32a** (50 mg, 0.094 mmol) was added to the system. After 1 hour of reaction, sodium cyanoborohydride (11 mg, 0.18 mmol) was added, and the mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 39%-59%, flow rate: 30 mL/min) to give the title compound **32** (25 mg, yield: 33%).

MS m/z (ESI): 790.3 [M+1].

¹H NMR (500 MHz, DMSO-*d₆*): δ 10.25 (s, 1H), 8.25 (s, 1H), 7.60 (d, 1H), 7.43 (d, 1H), 7.37 (dd, 1H), 7.14 (d, 2H), 6.92 (d, 2H), 6.80 (d, 1H), 6.66 (dd, 1H), 4.10-3.99 (m, 1H), 3.69 (t, 2H), 3.57-3.40 (m, 6H), 3.29-3.23 (m, 3H), 3.20-3.02 (s, 4H), 2.80-2.61 (m, 3H), 2.45-2.28 (m, 4H), 2.27-2.23 (m, 1H), 2.05-1.94 (m, 2H), 1.82-1.68 (m, 2H), 1.62-1.32 (m, 10H), 1.21 (d, 3H).

### Example 33 (S)-2-Chloro-4-(8-(5-(2-(4-(4-(2,4-dioxotetrahydropyrimidin-1(2H)-yl)phenyl)piperazin-1-yl)-7-azaspiro[3.5]nonane-7-carbonyl)pyridin-2-yl)-3-methyl-2,8-diazaspiro[4.5]decan-2-yl]benzonitrile 33

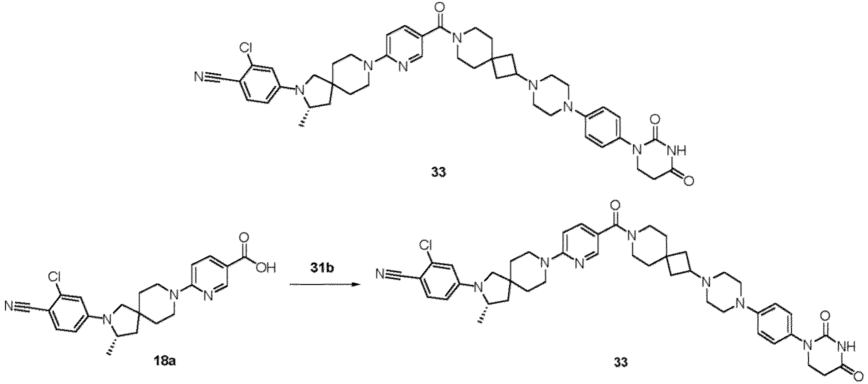

Compound **18a** (30 mg, 0.073 mmol) was dissolved in *N,N*-dimethylformamide (3 mL), and compound **31b** (45 mg, 0.072 mmol), *O*-(7-azabenzotriazol-1-yl)-*N,N,N*'*N*'-tetramethyluronium hexafluorophosphate (36 mg, 0.095 mmol), and *N,N-*diisopropylethylamine (38 mg, 0.294 mmol) were added. The mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 45%-65%, flow rate: 30 mL/min) to give the title compound **33** (15 mg, yield: 26%).

MS m/z (ESI): 790.3 [M+1].

¹H NMR (500 MHz, DMSO-*d₆*) δ: 10.25 (s, 1H), 8.14 (d, 1H), 7.60 (d, 1H), 7.53 (d, 1H), 7.14 (d, 2H), 6.92 (d, 2H), 6.83 (d, 1H), 6.79 (s, 1H), 6.65 (d, 1H), 4.04 (q, 1H), 3.76-3.72 (m, 1H), 3.69 (t, 2H), 3.57-3.42 (m, 6H), 3.39-3.33 (m, 2H), 3.11 (s, 4H), 2.72 (t, 1H), 2.67 (t, 2H), 2.38 (s, 4H), 2.25 (dd, 1H), 2.04-1.95 (m, 3H), 1.70-1.55 (m, 7H), 1.53-1.44 (m, 2H), 1.43-1.37 (m, 2H), 1.20 (d, 3H).

### Example 34 2-Chloro-4-((3S)-8-(4-(2-(4-(4-(2,6-dioxopiperidin-3-yl)phenyl)piperazin-1-yl)-7-azaspiro[3.5]nonane-7-carbonyl)phenyl)-3-methyl-2,8-diazaspiro[4.5]decan-2-yl)benzonitrile 34 (a mixture of diastereomers)

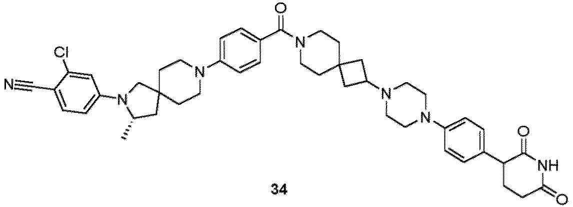

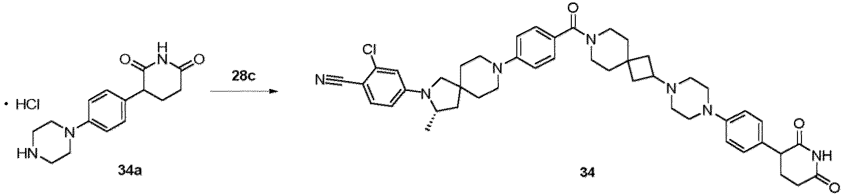

(±)-3-(4-(Piperazin-1-yl)phenyl)piperidine-2,6-dione **34a** (a racemate, a 1:1 ratio, 31 mg, 0.113 mmol, prepared using the method disclosed in "Example Ligase 70 on page 104 of the specification in the disclosed patent WO2021083949A1") was dissolved in a mixed solvent of dichloromethane and methanol (8 mL, V/V = 3/1), and anhydrous sodium acetate (47 mg, 0.573 mmol) was added. The mixture was left to react for 10 minutes. Compound **28c** (50 mg, 0.094 mmol) was added, and the mixture was left to react for 10 minutes. Sodium cyanoborohydride (11 mg, 0.184 mmol) was added, and the mixture was left to react for 12 hours. The reaction mixture was filtered and separated and purified by preparative high performance liquid chromatography (Waters-2545, elution system: 10 mmol/L aqueous ammonium bicarbonate solution and acetonitrile, gradient of acetonitrile: 50%-70%, flow rate: 30 mL/min) to give the title compound **34** (a mixture of diastereomers, a 1:1 ratio, 20 mg, yield: 27%).

MS m/z (ESI): 788.3 [M+1].

¹H NMR (500 MHz, DMSO-*d₆*): δ 10.35 (s, 1H), 7.58 (d, 1H), 7.23 (d, 2H), 7.04 (d, 2H), 6.93 (d, 2H), 6.87 (d, 2H), 6.78 (s, 1H), 6.65 (d, 1H), 4.02 (q, 1H), 3.71 (dd, 3H), 3.35-3.27 (m, 6H), 3.24-3.18 (m, 2H), 3.09 (s, 4H), 2.71 (t, 1H), 2.65-2.58 (m, 1H), 2.48-2.43 (m, 1H), 2.36 (s, 4H), 2.23 (dd, 1H), 2.14-2.09 (m, 1H), 2.03-1.95 (m, 3H), 1.78-1.67 (m, 2H), 1.64-1.52 (m, 5H), 1.50-1.42 (m, 4H), 1.19 (d, 3H).

### Example 35 (S)-2-Chloro-4-(8-(4-(2-(4-(3-(2,4-dioxotetrahydropyrimidin-1(2H)-yl)phenyl)piperazin-1-yl)-7-azaspiro[3.5]nonane-7-carbonyl)phenyl)-3-methyl-2,8-diazaspiro[4.5]decan-2-yl)benzonitrile 35

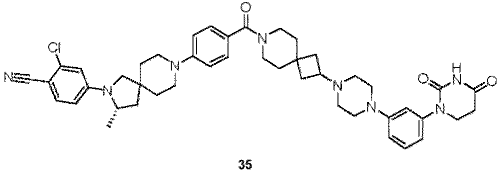

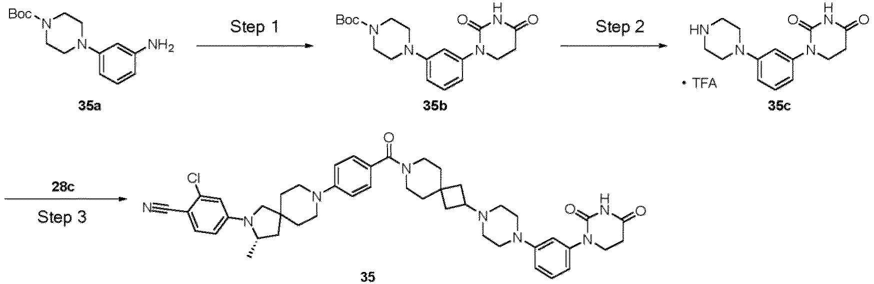

### Step 1 tert-Butyl 4-(3-(2,4-dioxotetrahydropyrimidin-1(2H)-yl)phenyl)piperazine-1-carboxylate 35b

tert-Butyl 4-(3-aminophenyl)piperazine-1-carboxylate **35a** (1 g, 3.60 mmol, prepared using the method disclosed in "Example SSTN-552 on page 312 of the specification in the disclosed patent WO2021237112A1") was dissolved in toluene (5 mL), and acrylic acid (286 mg, 3.97 mmol, Bide Pharmatech) was added. The mixture was heated to 100 °C and left to react for 3 hours. Toluene was removed under reduced pressure, and acetic acid (3 mL) and urea (867 mg, 14.4 mmol) were added. The mixture was heated to 100 °C and left to react for 12 hours. Acetic acid was removed under reduced pressure, and the resulting residue was purified by silica gel column chromatography with eluent system A to give the title compound **35b** (150 mg, yield: 11%).

MS m/z (ESI): 375.4 [M+1].

### Step 2 1-(3-(Piperazin-1-yl)phenyl)dihydropyrimidine-2,4(1H,3H)-dione trifluoroacetate 35c

Compound **35b** (150 mg, 0.40 mmol) was added to a 25 mL eggplant-shaped flask, and dichloromethane (10 mL) and trifluoroacetic acid (2 mL) were added. The mixture was left to react for 1 hour. The reaction mixture was concentrated under reduced pressure and dried *in vacuo* to give the title compound **35c** as a crude product (100 mg, yield: 91%). The crude product was directly used in the next step without purification.

MS m/z (ESI): 275.4 [M+1].

### Step 3 (S)-2-Chloro-4-(8-(4-(2-(4-(3-(2,4-dioxotetrahydropyrimidin-1(2H)-yl)phenyl)piperazin-1-yl)-7-azaspiro[3.5]nonane-7-carbonyl)phenyl)-3-methyl-2,8-diazaspiro[4.5]decan-2-yl)benzonitrile 35

Compound **28c** (50 mg, 0.094 mmol) was weighed into a 25 mL eggplant-shaped flask, and a mixed solvent of dichloromethane and methanol (4 mL, V/V = 3/1) and anhydrous sodium acetate (46 mg, 0.49 mmol) were added. After 15 minutes of reaction, compound **35c** (37 mg, 0.096 mmol) was added to the system. After 1 hour of reaction, sodium cyanoborohydride (15 mg, 0.25 mmol) was added, and the mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 50%-80%, flow rate: 30 mL/min) to give the title compound **35** (15 mg, yield: 20%).

MS m/z (ESI): 789.7 [M+1].

¹H NMR (500 MHz, DMSO-*d₆*) δ 10.29 (s, 1H), 7.59 (d, 1H), 7.23 (d, 2H), 7.20 (t, 1H), 6.94 (d, 2H), 6.87 (s, 1H), 6.82-6.77 (m, 2H), 6.71 (dd, 1H), 6.66 (dd, 1H), 4.03 (q, 1H), 3.74 (t, 2H), 3.43 (d, 4H), 3.31-3.26 (m, 2H), 3.25-3.15 (m, 3H), 3.12 (s, 4H), 2.75-2.70 (m,1H), 2.68 (t, 2H), 2.37 (s, 4H), 2.23 (dd, 1H), 2.04-1.95 (m, 3H), 1.78-1.66 (m, 2H), 1.64-1.53 (m, 5H), 1.51-1.42 (m, 4H), 1.20 (d, 3H).

### Example 36 2-Chloro-4-((3S)-8-(4-(2-(4-(3-((2,6-dioxopiperidin-3-yl)amino)phenyl)piperazin-1-yl)-7-azaspiro[3.5]nonane-7-carbonyl)-2-fluorophenyl)-3-methyl-2,8-diazaspiro[4.5]decan-2-yl)benzonitrile 36 (a mixture of diastereomers)

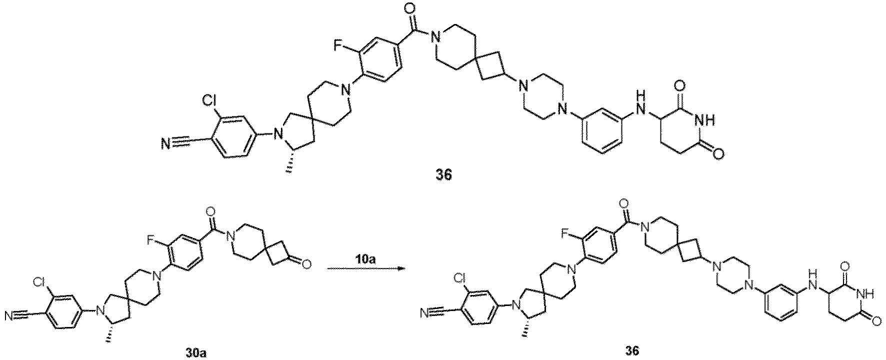

Compound **10a** (35 mg, 0.11 mmol) was dissolved in a mixed solvent of dichloromethane and methanol (8 mL, V/V = 3/1), and anhydrous sodium acetate (45 mg, 0.55 mmol) was added. After 10 minutes of reaction, compound **30a** (50 mg, 0.091 mmol) was added. After 30 minutes of reaction, sodium cyanoborohydride (11 mg, 0.18 mmol) was added, and the mixture was left to react for 12 hours. The reaction mixture was filtered and separated and purified by preparative high performance liquid chromatography (Waters-2545, elution system: 10 mmol/L aqueous ammonium bicarbonate solution and acetonitrile, gradient of acetonitrile: 53%-73%, flow rate: 30 mL/min) to give the title compound **36** (a mixture of diastereomers, a 1:1 ratio, 30 mg, yield: 40%).

MS m/z (ESI): 821.3 [M+1].

¹H NMR (500 MHz, DMSO-*d₆*) δ: 10.76 (s, 1H), 7.60 (d, 1H), 7.19-7.02 (m, 3H), 6.91 (t, 1H), 6.81 (s, 1H), 6.67 (d,1H), 6.25 (s, 1H), 6.16 (dd, 2H), 5.62 (d, 1H), 4.34 -4.27 (m, 1H), 4.04 (q, 1H), 3.45 (d, 4H), 3.17-3.08 (m, 2H), 3.07-2.97 (m, 6H), 2.78 -2.69 (m, 2H), 2.64-2.54 (m, 2H), 2.35 (s, 4H), 2.26 (dd, 1H), 2.13-2.05 (m, 1H), 2.04 -1.96 (m, 2H), 1.91-1.75 (m, 4H), 1.63-1.52 (m, 7H), 1.51-1.43 (m, 2H), 1.21 (d, 3H).

### Example 37 2-Chloro-4-((3S)-8-(4-(4-((1-(3-((2,6-dioxopiperidin-3-yl)amino)phenyl)piperidin-4-yl)methyl)piperazine-1-carbonyl)phenyl)-3-methyl-2,8-diazaspiro[4.5]decan-2-yl)benzonitrile 37 (a mixture of diastereomers)

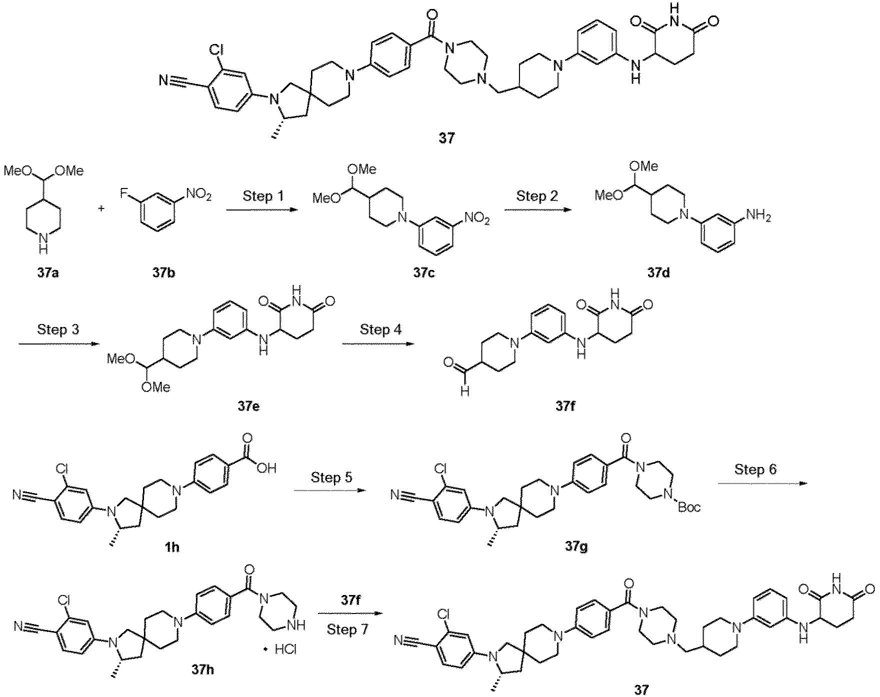

### Step 1 4-(Dimethoxymethyl)-1-(3-nitrophenyl)piperidine 37c

To a 50 mL single-necked flask were sequentially added 25 mL of dimethyl sulfoxide, m-fluoronitrobenzene **37b** (1 g, 7.09 mmol, Accela ChemBio (Shanghai) Co., Ltd.), and 4-(dimethoxymethyl)piperidine **37a** (1.7 g, 10.7 mmol, Accela ChemBio (Shanghai) Co., Ltd.). Potassium carbonate (1.46 g, 10.6 mmol) was slowly added, and the reaction flask was then placed in a microwave reactor. The mixture was heated to 120 °C and left to react for 4 hours. 50 mL of a saturated sodium bicarbonate solution was added to the reaction system under ice bath cooling, and extraction was performed with ethyl acetate (50 mL × 3). The organic phases were combined, washed with a saturated sodium chloride solution (50 mL × 3), dried over anhydrous sodium sulfate, and filtered. The filtrate was concentrated under reduced pressure, and the resulting residue was purified by silica gel column chromatography with eluent system B to give the title compound **37c** (374 mg, yield: 19%).

MS m/z (ESI): 281.1 [M+1].

### Step 2 3-(4-(Dimethoxymethyl)piperidin-1-yl)aniline 37d

Compound **37c** (370 mg, 1.32 mmol) was dissolved in methanol (5 mL), and 10% dry palladium on carbon (14 mg, Accela ChemBio (Shanghai) Co., Ltd.) was then added. The system was purged with hydrogen gas 3 times and left to react for 12 hours under a hydrogen atmosphere. The reaction mixture was filtered using celite. The resulting filtrate was concentrated under reduced pressure, and the resulting residue was purified by silica gel column chromatography with eluent system B to give the title compound **37d** (329 mg, yield: 99%).

MS m/z (ESI): 251.2 [M+1].

### Step 3 (±)-3-((3-(4-(Dimethoxymethyl)piperidin-1-yl)phenyl)amino)piperidine-2,6-dione 37e

Compound **37d** (330 mg, 1.32 mmol) was dissolved in acetonitrile (10 mL). Sodium bicarbonate (554 mg, 6.60 mmol) was added, and (±)-3-bromopiperidine-2,6-dione (303 mg, 1.58 mmol, Jiangsu Aikon Co., Ltd.) was added. The mixture was heated to 80 °C and left to react. After 2 hours of reaction, (±)-3-bromopiperidine-2,6-dione (303 mg, 1.58 mmol) was added again. After 2 hours of reaction, (±)-3-bromopiperidine-2,6-dione (303 mg, 1.58 mmol) was added again, and the mixture was left to react for 12 hours. The reaction mixture was concentrated under reduced pressure and filtered, and the filter cake was washed with ethyl acetate (15 mL). The filtrate was concentrated under reduced pressure, and the resulting residue was purified by silica gel column chromatography with eluent system A to give the title compound **37e** (a racemate, a 1:1 ratio, 40 mg, yield: 8%). MS m/z (ESI): 362.6 [M+1].

### Step 4 (±)-1-(3-((2,6-Dioxopiperidin-3-yl)amino)phenyl)piperidine-4-carbaldehyde 37f

Compound **37e** (40 mg, 0.11 mmol) was dissolved in tetrahydrofuran (5 mL), and a 2 M sulfuric acid solution (1 mL) was added. The mixture was heated to 60 °C and left to react for 4 hours in an oil bath. Solid sodium bicarbonate was added to the reaction mixture to adjust the pH of the reaction mixture to 8. Filtration was performed, and the filtrate was dried over anhydrous sodium sulfate and filtered. The filtrate was concentrated under reduced pressure and dried *in vacuo* to give the title compound **37f** as a crude product (a racemate, a 1:1 ratio, 30 mg, yield: 86%). The crude product was directly used in the next step without purification.

MS m/z (ESI): 316.2 [M+1].

### Step 5 tert-Butyl (S)-4-(4-(2-(3-chloro-4-cyanophenyl)-3-methyl-2,8-diazaspiro[4.5]decan-8-yl)benzoyl)piperazine-1-carboxylate 37g

Compound **1h** (100 mg, 0.24 mmol) was dissolved in *N,N*-dimethylformamide (2 mL). tert-Butyl piperazine-1-carboxylate (38 mg, 0.36 mmol) was added, and *O*-(7-azabenzotriazol-1-yl)-*N*,*N*,*N*,*N*-tetramethyluronium hexafluorophosphate (139 mg, 0.37 mmol) and *N*,*N*-diisopropylethylamine (95 mg, 0.74 mmol) were then added. After 1 hour of reaction, the reaction mixture was concentrated under reduced pressure, and the resulting residue was purified by silica gel column chromatography with eluent system A to give the title compound **37g** (139 mg, yield: 98%).

MS m/z (ESI): 578.2 [M+1].

### Step 6 (S)-2-Chloro-4-(3-methyl-8-(4-(piperazine-1-carbonyl)phenyl)-2,8-diazaspiro[4.5]decan-2-yl)benzonitrile hydrochloride 37h

Compound **37g** (139 mg, 0.24 mmol) was dissolved in a 4 M solution of hydrogen chloride in 1,4-dioxane (2 mL), and the mixture was left to react for 1 hour. The reaction mixture was concentrated and dried *in vacuo* to give the title compound **37h** as a crude product (123 mg, yield: 99%). The crude product was directly used in the next step without purification.

MS m/z (ESI): 478.2 [M+1].

### Step 7 2-Chloro-4-((3S)-8-(4-(4-((1-(3-((2,6-dioxopiperidin-3-yl)amino)phenyl)piperidin-4-yl)methyl)piperazine-1-carbonyl)phenyl)-3-methyl-2,8-diazaspiro[4.5]decan-2-yl)benzonitrile 37

Compound **37h** (60 mg, 0.12 mmol) was weighed into a 25 mL eggplant-shaped flask, and 4 mL of a mixed solvent of dichloromethane and methanol (V/V = 3/1) and anhydrous sodium acetate (109 mg, 1.30 mmol) were added. After 15 minutes of reaction, compound **37f** (41 mg, 0.13 mmol) was added to the system. After 1 hour of reaction, sodium triacetoxyborohydride (55 mg, 0.26 mmol) was added, and the mixture was left to react for 12 hours. The reaction mixture was filtered and separated and purified by preparative high performance liquid chromatography (Waters-2545, elution system: 10 mmol/L aqueous ammonium bicarbonate solution and acetonitrile, gradient of acetonitrile: 39%-59%, flow rate: 30 mL/min) to give the title compound **37** (a mixture of diastereomers, a 1:1 ratio, 5 mg, yield: 5%).

MS m/z (ESI): 777.3 [M+1].

¹H NMR (500 MHz, DMSO-*d₆*) δ 10.75 (s, 1H), 7.58 (d, 1H), 7.25 (d, 2H), 6.95 (d, 2H), 6.89 (t, 1H), 6.80 (d, 1H), 6.66 (dd, 1H), 6.25 (s, 1H), 6.18 (d, 1H), 6.10 (d, 1H), 5.59 (d, 1H), 4.32-4.26 (m, 1H), 4.04 (q, 1H), 3.59 (d, 2H), 3.49 (s, 3H), 3.43 (d, 2H), 3.39-3.35 (m, 3H), 3.37-3.16 (m, 3H), 2.78-2.69 (m, 1H), 2.65-2.54 (m, 3H), 2.35 (s, 4H), 2.23 (dd, 1H), 2.18 (d, 2H), 2.12-2.06 (m, 1H), 2.03-1.95 (m, 1H), 1.84 (qd, 1H), 1.79-1.62 (m, 5H), 1.57 (dd, 1H), 1.51-1.45 (m, 2H), 1.20 (d, 3H).

### Example 38 (±)-2-Chloro-4-(8-(4-(4-((4-(3-((2,6-dioxopiperidin-3-yl)amino)phenyl)piperazin-1-yl)methyl)piperidine-1-carbonyl)phenyl)-2,8-diazaspiro[4.5]decan-2-yl)-3-methylbenzonitrile 38 (a racemate)

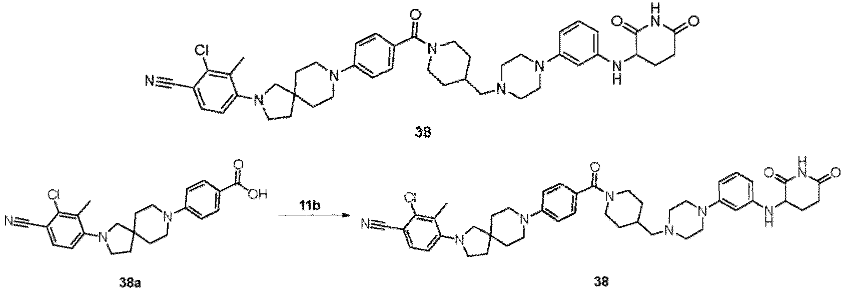

To a 25 mL three-necked flask were sequentially added 2 mL of *N,N*-dimethylformamide, compound **11b** (52 mg, 0.11 mmol), and 4-(2-(3-chloro-4-cyano-2-methylphenyl)-2,8-diazaspiro[4.5]decan-8-yl)benzoic acid **38a** (55 mg, 0.11 mmol, prepared using the method disclosed in "Example 11 on page 212 of the specification in the disclosed patent WO202155756 A1"). *N*,*N-*Diisopropylethylamine (118 mg, 0.91 mmol) and *O*-(7-azabenzotriazol-1-yl)-*N*,*N*,*N*,*N*-tetramethyluronium hexafluorophosphate (70 mg, 0.18 mmol) were slowly added under an ice bath, and the system was then warmed to room temperature and left to react for 16 hours. The reaction mixture was filtered and separated and purified by preparative high performance liquid chromatography (Waters 2545, elution system: 10 mmol/L aqueous ammonium bicarbonate solution and acetonitrile, gradient of acetonitrile: 55%-95%, flow rate: 30 mL/min) to give the title compound **38** (a racemate, a 1:1 ratio, 35 mg, yield: 39%).

MS m/z (ESI): 777.3 [M+1].

¹H NMR (500 MHz, DMSO-*d₆*): 10.75 (s, 1H), 7.56 (d, 1H), 7.24 (d, 2H), 6.95 (d, 2H), 6.90 (t, 1H), 6.85 (d, 1H), 6.25 (s, 1H), 6.18 (d, 1H), 6.13 (d, 1H), 5.61 (d, 1H), 4.34-4.26 (m, 1H), 4.24-3.69 (m, 3H), 3.46 (t, 2H), 3.30-3.23 (m, 5H), 3.05 (s, 4H), 2.95-2.81 (m, 2H), 2.78-2.69 (m, 1H), 2.61-2.54 (m, 1H), 2.46 (s, 4H), 2.37 (s, 3H), 2.19 (d, 2H), 2.13-2.05 (m, 1H), 1.88-1.78 (m, 4H), 1.77-1.70 (m, 2H), 1.69-1.61 (m, 4H), 1.11-1.01 (m, 2H).

### Example 39 2-Chloro-4-((3S)-8-(4-(4-((4-(2-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 39 (a mixture of diastereomers)

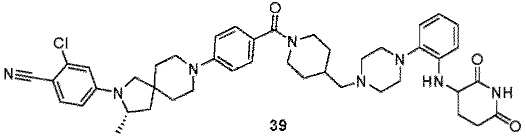

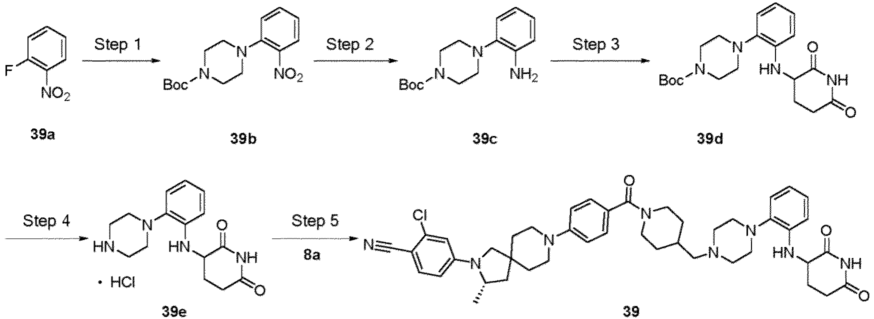

### Step 1 tert-Butyl 4-(2-nitrophenyl)piperazine-1-carboxylate 39b

2-Fluoronitrobenzene **39a** (1 g, 7.09 mmol, Bide Pharmatech) and tert-butyl piperazine-1-carboxylate (1.45 g, 7.79 mmol) were dissolved in *N,N-*dimethylformamide (10 mL), and cesium carbonate (4.62 g, 14.18 mmol) was added. The mixture was heated to 80 °C and left to react for 12 hours. The reaction mixture was concentrated under reduced pressure, and the resulting residue was purified by silica gel column chromatography with eluent system B to give the title compound **39b** (2.0 g, yield: 92%).

MS m/z (ESI): 308.2 [M+1].

### Step 2 tert-Butyl 4-(2-aminophenyl)piperazine-1-carboxylate 39c

Compound **39b** (2 g, 6.51 mmol) was dissolved in methanol (20 mL), and 10% dry palladium on carbon (692 mg) was added. The system was purged with hydrogen gas three times and left to react for 12 hours under a hydrogen atmosphere. The reaction mixture was filtered using celite. The filtrate was concentrated under reduced pressure, and the resulting residue was purified by silica gel column chromatography with eluent system A to give the title compound **39c** (1.8 g, yield: 100%).

MS m/z (ESI): 278.2 [M+1].

### Step 3 tert-Butyl (±)-4-(2-((2,6-dioxopiperidin-3-yl)amino)phenyl)piperazine-1-carboxylate 39d

Compound **39c** (500 mg, 1.80 mmol) was dissolved in acetonitrile (10 mL). Sodium bicarbonate (606 mg, 7.21 mmol) and (±)-3-bromopiperidine-2,6-dione (415 mg, 2.16 mmol) were added. The mixture was heated to 85 °C and left to react for 40 hours. The reaction mixture was filtered. The filtrate was concentrated under reduced pressure, and the resulting residue was purified by silica gel column chromatography with eluent system A to give the title compound **39d** (a racemate, a 1:1 ratio, 360 mg, yield: 51%).

MS m/z (ESI): 389.3 [M+1].

### Step 4 (±)-3-((2-(Piperazin-1-yl)phenyl)amino)piperidine-2,6-dione hydrochloride 39e Compound 39d (360 mg, 0.93 mmol) was dissolved in dichloromethane (3 mL), and a 4

M solution of hydrogen chloride in 1,4-dioxane (2 mL) was added. The mixture was left to react for 1 hour. The reaction mixture was concentrated under reduced pressure and dried *in vacuo* to give the title compound **39e** as a crude product (a racemate, a 1:1 ratio, 260 mg, yield: 86%). The crude product was directly used in the next step without purification.

MS m/z (ESI): 289.3 [M+1].

### Step 5 2-Chloro-4-((3S)-8-(4-(4-((4-(2-((2,6-dioxopiperidin-3-yl)amino)phenyl)piperazin-1-yl)methyl)piperidine-1-carbonyl)phenyl)-3-methyl-2,8-diazaspiro[4.5]decan-2-yl)benzonitrile 39

Compound **39e** (60 mg, 0.18 mmol) and anhydrous sodium acetate (71 mg, 0.87 mmol) were added to a mixed solvent of dichloromethane and methanol (8 mL, V/V = 3/1). After 15 minutes of reaction, compound **8a** (55 mg, 0.11 mmol) was added, and the mixture was left to react for 15 minutes. Sodium triacetoxyborohydride (46 mg, 0.22 mmol) was added, and the mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 55%-85%, flow rate: 30 mL/min) to give the title compound **39** (a mixture of diastereomers, a 1:1 ratio, 15 mg, yield: 18%).

MS m/z (ESI): 777.3 [M+1].

¹H NMR (500 MHz, DMSO-*d*₆) δ: 10.92 (s, 1H), 7.59 (d, 1H), 7.24 (d, 2H), 6.98 (d, 1H), 6.96-6.89 (m, 3H), 6.80 (d, 1H), 6.70-6.61 (m, 3H), 5.53 (d, 1H), 4.26-4.19 (m, 1H), 4.04 (q, 1H), 3.44 (d, 1H), 3.39-3.27 (m, 4H), 3.26-3.14 (m, 3H), 2.95-2.78 (m, 5H), 2.73 (s, 2H), 2.56 (d, 2H), 2.31-2.25 (m, 2H), 2.22 (m, 2H), 2.03-1.95 (m, 1H), 1.93-1.79 (m, 3H), 1.78-1.68 (m, 4H), 1.58 (dd, 1H), 1.53-1.41 (m, 3H), 1.20 (d, 3H), 1.12-1.00 (m, 2H).

### Example 40 2-Chloro-4-((3S)-8-(4-(4-((4-(3-((2,6-dioxopiperidin-3-yl)amino)phenyl)-3-oxopiperazin-1-yl)methyl)piperidine-1-carbonyl)phenyl)-3-methyl-2, 8-diazaspiro[4.5]decan-2-yl)benzonitrile 40 (a mixture of diastereomers)

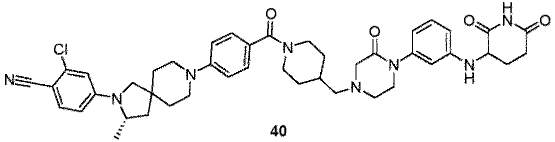

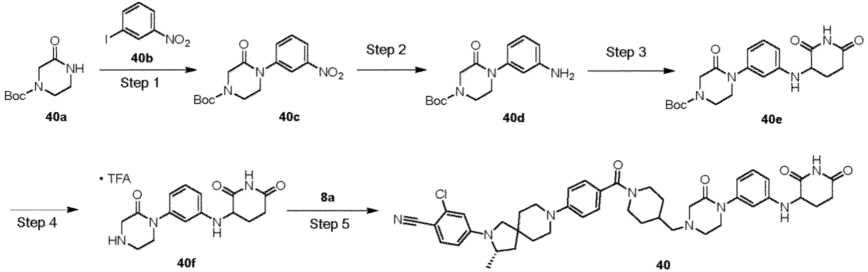

### Step 1 tert-Butyl 4-(3-nitrophenyl)-3-oxopiperazine-1-carboxylate 40c

tert-Butyl 3-oxo-1-piperazinecarboxylate **40a** (1.0 g, 4.99 mmol, Bide Pharmatech) was dissolved in 1,4-dioxane (10 mL), and 1-iodo-3-nitrobenzene **40b** (1.19 g, 4.79 mmol, Bide Pharmatech), cuprous iodide (143 mg, 0.75 mmol), anhydrous potassium carbonate (1.38 g, 9.99 mmol), and *N*,*N-*dimethyl-1,2-cyclohexanediamine (142 mg, 1.00 mmol) were added. The mixture was left to react for 15 minutes under a nitrogen atmosphere, heated to 110 °C, and left to react for 16 hours. The reaction mixture was concentrated under reduced pressure, and the resulting residue was purified by silica gel column chromatography with eluent system B to give the title compound **40c** (750 mg, yield: 47%).

MS m/z (ESI): 266.1 [M-55].

### Step 2 tert-Butyl 4-(3-aminophenyl)-3-oxopiperazine-1 -carboxylate 40d

To a 100 mL single-necked flask were added a mixed solvent of tetrahydrofuran and methanol (20 mL, V/V = 1/1), compound **40c** (750 mg, 2.33 mmol), and 10% wet palladium on carbon (containing 50% water, 210 mg, Accela ChemBio (Shanghai) Co., Ltd.). The system was purged with hydrogen gas 3 times and left to react for 3 hours under a hydrogen atmosphere. The reaction mixture was filtered using celite, and the filtrate was concentrated under reduced pressure to give the title compound **40d** as a crude product (680 mg, yield: 100%). The crude product was directly used in the next step without purification.

MS m/z (ESI): 292.1 [M+1].

### Step 3 tert-Butyl (±)-4-(3-((2,6-dioxopiperidin-3-yl)amino)phenyl)-3-oxopiperazine-1-carboxylate 40e

Compound **40d** (200 mg, 0.69 mmol) was dissolved in *N*,*N-*dimethylformamide (3 mL), and (±)-3-bromopiperidine-2,6-dione (264 mg, 1.37 mmol) was then added. Diisopropylethylamine (266 mg, 2.06 mmol) was added. The mixture was heated to 85 °C and left to react for 4 hours, and (±)-3-bromopiperidine-2,6-dione (264 mg, 1.37 mmol) was added again. After another 4 hours of reaction, (±)-3-bromopiperidine-2,6-dione (264 mg, 1.37 mmol) was added again, and the mixture was left to react for 4 hours. The reaction mixture was concentrated under reduced pressure, and the resulting residue was purified by silica gel column chromatography with eluent system A to give the title compound **40e** (a racemate, a 1:1 ratio, 50 mg, yield: 18%).

MS m/z (ESI): 403.1 [M+1].

### Step 4 (±)-3-((3-(2-Oxopiperazin-1-yl)phenyl)amino)piperidine-2,6-dione trifluoroacetate 40f

To a 50 mL single-necked flask were added a mixed solvent of trifluoroacetic acid and dichloromethane (2.5 mL, V/V = 1/4) and compound **40e** (50 mg, 0.12 mmol). The mixture was left to react for 1 hour. The reaction mixture was concentrated under reduced pressure and dried *in vacuo* to give the title compound **40f** as a crude product (a racemate, a 1:1 ratio, 51 mg, yield: 99%). The crude product was directly used in the next step without purification.

MS m/z (ESI): 303.1 [M+1].

### Step 5 2-Chloro-4-((3S)-8-(4-(4-((4-(3-((2,6-dioxopiperidin-3-yl)amino)phenyl)-3-oxopiperazin-1-yl)methyl)piperidine-1-carbonyl)phenyl)-3-methyl-2, 8-diazaspiro[4.5]decan-2-yl)benzonitrile 40

To a 25 mL single-necked flask were added a mixed solvent of dichloromethane and methanol (3 mL, V/V = 2/1), compound **40f** (52 mg, 0.12 mmol), and anhydrous sodium acetate (34 mg, 0.42 mmol). After 15 minutes of reaction, compound **8a** (35 mg, 0.069 mmol) was added. After 30 minutes of reaction, sodium triacetoxyborohydride (29 mg, 0.14 mmol) was slowly added, and the mixture was left to react for 16 hours. 10 mL of a saturated sodium bicarbonate solution (10 mL) was added to the reaction system under ice bath cooling, and extraction was performed with ethyl acetate (15 mL × 3). The organic phases were combined, washed with a saturated sodium chloride solution (15 mL), dried over anhydrous sodium sulfate, and filtered. The filtrate was concentrated under reduced pressure and separated and purified by preparative high performance liquid chromatography (Waters 2545, elution system: 10 mmol/L aqueous ammonium bicarbonate solution and acetonitrile, gradient of acetonitrile: 50%-95%, flow rate: 30 mL/min) to give the title compound **40** (a mixture of diastereomers, a 1:1 ratio, 6 mg, yield: 11%).

MS m/z (ESI): 791.3 [M+1].

¹H NMR (500 MHz, DMSO-*d₆*): δ 10.78 (s, 1H), 7.59 (d, 1H), 7.24 (d, 2H), 7.10-7.05 (m, 1H), 6.94 (d, 2H), 6.80 (d, 1H), 6.66 (dd, 1H), 6.60 (t, 1H), 6.57 (dd, 1H), 6.48 (dd, 1H), 5.96 (s, 1H), 4.37-4.30 (m, 1H), 4.07-4.00 (m, 2H), 3.64-3.51 (m, 2H), 3.44 (d, 1H), 3.39-3.35 (m, 1H), 3.25-3.15 (m, 3H), 3.12 (s, 2H), 2.95-2.84 (m, 2H), 2.79-2.68 (m, 3H), 2.61-2.53 (m, 1H), 2.32-2.19 (m, 3H), 2.12-2.05 (m, 1H), 2.00-1.95 (m, 1H), 1.93-1.81 (m, 2H), 1.79-1.67 (m, 4H), 1.58 (dd, 1H), 1.54-1.43 (m, 3H), 1.20 (d, 3H), 1.13-1.03 (m, 2H).

### Example 41 4-((3S)-8-(4-(4-((4-(3-((2,6-Dioxopiperidin-3-yl)amino)phenyl)piperazin-1-yl)methyl)piperidine-1-carbonyl)phenyl)-3-methyl-2,8-diazaspiro[4.5]decan-2-yl)-2-(trifluoromethyl)benzonitrile 41 (a mixture of diastereomers)

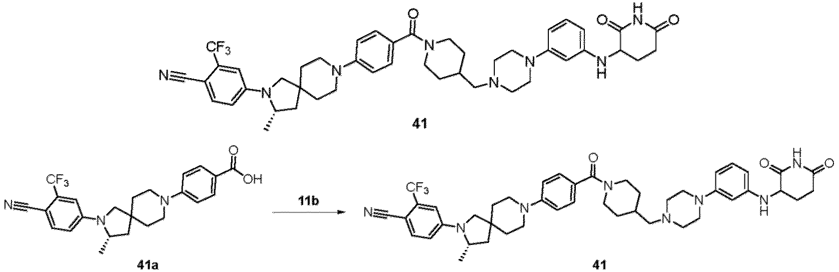

(*S*)-4-(2-(4-Cyano-3-(trifluoromethyl)phenyl)-3-methyl-2,8-diazaspiro[4.5]decan-8-yl)benzoic acid **41a** (30 mg, 0.068 mmol, prepared using the method disclosed in "Example 41 on page 253 of the specification in the disclosed patent WO2021055756A1") was dissolved in *N*,*N-*dimethylformamide (3 mL). Compound **11b** (38 mg, 0.083 mmol) was added, and *O*-(7-azabenzotriazol-1-yl)-*N*,*N*,*N'*,*N'-*tetramethyluronium hexafluorophosphate (39 mg, 0.10 mmol) and *N,N-*diisopropylethylamine (53 mg, 0.41 mmol) were then added. The mixture was left to react for 1 hour. The reaction mixture was filtered and separated and purified by preparative high performance liquid chromatography (Waters-2545, elution system: 10 mmol/L aqueous ammonium bicarbonate solution and acetonitrile, gradient of acetonitrile: 60%-80%, flow rate: 30 mL/min) to give the title compound **41** (a mixture of diastereomers, a 1:1 ratio, 10 mg, yield: 18%).

MS m/z (ESI): 811.3 [M+1].

¹H NMR (500 MHz, DMSO-*d₆*): δ 10.76 (s, 1H), 7.77 (d, 1H), 7.24 (d, 2H), 6.99-6.86 (m, 5H), 6.24 (s, 1H), 6.18 (d, 1H), 6.14 (d, 1H), 5.62 (d, 1H), 4.34-4.27 (m, 1H), 4.11 (q, 1H), 3.50 (d, 1H), 3.38 (d, 2H), 3.36-3.33 (m, 1H), 3.30-3.17 (m, 3H), 3.06 (s, 4H), 2.97-2.80 (m, 3H), 2.79-2.67 (m, 2H), 2.65-2.53 (m, 3H), 2.30-2.18 (m, 3H), 2.14-2.07 (m, 1H), 2.04-1.95 (m, 1H), 1.89-1.80 (m, 2H), 1.79-1.69 (m, 4H), 1.60 (dd, 1H), 1.57-1.44 (m, 2H), 1.22 (d, 3H), 1.12-1.02 (m, 2H).

### Example 42 2-(4-(4-((S)-2-(3-Chloro-4-cyanophenyl)-3-methyl-2,8-diazaspiro[4.5]decan-8-yl)benzoyl)piperazin-1-yl)-N-(3-((2,6-dioxopiperidin-3-yl)amino)phenyl)acetamide 42 a mixture of diastereomers

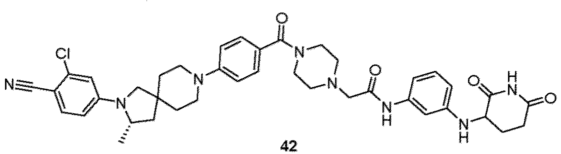

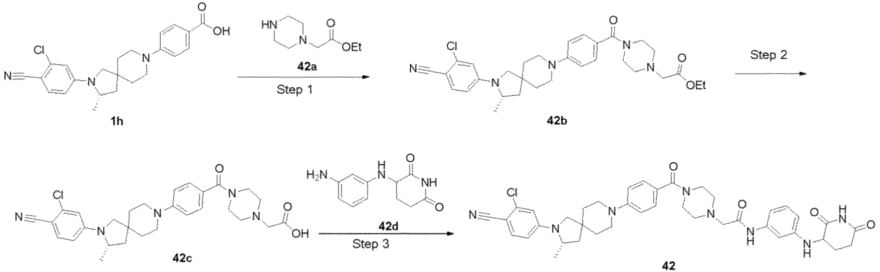

### Step 1 Ethyl (S)-2-(4-(4-(2-(3-chloro-4-cyanophenyl)-3-methyl-2,8-diazaspiro[4.5]decan-8-yl)benzoyl)piperazin-1-yl)acetate 42b

Compound **1h** (50 mg, 0.12 mmol) and ethyl 2-(piperazin-1-yl)acetate **42a** (30 mg, 0.14 mmol, Bide Pharmatech) were dissolved in *N*,*N*-dimethylformamide (4 mL), and *O*-(7-azabenzotriazol-1-yl)-*N*,*N*,*N'*,*N'-*tetramethyluronium hexafluorophosphate (70 mg, 0.18 mmol) and *N*,*N*-diisopropylethylamine (65 mg, 0.50 mmol) were added. The mixture was left to react for 1 hour. The reaction mixture was concentrated under reduced pressure, and the resulting residue was purified by silica gel column chromatography with eluent system A to give the title compound **42b** (68 mg, yield: 99%).

MS m/z (ESI): 564.2 [M+1].

### Step 2 (S)-2-(4-(4-(2-(3-Chloro-4-cyanophenyl)-3-methyl-2,8-diazaspiro[4.5]decan-8-yl)benzoyl)piperazin-1-yl)acetic acid 42c

Compound **42b** (68 mg, 0.12 mmol) was dissolved in a mixed solvent of tetrahydrofuran and water (6 mL, V/V = 1/1), and the mixture was left to react for 1 hour. The pH of the reaction mixture was adjusted to 7 with 1 M dilute hydrochloric acid, and extraction was performed with ethyl acetate (5 mL × 3). The organic phases were combined, washed with a saturated sodium chloride solution, dried over anhydrous sodium sulfate, and filtered. The filtrate was concentrated and dried *in vacuo* to give the title compound **42c** as a crude product (65 mg, yield: 99%). The crude product was directly used in the next step without purification.

MS m/z (ESI): 536.2 [M+1].

### Step 3 2-(4-(4-((S)-2-(3-Chloro-4-cyanophenyl)-3-methyl-2,8-diazaspiro[4.5]decan-8-yl)benzoyl)piperazin-1-yl)-N-(3-((2,6-dioxopiperidin-3-yl)amino)phenyl)acetamide 42

Compound **42c** (65 mg, 0.12 mmol) was weighed into a 25 mL eggplant-shaped flask, and *N*,*N*-dimethylformamide (2 mL), *N*,*N-*diisopropylethylamine (63 mg, 0.49 mmol), and *O*-(7-azabenzotriazol-1-yl)-*N*,*N*,*N'*,*N*'-tetramethyluronium hexafluorophosphate (70 mg, 0.18 mmol) were added. After 15 minutes of reaction, (±)-3-((3-aminophenyl)amino)piperidine-2,6-dione **42d** (a racemate, a 1:1 ratio, 35 mg, 0.16 mmol, prepared using the method disclosed in "Example 12 on page 108 of the specification in the disclosed patent WO2020132016A1") was added, and the mixture was left to react for 30 minutes. The reaction mixture was filtered and separated and purified by preparative high performance liquid chromatography (Waters 2545, elution system: 10 mmol/L aqueous ammonium bicarbonate solution and acetonitrile, gradient of acetonitrile: 64%-82%, flow rate: 30 mL/min) to give the title compound **42** (a mixture of diastereomers, a 1:1 ratio, 15 mg, yield: 17%).

MS m/z (ESI): 737.2 [M+1].

¹H NMR (500 MHz, DMSO-*d*₆) δ 10.77 (s, 1H), 9.48 (s, 1H), 7.59 (d, 1H), 7.27 (d, 2H), 7.02-6.92 (m, 4H), 6.84-6.78 (m, 2H), 6.66 (m, 1H), 6.39 (d, 1H), 5.87 (d, 1H), 4.28-4.22 (m, 1H), 4.07-4.02 (m, 1H), 3.56 (m, 4H), 3.43 (d, 2H), 3.25-3.19 (m, 2H), 3.14 (s, 2H), 2.76-2.68 (m, 4H), 2.65-2.58 (m, 4H), 2.26-2.19 (m, 1H), 2.13-2.06 (m, 1H), 1.94-1.86 (m, 1H), 1.76-1.68 (m, 3H), 1.58 (dd, 1H), 1.52-1.47 (m, 1H), 1.20 (d, 3H).

### Example 43 4-(4-((1-(4-((S)-2-(3-Chloro-4-cyanophenyl)-3-methyl-2,8-diazaspiro[4.5]decan-8-yl)benzoyl)piperidin-4-yl)methyl)piperazin-1-yl) N (2,6-dioxopiperidin-3-yl)benzamide 43 a mixture of diastereomers)

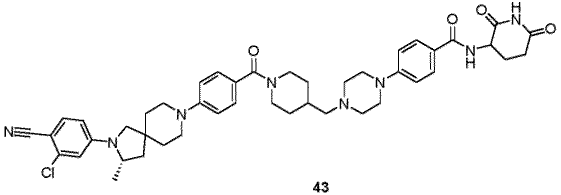

The synthesis scheme of Example 3 was used, and the starting material of step 1, compound **1d,** was replaced with (±)-*N*-(2,6-dioxopiperidin-3-yl)-4-(piperazin-1-yl)benzamide hydrochloride **43a** (prepared using the method disclosed in "Example 9 on page 169 of the specification in the disclosed patent WO2021127443A1"). After separation and purification by preparative high performance liquid chromatography (Waters 2545, elution system: 10 mmol/L aqueous ammonium bicarbonate solution and acetonitrile, gradient of acetonitrile: 55%-95%, flow rate: 30 mL/min), the title compound 43 (a mixture of diastereomers, a 1:1 ratio, 36 mg) was obtained.

MS m/z (ESI): 805.7 [M+1].

¹H NMR (500 MHz, DMSO-*d₆*): δ 10.82 (s, 1H), 8.46 (d, 1H), 7.74 (d, 2H), 7.59 (d, 1H), 7.24 (d, 2H), 7.04-6.87 (m, 4H), 6.80 (d, 1H), 6.65 (dd, 1H), 4.81-4.64 (m, 1H), 4.40-3.72 (m, 3H), 3.43 (d, 1H), 3.38-3.05 (m, 9H), 3.03-2.66 (m, 4H), 2.60-2.41 (m, 4H), 2.30-2.16 (m, 3H), 2.15-2.04 (m, 1H), 2.02-1.66 (m, 6H), 1.57 (dd, 1H), 1.53-1.42 (m, 2H), 1.20 (d, 3H), 1.14-0.98 (m, 2H).

### Biological Evaluations

The present disclosure is further described and explained below with reference to test examples. However, these test examples are not intended to limit the scope of the present disclosure.

### Test Example 1. Degradation Activity of Compounds Disclosed Herein on Androgen Receptor in LNCaP Cells

Through the *in vitro* cell assay described below, the degradation activity of the test compounds on the androgen receptor (AR) in the human prostate cancer cell strain can be determined, and their activity can be represented by DC₅₀ values. On the first day of the experiment, LNCaP cells (ATCC, CRL-1740) were seeded in a 96-well plate at a density of 20,000 cells/well using phenol red-free RPMI 1640 medium (Gibco, 11835-030) containing 5% charcoal-stripped fetal bovine serum (Shanghai Biosun Biotechnology Co., Ltd., S-FBS-AU-045), with 200 µL of cell suspension in each well. The plate was incubated in a 37 °C, 5% CO₂ cell incubator for three days. On the fourth day, serially diluted test compounds prepared with a plating medium containing 1 nM R1881 (Aladdin, M305037) were added at 22 µL/well. The final concentration of R1881 was 0.1 nM, and the final concentrations of the compounds were 7 concentration points obtained by 6-fold serial dilution starting from 2.5 µM. A blank control cell well containing 0.5% DMSO was set up. The plate was incubated in a 37 °C, 5% CO₂ cell incubator for 24 hours. On the fifth day, the 96-well cell culture plate was taken out, and the cell culture supernatant was removed from the plate using a pipette and discarded. The plate was washed with 300 µL of ice-cold PBS once, and 50 µL of 1× lysis buffer diluted with ddH₂O and containing 1 mM PMSF (Cell Signaling Technology, #9803 S) was then added. After 1 minute of shaking on a micro-shaker, the cell plate was placed on ice for 15-20 minutes of lysis. The lysate was well mixed by pipetting up and down and then centrifuged at 4000 rpm at 4 °C for 10 minutes. AR protein levels were measured using an AR ELISA kit (Cell Signaling Technology, #12850) according to the instructions. 96 µL of sample dilution and 4 µL of sample lysate or blank lysis buffer were added to a 96-well microplate. The plate was sealed, shaken to mix well the contents, and then incubated in a 37 °C incubator for 2 hours. The liquid in the wells was discarded, and 300 µL of wash buffer was added to each well to wash the plate. After five washes, 100 µL of detection antibody working solution was added. The plate was sealed and incubated in a 37 °C incubator for 1 hour. The liquid in the wells was discarded, and 300 µL of wash buffer was added to each well to wash the plate. After five washes, 100 µL of enzyme conjugate working solution was added. The plate was sealed and then incubated in a 37 °C incubator for 30 minutes. The liquid in the wells was discarded, and 300 µL of wash buffer was added to each well to wash the plate. After five washes, 100 µL of substrate solution was added. The plate was sealed and then incubated at room temperature in the dark for 5 minutes, and 100 µL of stop solution was added to each well. Immediately after the contents were well mixed, the plate was placed on a microplate reader (BMG Labtech, PHERAstar FS), and the OD450 and OD540 values were recorded. The OD540 absorbance values of the corresponding wells were subtracted from the OD450 absorbance values of all the wells to obtain corrected OD450 absorbance values (OD450-correction). The degradation rate of each concentration of the compounds was calculated using the following formula. The maximums (Max) represent the maximum AR degradation rates of the compounds. A curve was fit to the logarithmic concentration and degradation efficiency of each of the compounds using GraphPad Prism, and the DC₅₀ value was calculated. Degradation rate (%) = (OD450-correctionblank control-OD450-correctioncompound)/(OD450-correctionblanck control-OD450-correctionlysis buffer control) × 100%

The biological activity of the compounds of the present disclosure was obtained from the above analysis. The calculated DC₅₀ and Max values are shown in Table 1 below.

**Table 1. The degradation activity of the compounds of the present disclosure on the androgen receptor in LNCaP cells**

| Example No. | LNCaP DC₅₀ (nM) | Max (%) |
|---|---|---|
| 1 | 10.6 | 89 |
| 2 | 5.1 | 90 |
| 3 | 22.1 | 89 |
| 4 | 20.2 | 92 |
| 5 | 30.5 | 86 |
| 6 | 5.7 | 86 |
| 7 | 1.0 | 93 |
| 8 | 1.6 | 85 |
| 9 | 12.9 | 92 |
| 10 | 2.5 | 97 |
| 11 | 7.3 | 98 |
| 11-1 or 11-2 (shorter retention time) | 6.6 | 96 |
| 11-2 or 11-1 (longer retention time) | 1.6 | 97 |
| 12 | 1.3 | 97 |
| 12-1 or 12-2 (shorter retention time) | 3.5 | 95 |
| 12-2 or 12-1 (longer retention time) | 1.3 | 97 |
| 13 | 0.5 | 96 |
| 14 | 1.9 | 96 |
| 15 | 2.5 | 96 |
| 15-2 | 14.2 | 97 |
| 15-1 | 4.2 | 97 |
| 16 | 3.9 | 97 |
| 17 | 3.0 | 97 |
| 18 | 5.8 | 96 |
| 19 | 1.8 | 96 |
| 20 | 0.4 | 92 |
| 21 | 0.5 | 94 |
| 22 | 0.4 | 97 |
| 23 | 6.1 | 96 |
| 24 | 9.1 | 94 |
| 25 | 1.8 | 96 |
| 26 | 2.5 | 94 |
| 27 | 7.7 | 96 |
| 28 | 5.8 | 97 |
| 29 | 12.0 | 93 |
| 30 | 4.2 | 96 |
| 31 | 8.7 | 94 |
| 32 | 1.0 | 95 |
| 33 | 2.5 | 95 |
| 34 | 5.0 | 96 |
| 35 | 5.2 | 88 |
| 36 | 5.3 | 97 |
| 37 | 7.6 | 97 |
| 38 | 11.7 | 96 |
| 39 | 13.9 | 86 |
| 40 | 7.9 | 97 |
| 41 | 17.1 | 96 |
| 42 | 4.2 | 90 |
| 43 | 9.3 | 92 |

Conclusion: The compounds of the present disclosure have significant degradation activity on the androgen receptor in LNCaP cells.

### Test Example 2. Inhibition of LNCaP Proliferation by Compounds Disclosed Herein

Through the *in vitro* cell assay described below, the proliferation inhibition activity of the test compounds on the human prostate cancer cell strain can be determined, and their activity can be represented by IC₅₀ values. On the first day of the experiment, LNCaP cells (ATCC, CRL-1740) were seeded in a 96-well plate at a density of 7500 cells/well using phenol red-free RPMI 1640 medium (Gibco, 11835-030) containing 5% charcoal-stripped fetal bovine serum (Shanghai Biosun Biotechnology Co., Ltd., S-FBS-AU-045), with 200 µL of cell suspension in each well. The plate was incubated in a 37 °C, 5% CO₂ cell incubator for three days. On the fourth day, serially diluted test compounds prepared with a plating medium containing 1 nM R1881 (Aladdin, M305037) were added at 22 µL/well. The final concentration of R1881 was 0.1 nM, and the final concentrations of the compounds were 9 concentration points obtained by 4-fold serial dilution starting from 10 µM. A blank control cell well containing 0.5% DMSO and a cell-free vehicle control well were set up. The plate was incubated in a 37 °C, 5% CO₂ cell incubator for six days. On the tenth day, the 96-well cell culture plate was taken out, and 110 µL of cell culture supernatant was removed from each well using a pipette and discarded. Then 50 µL of CellTiter-Glo^{®} Luminescent Cell Viability Assay (Promega, G7573) was added. After 10 minutes of standing at room temperature, luminescence signal values (RLU) were obtained using a multifunctional microplate reader (PerkinElmer, VICTOR 3). The inhibition rate of each concentration of the compounds was calculated using the following formula. The maximums (Max) represent the maximum proliferation inhibition rates of the compounds. A curve was fit to the logarithmic concentration and inhibition rate of each of the compounds using GraphPad Prism, and the IC₅₀ value was calculated. Degradation rate (%) = (RLUblank control-RLUcompound)/(RLUblanck control-RLUvehicle control) × 100%

The biological activity of the compounds of the present disclosure was obtained from the above analysis. The calculated IC₅₀ values are shown in Table 2 below.

**Table 2. The proliferation inhibition activity of the compounds of the present disclosure on LNCaP cells**

| Compound No. | LNCaP IC₅₀ (nM) | Max (%) |
|---|---|---|
| 1 | 183 | 63 |
| 2 | 164 | 62 |
| 3 | 286 | 60 |
| 4 | 135 | 58 |
| 5 | 189 | 72 |
| 6 | 24.6 | 50 |
| 7 | 4.6 | 53 |
| 8 | 9.8 | 31 |
| 9 | 69 | 59 |
| 10 | 93 | 70 |
| 11 | 82 | 68 |
| 11-1 or 11-2 (shorter retention time) | 119 | 72 |
| 11-2 or 11-1 (longer retention time) | 56 | 72 |
| 12 | 48 | 73 |
| 13 | 106 | 56 |
| 14 | 57 | 69 |
| 15 | 65 | 74 |
| 15-2 | 74 | 68 |
| 15-1 | 15 | 64 |
| 16 | 84 | 70 |
| 17 | 112 | 74 |
| 18 | 123 | 72 |
| 19 | 60 | 68 |
| 20 | 15 | 59 |
| 21 | 16 | 60 |
| 23 | 41 | 76 |
| 24 | 659 | 54 |
| 25 | 24 | 60 |
| 26 | 7.8 | 63 |
| 27 | 7.9 | 64 |
| 28 | 17 | 61 |
| 29 | 81 | 45 |
| 30 | 7.2 | 52 |
| 31 | 12 | 57 |
| 32 | 3.1 | 63 |
| 33 | 3.5 | 58 |
| 34 | 12 | 60 |
| 35 | 31 | 57 |
| 36 | 35 | 57 |
| 37 | 125 | 73 |
| 38 | 49 | 60 |
| 41 | 98 | 56 |
| 42 | 39 | 50 |

Conclusion: The compounds of the present disclosure have good proliferation inhibition activity on LNCaP cells.

### Test Example 3. Pharmacokinetic Evaluation

Comparative Example 1 (synthesized by reference to Example 307 of the patent WO2021055756A1) has the following structure:

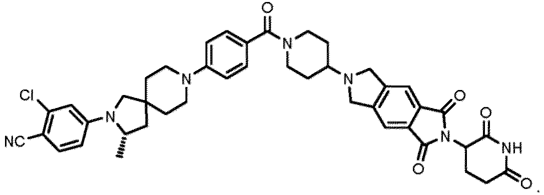

ARV-110 (synthesized by reference to Example 406 of the patent WO2018071606A1) has the following structure:

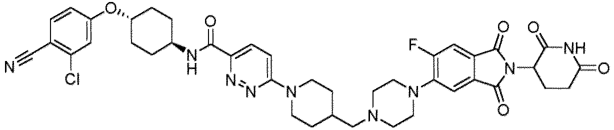

### I. SD rat testing

### 1. Abstract

SD rats were used as test animals. The plasma concentrations of compounds of the present disclosure in SD rats at different time points after intragastric (i.g.) administration were determined using an LC/MS/MS method. The pharmacokinetic behavior of the compounds of the present disclosure in SD rats was studied, and their pharmacokinetic profiles were evaluated.

### 2. Protocol

### 2.1. Test compounds

Example 11 compound;
Example 11-2 or 11-1 (longer retention time);
Example 12 compound;
Example 15 compound;
Example 15-1 compound;
Example 23 compound;
Example 28 compound; and
Comparative Example 1 compound.

### 2.2. Test animals

32 SD rats, provided by Vital River Laboratory Animal Co., Ltd., with an equal number of males and females, were evenly divided into 8 groups. The animals were fasted overnight and then intragastrically dosed.

### 2.3. Compound solution preparation

A certain amount of test compound was weighed out and dissolved in 5% DMSO + 5% tween 80 + 90% normal saline to prepare a 0.2 mg/mL clear solution.

### 2.4. Administration

The dose administered was 2.0 mg/kg, and the volume was 10.0 mL/kg.

### 3. Procedure

0.2-mL blood samples were collected from the orbit before administration and 0.25, 0.5, 1.0, 2.0, 4.0, 6.0, 8.0, 11.0, and 24.0 hours after administration. The blood samples were placed into EDTA-K2 anticoagulation test tubes and centrifuged at 10,000 rpm for 1 minute (4 °C), and plasma was separated within 1 hour and stored at -20 °C before analysis. The procedure starting from the blood collection to the centrifugation was performed under ice-bath conditions. Access to food was given 2 hours after administration.

After administration of different concentrations of the test compounds, the plasma concentrations of the test compounds in the SD rats were determined: 25 µL of each of the SD rat plasma samples of the various time points after administration was taken; 200 µL of acetonitrile was added to precipitate protein, and 50 µL of camptothecin (100 ng/mL) was added; after 5 minutes of vortexing and 10 minutes of centrifugation at 4000 rpm, 30 µL of the supernatant was taken and diluted with 120 µL of water, and 1 µL of the dilution was taken for LC/MS/MS analysis.

### 4. Pharmacokinetic parameters

**Table 3. Pharmacokinetic parameters of the compounds of the present disclosure in SD rats**

| Compound No. | Route of administration/dose (mg/kg) | Plasma concentration Cmax (ng/mL) | Area under curve AUC₀₋ₜ (h* ng/mL) | Half-life T1/2 (h) | Clearance rate CL/F (mL/min/kg) | Apparent distribution volume Vz/F (mL/kg) |
|---|---|---|---|---|---|---|
| 11 | i.g. /2.0 | 69.4 | 644 | 6.75 | 60.2 | 34816 |
| 11-2 or 11-1 (longer retention time) | i.g. /2.0 | 77.1 | 685 | 6.26 | 63.7 | 39568 |
| 12 | i.g. /2.0 | 37.4 | 304 | 5.19 | 139 | 63103 |
| 15 | i.g. /2.0 | 227 | 2137 | 6.75 | 14.6 | 8504 |
| 15-1 | i.g. /2.0 | 110 | 1126 | 6.66 | 39.5 | 23003 |
| 23 | i.g. /2.0 | 81.6 | 703 | 5.93 | 51.2 | 26276 |
| 28 | i.g. /2.0 | 25.5 | 315 | 7.17 | 108 | 66596 |
| Comparative Example 1 | i.g. /2.0 | 2.98 | 22 | 4.9 | 1166 | 506710 |

Conclusion: The compounds of the present disclosure exhibited larger areas under the curves, longer half-lives, and lower clearance rates in SD rats than Comparative Example 1, have excellent pharmacokinetic absorption activity, and have significant pharmacokinetic advantages.

### II. Dog testing

### 1. Abstract

Beagles were used as test animals. The plasma concentrations of a compound of the present disclosure in beagles at different time points after intragastric (i.g.) administration were determined using an LC/MS/MS method. The pharmacokinetic behavior of the compound of the present disclosure in beagles was studied, and its pharmacokinetic profile was evaluated.

### 2. Protocol

### 2.1. Test compounds

Example 15-1 compound; and
Compound ARV-110.

### 2.2. Test animals

8 beagles, provided by Jiangsu Johnbio Biotechnology Co., Ltd., with an equal number of males and females, were evenly divided into 2 groups. The animals were fasted overnight and then intragastrically dosed.

### 2.3. Compound solution preparation

A certain amount of test compound was weighed out and dissolved in 5% DMSO + 30% PG + 30% PEG400 + 35% normal saline to prepare a 0.4 mg/mL solution.

### 2.4. Administration

The dose administered was 2.0 mg/kg, and the volume was 5.0 mL/kg.

### 3. Procedure

0.5-mL blood samples were collected from the forelimb vein before administration and 0.25, 0.5, 1.0, 2.0, 4.0, 6.0, 8.0, 11.0, and 24.0 hours after administration. The blood samples were placed into EDTA-K2 anticoagulation test tubes and centrifuged at 10,000 rpm for 2 minutes (4 °C), and plasma was separated within 1 hour and stored at -80 °C before analysis. The procedure starting from the blood collection to the centrifugation was performed under ice-bath conditions. Access to food was given 3 hours after administration.

After administration of different concentrations of the test compounds, the plasma concentrations of the test compounds in the beagles were determined: 50 µL of each of the beagle plasma samples of the various time points after administration was taken, and 25 µL of camptothecin (1 µg/mL) and 450 µL of acetonitrile were added to precipitate protein; after vortexing and 10 minutes of centrifugation at 3700 rpm, 0.5 µL of the supernatant was taken for LC/MS/MS analysis.

### 4. Pharmacokinetic parameters

**Table 4. Pharmacokinetic parameters of the compound of the present disclosure in beagles**

| Compound No. | Route of administration/dose (mg/kg) | Plasma concentration Cmax (ng/mL) | Area under curve AUC₀₋ₜ (h* ng/mL) | Half-life T1/2 (h) | Clearance rate CL/F (mL/min/kg) | Apparent distribution volume Vz/F (mL/kg) |
|---|---|---|---|---|---|---|
| 15-1 | i.g. /2.0 | 75.6 | 1137 | 22.5 | 15.8 | 29843 |
| ARV-110 | i.g. /2.0 | 13.0 | 239 | 48.8 | 70.9 | 278328 |

Conclusion: The compound of the present disclosure exhibited a larger area under the curve and a lower clearance rate in beagles than compound ARV-110, has excellent pharmacokinetic absorption activity, and has significant pharmacokinetic advantages.

### III. Monkey testing

### 1. Abstract

Cynomolgus monkeys were used as test animals. The plasma concentrations of a compound of the present disclosure in cynomolgus monkeys at different time points after intragastric (i.g.) administration were determined using an LC/MS/MS method. The pharmacokinetic behavior of the compound of the present disclosure in cynomolgus monkeys was studied, and its pharmacokinetic profile was evaluated.

### 2. Protocol

### 2.1. Test compounds

Example 15-1 compound; and
ARV-110.

### 2.2. Test animals

8 cynomolgus monkeys, provided by Shanghai Medicilon Inc., with an equal number of males and females, were evenly divided into 2 groups. The animals were fasted overnight and then intragastrically dosed.

### 2.3. Compound solution preparation

A certain amount of test compound was weighed out and dissolved in 5% DMSO + 30% PG + 30% PEG400 + 35% normal saline to prepare a 0.4 mg/mL clear solution.

### 2.4. Administration

The dose administered was 2.0 mg/kg, and the volume was 5.0 mL/kg.

### 3. Procedure

1-mL blood samples were collected from the forelimb vein before administration and 0.25, 0.5, 1.0, 2.0, 4.0, 6.0, 8.0, 12.0, and 24.0 hours after administration. The blood samples were placed into EDTA-K2 anticoagulation test tubes and centrifuged at 2200 g for 10 minutes (2-8 °C), and plasma was separated within 1 hour and stored at -80 °C before analysis. The procedure starting from the blood collection to the centrifugation was performed under ice-bath conditions. Access to food and *ad libitum* access to water were given 3 hours after administration.

After administration of different concentrations of the test compounds, the plasma concentrations of the test compounds in the cynomolgus monkeys were determined: 20 µL of each of the cynomolgus monkey plasma samples of the various time points after administration was taken, and 400 µL of methanol was added to precipitate protein (containing 10 ng/mL verapamil); after 1 minute of vortexing and 7 minutes of centrifugation at 18,000 g, 5 µL of the supernatant was taken for LC/MS/MS analysis.

### 4. Pharmacokinetic parameters

**Table 5. Pharmacokinetic parameters of the compound of the present disclosure in cynomolgus monkeys**

| Compound No. | Route of administration/dose (mg/kg) | Plasma concentration Cmax (ng/mL) | Area under curve AUC₀₋ₜ (h* ng/mL) | Half-life T1/2 (h) | Clearance rate CL/F (mL/min/kg) | Apparent distribution volume Vz/F (mL/kg) |
|---|---|---|---|---|---|---|
| 15-1 | i.g. /2.0 | 200.5 | 2086.0 | 14.6 | 11.0 | 14494.8 |
| ARV 110 | i.g. /2.0 | 15.9 | 282.0 | 42.0 | 44.6 | 165179.6 |

Conclusion: The compound of the present disclosure exhibited a larger area under the curve and a lower clearance rate in cynomolgus monkeys than ARV-110, has excellent pharmacokinetic absorption activity, and has significant pharmacokinetic advantages.

### Test Example 4. Pharmacodynamic Testing

### 1. Objective

To evaluate the inhibition of the growth of castration-resistant prostate cancer LNCap-FGC subcutaneous xenograft tumors in male CB17-SCID mice by compounds of the present disclosure.

### 2. Test compounds

Example 15-1 compound;
Example 11-2 or 11-1 (longer retention time) compound;
ARV 110; and
20% PEG400 + 75% (10% TPGS) + 5% (1% HPMC K100LV) solution.

### 3. Method and materials

### 3.1. Test animals and housing conditions

Test animals: CB17-SCID male mice, purchased from Beijing Vital River Laboratory Animal Technology Co., Ltd. (certificate No. SCXK(Beijing)2016-0006; animal certification No. 110011220107542683), weighing about 20.54-26.72 g on purchase. Housing conditions: 5 animals/cage, a 12/12-hour light/dark cycle, a constant temperature of 23 ± 1 °C with humidity at 50 to 60%, *ad libitum* access to food and water.

### 3.2. Grouping of animals

Following acclimatization, the CB17-SCID mice were grouped and dosed as shown in Table 6 below.

**Table 6. The animal groups and administration regimen for the in vivo pharmacodynamic testing**

| Group | Number of animals | Route of administration |
|---|---|---|
| Vehicle control | 8 | 20% PEG400 + 75% (10% TPGS) + 5% (1% HPMC K100LV) solution (i.g./qd) |
| ARV-110 | 8 | 1 mpk (i.g./qd) |
| ARV-110 | 8 | 10 mpk (i.g./qd) |
| Example 15-1 | 8 | 1.5 mpk (i.g./qd) |
| Example 15-1 | 8 | 5 mpk (i.g./qd) |
| Example 15-1 | 8 | 15 mpk (i.g./qd) |
| 11-2 or 11-1 (longer retention time) | 8 | 1.5 mpk (i.g./qd) |
| 11-2 or 11-1 (longer retention time) | 8 | 5 mpk (i.g./qd) |
| 11-2 or 11-1 (longer retention time) | 8 | 15 mpk (i.g./qd) |

Notes: qd stands for "once a day"; i.g. stands for intragastric administration.

### 3.3. Method

0.2 mL (10 × 10⁶ cells + Matrigel) of LNCaP FGC cells (Matrigel was added, in a 1:1 ratio by volume) in the logarithmic growth phase was subcutaneously inoculated into the right upper limb of each mouse. When the mean tumor volume reached 77 mm³, male mice with stable tumor growth were selected and subjected to orchiectomy followed by three days of analgesic care. When the mean tumor volume reached 172 mm³, grouping and administration were started. The mice were randomized into 9 groups of 8 according to tumor volume and body weight, as shown in Table 6. The day of grouping was defined as D0, and intragastric administration was started and performed once a day for 21 days. The 21th day after administration was defined as D21 (Table 7). For tumor-bearing mice, the tumor volume was measured with a vernier caliper and the body weight was measured with a balance twice a week, and data were recorded.

### 3.4. Statistical analysis

All data were plotted and statistically analyzed using Excel and GraphPad Prism 8 software.

The calculation formula for tumor volume (V) was: V = 1/2 × a × b², where a and b represent length and width, respectively.

Relative tumor proliferation rate T/C(%) = (T-T₀)/(C-C₀) × 100 (%), where T and C represent the tumor volumes of a treatment group and the control group at the end of the experiment; T₀ and C₀ represent the tumor volumes at the beginning of the experiment. Tumor growth inhibition rate TGI (%) = 1-T/C (%).

### 4. Results

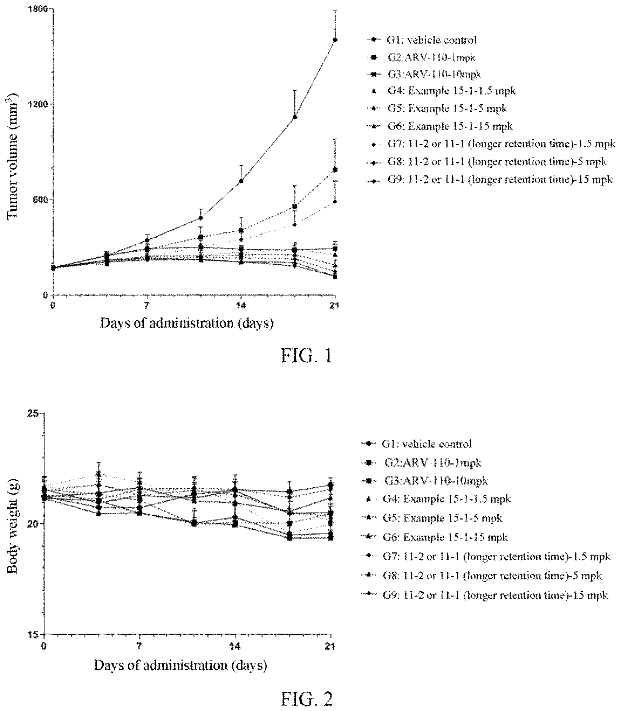

Data on the efficacy of Example 15-1 and 11-2 or 11-1 (longer retention time) compounds against LNCap-FGC xenograft tumors in castrated CB17-SCID mice are shown in Table 7 and FIG. 1.

The effects of Example 15-1 and 11-2 or 11-1 (longer retention time) compounds on the body weight of CB17-SCID mice are shown in FIG. 2.

**Table 7. The efficacy of the compounds of the present disclosure against LNCap-FGC xenograft tumors in castrated CB17-SCID mice**

| Group | Administration | Route Dose (mpk) | Mean tumor volume (mm³) | | | | TGI (%) D21 | P | Number of remaining animals/group |
|---|---|---|---|---|---|---|---|---|---|
| | | | D0 | SEM | D21 | SEM | | (vs. vehicle control) | |
| Vehicle control | qd/21d | i.g., 0 | 172 | 11 | 1,606 | 187 | / | / | 8/8 |
| ARV-110 | qd/21d | i.g., 1 | 172 | 10 | 789 | 193 | 57 | <0.0001 | 8/8 |
| ARV-110 | qd/21d | i.g., 10 | 172 | 10 | 293 | 43 | 92 | <0.0001 | 8/8 |
| Example 15-1 | qd/21d | i.g., 1.5 | 172 | 10 | 255 | 64 | 94 | <0.0001 | 8/8 |
| Example 15-1 | qd/21d | i.g., 5 | 172 | 11 | 188 | 32 | 99 | <0.0001 | 8/8 |
| Example 15-1 | qd/21d | i.g., 15 | 172 | 11 | 117 | 17 | 104 | <0.0001 | 8/8 |
| 11-2 or 11-1 (longer retention time) | qd/21d | i.g., 1.5 | 172 | 11 | 587 | 131 | 71 | <0.0001 | 8/8 |
| 11-2 or 11-1 (longer retention time) | qd/21d | i.g., 5 | 172 | 10 | 147 | 35 | 102 | <0.0001 | 8/8 |
| 11-2 or 11-1 (longer retention time) | qd/21d | i.g., 15 | 172 | 10 | 120 | 10 | 104 | <0.0001 | 8/8 |

| | | | | | | | | | |
|---|---|---|---|---|---|---|---|---|---|
| Notes: qd stands for "once a day"; d stands for day; i.g. stands for intragastric administration; SEM stands for standard error of measurement. | | | | | | | | | |

### 5. Conclusion

After 21 days of once-daily administration, Example 15-1 compound exhibited a tumor growth inhibition rate of 94% in the low-dose 1.5 mpk group, a tumor growth inhibition rate of 99% in the medium-dose 5 mpk group, and a tumor growth inhibition rate of 104% in the high-dose 15 mpk group, and showed little effect on the body weight of the mice. After 21 days of once-daily administration, Example 11-2 or 11-1 (longer retention time) exhibited a tumor growth inhibition rate of 71% in the low-dose 1.5 mpk group, a tumor growth inhibition rate of 102% in the medium-dose 5 mpk group, and a tumor growth inhibition rate of 104% in the high-dose 15 mpk group, and showed little effect on the body weight of the mice.

After 21 days of once-daily administration under the same conditions, ARV-110 exhibited a tumor growth inhibition rate of 57% in the low-dose 1 mpk group and a tumor growth inhibition rate of 92% in the high-dose 10 mpk group. Example 15-1 and Examples 11-2 or 11-1 (longer retention time) have some advantages over ARV-110.

## Claims

1. A compound represented by general formula (I) or a pharmaceutically acceptable salt thereof,
R-X¹-X²-X³-X⁴-A (I)
wherein:
R is aryl or heteroaryl, wherein the aryl and heteroaryl are each independently optionally substituted with one or more substituents selected from the group consisting of halogen, alkyl, alkoxy, haloalkyl, haloalkoxy, cyano, -(CH₂)ₙNR^{a}R^{b}, nitro, hydroxy, hydroxyalkyl, aminoalkyl, alkoxyalkyl, cycloalkylalkyl, heterocyclylalkyl, cycloalkyl, heterocyclyl, aryl, and heteroaryl;
A is selected from the group consisting of 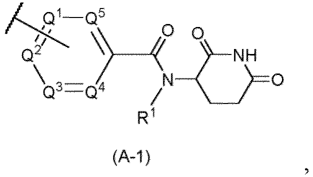 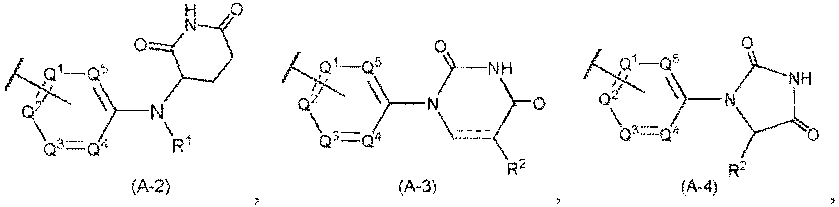 and 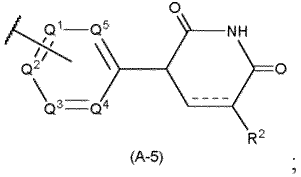
- is a single bond or a double bond;
one of Q¹, Q², Q³, Q⁴, and Q⁵ is a carbon atom, and the other four are identical or different and are each independently a nitrogen atom or CR';
each R' is identical or different and is independently selected from the group consisting of a hydrogen atom, halogen, alkyl, alkoxy, haloalkyl, haloalkoxy, hydroxyalkyl, aminoalkyl, alkoxyalkyl, cyano, hydroxy, nitro, and -(CH₂)ₙNR^{c}R^{d};
R¹ is selected from the group consisting of a hydrogen atom, alkyl, haloalkyl, hydroxyalkyl, aminoalkyl, and alkoxyalkyl;
R² is selected from the group consisting of a hydrogen atom, halogen, alkyl, haloalkyl, hydroxyalkyl, alkoxy, haloalkoxy, hydroxy, cyano, aminoalkyl, and alkoxyalkyl;
X¹ is spirocyclyl, wherein the spirocyclyl is optionally substituted with one or more substituents selected from the group consisting of halogen, alkyl, alkoxy, haloalkyl, haloalkoxy, cyano, nitro, hydroxy, hydroxyalkyl, aminoalkyl, alkoxyalkyl, cycloalkylalkyl, heterocyclylalkyl, cycloalkyl, heterocyclyl, aryl, heteroaryl, -OR³, - C(O)R³, -C(O)OR³, -S(O)ₘR³, -NR⁴R⁵, -C(O)NR⁴R⁵, -S(O)ₘNR⁴R⁵, =O, and =S;
X² is selected from the group consisting of -C(O)-, -S(O)₂-, and -(CR^{2a}R^{2b})ₘ₁-;
X³ is selected from the group consisting of cycloalkyl, heterocyclyl, aryl, and heteroaryl, wherein the cycloalkyl, heterocyclyl, aryl, and heteroaryl are each independently optionally substituted with one or more substituents selected from the group consisting of halogen, alkyl, alkoxy, haloalkyl, haloalkoxy, cyano, -(CH₂)ₙNR^{e}R^{f}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
X⁴ is -J¹-J²-J³-J⁴-J⁵-, wherein J¹ is attached to X³;
J¹ is selected from the group consisting of -O-, -S-, -NR⁶-, -C(O)-, -S(O)₂-, -(CR⁷R⁸)ₘ₂-, alkenyl, and alkynyl;
J² is cycloalkyl or heterocyclyl, wherein the cycloalkyl and heterocyclyl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J³ is selected from the group consisting of -O-, -S-, -NR^{6a}-, -C(O)-, -S(O)₂-, -(CR^{7a}R^{8a})ₘ₃-, alkenyl, alkynyl, cycloalkyl, and heterocyclyl, wherein the cycloalkyl and heterocyclyl are each independently optionally substituted with one or more substituents selected from the group consisting of halogen, alkyl, alkoxy, haloalkyl, haloalkoxy, cyano, - (CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J⁴ is selected from the group consisting of a bond, cycloalkyl, heterocyclyl, aryl, and heteroaryl, wherein the cycloalkyl, heterocyclyl, aryl, and heteroaryl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
J⁵ is selected from the group consisting of -C(O)NR^{6b}-, -NR^{6b}C(O)-, -O-, -S-, -NR^{6b}-, - C(O)-, -S(O)₂-, -(CR^{7b}R^{8b})ₘ₄-, alkenyl, alkynyl, cycloalkyl, and heterocyclyl, wherein the cycloalkyl and heterocyclyl are each independently optionally substituted with one or more substituents selected from the group consisting of halogen, alkyl, alkoxy, haloalkyl, haloalkoxy, cyano, -(CH₂)ₙNR^{g}R^{h}, nitro, hydroxy, hydroxyalkyl, aminoalkyl, alkoxyalkyl, cycloalkylalkyl, heterocyclylalkyl, cycloalkyl, heterocyclyl, aryl, heteroaryl, and =O;
R^{a}, R^{b}, R^{c}, R^{d}, R^{e}, R^{f}, R^{g}, R^{h}, R⁶, R^{6a}, and R^{6b} are identical or different and are each independently selected from the group consisting of a hydrogen atom, alkyl, haloalkyl, hydroxyalkyl, aminoalkyl, alkoxyalkyl, cycloalkylalkyl, and heterocyclylalkyl;
R^{2a}, R^{2b}, R⁷, R⁸, R^{7a}, R^{8a}, R^{7b}, and R^{8b} are identical or different and are each independently selected from the group consisting of a hydrogen atom, halogen, alkyl, alkoxy, hydroxy, amino, cyano, haloalkyl, haloalkoxy, hydroxyalkyl, aminoalkyl, alkoxyalkyl, cycloalkylalkyl, and heterocyclylalkyl;
R³ is selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
R⁴ and R⁵ are identical or different and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
or R⁴ and R⁵, together with the nitrogen atom to which they are attached, form heterocyclyl, and the heterocyclyl is optionally substituted with one or more substituents selected from the group consisting of halogen, alkyl, alkoxy, hydroxy, amino, cyano, nitro, haloalkyl, haloalkoxy, hydroxyalkyl, =O, aminoalkyl, alkoxyalkyl, cycloalkylalkyl, heterocyclylalkyl, cycloalkyl, heterocyclyl, aryl, and heteroaryl;
n is 0, 1, 2, or 3;
m is 0, 1, or 2;
m1 is 0, 1, 2, or 3;
m2 is 0, 1, 2, or 3;
m3 is 0, 1, 2, or 3; and
m4 is 0, 1, 2, or 3.

2. The compound represented by general formula (I) or the pharmaceutically acceptable salt thereof according to claim 1, wherein R is phenyl or 5- or 6-membered heteroaryl, wherein the phenyl and 5- or 6-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl; preferably, R is selected from the group consisting of 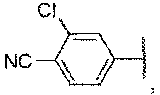 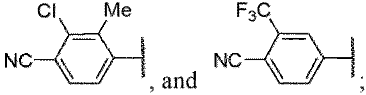 more preferably, R is

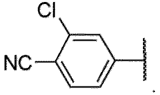

3. The compound represented by general formula (I) or the pharmaceutically acceptable salt thereof according to claim 1 or 2, wherein X¹ is 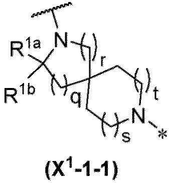 or 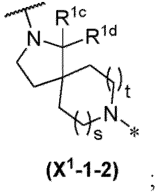 the bond with * is attached to X²; R^{1a}, R^{1b}, R^{1c}, and R^{1d} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
or, R^{1a} and R^{1b}, together with the carbon atom to which they are attached, form C=O;
or, R^{1c} and R^{1d}, together with the carbon atom to which they are attached, form C=O;
q is 0, 1, 2, or 3; r is 0, 1, 2, or 3; s is 0, 1, 2, 3, or 4; and t is 0, 1, 2, 3, or 4.

4. The compound represented by general formula (I) or the pharmaceutically acceptable salt thereof according to any one of claims 1 to 3, wherein X³ is 6- to 10-membered aryl or 5- to 10-membered heteroaryl, wherein the 6- to 10-membered aryl and 5- to 10-membered heteroaryl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably, X³ is phenyl or 6-membered heteroaryl, wherein the phenyl and 6-membered heteroaryl are each independently optionally substituted with one or more halogens.

5. The compound represented by general formula (I) or the pharmaceutically acceptable salt thereof according to any one of claims 1 to 4, wherein J² is 3- to 12-membered heterocyclyl, wherein the 3- to 12-membered heterocyclyl is optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O; preferably, J² is selected from the group consisting of 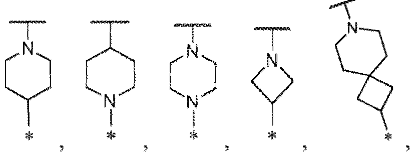 and 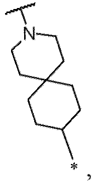 wherein the bond with * is attached to J³.

6. The compound represented by general formula (I) or the pharmaceutically acceptable salt thereof according to any one of claims 1 to 5, wherein J³ is selected from the group consisting of -O-, -S-, -NR^{6a}-, -C(O)-, -S(O)₂-, -(CR^{7a}R^{8a})ₘ₃-, C₂₋₆ alkenyl, C₂₋₆ alkynyl, 3- to 8-membered cycloalkyl, and 3- to 8-membered heterocyclyl; R^{6a} is a hydrogen atom or C₁₋₆ alkyl; R^{7a} and R^{8a} are identical or different and are each independently selected from the group consisting of a hydrogen atom, halogen, hydroxy, and C₁₋₆ alkyl; m3 is 0, 1, 2, or 3; preferably, J³ is a bond or CH₂.

7. The compound represented by general formula (I) or the pharmaceutically acceptable salt thereof according to any one of claims 1 to 6, wherein J⁴ is 3- to 8-membered cycloalkyl or 3- to 8-membered heterocyclyl, wherein the 3- to 8-membered cycloalkyl and 3- to 8-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O; preferably, J⁴ is selected from the group consisting of 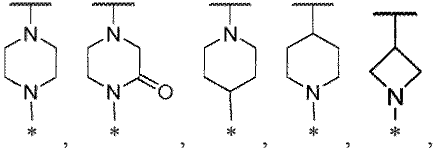 and 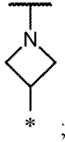 the bond with * is attached to J⁵.

8. The compound represented by general formula (I) or the pharmaceutically acceptable salt thereof according to any one of claims 1 to 4 and 6, being a compound represented by general formula (G) or a pharmaceutically acceptable salt thereof: 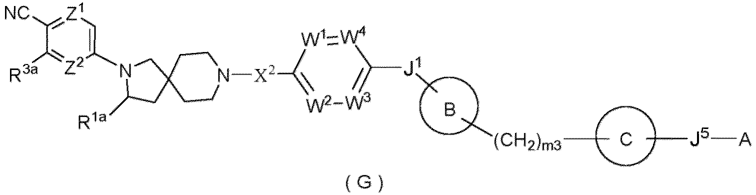 wherein:
R^{1a} is selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z¹ is N or CR^{3b}; Z² is N or CR^{3c}; R^{3a}, R^{3b}, and R^{3c} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, nitro, hydroxy, and C₁₋₆ hydroxyalkyl;
ring B and ring C are identical or different and are each independently 3- to 12-membered cycloalkyl or 3- to 12-membered heterocyclyl, and the 3- to 12-membered cycloalkyl and 3- to 12-membered heterocyclyl are each independently optionally substituted with one or more substituents selected from the group consisting of halogen, C₁₋₆ alkyl, C₁₋₆ alkoxy, C₁₋₆ haloalkyl, C₁₋₆ haloalkoxy, cyano, amino, hydroxy, and =O;
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
m3, X², J¹, J⁵, and A are as defined in claim 1.

9. The compound represented by general formula (I) or the pharmaceutically acceptable salt thereof according to any one of claims 1 to 4 and 6 to 8, being a compound represented by general formula (III') or a pharmaceutically acceptable salt thereof: 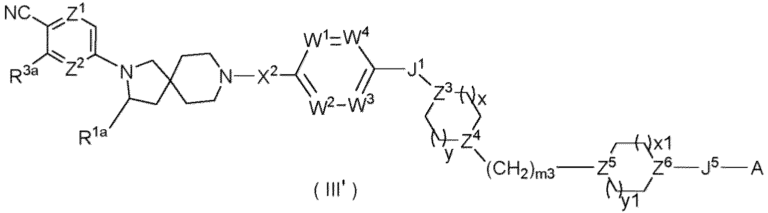 wherein:
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z³, Z⁴, Z⁵, and Z⁶ are identical or different and are each independently a N atom or CH;
m3 is 0, 1, 2, or 3;
x, x1, y, and y1 are each independently 0, 1, or 2;
J⁵ is selected from the group consisting of -C(O)NR^{6b}-, -NR^{6b}C(O)-, -O-, -S-, -NR^{6b}-, - C(O)-, -S(O)₂-, -(CR^{7b}R^{8b})ₘ₄-, C₂₋₆ alkenyl, C₂₋₆ alkynyl, 3- to 8-membered cycloalkyl, and 3- to 8-membered heterocyclyl; R^{6b} is a hydrogen atom or C₁₋₆ alkyl; R^{7b} and R^{8b} are identical or different and are each independently selected from the group consisting of a hydrogen atom, halogen, hydroxy, and C₁₋₆ alkyl; m4 is 0, 1, 2, or 3; preferably, J⁵ is selected from the group consisting of a bond, 3- to 8-membered heterocyclyl, and 3- to 8-membered cycloalkyl;
Z¹, Z², R^{1a}, R^{3a}, X², J¹, and A are as defined in claim 8.

10. The compound represented by general formula (I) or the pharmaceutically acceptable salt thereof according to any one of claims 1 to 9, being a compound represented by general formula (III) or a pharmaceutically acceptable salt thereof: 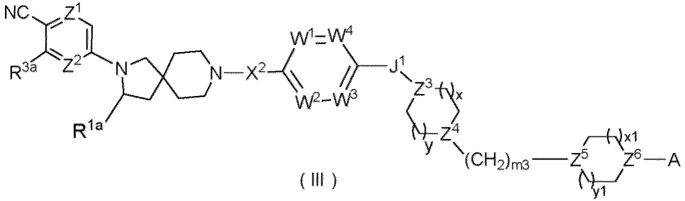 wherein:
W¹ is N or CR^{3d}; W² is N or CR^{3e}; W³ is N or CR^{3f}; W⁴ is N or CR^{3g};
R^{3d}, R^{3e}, R^{3f}, and R^{3g} are identical or different and are each independently selected from the group consisting of a hydrogen atom, halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl;
Z³, Z⁴, Z⁵, and Z⁶ are identical or different and are each independently a N atom or CH, provided that at least one of Z³ and Z⁴ is a N atom;
m3 is 0, 1, 2, or 3;
x, x1, y, and y1 are each independently 0, 1, or 2;
Z¹, Z², R^{1a}, R^{3a}, X², J¹, and A are as defined in claim 8.

11. The compound represented by general formula (I) or the pharmaceutically acceptable salt thereof according to any one of claims 1 to 10, wherein X² is selected from the group consisting of -C(O)-, -S(O)₂-, and -(CR^{2a}R^{2b})ₘ₁-; R^{2a} and R^{2b} are identical or different and are each independently selected from the group consisting of a hydrogen atom, halogen, hydroxy, and C₁₋₆ alkyl; m1 is 0, 1, 2, or 3; preferably, X² is a bond or -C(O)-; more preferably, X² is a bond.

12. The compound represented by general formula (I) or the pharmaceutically acceptable salt thereof according to any one of claims 1 to 11, wherein J¹ is selected from the group consisting of -O-, -S-, -NR⁶-, -C(O)-, -S(O)₂-, -(CR⁷R⁸)ₘ₂-, C₂₋₆ alkenyl, and C₂₋₆ alkynyl; R⁶ is a hydrogen atom or C₁₋₆ alkyl; R⁷ and R⁸ are identical or different and are each independently selected from the group consisting of a hydrogen atom, halogen, hydroxy, and C₁₋₆ alkyl; m2 is 0, 1, 2, or 3; preferably, J¹ is -S- or -C(O)-; more preferably, J¹ is - C(O)-.

13. The compound represented by general formula (I) or the pharmaceutically acceptable salt thereof according to any one of claims 1 to 12, being a compound represented by general formula (IV'-1) or a pharmaceutically acceptable salt thereof: 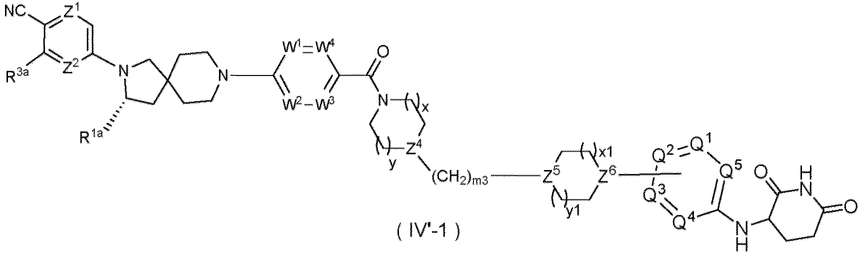 wherein:
R^{1a} is selected from the group consisting of halogen, C₁₋₆ alkyl, C₁₋₆ alkoxy, C₁₋₆ haloalkyl, C₁₋₆ haloalkoxy, cyano, amino, hydroxy, and C₁₋₆ hydroxyalkyl; preferably, R^{1a} is C₁₋₆ alkyl; more preferably, R^{1a} is methyl;
Q² is a carbon atom, and Q¹, Q³, Q⁴, and Q⁵ are identical or different and are each independently a nitrogen atom or CR'; or, Q³ is a carbon atom, and Q¹, Q², Q⁴, and Q⁵ are identical or different and are each independently a nitrogen atom or CR';
each R' is identical or different and is independently selected from the group consisting of a hydrogen atom, halogen, C₁₋₆ alkyl, C₁₋₆ alkoxy, C₁₋₆ haloalkyl, C₁₋₆ haloalkoxy, C₁₋₆ hydroxyalkyl, cyano, hydroxy, and amino; and
Z¹, Z², R^{3a}, W¹, W², W³, W⁴, Z⁴, Z⁵, Z⁶, m3, x, x1, y, and y1 are as defined in claim 9.

14. The compound represented by general formula (I) or the pharmaceutically acceptable salt thereof according to any one of claims 8 to 13, wherein Z¹ is CR^{3b}; Z² is CR^{3c}; R^{3a}, R^{3b}, and R^{3c} are identical or different and are each independently selected from the group consisting of a hydrogen atom, halogen, C₁₋₆ alkyl, C₁₋₆ alkoxy, cyano, and C₁₋₆ haloalkyl; preferably, Z¹ is CR^{3b}; Z² is CR^{3c}; R^{3a} is halogen or C₁₋₆ haloalkyl; R^{3b} is a hydrogen atom; R^{3c} is a hydrogen atom or C₁₋₆ alkyl.

15. The compound represented by general formula (I) or the pharmaceutically acceptable salt thereof according to any one of claims 8 to 14, wherein W¹ is N or CR^{3d}; W² is N or CR^{3e}; W³ is N or CR^{3f}; W⁴ is N or CR^{3g}; R^{3d}, R^{3e}, R^{3f}, and R^{3g} are identical or different and are each independently a hydrogen atom or halogen; preferably, W¹ is CR^{3d}; W² is CR^{3e}; W³ is CR^{3f}; W⁴ is CR^{3g}; R^{3d}, R^{3e}, R^{3f}, and R^{3g} are identical or different and are each independently a hydrogen atom or halogen.

16. The compound represented by general formula (I) or the pharmaceutically acceptable salt thereof according to any one of claims 1 to 15, being selected from the group consisting of the following compounds: 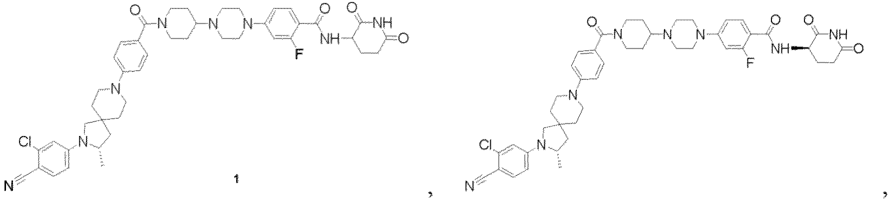 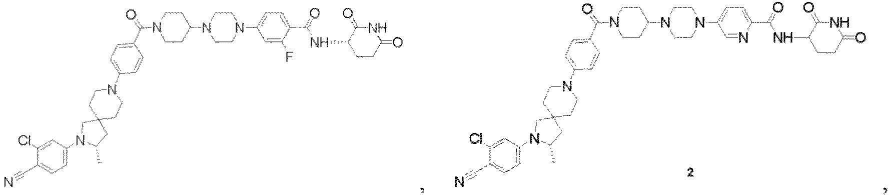 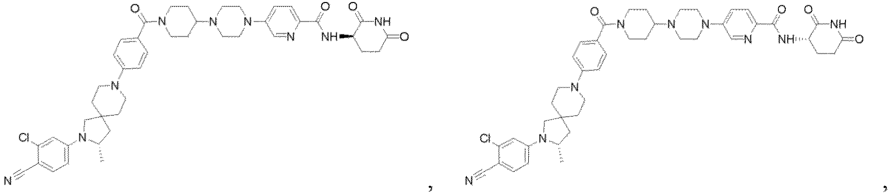 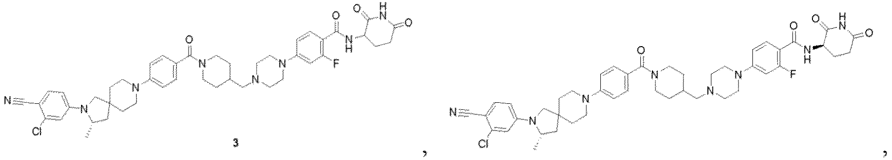 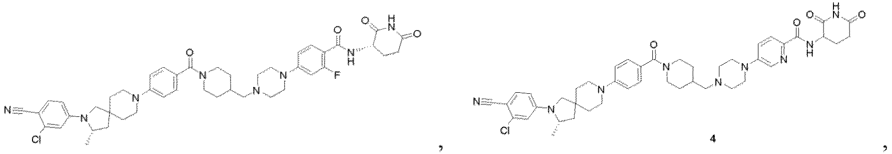 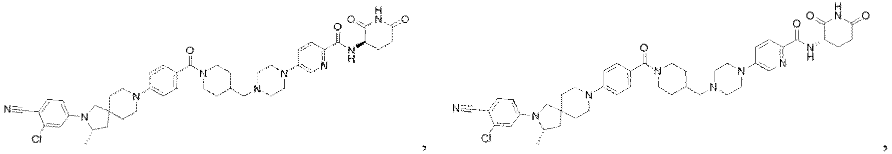 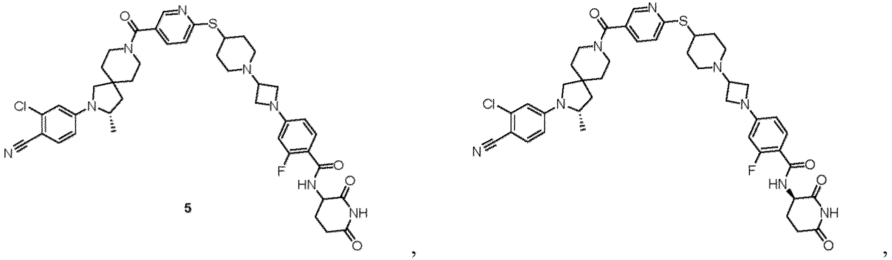 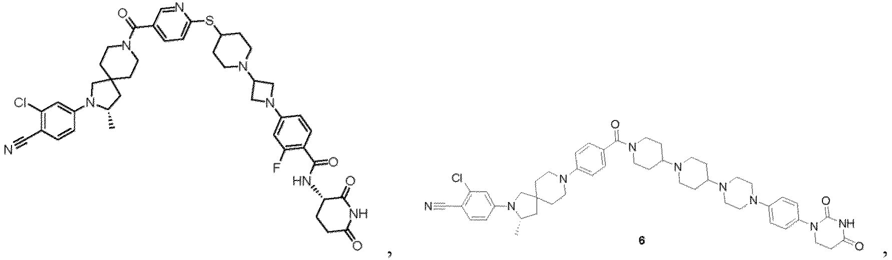 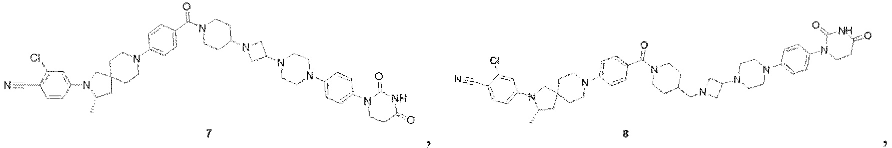 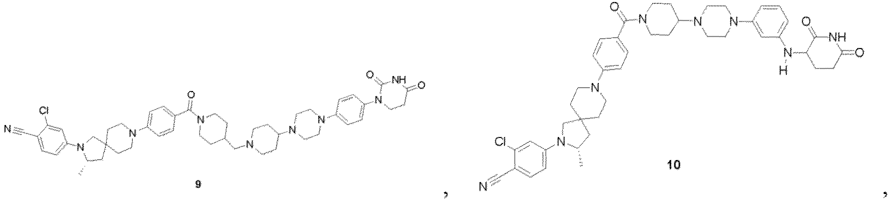 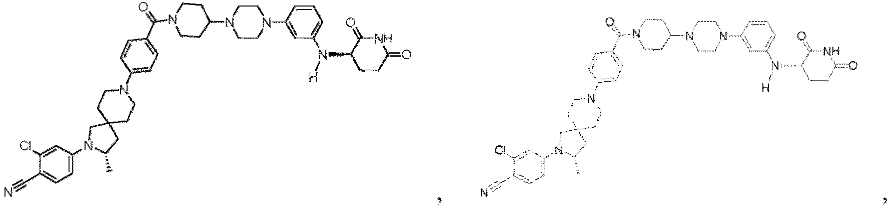 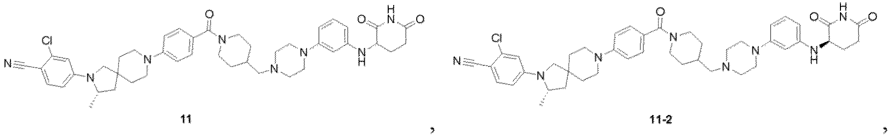 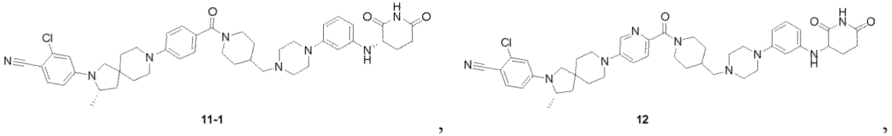 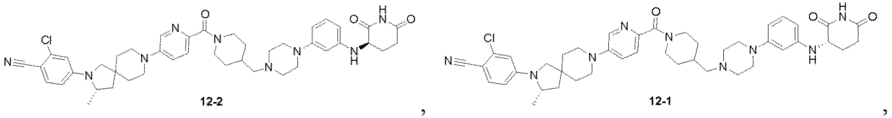 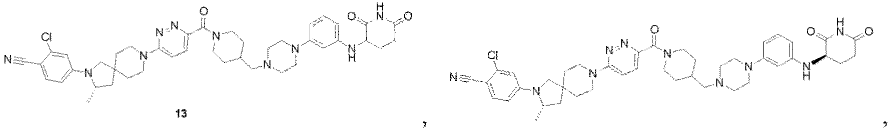 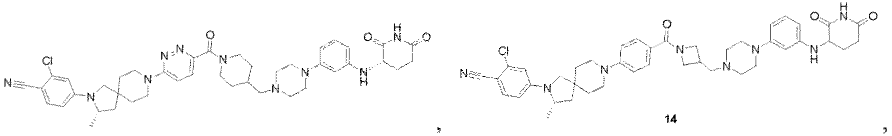 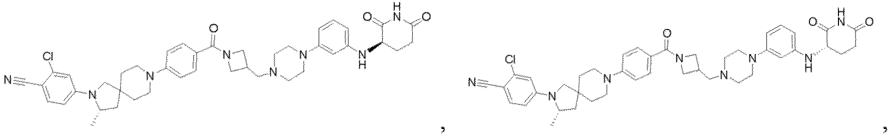 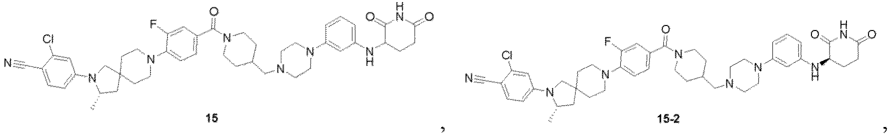 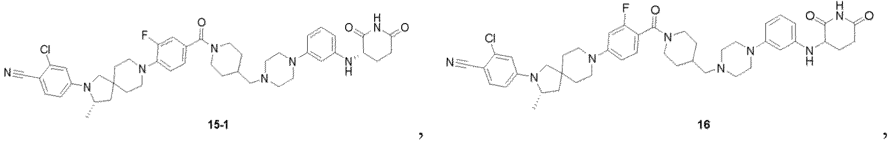 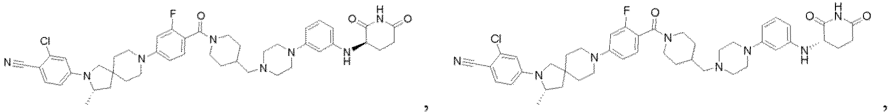 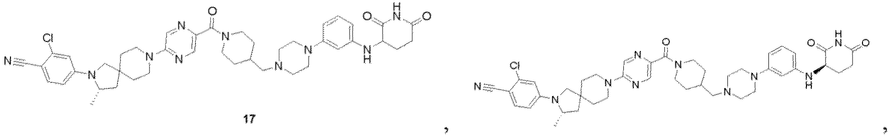 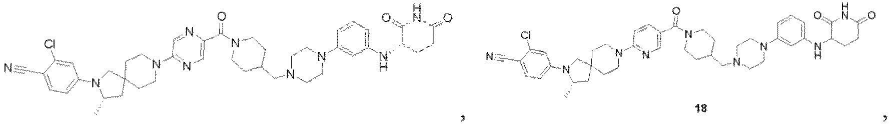 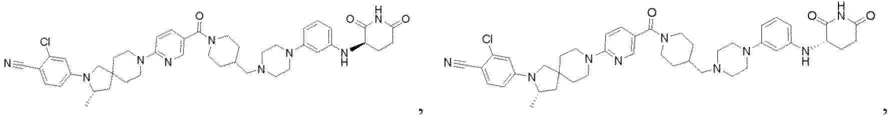 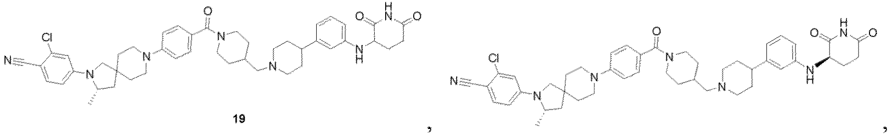 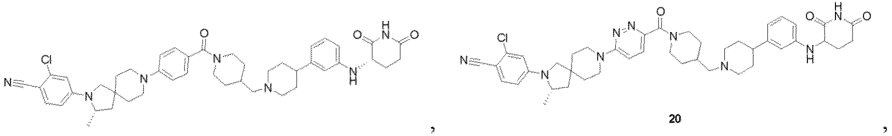 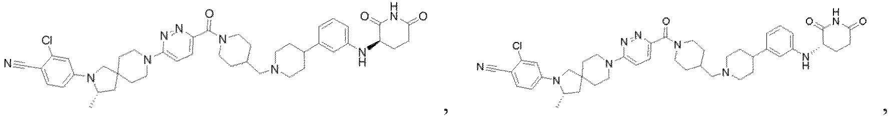 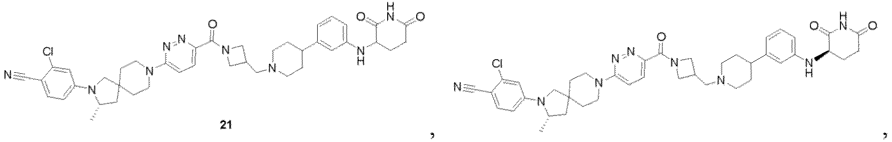 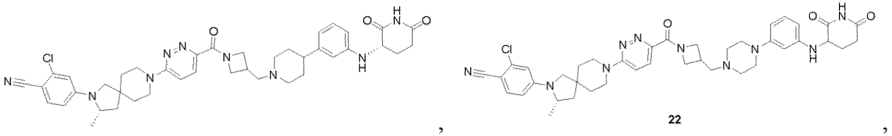 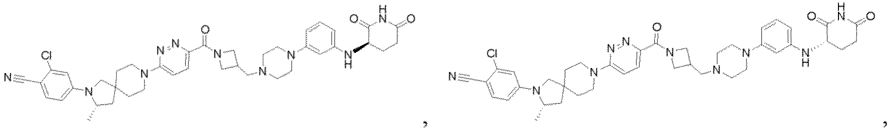 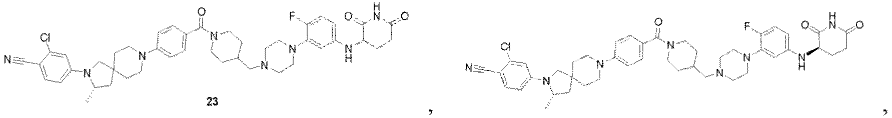 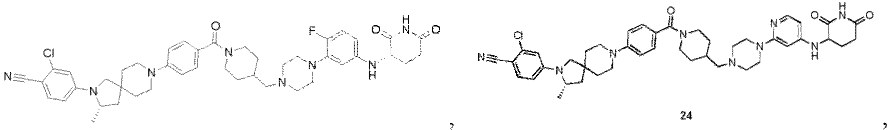 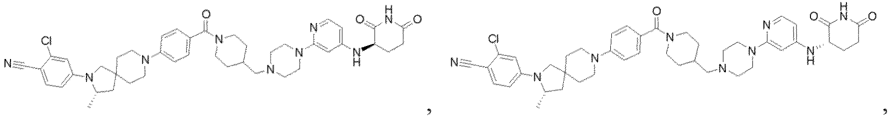 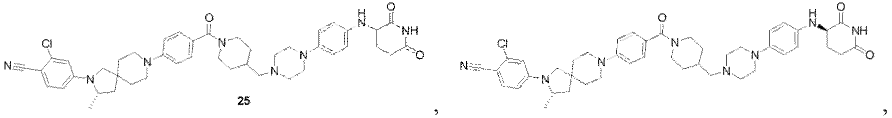 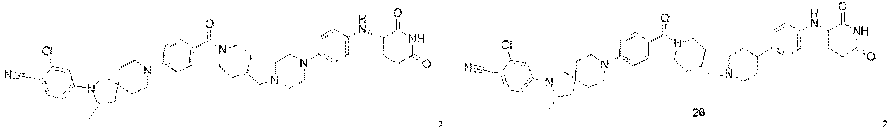 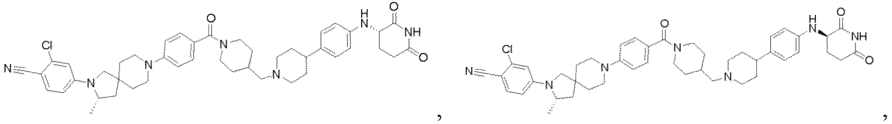 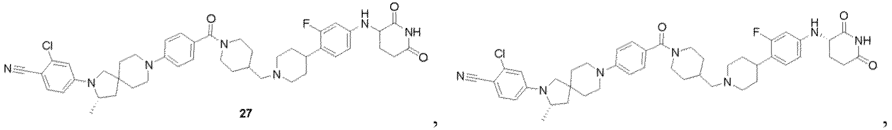 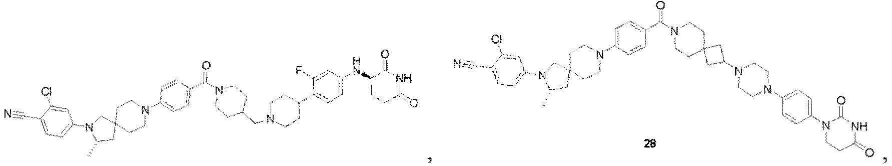 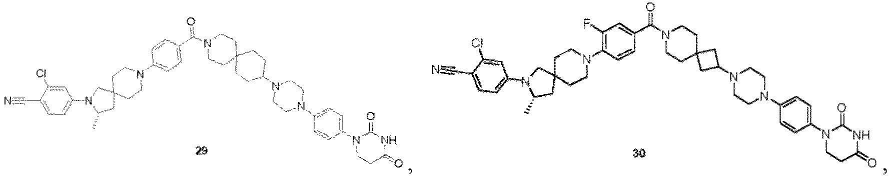 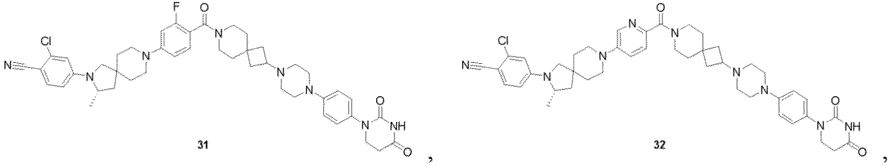 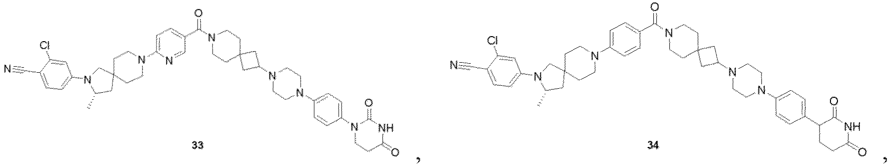 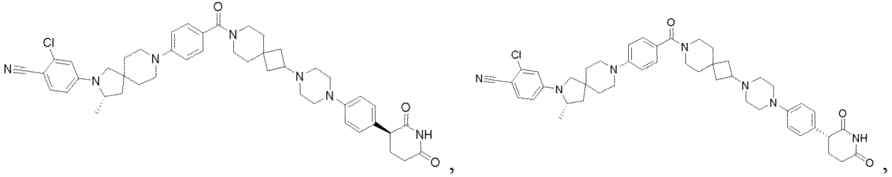 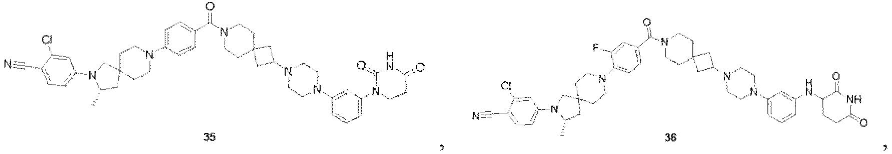 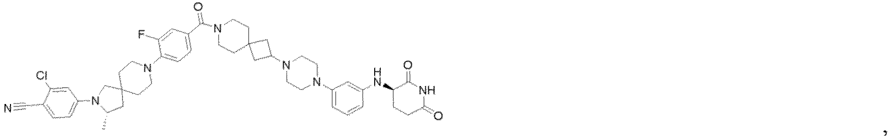 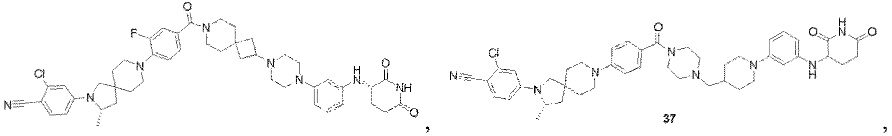 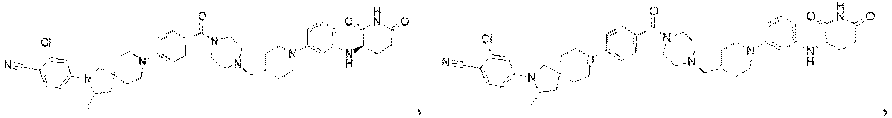 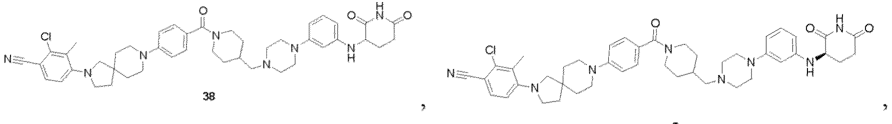 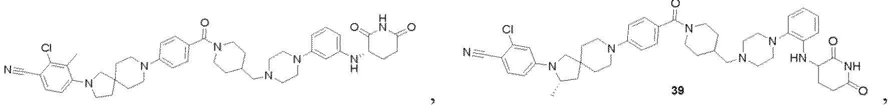 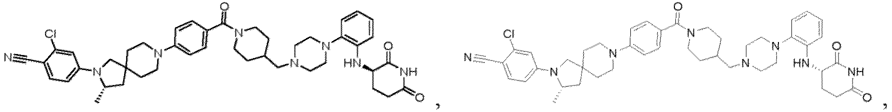 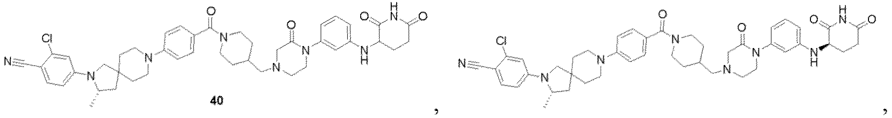 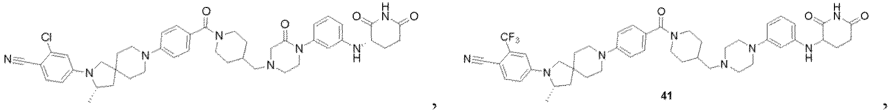 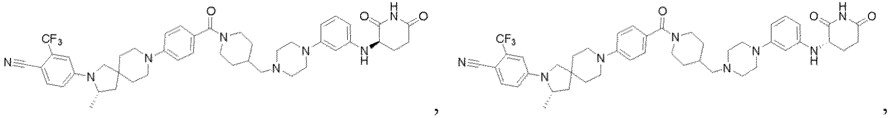 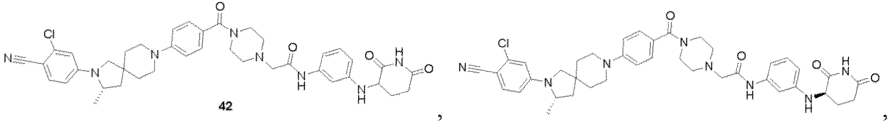 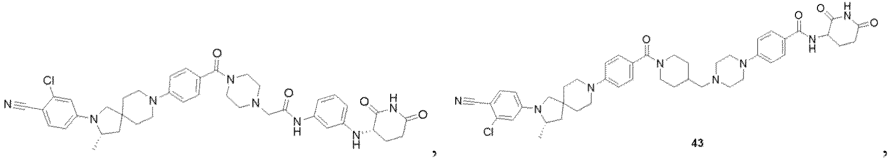 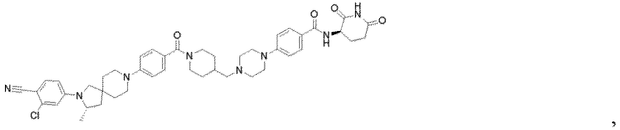 and

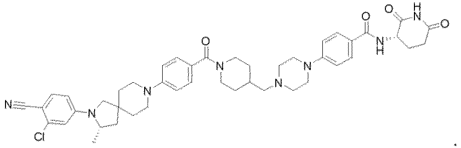

17. A compound represented by general formula (MB) or a salt thereof, 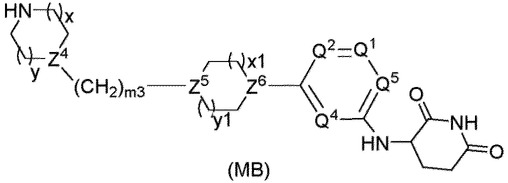 wherein:
Q¹, Q², Q⁴, Q⁵, Z⁴, Z⁵, Z⁶, x, x1, y, y1, and m3 are as defined in claim 13.

18. A compound, being selected from the group consisting of the following compounds:

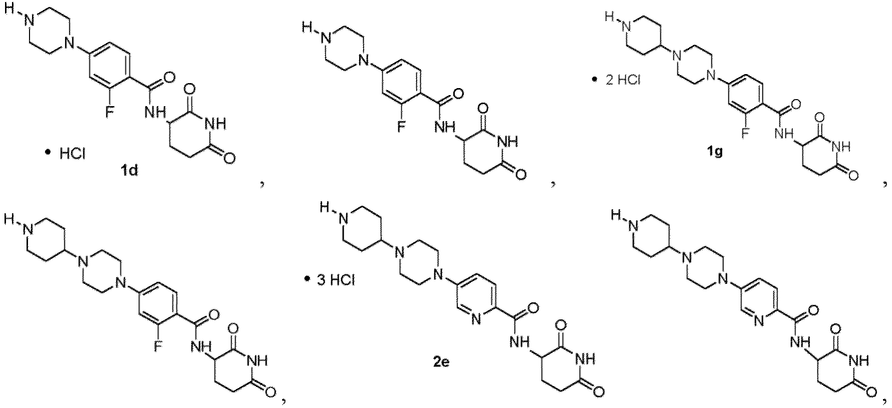

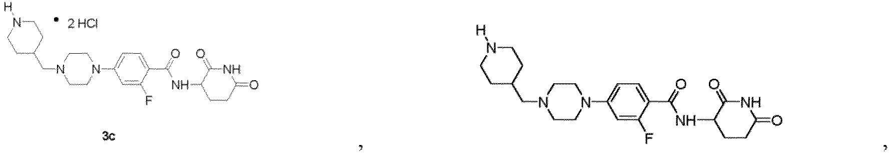

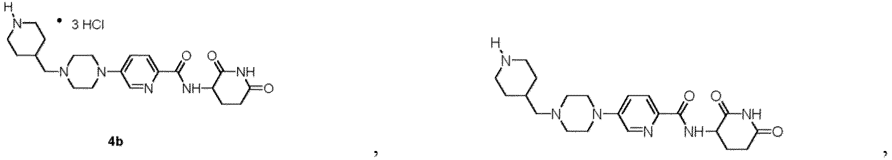

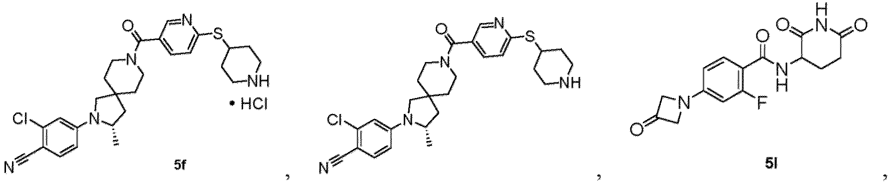

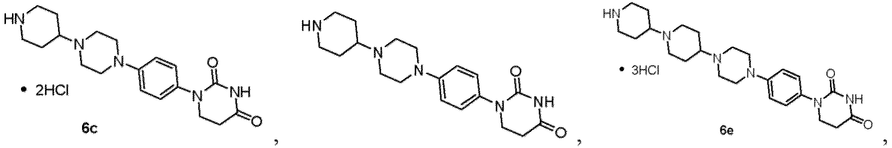

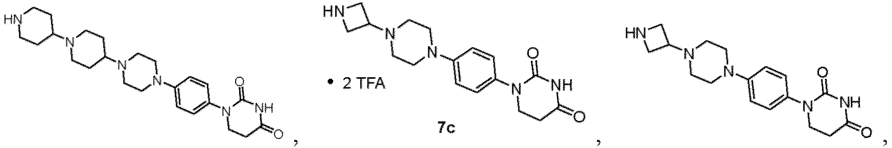

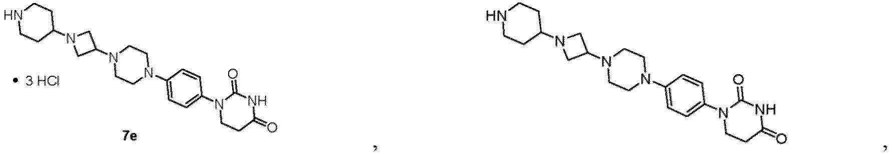

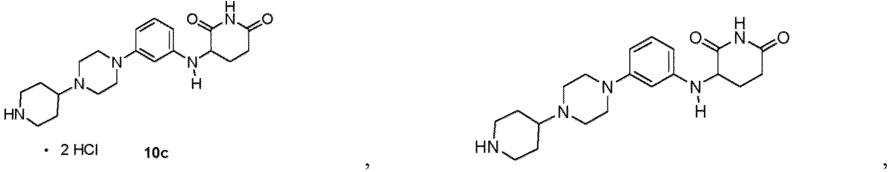

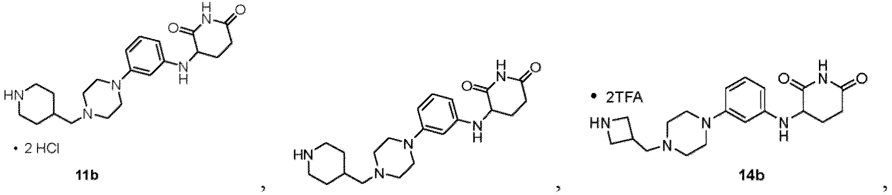

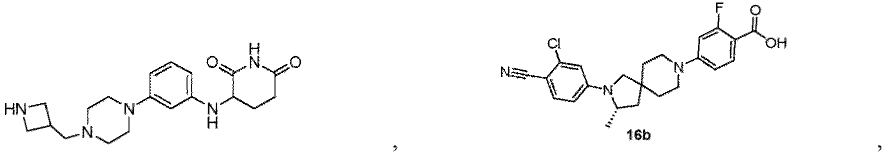

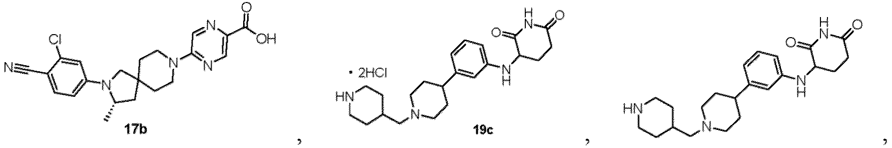

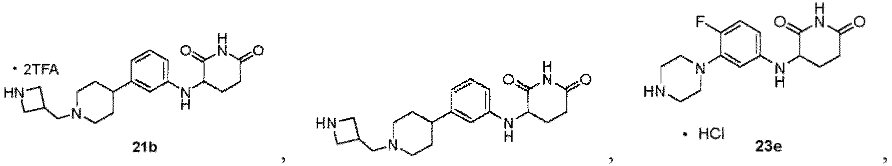

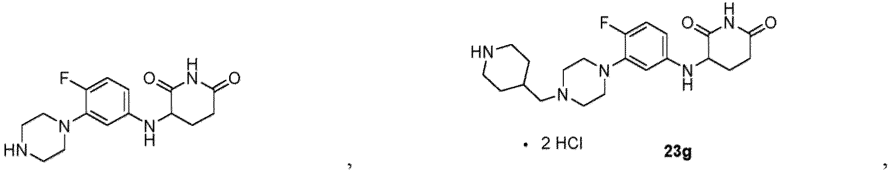

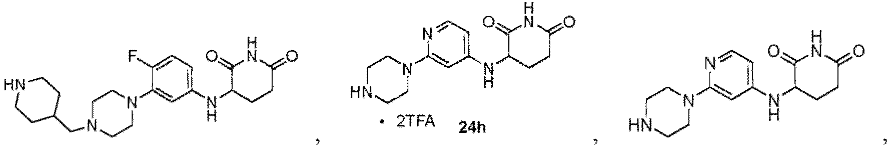

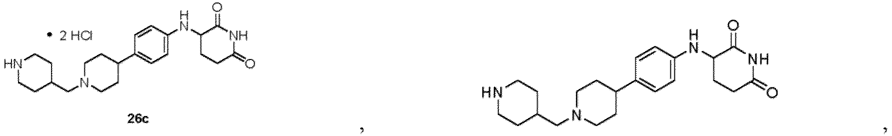

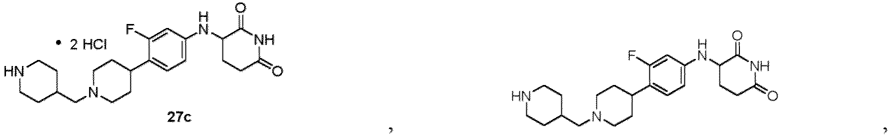

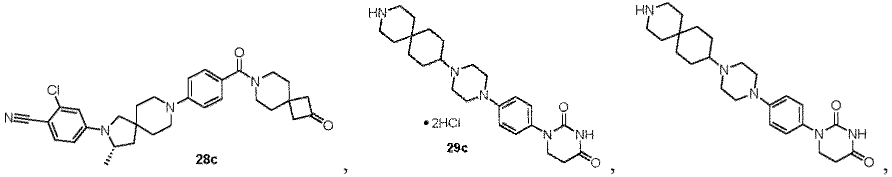

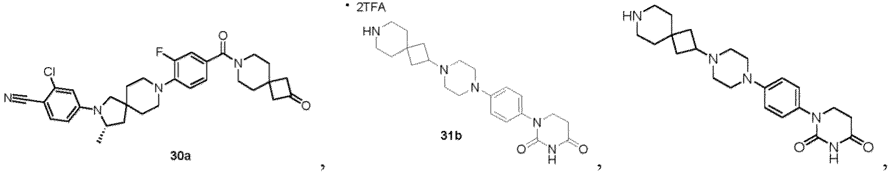

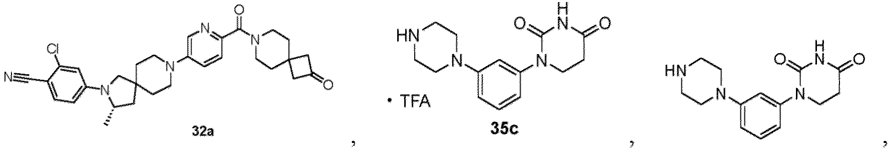

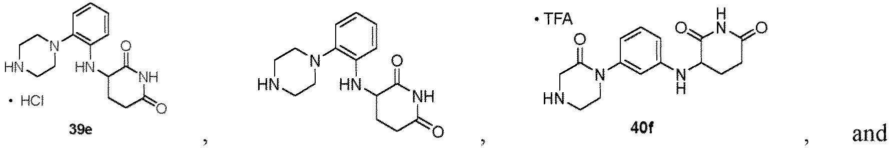

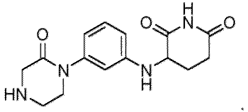

19. A method for preparing the compound represented by general formula (IV'-1) or the pharmaceutically acceptable salt thereof according to claim 13, comprising: 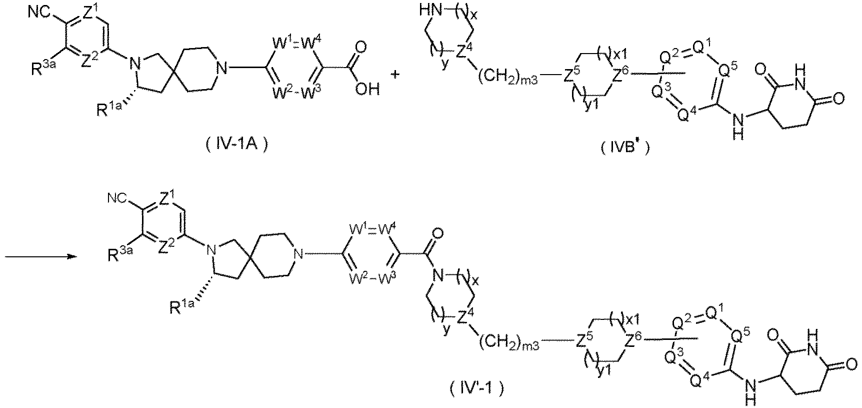 conducting a condensation reaction of a compound represented by general formula (IV-1A) or a salt thereof with a compound represented by general formula (IVB') or a salt thereof to give the compound represented by general formula (IV'-1) or the pharmaceutically acceptable salt thereof;
wherein:
R^{1a}, Q¹, Q², Q³, Q⁴, Q⁵, R^{3a}, W¹, W², W³, W⁴, Z¹, Z², Z⁴, Z⁵, Z⁶, x, x1, y, y1, and m3 are as defined in claim 13.

20. A pharmaceutical composition, wherein the pharmaceutical composition comprises the compound or the pharmaceutically acceptable salt thereof according to any one of claims 1 to 16, and one or more pharmaceutically acceptable carriers, diluents, or excipients.

21. Use of the compound or the pharmaceutically acceptable salt thereof according to any one of claims 1 to 16 or the pharmaceutical composition according to claim 20 in the preparation of a medicament for regulating the ubiquitination and degradation of the androgen receptor (AR) protein.

22. Use of the compound or the pharmaceutically acceptable salt thereof according to any one of claims 1 to 16 or the pharmaceutical composition according to claim 20 in the preparation of a medicament for treating and/or preventing an estrogen receptor-mediated or -dependent disease or disorder, wherein the estrogen receptor-mediated or -dependent disease or disorder is preferably selected from the group consisting of a tumor, male sexual dysfunction, and Kennedy's disease.

23. The use according to claim 22, wherein the estrogen receptor-mediated or -dependent disease or disorder is selected from the group consisting of prostate cancer, prostatic hyperplasia, hirsutism, alopecia, anorexia nervosa, breast cancer, acne, male sexual dysfunction, Kennedy's disease, and AIDS, preferably prostate cancer, more preferably hormone-sensitive prostate cancer or hormone-refractory prostate cancer.
